(19) Europäisches Patentamt
European Patent Office
Office européen des brevets

(11) EP 3 613 850 A1

(12) EUROPEAN PATENT APPLICATION

(43) Date of publication:
26.02.2020 Bulletin 2020/09

(21) Application number: 19192017.2

(22) Date of filing: 16.08.2019

(51) Int Cl.:
*C12N 9/02* (2006.01) *C12N 9/04* (2006.01)
*C12N 9/00* (2006.01) *C12N 9/12* (2006.01)
*C12N 9/10* (2006.01) *C12N 9/06* (2006.01)
*C12N 9/88* (2006.01) *C12P 13/04* (2006.01)
*C12P 13/06* (2006.01) *C12P 13/08* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 22.08.2018 EP 18190216

(71) Applicant: **Evonik Operations GmbH**
**45128 Essen (DE)**

(72) Inventors:
- **Engel, Philip**
**45259 Essen (DE)**
- **Schaffer, Steffen**
**45699 Herten (DE)**
- **Thum, Oliver**
**40880 Ratingen (DE)**
- **Andrea, Heiko**
**45768 Marl (DE)**
- **Gehring, Christian**
**45770 Marl (DE)**
- **Grund, Bastian**
**Shanghai, Shanghai 201702 (CN)**

(74) Representative: **Evonik Patent Association**
**c/o Evonik Industries AG**
**IP Management**
**Bau 1042A/PB 15**
**Paul-Baumann-Straße 1**
**45772 Marl (DE)**

# (54) AMINO ACID PRODUCTION

(57) The present invention relates to a microbial cell for producing at least one L-amino acid from at least one C1-C4 alkane, wherein the cell comprises:
(v) an increased expression relative to the wild type cell of Enzyme $E_1$ capable of converting the alkane to a corresponding 1-alkanol;
(vi) an increased expression relative to the wild type cell of Enzyme $E_2$ capable of converting the 1-alkanol of (i) to a corresponding aldehyde; and either
(vii)
(A)
- an increased expression relative to the wild type cell of Enzyme $E_3$ capable of converting the aldehyde of (ii) to a corresponding alkanoic acid; and
- a wild-type level expression of Enzyme $E_4$ or an increased expression relative to the wild type cell of Enzyme $E_4$ capable of converting the alkanoic acid of (iii) to a corresponding fatty acyl thioester; or

(B)
- an increased expression relative to the wild type cell of Enzyme $E_5$ capable of converting the aldehyde of (ii) to a corresponding fatty acyl thioester;
and

(viii) an increased expression relative to the wild type cell of Enzyme $E_6$ capable of converting the fatty acyl thioester of (iii) to a corresponding amino acid

EP 3 613 850 A1

SEQUENCE LISTING

<110> Evonik Degussa GmbH

<120> AMINO ACID PRODUCTION

<130> 201500304

<160> 101

<170> PatentIn version 3.5

<210> 1
<211> 449
<212> PRT
<213> Escherichia coli

<220>
<221> Lysine-sensitive aspartokinase 3
<222> (1)..(449)

<400> 1

```
Met Ser Glu Ile Val Val Ser Lys Phe Gly Gly Thr Ser Val Ala Asp
1               5                   10                  15

Phe Asp Ala Met Asn Arg Ser Ala Asp Ile Val Leu Ser Asp Ala Asn
            20                  25                  30

Val Arg Leu Val Val Leu Ser Ala Ser Ala Gly Ile Thr Asn Leu Leu
        35                  40                  45

Val Ala Leu Ala Glu Gly Leu Glu Pro Gly Glu Arg Phe Glu Lys Leu
    50                  55                  60

Asp Ala Ile Arg Asn Ile Gln Phe Ala Ile Leu Glu Arg Leu Arg Tyr
65                  70                  75                  80

Pro Asn Val Ile Arg Glu Glu Ile Glu Arg Leu Leu Glu Asn Ile Thr
                85                  90                  95

Val Leu Ala Glu Ala Ala Ala Leu Ala Thr Ser Pro Ala Leu Thr Asp
            100                 105                 110

Glu Leu Val Ser His Gly Glu Leu Met Ser Thr Leu Leu Phe Val Glu
        115                 120                 125

Ile Leu Arg Glu Arg Asp Val Gln Ala Gln Trp Phe Asp Val Arg Lys
    130                 135                 140

Val Met Arg Thr Asn Asp Arg Phe Gly Arg Ala Glu Pro Asp Ile Ala
145                 150                 155                 160
```

```
Ala Leu Ala Glu Leu Ala Ala Leu Gln Leu Leu Pro Arg Leu Asn Glu
                165                 170                 175

Gly Leu Val Ile Thr Gln Gly Phe Ile Gly Ser Glu Asn Lys Gly Arg
            180                 185                 190

Thr Thr Thr Leu Gly Arg Gly Gly Ser Asp Tyr Thr Ala Ala Leu Leu
        195                 200                 205

Ala Glu Ala Leu His Ala Ser Arg Val Asp Ile Trp Thr Asp Val Pro
    210                 215                 220

Gly Ile Tyr Thr Thr Asp Pro Arg Val Val Ser Ala Ala Lys Arg Ile
225                 230                 235                 240

Asp Glu Ile Ala Phe Ala Glu Ala Ala Glu Met Ala Thr Phe Gly Ala
                245                 250                 255

Lys Val Leu His Pro Ala Thr Leu Leu Pro Ala Val Arg Ser Asp Ile
            260                 265                 270

Pro Val Phe Val Gly Ser Ser Lys Asp Pro Arg Ala Gly Gly Thr Leu
        275                 280                 285

Val Cys Asn Lys Thr Glu Asn Pro Pro Leu Phe Arg Ala Leu Ala Leu
    290                 295                 300

Arg Arg Asn Gln Thr Leu Leu Thr Leu His Ser Leu Asn Met Leu His
305                 310                 315                 320

Ser Arg Gly Phe Leu Ala Glu Val Phe Gly Ile Leu Ala Arg His Asn
                325                 330                 335

Ile Ser Val Asp Leu Ile Thr Thr Ser Glu Val Ser Val Ala Leu Thr
            340                 345                 350

Leu Asp Thr Thr Gly Ser Thr Ser Thr Gly Asp Thr Leu Leu Thr Gln
        355                 360                 365

Ser Leu Leu Met Glu Leu Ser Ala Leu Cys Arg Val Glu Val Glu Glu
    370                 375                 380

Gly Leu Ala Leu Val Ala Leu Ile Gly Asn Asp Leu Ser Lys Ala Cys
385                 390                 395                 400

Gly Val Gly Lys Glu Val Phe Gly Val Leu Glu Pro Phe Asn Ile Arg
                405                 410                 415
```

Met Ile Cys Tyr Gly Ala Ser Ser His Asn Leu Cys Phe Leu Val Pro
420 425 430

Gly Glu Asp Ala Glu Gln Val Val Gln Lys Leu His Ser Asn Leu Phe
435 440 445

Glu

<210> 2
<211> 366
<212> PRT
<213> Escherichia coli

<220>
<221> Aspartate-semialdehyde dehydrogenase
<222> (1)..(366)

<400> 2

Met Lys Asn Val Gly Phe Ile Gly Trp Arg Gly Met Val Gly Ser Val
1 5 10 15

Leu Met Gln Arg Met Val Glu Glu Arg Asp Phe Asp Ala Ile Arg Pro
20 25 30

Val Phe Phe Ser Thr Ser Gln Leu Gly Gln Ala Ala Pro Ser Phe Gly
35 40 45

Gly Thr Thr Gly Thr Leu Gln Asp Ala Phe Asp Leu Glu Ala Leu Lys
50 55 60

Ala Leu Asp Ile Ile Val Thr Cys Gln Gly Gly Asp Tyr Thr Asn Glu
65 70 75 80

Ile Tyr Pro Lys Leu Arg Glu Ser Gly Trp Gln Gly Tyr Trp Ile Asp
85 90 95

Ala Ala Ser Ser Leu Arg Met Lys Asp Asp Ala Ile Ile Ile Leu Asp
100 105 110

Pro Val Asn Gln Asp Val Ile Thr Asp Gly Leu Asn Asn Gly Ile Arg
115 120 125

Thr Phe Val Gly Gly Asn Cys Thr Val Ser Leu Met Leu Met Ser Leu
130 135 140

Gly Gly Leu Phe Ala Asn Asp Leu Val Asp Trp Val Ser Val Ala Thr

145 150 155 160

Tyr Gln Ala Ala Ser Gly Gly Gly Ala Arg His Met Arg Glu Leu Leu
165 170 175

Thr Gln Met Gly His Leu Tyr Gly His Val Ala Asp Glu Leu Ala Thr
180 185 190

Pro Ser Ser Ala Ile Leu Asp Ile Glu Arg Lys Val Thr Thr Leu Thr
195 200 205

Arg Ser Gly Glu Leu Pro Val Asp Asn Phe Gly Val Pro Leu Ala Gly
210 215 220

Ser Leu Ile Pro Trp Ile Asp Lys Gln Leu Asp Asn Gly Gln Ser Arg
225 230 235 240

Glu Glu Trp Lys Gly Gln Ala Glu Thr Asn Lys Ile Leu Asn Thr Ser
245 250 255

Ser Val Ile Pro Val Asp Gly Leu Cys Val Arg Val Gly Ala Leu Arg
260 265 270

Cys His Ser Gln Ala Phe Thr Ile Lys Leu Lys Lys Asp Val Ser Ile
275 280 285

Pro Thr Val Glu Glu Leu Leu Ala Ala His Asn Pro Trp Ala Lys Val
290 295 300

Val Pro Asn Asp Arg Glu Ile Thr Met Arg Glu Leu Thr Pro Ala Ala
305 310 315 320

Val Thr Gly Thr Leu Thr Thr Pro Val Gly Arg Leu Arg Lys Leu Asn
325 330 335

Met Gly Pro Glu Phe Leu Ser Ala Phe Thr Val Gly Asp Gln Leu Leu
340 345 350

Trp Gly Ala Ala Glu Pro Leu Arg Arg Met Leu Arg Gln Leu
355 360 365

<210> 3
<211> 292
<212> PRT
<213> Escherichia coli

<220>
<221> 4-hydroxy-tetrahydrodipicolinate synthase

<222> (1)..(292)

<400> 3

Met Phe Thr Gly Ser Ile Val Ala Ile Val Thr Pro Met Asp Glu Lys
1 5 10 15

Gly Asn Val Cys Arg Ala Ser Leu Lys Lys Leu Ile Asp Tyr His Val
20 25 30

Ala Ser Gly Thr Ser Ala Ile Val Ser Val Gly Thr Thr Gly Glu Ser
35 40 45

Ala Thr Leu Asn His Asp Glu His Ala Asp Val Val Met Met Thr Leu
50 55 60

Asp Leu Ala Asp Gly Arg Ile Pro Val Ile Ala Gly Thr Gly Ala Asn
65 70 75 80

Ala Thr Ala Glu Ala Ile Ser Leu Thr Gln Arg Phe Asn Asp Ser Gly
85 90 95

Ile Val Gly Cys Leu Thr Val Thr Pro Tyr Tyr Asn Arg Pro Ser Gln
100 105 110

Glu Gly Leu Tyr Gln His Phe Lys Ala Ile Ala Glu His Thr Asp Leu
115 120 125

Pro Gln Ile Leu Tyr Asn Val Pro Ser Arg Thr Gly Cys Asp Leu Leu
130 135 140

Pro Glu Thr Val Gly Arg Leu Ala Lys Val Lys Asn Ile Ile Gly Ile
145 150 155 160

Lys Glu Ala Thr Gly Asn Leu Thr Arg Val Asn Gln Ile Lys Glu Leu
165 170 175

Val Ser Asp Asp Phe Val Leu Leu Ser Gly Asp Asp Ala Ser Ala Leu
180 185 190

Asp Phe Met Gln Leu Gly Gly His Gly Val Ile Ser Val Thr Ala Asn
195 200 205

Val Ala Ala Arg Asp Met Ala Gln Met Cys Lys Leu Ala Ala Glu Gly
210 215 220

His Phe Ala Glu Ala Arg Val Ile Asn Gln Arg Leu Met Pro Leu His
225 230 235 240

Asn Lys Leu Phe Val Glu Pro Asn Pro Ile Pro Val Lys Trp Ala Cys
245 250 255

Lys Glu Leu Gly Leu Val Ala Thr Asp Thr Leu Arg Leu Pro Met Thr
260 265 270

Pro Ile Thr Asp Ser Gly Arg Glu Thr Val Arg Ala Ala Leu Lys His
275 280 285

Ala Gly Leu Leu
290

<210> 4
<211> 397
<212> PRT
<213> Corynebacterium glutamicum

<400> 4

Met Ala Leu Ala Leu Val Leu Asn Ser Gly Ser Ser Ser Ile Lys Phe
1 5 10 15

Gln Leu Val Asn Pro Glu Asn Ser Ala Ile Asp Glu Pro Tyr Val Ser
20 25 30

Gly Leu Val Glu Gln Ile Gly Glu Pro Asn Gly Arg Ile Val Leu Lys
35 40 45

Ile Glu Gly Glu Lys Tyr Thr Leu Glu Thr Pro Ile Ala Asp His Ser
50 55 60

Glu Gly Leu Asn Leu Ala Phe Asp Leu Met Asp Gln His Asn Cys Gly
65 70 75 80

Pro Ser Gln Leu Glu Ile Thr Ala Val Gly His Arg Val Val His Gly
85 90 95

Gly Ile Leu Phe Ser Ala Pro Glu Leu Ile Thr Asp Glu Ile Val Glu
100 105 110

Met Ile Arg Asp Leu Ile Pro Leu Ala Pro Leu His Asn Pro Ala Asn
115 120 125

Val Asp Gly Ile Asp Val Ala Arg Lys Ile Leu Pro Asp Val Pro His
130 135 140

Val Ala Val Phe Asp Thr Gly Phe Phe His Ser Leu Pro Pro Ala Ala
145 150 155 160

```
Ala Leu Tyr Ala Ile Asn Lys Asp Val Ala Ala Glu His Gly Ile Arg
                165                 170                 175

Arg Tyr Gly Phe His Gly Thr Ser His Glu Phe Val Ser Lys Arg Val
            180                 185                 190

Val Glu Ile Leu Glu Lys Pro Thr Glu Asp Ile Asn Thr Ile Thr Phe
        195                 200                 205

His Leu Gly Asn Gly Ala Ser Met Ala Ala Val Gln Gly Gly Arg Ala
    210                 215                 220

Val Asp Thr Ser Met Gly Met Thr Pro Leu Ala Gly Leu Val Met Gly
225                 230                 235                 240

Thr Arg Ser Gly Asp Ile Asp Pro Gly Ile Val Phe His Leu Ser Arg
                245                 250                 255

Thr Ala Gly Met Ser Ile Asp Glu Ile Asp Asn Leu Leu Asn Lys Lys
            260                 265                 270

Ser Gly Val Lys Gly Leu Ser Gly Val Asn Asp Phe Arg Glu Leu Arg
        275                 280                 285

Glu Met Ile Asp Asn Asn Asp Gln Asp Ala Trp Ser Ala Tyr Asn Ile
    290                 295                 300

Tyr Ile His Gln Leu Arg Arg Tyr Leu Gly Ser Tyr Met Val Ala Leu
305                 310                 315                 320

Gly Arg Val Asp Thr Ile Val Phe Thr Ala Gly Val Gly Glu Asn Ala
                325                 330                 335

Gln Phe Val Arg Glu Asp Ala Leu Ala Gly Leu Glu Met Tyr Gly Ile
            340                 345                 350

Glu Ile Asp Pro Glu Arg Asn Ala Leu Pro Asn Asp Gly Pro Arg Leu
        355                 360                 365

Ile Ser Thr Asp Ala Ser Lys Val Lys Val Phe Val Ile Pro Thr Asn
    370                 375                 380

Glu Glu Leu Ala Ile Ala Arg Tyr Ala Val Lys Phe Ala
385                 390                 395
```

<210> 5
<211> 273

<212> PRT
<213> Escherichia coli

<220>
<221> 4-hydroxy-tetrahydrodipicolinate reductase
<222> (1)..(273)

<400> 5

Met His Asp Ala Asn Ile Arg Val Ala Ile Ala Gly Ala Gly Gly Arg
1 5 10 15

Met Gly Arg Gln Leu Ile Gln Ala Ala Leu Ala Leu Glu Gly Val Gln
20 25 30

Leu Gly Ala Ala Leu Glu Arg Glu Gly Ser Ser Leu Leu Gly Ser Asp
35 40 45

Ala Gly Glu Leu Ala Gly Ala Gly Lys Thr Gly Val Thr Val Gln Ser
50 55 60

Ser Leu Asp Ala Val Lys Asp Asp Phe Asp Val Phe Ile Asp Phe Thr
65 70 75 80

Arg Pro Glu Gly Thr Leu Asn His Leu Ala Phe Cys Arg Gln His Gly
85 90 95

Lys Gly Met Val Ile Gly Thr Thr Gly Phe Asp Glu Ala Gly Lys Gln
100 105 110

Ala Ile Arg Asp Ala Ala Ala Asp Ile Ala Ile Val Phe Ala Ala Asn
115 120 125

Phe Ser Val Gly Val Asn Val Met Leu Lys Leu Leu Glu Lys Ala Ala
130 135 140

Lys Val Met Gly Asp Tyr Thr Asp Ile Glu Ile Ile Glu Ala His His
145 150 155 160

Arg His Lys Val Asp Ala Pro Ser Gly Thr Ala Leu Ala Met Gly Glu
165 170 175

Ala Ile Ala His Ala Leu Asp Lys Asp Leu Lys Asp Cys Ala Val Tyr
180 185 190

Ser Arg Glu Gly His Thr Gly Glu Arg Val Pro Gly Thr Ile Gly Phe
195 200 205

Ala Thr Val Arg Ala Gly Asp Ile Val Gly Glu His Thr Ala Met Phe

```
    210                 215                 220

Ala Asp Ile Gly Glu Arg Leu Glu Ile Thr His Lys Ala Ser Ser Arg
225                 230                 235                 240

Met Thr Phe Ala Asn Gly Ala Val Arg Ser Ala Leu Trp Leu Ser Gly
                245                 250                 255

Lys Glu Ser Gly Leu Phe Asp Met Arg Asp Val Leu Asp Leu Asn Asn
            260                 265                 270

Leu
```

```
<210>  6
<211>  420
<212>  PRT
<213>  Escherichia coli

<220>
<221>  Diaminopimelate decarboxylase
<222>  (1)..(420)

<400>  6

Met Pro His Ser Leu Phe Ser Thr Asp Thr Asp Leu Thr Ala Glu Asn
1               5                   10                  15

Leu Leu Arg Leu Pro Ala Glu Phe Gly Cys Pro Val Trp Val Tyr Asp
            20                  25                  30

Ala Gln Ile Ile Arg Arg Gln Ile Ala Ala Leu Lys Gln Phe Asp Val
        35                  40                  45

Val Arg Phe Ala Gln Lys Ala Cys Ser Asn Ile His Ile Leu Arg Leu
    50                  55                  60

Met Arg Glu Gln Gly Val Lys Val Asp Ser Val Ser Leu Gly Glu Ile
65                  70                  75                  80

Glu Arg Ala Leu Ala Ala Gly Tyr Asn Pro Gln Thr His Pro Asp Asp
                85                  90                  95

Ile Val Phe Thr Ala Asp Val Ile Asp Gln Ala Thr Leu Glu Arg Val
            100                 105                 110

Ser Glu Leu Gln Ile Pro Val Asn Ala Gly Ser Val Asp Met Leu Asp
        115                 120                 125
```

```
Gln Leu Gly Gln Val Ser Pro Gly His Arg Val Trp Leu Arg Val Asn
    130                 135                 140

Pro Gly Phe Gly His Gly His Ser Gln Lys Thr Asn Thr Gly Gly Glu
145                 150                 155                 160

Asn Ser Lys His Gly Ile Trp Tyr Thr Asp Leu Pro Ala Ala Leu Asp
                165                 170                 175

Val Ile Gln Arg His His Leu Gln Leu Val Gly Ile His Met His Ile
            180                 185                 190

Gly Ser Gly Val Asp Tyr Ala His Leu Glu Gln Val Cys Gly Ala Met
        195                 200                 205

Val Arg Gln Val Ile Glu Phe Gly Gln Asp Leu Gln Ala Ile Ser Ala
    210                 215                 220

Gly Gly Gly Leu Ser Val Pro Tyr Gln Gln Gly Glu Glu Ala Val Asp
225                 230                 235                 240

Thr Glu His Tyr Tyr Gly Leu Trp Asn Ala Ala Arg Glu Gln Ile Ala
                245                 250                 255

Arg His Leu Gly His Pro Val Lys Leu Glu Ile Glu Pro Gly Arg Phe
            260                 265                 270

Leu Val Ala Gln Ser Gly Val Leu Ile Thr Gln Val Arg Ser Val Lys
        275                 280                 285

Gln Met Gly Ser Arg His Phe Val Leu Val Asp Ala Gly Phe Asn Asp
    290                 295                 300

Leu Met Arg Pro Ala Met Tyr Gly Ser Tyr His His Ile Ser Ala Leu
305                 310                 315                 320

Ala Ala Asp Gly Arg Ser Leu Glu His Ala Pro Thr Val Glu Thr Val
                325                 330                 335

Val Ala Gly Pro Leu Cys Glu Ser Gly Asp Val Phe Thr Gln Gln Glu
            340                 345                 350

Gly Gly Asn Val Glu Thr Arg Ala Leu Pro Glu Val Lys Ala Gly Asp
        355                 360                 365

Tyr Leu Val Leu His Asp Thr Gly Ala Tyr Gly Ala Ser Met Ser Ser
    370                 375                 380
```

Asn Tyr Asn Ser Arg Pro Leu Leu Pro Glu Val Leu Phe Asp Asn Gly
385 390 395 400

Gln Ala Arg Leu Ile Arg Arg Arg Gln Thr Ile Glu Glu Leu Leu Ala
405 410 415

Leu Glu Leu Leu
420

<210> 7
<211> 299
<212> PRT
<213> Escherichia coli

<220>
<221> Lysine exporter LysO
<222> (1)..(299)

<400> 7

Met Phe Ser Gly Leu Leu Ile Ile Leu Val Pro Leu Ile Val Gly Tyr
1 5 10 15

Leu Ile Pro Leu Arg Gln Gln Ala Ala Leu Lys Val Ile Asn Gln Leu
20 25 30

Leu Ser Trp Met Val Tyr Leu Ile Leu Phe Phe Met Gly Ile Ser Leu
35 40 45

Ala Phe Leu Asp Asn Leu Ala Ser Asn Leu Leu Ala Ile Leu His Tyr
50 55 60

Ser Ala Val Ser Ile Thr Val Ile Leu Leu Cys Asn Ile Ala Ala Leu
65 70 75 80

Met Trp Leu Glu Arg Gly Leu Pro Trp Arg Asn His His Gln Gln Glu
85 90 95

Lys Leu Pro Ser Arg Ile Ala Met Ala Leu Glu Ser Leu Lys Leu Cys
100 105 110

Gly Val Val Val Ile Gly Phe Ala Ile Gly Leu Ser Gly Leu Ala Phe
115 120 125

Leu Gln His Ala Thr Glu Ala Ser Glu Tyr Thr Leu Ile Leu Leu Leu
130 135 140

Phe Leu Val Gly Ile Gln Leu Arg Asn Asn Gly Met Thr Leu Lys Gln
145 150 155 160

Ile Val Leu Asn Arg Arg Gly Met Ile Val Ala Val Val Val Val Val
165 170 175

Ser Ser Leu Ile Gly Gly Leu Ile Asn Ala Phe Ile Leu Asp Leu Pro
180 185 190

Ile Asn Thr Ala Leu Ala Met Ala Ser Gly Phe Gly Trp Tyr Ser Leu
195 200 205

Ser Gly Ile Leu Leu Thr Glu Ser Phe Gly Pro Val Ile Gly Ser Ala
210 215 220

Ala Phe Phe Asn Asp Leu Ala Arg Glu Leu Ile Ala Ile Met Leu Ile
225 230 235 240

Pro Gly Leu Ile Arg Arg Ser Arg Ser Thr Ala Leu Gly Leu Cys Gly
245 250 255

Ala Thr Ser Met Asp Phe Thr Leu Pro Val Leu Gln Arg Thr Gly Gly
260 265 270

Leu Asp Met Val Pro Ala Ala Ile Val His Gly Phe Ile Leu Ser Leu
275 280 285

Leu Val Pro Ile Leu Ile Ala Phe Phe Ser Ala
290 295

<210> 8
<211> 223
<212> PRT
<213> Escherichia coli

<220>
<221> Uncharacterized membrane protein YahN
<222> (1)..(223)

<400> 8

Met Met Gln Leu Val His Leu Phe Met Asp Glu Ile Thr Met Asp Pro
1 5 10 15

Leu His Ala Val Tyr Leu Thr Val Gly Leu Phe Val Ile Thr Phe Phe
20 25 30

Asn Pro Gly Ala Asn Leu Phe Val Val Val Gln Thr Ser Leu Ala Ser
35 40 45

Gly Arg Arg Ala Gly Val Leu Thr Gly Leu Gly Val Ala Leu Gly Asp

50 55 60

Ala Phe Tyr Ser Gly Leu Gly Leu Phe Gly Leu Ala Thr Leu Ile Thr
65 70 75 80

Gln Cys Glu Glu Ile Phe Ser Leu Ile Arg Ile Val Gly Gly Ala Tyr
85 90 95

Leu Leu Trp Phe Ala Trp Cys Ser Met Arg Arg Gln Ser Thr Pro Gln
100 105 110

Met Ser Thr Leu Gln Gln Pro Ile Ser Ala Pro Trp Tyr Val Phe Phe
115 120 125

Arg Arg Gly Leu Ile Thr Asp Leu Ser Asn Pro Gln Thr Val Leu Phe
130 135 140

Phe Ile Ser Ile Phe Ser Val Thr Leu Asn Ala Glu Thr Pro Thr Trp
145 150 155 160

Ala Arg Leu Met Ala Trp Ala Gly Ile Val Leu Ala Ser Ile Ile Trp
165 170 175

Arg Val Phe Leu Ser Gln Ala Phe Ser Leu Pro Ala Val Arg Arg Ala
180 185 190

Tyr Gly Arg Met Gln Arg Val Ala Ser Arg Val Ile Gly Ala Ile Ile
195 200 205

Gly Val Phe Ala Leu Arg Leu Ile Tyr Glu Gly Val Thr Gln Arg
210 215 220

<210> 9
<211> 361
<212> PRT
<213> Corynebacterium glutamicum

<220>
<221> Lysine exporter protein
<222> (1)..(361)

<400> 9

Met Ala Val Met Ala Tyr Gln Pro Ala Asp Asn Arg Tyr Asp Asp Met
1 5 10 15

Ile Tyr Arg Arg Val Gly Asn Ser Gly Leu Lys Leu Pro Ala Ile Ser
20 25 30

```
Leu Gly Leu Trp His Asn Phe Gly Asp Asp Lys Pro Leu Ser Thr Gln
        35                  40                  45

Arg Ser Ile Ile His Arg Ala Phe Asp Arg Gly Val Thr His Phe Asp
    50                  55                  60

Leu Ala Asn Asn Tyr Gly Pro Pro Ala Gly Ser Ala Glu Thr Asn Phe
65                  70                  75                  80

Gly Arg Ile Leu Arg Glu Asp Leu Lys Ser His Arg Asp Glu Leu Ile
                85                  90                  95

Ile Ser Ser Lys Ala Gly Trp Asp Met Trp Pro Gly Pro Tyr Gly Phe
            100                 105                 110

Gly Gly Ser Arg Lys Tyr Leu Val Ser Ser Leu Asp Gln Ser Leu Thr
        115                 120                 125

Arg Leu Gly Leu Asp Tyr Val Asp Ile Phe Tyr His His Arg Pro Asp
    130                 135                 140

Pro Asp Thr Pro Leu Glu Glu Thr Met Tyr Ala Leu Arg Asp Ile Val
145                 150                 155                 160

Ala Ser Gly Lys Ala Leu Tyr Val Gly Ile Ser Ser Tyr Gly Pro Glu
                165                 170                 175

Leu Thr Ala Glu Ala Ala Glu Phe Met Ala Glu Glu Gly Cys Pro Leu
            180                 185                 190

Leu Ile His Gln Pro Ser Tyr Ser Ile Ile Asn Arg Trp Val Glu Glu
        195                 200                 205

Pro Gly Asp Asp Gly Glu Asn Leu Leu Gln Ser Ala Ala Asn Asn Gly
    210                 215                 220

Leu Gly Val Ile Ala Phe Ser Pro Leu Ala Gln Gly Leu Leu Thr Asp
225                 230                 235                 240

Lys Tyr Leu Asp Gly Ile Pro Glu Gly Ser Arg Ala Ser Gln Gly Lys
                245                 250                 255

Ser Leu Ser Glu Gly Met Leu Asn Val Asn Asn Ile Asp Met Val Arg
            260                 265                 270

Lys Leu Asn Asp Ile Ala Gln Glu Arg Gly Gln Ser Leu Ala Gln Met
        275                 280                 285
```

Ala Leu Ala Trp Val Leu Arg Glu Gln Gly Glu Tyr Gly Ala Asp Thr
290 295 300

Val Thr Ser Ala Leu Ile Gly Ala Ser Ser Val Glu Gln Leu Asp Asn
305 310 315 320

Ser Leu Asp Ser Leu Asn Asn Leu Glu Phe Ser Asp Ala Glu Leu Glu
325 330 335

Ala Ile Asp Glu Ile Ser His Asp Ala Gly Ile Asn Ile Trp Ala Lys
340 345 350

Ala Thr Asp Ser Lys Thr Arg Glu Asn
355 360

<210> 10
<211> 883
<212> PRT
<213> Escherichia coli

<220>
<221> Phosphoenolpyruvate carboxylase
<222> (1)..(883)

<400> 10

Met Asn Glu Gln Tyr Ser Ala Leu Arg Ser Asn Val Ser Met Leu Gly
1 5 10 15

Lys Val Leu Gly Glu Thr Ile Lys Asp Ala Leu Gly Glu His Ile Leu
20 25 30

Glu Arg Val Glu Thr Ile Arg Lys Leu Ser Lys Ser Ser Arg Ala Gly
35 40 45

Asn Asp Ala Asn Arg Gln Glu Leu Leu Thr Thr Leu Gln Asn Leu Ser
50 55 60

Asn Asp Glu Leu Leu Pro Val Ala Arg Ala Phe Ser Gln Phe Leu Asn
65 70 75 80

Leu Ala Asn Thr Ala Glu Gln Tyr His Ser Ile Ser Pro Lys Gly Glu
85 90 95

Ala Ala Ser Asn Pro Glu Val Ile Ala Arg Thr Leu Arg Lys Leu Lys
100 105 110

Asn Gln Pro Glu Leu Ser Glu Asp Thr Ile Lys Lys Ala Val Glu Ser
115 120 125

```
Leu Ser Leu Glu Leu Val Leu Thr Ala His Pro Thr Glu Ile Thr Arg
    130                 135                 140

Arg Thr Leu Ile His Lys Met Val Glu Val Asn Ala Cys Leu Lys Gln
145                 150                 155                 160

Leu Asp Asn Lys Asp Ile Ala Asp Tyr Glu His Asn Gln Leu Met Arg
                165                 170                 175

Arg Leu Arg Gln Leu Ile Ala Gln Ser Trp His Thr Asp Glu Ile Arg
            180                 185                 190

Lys Leu Arg Pro Ser Pro Val Asp Glu Ala Lys Trp Gly Phe Ala Val
        195                 200                 205

Val Glu Asn Ser Leu Trp Gln Gly Val Pro Asn Tyr Leu Arg Glu Leu
    210                 215                 220

Asn Glu Gln Leu Glu Glu Asn Leu Gly Tyr Lys Leu Pro Val Glu Phe
225                 230                 235                 240

Val Pro Val Arg Phe Thr Ser Trp Met Gly Gly Asp Arg Asp Gly Asn
                245                 250                 255

Pro Asn Val Thr Ala Asp Ile Thr Arg His Val Leu Leu Leu Ser Arg
            260                 265                 270

Trp Lys Ala Thr Asp Leu Phe Leu Lys Asp Ile Gln Val Leu Val Ser
        275                 280                 285

Glu Leu Ser Met Val Glu Ala Thr Pro Glu Leu Leu Ala Leu Val Gly
    290                 295                 300

Glu Glu Gly Ala Ala Glu Pro Tyr Arg Tyr Leu Met Lys Asn Leu Arg
305                 310                 315                 320

Ser Arg Leu Met Ala Thr Gln Ala Trp Leu Glu Ala Arg Leu Lys Gly
                325                 330                 335

Glu Glu Leu Pro Lys Pro Glu Gly Leu Leu Thr Gln Asn Glu Glu Leu
            340                 345                 350

Trp Glu Pro Leu Tyr Ala Cys Tyr Gln Ser Leu Gln Ala Cys Gly Met
        355                 360                 365

Gly Ile Ile Ala Asn Gly Asp Leu Leu Asp Thr Leu Arg Arg Val Lys
```

370 375 380

Cys Phe Gly Val Pro Leu Val Arg Ile Asp Ile Arg Gln Glu Ser Thr
385 390 395 400

Arg His Thr Glu Ala Leu Gly Glu Leu Thr Arg Tyr Leu Gly Ile Gly
405 410 415

Asp Tyr Glu Ser Trp Ser Glu Ala Asp Lys Gln Ala Phe Leu Ile Arg
420 425 430

Glu Leu Asn Ser Lys Arg Pro Leu Leu Pro Arg Asn Trp Gln Pro Ser
435 440 445

Ala Glu Thr Arg Glu Val Leu Asp Thr Cys Gln Val Ile Ala Glu Ala
450 455 460

Pro Gln Gly Ser Ile Ala Ala Tyr Val Ile Ser Met Ala Lys Thr Pro
465 470 475 480

Ser Asp Val Leu Ala Val His Leu Leu Leu Lys Glu Ala Gly Ile Gly
485 490 495

Phe Ala Met Pro Val Ala Pro Leu Phe Glu Thr Leu Asp Asp Leu Asn
500 505 510

Asn Ala Asn Asp Val Met Thr Gln Leu Leu Asn Ile Asp Trp Tyr Arg
515 520 525

Gly Leu Ile Gln Gly Lys Gln Met Val Met Ile Gly Tyr Ser Asp Ser
530 535 540

Ala Lys Asp Ala Gly Val Met Ala Ala Ser Trp Ala Gln Tyr Gln Ala
545 550 555 560

Gln Asp Ala Leu Ile Lys Thr Cys Glu Lys Ala Gly Ile Glu Leu Thr
565 570 575

Leu Phe His Gly Arg Gly Gly Ser Ile Gly Arg Gly Gly Ala Pro Ala
580 585 590

His Ala Ala Leu Leu Ser Gln Pro Pro Gly Ser Leu Lys Gly Gly Leu
595 600 605

Arg Val Thr Glu Gln Gly Glu Met Ile Arg Phe Lys Tyr Gly Leu Pro
610 615 620

```
Glu Ile Thr Val Ser Ser Leu Ser Leu Tyr Thr Gly Ala Ile Leu Glu
625                 630                 635                 640

Ala Asn Leu Leu Pro Pro Pro Glu Pro Lys Glu Ser Trp Arg Arg Ile
                645                 650                 655

Met Asp Glu Leu Ser Val Ile Ser Cys Asp Val Tyr Arg Gly Tyr Val
            660                 665                 670

Arg Glu Asn Lys Asp Phe Val Pro Tyr Phe Arg Ser Ala Thr Pro Glu
        675                 680                 685

Gln Glu Leu Gly Lys Leu Pro Leu Gly Ser Arg Pro Ala Lys Arg Arg
    690                 695                 700

Pro Thr Gly Gly Val Glu Ser Leu Arg Ala Ile Pro Trp Ile Phe Ala
705                 710                 715                 720

Trp Thr Gln Asn Arg Leu Met Leu Pro Ala Trp Leu Gly Ala Gly Thr
                725                 730                 735

Ala Leu Gln Lys Val Val Glu Asp Gly Lys Gln Ser Glu Leu Glu Ala
            740                 745                 750

Met Cys Arg Asp Trp Pro Phe Phe Ser Thr Arg Leu Gly Met Leu Glu
        755                 760                 765

Met Val Phe Ala Lys Ala Asp Leu Trp Leu Ala Glu Tyr Tyr Asp Gln
    770                 775                 780

Arg Leu Val Asp Lys Ala Leu Trp Pro Leu Gly Lys Glu Leu Arg Asn
785                 790                 795                 800

Leu Gln Glu Glu Asp Ile Lys Val Val Leu Ala Ile Ala Asn Asp Ser
                805                 810                 815

His Leu Met Ala Asp Leu Pro Trp Ile Ala Glu Ser Ile Gln Leu Arg
            820                 825                 830

Asn Ile Tyr Thr Asp Pro Leu Asn Val Leu Gln Ala Glu Leu Leu His
        835                 840                 845

Arg Ser Arg Gln Ala Glu Lys Glu Gly Gln Glu Pro Asp Pro Arg Val
    850                 855                 860

Glu Gln Ala Leu Met Val Thr Ile Ala Gly Ile Ala Ala Gly Met Arg
865                 870                 875                 880
```

Asn Thr Gly

<210> 11
<211> 510
<212> PRT
<213> Escherichia coli

<220>
<221> proton-translocating transhydrogenase
<222> (1)..(510)

<220>
<221> proton-translocating transhydrogenase (pntA)
<222> (1)..(510)

<400> 11

```
Met Arg Ile Gly Ile Pro Arg Glu Arg Leu Thr Asn Glu Thr Arg Val
1               5                   10                  15

Ala Ala Thr Pro Lys Thr Val Glu Gln Leu Leu Lys Leu Gly Phe Thr
            20                  25                  30

Val Ala Val Glu Ser Gly Ala Gly Gln Leu Ala Ser Phe Asp Asp Lys
        35                  40                  45

Ala Phe Val Gln Ala Gly Ala Glu Ile Val Glu Gly Asn Ser Val Trp
    50                  55                  60

Gln Ser Glu Ile Ile Leu Lys Val Asn Ala Pro Leu Asp Asp Glu Ile
65                  70                  75                  80

Ala Leu Leu Asn Pro Gly Thr Thr Leu Val Ser Phe Ile Trp Pro Ala
                85                  90                  95

Gln Asn Pro Glu Leu Met Gln Lys Leu Ala Glu Arg Asn Val Thr Val
            100                 105                 110

Met Ala Met Asp Ser Val Pro Arg Ile Ser Arg Ala Gln Ser Leu Asp
        115                 120                 125

Ala Leu Ser Ser Met Ala Asn Ile Ala Gly Tyr Arg Ala Ile Val Glu
    130                 135                 140

Ala Ala His Glu Phe Gly Arg Phe Phe Thr Gly Gln Ile Thr Ala Ala
145                 150                 155                 160

Gly Lys Val Pro Pro Ala Lys Val Met Val Ile Gly Ala Gly Val Ala
                165                 170                 175
```

```
Gly Leu Ala Ala Ile Gly Ala Ala Asn Ser Leu Gly Ala Ile Val Arg
            180                 185                 190

Ala Phe Asp Thr Arg Pro Glu Val Lys Glu Gln Val Gln Ser Met Gly
        195                 200                 205

Ala Glu Phe Leu Glu Leu Asp Phe Lys Glu Glu Ala Gly Ser Gly Asp
    210                 215                 220

Gly Tyr Ala Lys Val Met Ser Asp Ala Phe Ile Lys Ala Glu Met Glu
225                 230                 235                 240

Leu Phe Ala Ala Gln Ala Lys Glu Val Asp Ile Ile Val Thr Thr Ala
                245                 250                 255

Leu Ile Pro Gly Lys Pro Ala Pro Lys Leu Ile Thr Arg Glu Met Val
            260                 265                 270

Asp Ser Met Lys Ala Gly Ser Val Ile Val Asp Leu Ala Ala Gln Asn
        275                 280                 285

Gly Gly Asn Cys Glu Tyr Thr Val Pro Gly Glu Ile Phe Thr Thr Glu
    290                 295                 300

Asn Gly Val Lys Val Ile Gly Tyr Thr Asp Leu Pro Gly Arg Leu Pro
305                 310                 315                 320

Thr Gln Ser Ser Gln Leu Tyr Gly Thr Asn Leu Val Asn Leu Leu Lys
                325                 330                 335

Leu Leu Cys Lys Glu Lys Asp Gly Asn Ile Thr Val Asp Phe Asp Asp
            340                 345                 350

Val Val Ile Arg Gly Val Thr Val Ile Arg Ala Gly Glu Ile Thr Trp
        355                 360                 365

Pro Ala Pro Pro Ile Gln Val Ser Ala Gln Pro Gln Ala Ala Gln Lys
    370                 375                 380

Ala Ala Pro Glu Val Lys Thr Glu Glu Lys Cys Thr Cys Ser Pro Trp
385                 390                 395                 400

Arg Lys Tyr Ala Leu Met Ala Leu Ala Ile Ile Leu Phe Gly Trp Met
                405                 410                 415

Ala Ser Val Ala Pro Lys Glu Phe Leu Gly His Phe Thr Val Phe Ala
```

420 425 430

Leu Ala Cys Val Val Gly Tyr Tyr Val Val Trp Asn Val Ser His Ala
435 440 445

Leu His Thr Pro Leu Met Ser Val Thr Asn Ala Ile Ser Gly Ile Ile
450 455 460

Val Val Gly Ala Leu Leu Gln Ile Gly Gln Gly Gly Trp Val Ser Phe
465 470 475 480

Leu Ser Phe Ile Ala Val Leu Ile Ala Ser Ile Asn Ile Phe Gly Gly
485 490 495

Phe Thr Val Thr Gln Arg Met Leu Lys Met Phe Arg Lys Asn
500 505 510

<210> 12
<211> 1140
<212> PRT
<213> Corynebacterium glutamicum

<220>
<221> Pyruvate carboxylase
<222> (1)..(1140)

<400> 12

Met Ser Thr His Thr Ser Ser Thr Leu Pro Ala Phe Lys Lys Ile Leu
1 5 10 15

Val Ala Asn Arg Gly Glu Ile Ala Val Arg Ala Phe Arg Ala Ala Leu
20 25 30

Glu Thr Gly Ala Ala Thr Val Ala Ile Tyr Pro Arg Glu Asp Arg Gly
35 40 45

Ser Phe His Arg Ser Phe Ala Ser Glu Ala Val Arg Ile Gly Thr Glu
50 55 60

Gly Ser Pro Val Lys Ala Tyr Leu Asp Ile Asp Glu Ile Ile Gly Ala
65 70 75 80

Ala Lys Lys Val Lys Ala Asp Ala Ile Tyr Pro Gly Tyr Gly Phe Leu
85 90 95

Ser Glu Asn Ala Gln Leu Ala Arg Glu Cys Ala Glu Asn Gly Ile Thr
100 105 110

Phe Ile Gly Pro Thr Pro Glu Val Leu Asp Leu Thr Gly Asp Lys Ser
115 120 125

Arg Ala Val Thr Ala Ala Lys Lys Ala Gly Leu Pro Val Leu Ala Glu
130 135 140

Ser Thr Pro Ser Lys Asn Ile Asp Glu Ile Val Lys Ser Ala Glu Gly
145 150 155 160

Gln Thr Tyr Pro Ile Phe Val Lys Ala Val Ala Gly Gly Gly Gly Arg
165 170 175

Gly Met Arg Phe Val Ala Ser Pro Asp Glu Leu Arg Lys Leu Ala Thr
180 185 190

Glu Ala Ser Arg Glu Ala Glu Ala Ala Phe Gly Asp Gly Ala Val Tyr
195 200 205

Val Glu Arg Ala Val Ile Asn Pro Gln His Ile Glu Val Gln Ile Leu
210 215 220

Gly Asp His Thr Gly Glu Val Val His Leu Tyr Glu Arg Asp Cys Ser
225 230 235 240

Leu Gln Arg Arg His Gln Lys Val Val Glu Ile Ala Pro Ala Gln His
245 250 255

Leu Asp Pro Glu Leu Arg Asp Arg Ile Cys Ala Asp Ala Val Lys Phe
260 265 270

Cys Arg Ser Ile Gly Tyr Gln Gly Ala Gly Thr Val Glu Phe Leu Val
275 280 285

Asp Glu Lys Gly Asn His Val Phe Ile Glu Met Asn Pro Arg Ile Gln
290 295 300

Val Glu His Thr Val Thr Glu Glu Val Thr Glu Val Asp Leu Val Lys
305 310 315 320

Ala Gln Met Arg Leu Ala Ala Gly Ala Thr Leu Lys Glu Leu Gly Leu
325 330 335

Thr Gln Asp Lys Ile Lys Thr His Gly Ala Ala Leu Gln Cys Arg Ile
340 345 350

Thr Thr Glu Asp Pro Asn Asn Gly Phe Arg Pro Asp Thr Gly Thr Ile
355 360 365

```
Thr Ala Tyr Arg Ser Pro Gly Gly Ala Gly Val Arg Leu Asp Gly Ala
    370                 375                 380

Ala Gln Leu Gly Gly Glu Ile Thr Ala His Phe Asp Ser Met Leu Val
385                 390                 395                 400

Lys Met Thr Cys Arg Gly Ser Asp Phe Glu Thr Ala Val Ala Arg Ala
                405                 410                 415

Gln Arg Ala Leu Ala Glu Phe Thr Val Ser Gly Val Ala Thr Asn Ile
            420                 425                 430

Gly Phe Leu Arg Ala Leu Leu Arg Glu Glu Asp Phe Thr Ser Lys Arg
        435                 440                 445

Ile Ala Thr Gly Phe Ile Ala Asp His Pro His Leu Leu Gln Ala Pro
    450                 455                 460

Pro Ala Asp Asp Glu Gln Gly Arg Ile Leu Asp Tyr Leu Ala Asp Val
465                 470                 475                 480

Thr Val Asn Lys Pro His Gly Val Arg Pro Lys Asp Val Ala Ala Pro
                485                 490                 495

Ile Asp Lys Leu Pro Asn Ile Lys Asp Leu Pro Leu Pro Arg Gly Ser
            500                 505                 510

Arg Asp Arg Leu Lys Gln Leu Gly Pro Ala Ala Phe Ala Arg Asp Leu
        515                 520                 525

Arg Glu Gln Asp Ala Leu Ala Val Thr Asp Thr Thr Phe Arg Asp Ala
    530                 535                 540

His Gln Ser Leu Leu Ala Thr Arg Val Arg Ser Phe Ala Leu Lys Pro
545                 550                 555                 560

Ala Ala Glu Ala Val Ala Lys Leu Thr Pro Glu Leu Leu Ser Val Glu
                565                 570                 575

Ala Trp Gly Gly Ala Thr Tyr Asp Val Ala Met Arg Phe Leu Phe Glu
            580                 585                 590

Asp Pro Trp Asp Arg Leu Asp Glu Leu Arg Glu Ala Met Pro Asn Val
        595                 600                 605

Asn Ile Gln Met Leu Leu Arg Gly Arg Asn Thr Val Gly Tyr Thr Pro
    610                 615                 620
```

```
Tyr Pro Asp Ser Val Cys Arg Ala Phe Val Lys Glu Ala Ala Ser Ser
625                 630                 635                 640

Gly Val Asp Ile Phe Arg Ile Phe Asp Ala Leu Asn Asp Val Ser Gln
                645                 650                 655

Met Arg Pro Ala Ile Asp Ala Val Leu Glu Thr Asn Thr Ala Val Ala
            660                 665                 670

Glu Val Ala Met Ala Tyr Ser Gly Asp Leu Ser Asp Pro Asn Glu Lys
        675                 680                 685

Leu Tyr Thr Leu Asp Tyr Tyr Leu Lys Met Ala Glu Glu Ile Val Lys
    690                 695                 700

Ser Gly Ala His Ile Leu Ala Ile Lys Asp Met Ala Gly Leu Leu Arg
705                 710                 715                 720

Pro Ala Ala Val Thr Lys Leu Val Thr Ala Leu Arg Arg Glu Phe Asp
                725                 730                 735

Leu Pro Val His Val His Thr His Asp Thr Ala Gly Gly Gln Leu Ala
            740                 745                 750

Thr Tyr Phe Ala Ala Ala Gln Ala Gly Ala Asp Ala Val Asp Gly Ala
        755                 760                 765

Ser Ala Pro Leu Ser Gly Thr Thr Ser Gln Pro Ser Leu Ser Ala Ile
    770                 775                 780

Val Ala Ala Phe Ala His Thr Arg Arg Asp Thr Gly Leu Ser Leu Glu
785                 790                 795                 800

Ala Val Ser Asp Leu Glu Pro Tyr Trp Glu Ala Val Arg Gly Leu Tyr
                805                 810                 815

Leu Pro Phe Glu Ser Gly Thr Pro Gly Pro Thr Gly Arg Val Tyr Arg
            820                 825                 830

His Glu Ile Pro Gly Gly Gln Leu Ser Asn Leu Arg Ala Gln Ala Thr
        835                 840                 845

Ala Leu Gly Leu Ala Asp Arg Phe Glu Leu Ile Glu Asp Asn Tyr Ala
    850                 855                 860

Ala Val Asn Glu Met Leu Gly Arg Pro Thr Lys Val Thr Pro Ser Ser
```

865 870 875 880

Lys Val Val Gly Asp Leu Ala Leu His Leu Val Gly Ala Gly Val Asp
885 890 895

Pro Ala Asp Phe Ala Ala Asp Pro Gln Lys Tyr Asp Ile Pro Asp Ser
900 905 910

Val Ile Ala Phe Leu Arg Gly Glu Leu Gly Asn Pro Pro Gly Gly Trp
915 920 925

Pro Glu Pro Leu Arg Thr Arg Ala Leu Glu Gly Arg Ser Glu Gly Lys
930 935 940

Ala Pro Leu Thr Glu Val Pro Glu Glu Glu Gln Ala His Leu Asp Ala
945 950 955 960

Asp Asp Ser Lys Glu Arg Arg Asn Ser Leu Asn Arg Leu Leu Phe Pro
965 970 975

Lys Pro Thr Glu Glu Phe Leu Glu His Arg Arg Arg Phe Gly Asn Thr
980 985 990

Ser Ala Leu Asp Asp Arg Glu Phe Phe Tyr Gly Leu Val Glu Gly Arg
995 1000 1005

Glu Thr Leu Ile Arg Leu Pro Asp Val Arg Thr Pro Leu Leu Val
1010 1015 1020

Arg Leu Asp Ala Ile Ser Glu Pro Asp Asp Lys Gly Met Arg Asn
1025 1030 1035

Val Val Ala Asn Val Asn Gly Gln Ile Arg Pro Met Arg Val Arg
1040 1045 1050

Asp Arg Ser Val Glu Ser Val Thr Ala Thr Ala Glu Lys Ala Asp
1055 1060 1065

Ser Ser Asn Lys Gly His Val Ala Ala Pro Phe Ala Gly Val Val
1070 1075 1080

Thr Val Thr Val Ala Glu Gly Asp Glu Val Lys Ala Gly Asp Ala
1085 1090 1095

Val Ala Ile Ile Glu Ala Met Lys Met Glu Ala Thr Ile Thr Ala
1100 1105 1110

Ser Val Asp Gly Lys Ile Asp Arg Val Val Val Pro Ala Ala Thr
1115 1120 1125

Lys Val Glu Gly Gly Asp Leu Ile Val Val Val Ser
1130 1135 1140

<210> 13
<211> 331
<212> PRT
<213> Escherichia coli

<220>
<221> glyceraldehyde-3-phosphate dehydrogenase (NADP-dependent)
<222> (1)..(331)

<400> 13

Met Thr Ile Lys Val Gly Ile Asn Gly Phe Gly Arg Ile Gly Arg Ile
1 5 10 15

Val Phe Arg Ala Ala Gln Lys Arg Ser Asp Ile Glu Ile Val Ala Ile
20 25 30

Asn Asp Leu Leu Asp Ala Asp Tyr Met Ala Tyr Met Leu Lys Tyr Asp
35 40 45

Ser Thr His Gly Arg Phe Asp Gly Thr Val Glu Val Lys Asp Gly His
50 55 60

Leu Ile Val Asn Gly Lys Lys Ile Arg Val Thr Ala Glu Arg Asp Pro
65 70 75 80

Ala Asn Leu Lys Trp Asp Glu Val Gly Val Asp Val Val Ala Glu Ala
85 90 95

Thr Gly Leu Phe Leu Thr Asp Glu Thr Ala Arg Lys His Ile Thr Ala
100 105 110

Gly Ala Lys Lys Val Val Met Thr Gly Pro Ser Lys Asp Asn Thr Pro
115 120 125

Met Phe Val Lys Gly Ala Asn Phe Asp Lys Tyr Ala Gly Gln Asp Ile
130 135 140

Val Ser Asn Ala Ser Cys Thr Thr Asn Cys Leu Ala Pro Leu Ala Lys
145 150 155 160

Val Ile Asn Asp Asn Phe Gly Ile Ile Glu Gly Leu Met Thr Thr Val
165 170 175

```
His Ala Thr Thr Ala Thr Gln Lys Thr Val Asp Gly Pro Ser His Lys
            180                 185                 190

Asp Trp Arg Gly Gly Arg Gly Ala Ser Gln Asn Ile Ile Pro Ser Ser
        195                 200                 205

Thr Gly Ala Ala Lys Ala Val Gly Lys Val Leu Pro Glu Leu Asn Gly
    210                 215                 220

Lys Leu Thr Gly Met Ala Phe Arg Val Pro Thr Pro Asn Val Ser Val
225                 230                 235                 240

Val Asp Leu Thr Val Arg Leu Glu Lys Ala Ala Thr Tyr Glu Gln Ile
                245                 250                 255

Lys Ala Ala Val Lys Ala Ala Ala Glu Gly Glu Met Lys Gly Val Leu
            260                 265                 270

Gly Tyr Thr Glu Asp Asp Val Val Ser Thr Asp Phe Asn Gly Glu Val
        275                 280                 285

Cys Thr Ser Val Phe Asp Ala Lys Ala Gly Ile Ala Leu Asn Asp Asn
    290                 295                 300

Phe Val Lys Leu Val Ser Trp Tyr Asp Asn Glu Thr Gly Tyr Ser Asn
305                 310                 315                 320

Lys Val Leu Asp Leu Ile Ala His Ile Ser Lys
                325                 330


<210>  14
<211>  445
<212>  PRT
<213>  Corynebacterium glutamicum


<220>
<221>  Homoserine dehydrogenase
<222>  (1)..(445)

<400>  14

Met Thr Ser Ala Ser Ala Pro Ser Phe Asn Pro Gly Lys Gly Pro Gly
1               5                   10                  15

Ser Ala Val Gly Ile Ala Leu Leu Gly Phe Gly Thr Val Gly Thr Glu
            20                  25                  30

Val Met Arg Leu Met Thr Glu Tyr Gly Asp Glu Leu Ala His Arg Ile
        35                  40                  45
```

```
Gly Gly Pro Leu Glu Val Arg Gly Ile Ala Val Ser Asp Ile Ser Lys
    50                  55                  60

Pro Arg Glu Gly Val Ala Pro Glu Leu Leu Thr Glu Asp Ala Phe Ala
65                  70                  75                  80

Leu Ile Glu Arg Glu Asp Val Asp Ile Val Val Glu Val Ile Gly Gly
                85                  90                  95

Ile Glu Tyr Pro Arg Glu Val Val Leu Ala Ala Leu Lys Ala Gly Lys
            100                 105                 110

Ser Val Val Thr Ala Asn Lys Ala Leu Val Ala Ala His Ser Ala Glu
        115                 120                 125

Leu Ala Asp Ala Ala Glu Ala Ala Asn Val Asp Leu Tyr Phe Glu Ala
    130                 135                 140

Ala Val Ala Gly Ala Ile Pro Val Val Gly Pro Leu Arg Arg Ser Leu
145                 150                 155                 160

Ala Gly Asp Gln Ile Gln Ser Val Met Gly Ile Val Asn Gly Thr Thr
                165                 170                 175

Asn Phe Ile Leu Asp Ala Met Asp Ser Thr Gly Ala Asp Tyr Ala Asp
            180                 185                 190

Ser Leu Ala Glu Ala Thr Arg Leu Gly Tyr Ala Glu Ala Asp Pro Thr
        195                 200                 205

Ala Asp Val Glu Gly His Asp Ala Ala Ser Lys Ala Ala Ile Leu Ala
    210                 215                 220

Ser Ile Ala Phe His Thr Arg Val Thr Ala Asp Asp Val Tyr Cys Glu
225                 230                 235                 240

Gly Ile Ser Asn Ile Ser Ala Ala Asp Ile Glu Ala Ala Gln Gln Ala
                245                 250                 255

Gly His Thr Ile Lys Leu Leu Ala Ile Cys Glu Lys Phe Thr Asn Lys
            260                 265                 270

Glu Gly Lys Ser Ala Ile Ser Ala Arg Val His Pro Thr Leu Leu Pro
        275                 280                 285

Val Ser His Pro Leu Ala Ser Val Asn Lys Ser Phe Asn Ala Ile Phe
```

290 295 300

Val Glu Ala Glu Ala Ala Gly Arg Leu Met Phe Tyr Gly Asn Gly Ala
305 310 315 320

Gly Gly Ala Pro Thr Ala Ser Ala Val Leu Gly Asp Val Val Gly Ala
325 330 335

Ala Arg Asn Lys Val His Gly Gly Arg Ala Pro Gly Glu Ser Thr Tyr
340 345 350

Ala Asn Leu Pro Ile Ala Asp Phe Gly Glu Thr Thr Thr Arg Tyr His
355 360 365

Leu Asp Met Asp Val Glu Asp Arg Val Gly Val Leu Ala Glu Leu Ala
370 375 380

Ser Leu Phe Ser Glu Gln Gly Ile Ser Leu Arg Thr Ile Arg Gln Glu
385 390 395 400

Glu Arg Asp Asp Asp Ala Arg Leu Ile Val Val Thr His Ser Ala Leu
405 410 415

Glu Ser Asp Leu Ser Arg Thr Val Glu Leu Leu Lys Ala Lys Pro Val
420 425 430

Val Lys Ala Ile Asn Ser Val Ile Arg Leu Glu Arg Asp
435 440 445

<210> 15
<211> 310
<212> PRT
<213> Escherichia coli

<220>
<221> Homoserine kinase
<222> (1)..(310)

<400> 15

Met Val Lys Val Tyr Ala Pro Ala Ser Ser Ala Asn Met Ser Val Gly
1 5 10 15

Phe Asp Val Leu Gly Ala Ala Val Thr Pro Val Asp Gly Ala Leu Leu
20 25 30

Gly Asp Val Val Thr Val Glu Ala Ala Glu Thr Phe Ser Leu Asn Asn
35 40 45

```
Leu Gly Arg Phe Ala Asp Lys Leu Pro Ser Glu Pro Arg Glu Asn Ile
    50                  55                  60

Val Tyr Gln Cys Trp Glu Arg Phe Cys Gln Glu Leu Gly Lys Gln Ile
65                  70                  75                  80

Pro Val Ala Met Thr Leu Glu Lys Asn Met Pro Ile Gly Ser Gly Leu
                85                  90                  95

Gly Ser Ser Ala Cys Ser Val Val Ala Ala Leu Met Ala Met Asn Glu
            100                 105                 110

His Cys Gly Lys Pro Leu Asn Asp Thr Arg Leu Leu Ala Leu Met Gly
        115                 120                 125

Glu Leu Glu Gly Arg Ile Ser Gly Ser Ile His Tyr Asp Asn Val Ala
    130                 135                 140

Pro Cys Phe Leu Gly Gly Met Gln Leu Met Ile Glu Glu Asn Asp Ile
145                 150                 155                 160

Ile Ser Gln Gln Val Pro Gly Phe Asp Glu Trp Leu Trp Val Leu Ala
                165                 170                 175

Tyr Pro Gly Ile Lys Val Ser Thr Ala Glu Ala Arg Ala Ile Leu Pro
            180                 185                 190

Ala Gln Tyr Arg Arg Gln Asp Cys Ile Ala His Gly Arg His Leu Ala
        195                 200                 205

Gly Phe Ile His Ala Cys Tyr Ser Arg Gln Pro Glu Leu Ala Ala Lys
    210                 215                 220

Leu Met Lys Asp Val Ile Ala Glu Pro Tyr Arg Glu Arg Leu Leu Pro
225                 230                 235                 240

Gly Phe Arg Gln Ala Arg Gln Ala Val Ala Glu Ile Gly Ala Val Ala
                245                 250                 255

Ser Gly Ile Ser Gly Ser Gly Pro Thr Leu Phe Ala Leu Cys Asp Lys
            260                 265                 270

Pro Glu Thr Ala Gln Arg Val Ala Asp Trp Leu Gly Lys Asn Tyr Leu
        275                 280                 285

Gln Asn Gln Glu Gly Phe Val His Ile Cys Arg Leu Asp Thr Ala Gly
    290                 295                 300
```

Ala Arg Val Leu Glu Asn
305 310

<210> 16
<211> 377
<212> PRT
<213> Corynebacterium glutamicum

<220>
<221> Homoserin O-Acetyltransferase
<222> (1)..(377)

<400> 16

Met Pro Thr Leu Ala Pro Ser Gly Gln Leu Glu Ile Gln Ala Ile Gly
1 5 10 15

Asp Val Ser Thr Glu Ala Gly Ala Ile Ile Thr Asn Ala Glu Ile Ala
20 25 30

Tyr His Arg Trp Gly Glu Tyr Arg Val Asp Lys Glu Gly Arg Ser Asn
35 40 45

Val Val Leu Ile Glu His Ala Leu Thr Gly Asp Ser Asn Ala Ala Asp
50 55 60

Trp Trp Ala Asp Leu Leu Gly Pro Gly Lys Ala Ile Asn Thr Asp Ile
65 70 75 80

Tyr Cys Val Ile Cys Thr Asn Val Ile Gly Gly Cys Asn Gly Ser Thr
85 90 95

Gly Pro Gly Ser Met His Pro Asp Gly Asn Phe Trp Gly Asn Arg Phe
100 105 110

Pro Ala Thr Ser Ile Arg Asp Gln Val Asn Ala Glu Lys Gln Phe Leu
115 120 125

Asp Ala Leu Gly Ile Thr Thr Val Ala Ala Val Leu Gly Gly Ser Met
130 135 140

Gly Gly Ala Arg Thr Leu Glu Trp Ala Ala Met Tyr Pro Glu Thr Val
145 150 155 160

Gly Ala Ala Ala Val Leu Ala Val Ser Ala Arg Ala Ser Ala Trp Gln
165 170 175

Ile Gly Ile Gln Ser Ala Gln Ile Lys Ala Ile Glu Asn Asp His His
180 185 190

```
Trp His Glu Gly Asn Tyr Tyr Glu Ser Gly Cys Asn Pro Ala Thr Gly
        195                 200                 205

Leu Gly Ala Ala Arg Arg Ile Ala His Leu Thr Tyr Arg Gly Glu Leu
    210                 215                 220

Glu Ile Asp Glu Arg Phe Gly Thr Lys Ala Gln Lys Asn Glu Asn Pro
225                 230                 235                 240

Leu Gly Pro Tyr Arg Lys Pro Asp Gln Arg Phe Ala Val Glu Ser Tyr
                245                 250                 255

Leu Asp Tyr Gln Ala Asp Lys Leu Val Gln Arg Phe Asp Ala Gly Ser
            260                 265                 270

Tyr Val Leu Leu Thr Asp Ala Leu Asn Arg His Asp Ile Gly Arg Asp
        275                 280                 285

Arg Gly Gly Leu Asn Lys Ala Leu Glu Ser Ile Lys Val Pro Val Leu
    290                 295                 300

Val Ala Gly Val Asp Thr Asp Ile Leu Tyr Pro Tyr His Gln Gln Glu
305                 310                 315                 320

His Leu Ser Arg Asn Leu Gly Asn Leu Leu Ala Met Ala Lys Ile Val
                325                 330                 335

Ser Pro Val Gly His Asp Ala Phe Leu Thr Glu Ser Arg Gln Met Asp
            340                 345                 350

Arg Ile Val Arg Asn Phe Phe Ser Leu Ile Ser Pro Asp Glu Asp Asn
        355                 360                 365

Pro Ser Thr Tyr Ile Glu Phe Tyr Ile
    370                 375


<210>  17
<211>  502
<212>  PRT
<213>  Corynebacterium glutamicum

<400>  17

Met Ser Asp Arg Ile Ala Ser Glu Lys Leu Arg Ser Lys Leu Met Ser
1               5                   10                  15

Ala Asp Glu Ala Ala Gln Phe Val Asn His Gly Asp Lys Val Gly Phe
            20                  25                  30
```

Ser Gly Phe Thr Gly Ala Gly Tyr Pro Lys Ala Leu Pro Thr Ala Ile
        35                  40                  45

Ala Asn Arg Ala Lys Glu Ala His Gly Ala Gly Asn Asp Tyr Ala Ile
    50                  55                  60

Asp Leu Phe Thr Gly Ala Ser Thr Ala Pro Asp Cys Asp Gly Val Leu
65                  70                  75                  80

Ala Glu Ala Asp Ala Ile Arg Trp Arg Met Pro Tyr Ala Ser Asp Pro
                85                  90                  95

Ile Met Arg Asn Lys Ile Asn Ser Gly Ser Met Gly Tyr Ser Asp Ile
            100                 105                 110

His Leu Ser His Ser Gly Gln Gln Val Glu Glu Gly Phe Phe Gly Gln
        115                 120                 125

Leu Asn Val Ala Val Ile Glu Ile Thr Arg Ile Thr Glu Glu Gly Tyr
    130                 135                 140

Ile Ile Pro Ser Ser Ser Val Gly Asn Asn Val Glu Trp Leu Asn Ala
145                 150                 155                 160

Ala Glu Lys Val Ile Leu Glu Val Asn Ser Trp Gln Ser Glu Asp Leu
                165                 170                 175

Glu Gly Met His Asp Ile Trp Ser Val Pro Ala Leu Pro Asn Arg Ile
            180                 185                 190

Ala Val Pro Ile Asn Lys Pro Gly Asp Arg Ile Gly Lys Thr Tyr Ile
        195                 200                 205

Glu Phe Asp Thr Asp Lys Val Val Ala Val Val Glu Thr Asn Thr Ala
    210                 215                 220

Asp Arg Asn Ala Pro Phe Lys Pro Val Asp Asp Ile Ser Lys Lys Ile
225                 230                 235                 240

Ala Gly Asn Phe Leu Asp Phe Leu Glu Ser Glu Val Ala Ala Gly Arg
                245                 250                 255

Leu Ser Tyr Asp Gly Tyr Ile Met Gln Ser Gly Val Gly Asn Val Pro
            260                 265                 270

Asn Ala Val Met Ala Gly Leu Leu Glu Ser Lys Phe Glu Asn Ile Gln

275 280 285

Ala Tyr Thr Glu Val Ile Gln Asp Gly Met Val Asp Leu Ile Asp Ala
290 295 300

Gly Lys Met Thr Val Ala Ser Ala Thr Ser Phe Ser Leu Ser Pro Glu
305 310 315 320

Tyr Ala Glu Lys Met Asn Asn Glu Ala Lys Arg Tyr Arg Glu Ser Ile
325 330 335

Ile Leu Arg Pro Gln Gln Ile Ser Asn His Pro Glu Val Ile Arg Arg
340 345 350

Val Gly Leu Ile Ala Thr Asn Gly Leu Ile Glu Ala Asp Ile Tyr Gly
355 360 365

Asn Val Asn Ser Thr Asn Val Ser Gly Ser Arg Val Met Asn Gly Ile
370 375 380

Gly Gly Ser Gly Asp Phe Thr Arg Asn Gly Tyr Ile Ser Ser Phe Ile
385 390 395 400

Thr Pro Ser Glu Ala Lys Gly Gly Ala Ile Ser Ala Ile Val Pro Phe
405 410 415

Ala Ser His Ile Asp His Thr Glu His Asp Val Met Val Val Ile Ser
420 425 430

Glu Tyr Gly Tyr Ala Asp Leu Arg Gly Leu Ala Pro Arg Glu Arg Val
435 440 445

Ala Lys Met Ile Gly Leu Ala His Pro Asp Tyr Arg Pro Leu Leu Glu
450 455 460

Glu Tyr Tyr Ala Arg Ala Thr Ser Gly Asp Asn Lys Tyr Met Gln Thr
465 470 475 480

Pro His Asp Leu Ala Thr Ala Phe Asp Phe His Ile Asn Leu Ala Lys
485 490 495

Asn Gly Ser Met Lys Ala
500

<210> 18
<211> 428
<212> PRT
<213> Escherichia coli

<220>
<221>  threonine synthase
<222>  (1)..(428)

<400>  18

```
Met Lys Leu Tyr Asn Leu Lys Asp His Asn Glu Gln Val Ser Phe Ala
1               5                   10                  15

Gln Ala Val Thr Gln Gly Leu Gly Lys Asn Gln Gly Leu Phe Phe Pro
            20                  25                  30

His Asp Leu Pro Glu Phe Ser Leu Thr Glu Ile Asp Glu Met Leu Lys
        35                  40                  45

Leu Asp Phe Val Thr Arg Ser Ala Lys Ile Leu Ser Ala Phe Ile Gly
    50                  55                  60

Asp Glu Ile Pro Gln Glu Ile Leu Glu Glu Arg Val Arg Ala Ala Phe
65                  70                  75                  80

Ala Phe Pro Ala Pro Val Ala Asn Val Glu Ser Asp Val Gly Cys Leu
                85                  90                  95

Glu Leu Phe His Gly Pro Thr Leu Ala Phe Lys Asp Phe Gly Gly Arg
            100                 105                 110

Phe Met Ala Gln Met Leu Thr His Ile Ala Gly Asp Lys Pro Val Thr
        115                 120                 125

Ile Leu Thr Ala Thr Ser Gly Asp Thr Gly Ala Ala Val Ala His Ala
    130                 135                 140

Phe Tyr Gly Leu Pro Asn Val Lys Val Val Ile Leu Tyr Pro Arg Gly
145                 150                 155                 160

Lys Ile Ser Pro Leu Gln Glu Lys Leu Phe Cys Thr Leu Gly Gly Asn
                165                 170                 175

Ile Glu Thr Val Ala Ile Asp Gly Asp Phe Asp Ala Cys Gln Ala Leu
            180                 185                 190

Val Lys Gln Ala Phe Asp Asp Glu Glu Leu Lys Val Ala Leu Gly Leu
        195                 200                 205

Asn Ser Ala Asn Ser Ile Asn Ile Ser Arg Leu Leu Ala Gln Ile Cys
    210                 215                 220
```

```
Tyr Tyr Phe Glu Ala Val Ala Gln Leu Pro Gln Glu Thr Arg Asn Gln
225                 230                 235                 240

Leu Val Val Ser Val Pro Ser Gly Asn Phe Gly Asp Leu Thr Ala Gly
                245                 250                 255

Leu Leu Ala Lys Ser Leu Gly Leu Pro Val Lys Arg Phe Ile Ala Ala
            260                 265                 270

Thr Asn Val Asn Asp Thr Val Pro Arg Phe Leu His Asp Gly Gln Trp
        275                 280                 285

Ser Pro Lys Ala Thr Gln Ala Thr Leu Ser Asn Ala Met Asp Val Ser
    290                 295                 300

Gln Pro Asn Asn Trp Pro Arg Val Glu Glu Leu Phe Arg Arg Lys Ile
305                 310                 315                 320

Trp Gln Leu Lys Glu Leu Gly Tyr Ala Ala Val Asp Asp Glu Thr Thr
                325                 330                 335

Gln Gln Thr Met Arg Glu Leu Lys Glu Leu Gly Tyr Thr Ser Glu Pro
            340                 345                 350

His Ala Ala Val Ala Tyr Arg Ala Leu Arg Asp Gln Leu Asn Pro Gly
        355                 360                 365

Glu Tyr Gly Leu Phe Leu Gly Thr Ala His Pro Ala Lys Phe Lys Glu
    370                 375                 380

Ser Val Glu Ala Ile Leu Gly Glu Thr Leu Asp Leu Pro Lys Glu Leu
385                 390                 395                 400

Ala Glu Arg Ala Asp Leu Pro Leu Leu Ser His Asn Leu Pro Ala Asp
                405                 410                 415

Phe Ala Ala Leu Arg Lys Leu Met Met Asn His Gln
            420                 425
```

<210>  19
<211>  206
<212>  PRT
<213>  Escherichia coli

<220>
<221>  threonine exporter
<222>  (1)..(206)

<400> 19

Met Leu Met Leu Phe Leu Thr Val Ala Met Val His Ile Val Ala Leu
1 5 10 15

Met Ser Pro Gly Pro Asp Phe Phe Phe Val Ser Gln Thr Ala Val Ser
20 25 30

Arg Ser Arg Lys Glu Ala Met Met Gly Val Leu Gly Ile Thr Cys Gly
35 40 45

Val Met Val Trp Ala Gly Ile Ala Leu Leu Gly Leu His Leu Ile Ile
50 55 60

Glu Lys Met Ala Trp Leu His Thr Leu Ile Met Val Gly Gly Gly Leu
65 70 75 80

Tyr Leu Cys Trp Met Gly Tyr Gln Met Leu Arg Gly Ala Leu Lys Lys
85 90 95

Glu Ala Val Ser Ala Pro Ala Pro Gln Val Glu Leu Ala Lys Ser Gly
100 105 110

Arg Ser Phe Leu Lys Gly Leu Leu Thr Asn Leu Ala Asn Pro Lys Ala
115 120 125

Ile Ile Tyr Phe Gly Ser Val Phe Ser Leu Phe Val Gly Asp Asn Val
130 135 140

Gly Thr Thr Ala Arg Trp Gly Ile Phe Ala Leu Ile Ile Val Glu Thr
145 150 155 160

Leu Ala Trp Phe Thr Val Val Ala Ser Leu Phe Ala Leu Pro Gln Met
165 170 175

Arg Arg Gly Tyr Gln Arg Leu Ala Lys Trp Ile Asp Gly Phe Ala Gly
180 185 190

Ala Leu Phe Ala Gly Phe Gly Ile His Leu Ile Ile Ser Arg
195 200 205

<210> 20
<211> 462
<212> PRT
<213> Escherichia coli

<220>
<221> proton-translocating transhydrogenase (pntB)
<222> (1)..(462)

<400> 20

Met Ser Gly Gly Leu Val Thr Ala Ala Tyr Ile Val Ala Ala Ile Leu
1               5                   10                  15

Phe Ile Phe Ser Leu Ala Gly Leu Ser Lys His Glu Thr Ser Arg Gln
            20                  25                  30

Gly Asn Asn Phe Gly Ile Ala Gly Met Ala Ile Ala Leu Ile Ala Thr
        35                  40                  45

Ile Phe Gly Pro Asp Thr Gly Asn Val Gly Trp Ile Leu Leu Ala Met
    50                  55                  60

Val Ile Gly Gly Ala Ile Gly Ile Arg Leu Ala Lys Lys Val Glu Met
65                  70                  75                  80

Thr Glu Met Pro Glu Leu Val Ala Ile Leu His Ser Phe Val Gly Leu
                85                  90                  95

Ala Ala Val Leu Val Gly Phe Asn Ser Tyr Leu His His Asp Ala Gly
            100                 105                 110

Met Ala Pro Ile Leu Val Asn Ile His Leu Thr Glu Val Phe Leu Gly
        115                 120                 125

Ile Phe Ile Gly Ala Val Thr Phe Thr Gly Ser Val Val Ala Phe Gly
    130                 135                 140

Lys Leu Cys Gly Lys Ile Ser Ser Lys Pro Leu Met Leu Pro Asn Arg
145                 150                 155                 160

His Lys Met Asn Leu Ala Ala Leu Val Val Ser Phe Leu Leu Leu Ile
                165                 170                 175

Val Phe Val Arg Thr Asp Ser Val Gly Leu Gln Val Leu Ala Leu Leu
            180                 185                 190

Ile Met Thr Ala Ile Ala Leu Val Phe Gly Trp His Leu Val Ala Ser
        195                 200                 205

Ile Gly Gly Ala Asp Met Pro Val Val Val Ser Met Leu Asn Ser Tyr
    210                 215                 220

Ser Gly Trp Ala Ala Ala Ala Ala Gly Phe Met Leu Ser Asn Asp Leu
225                 230                 235                 240

```
Leu Ile Val Thr Gly Ala Leu Val Gly Ser Ser Gly Ala Ile Leu Ser
                245                 250                 255

Tyr Ile Met Cys Lys Ala Met Asn Arg Ser Phe Ile Ser Val Ile Ala
            260                 265                 270

Gly Gly Phe Gly Thr Asp Gly Ser Ser Thr Gly Asp Asp Gln Glu Val
        275                 280                 285

Gly Glu His Arg Glu Ile Thr Ala Glu Glu Thr Ala Glu Leu Leu Lys
    290                 295                 300

Asn Ser His Ser Val Ile Ile Thr Pro Gly Tyr Gly Met Ala Val Ala
305                 310                 315                 320

Gln Ala Gln Tyr Pro Val Ala Glu Ile Thr Glu Lys Leu Arg Ala Arg
                325                 330                 335

Gly Ile Asn Val Arg Phe Gly Ile His Pro Val Ala Gly Arg Leu Pro
            340                 345                 350

Gly His Met Asn Val Leu Leu Ala Glu Ala Lys Val Pro Tyr Asp Ile
        355                 360                 365

Val Leu Glu Met Asp Glu Ile Asn Asp Asp Phe Ala Asp Thr Asp Thr
    370                 375                 380

Val Leu Val Ile Gly Ala Asn Asp Thr Val Asn Pro Ala Ala Gln Asp
385                 390                 395                 400

Asp Pro Lys Ser Pro Ile Ala Gly Met Pro Val Leu Glu Val Trp Lys
                405                 410                 415

Ala Gln Asn Val Ile Val Phe Lys Arg Ser Met Asn Thr Gly Tyr Ala
            420                 425                 430

Gly Val Gln Asn Pro Leu Phe Phe Lys Glu Asn Thr His Met Leu Phe
        435                 440                 445

Gly Asp Ala Lys Ala Ser Val Asp Ala Ile Leu Lys Ala Leu
    450                 455                 460
```

<210> 21
<211> 719
<212> PRT
<213> Escherichia coli

<220>

<221> acyl-ACP synthetase
<222> (1)..(719)

<400> 21

Met Leu Phe Ser Phe Phe Arg Asn Leu Cys Arg Val Leu Tyr Arg Val
1 5 10 15

Arg Val Thr Gly Asp Thr Gln Ala Leu Lys Gly Glu Arg Val Leu Ile
20 25 30

Thr Pro Asn His Val Ser Phe Ile Asp Gly Ile Leu Leu Gly Leu Phe
35 40 45

Leu Pro Val Arg Pro Val Phe Ala Val Tyr Thr Ser Ile Ser Gln Gln
50 55 60

Trp Tyr Met Arg Trp Leu Lys Ser Phe Ile Asp Phe Val Pro Leu Asp
65 70 75 80

Pro Thr Gln Pro Met Ala Ile Lys His Leu Val Arg Leu Val Glu Gln
85 90 95

Gly Arg Pro Val Val Ile Phe Pro Glu Gly Arg Ile Thr Thr Thr Gly
100 105 110

Ser Leu Met Lys Ile Tyr Asp Gly Ala Gly Phe Val Ala Ala Lys Ser
115 120 125

Gly Ala Thr Val Ile Pro Val Arg Ile Glu Gly Ala Glu Leu Thr His
130 135 140

Phe Ser Arg Leu Lys Gly Leu Val Lys Arg Arg Leu Phe Pro Gln Ile
145 150 155 160

Thr Leu His Ile Leu Pro Pro Thr Gln Val Ala Met Pro Asp Ala Pro
165 170 175

Arg Ala Arg Asp Arg Arg Lys Ile Ala Gly Glu Met Leu His Gln Ile
180 185 190

Met Met Glu Ala Arg Met Ala Val Arg Pro Arg Glu Thr Leu Tyr Glu
195 200 205

Ser Leu Leu Ser Ala Met Tyr Arg Phe Gly Ala Gly Lys Lys Cys Val
210 215 220

Glu Asp Val Asn Phe Thr Pro Asp Ser Tyr Arg Lys Leu Leu Thr Lys
225 230 235 240

Thr Leu Phe Val Gly Arg Ile Leu Glu Lys Tyr Ser Val Glu Gly Glu
245 250 255

Arg Ile Gly Leu Met Leu Pro Asn Ala Gly Ile Ser Ala Ala Val Ile
260 265 270

Phe Gly Ala Ile Ala Arg Arg Arg Met Pro Ala Met Met Asn Tyr Thr
275 280 285

Ala Gly Val Lys Gly Leu Thr Ser Ala Ile Thr Ala Ala Glu Ile Lys
290 295 300

Thr Ile Phe Thr Ser Arg Gln Phe Leu Asp Lys Gly Lys Leu Trp His
305 310 315 320

Leu Pro Glu Gln Leu Thr Gln Val Arg Trp Val Tyr Leu Glu Asp Leu
325 330 335

Lys Ala Asp Val Thr Thr Ala Asp Lys Val Trp Ile Phe Ala His Leu
340 345 350

Leu Met Pro Arg Leu Ala Gln Val Lys Gln Gln Pro Glu Glu Glu Ala
355 360 365

Leu Ile Leu Phe Thr Ser Gly Ser Glu Gly His Pro Lys Gly Val Val
370 375 380

His Ser His Lys Ser Ile Leu Ala Asn Val Glu Gln Ile Lys Thr Ile
385 390 395 400

Ala Asp Phe Thr Thr Asn Asp Arg Phe Met Ser Ala Leu Pro Leu Phe
405 410 415

His Ser Phe Gly Leu Thr Val Gly Leu Phe Thr Pro Leu Leu Thr Gly
420 425 430

Ala Glu Val Phe Leu Tyr Pro Ser Pro Leu His Tyr Arg Ile Val Pro
435 440 445

Glu Leu Val Tyr Asp Arg Ser Cys Thr Val Leu Phe Gly Thr Ser Thr
450 455 460

Phe Leu Gly His Tyr Ala Arg Phe Ala Asn Pro Tyr Asp Phe Tyr Arg
465 470 475 480

Leu Arg Tyr Val Val Ala Gly Ala Glu Lys Leu Gln Glu Ser Thr Lys

485 490 495

Gln Leu Trp Gln Asp Lys Phe Gly Leu Arg Ile Leu Glu Gly Tyr Gly
500 505 510

Val Thr Glu Cys Ala Pro Val Val Ser Ile Asn Val Pro Met Ala Ala
515 520 525

Lys Pro Gly Thr Val Gly Arg Ile Leu Pro Gly Met Asp Ala Arg Leu
530 535 540

Leu Ser Val Pro Gly Ile Glu Glu Gly Gly Arg Leu Gln Leu Lys Gly
545 550 555 560

Pro Asn Ile Met Asn Gly Tyr Leu Arg Val Glu Lys Pro Gly Val Leu
565 570 575

Glu Val Pro Thr Ala Glu Asn Val Arg Gly Glu Met Glu Arg Gly Trp
580 585 590

Tyr Asp Thr Gly Asp Ile Val Arg Phe Asp Glu Gln Gly Phe Val Gln
595 600 605

Ile Gln Gly Arg Ala Lys Arg Phe Ala Lys Ile Ala Gly Glu Met Val
610 615 620

Ser Leu Glu Met Val Glu Gln Leu Ala Leu Gly Val Ser Pro Asp Lys
625 630 635 640

Val His Ala Thr Ala Ile Lys Ser Asp Ala Ser Lys Gly Glu Ala Leu
645 650 655

Val Leu Phe Thr Thr Asp Asn Glu Leu Thr Arg Asp Lys Leu Gln Gln
660 665 670

Tyr Ala Arg Glu His Gly Val Pro Glu Leu Ala Val Pro Arg Asp Ile
675 680 685

Arg Tyr Leu Lys Gln Met Pro Leu Leu Gly Ser Gly Lys Pro Asp Phe
690 695 700

Val Thr Leu Lys Ser Trp Val Asp Glu Ala Glu Gln His Asp Glu
705 710 715

<210> 22
<211> 561
<212> PRT
<213> Escherichia coli

<220>
<221> acyl-CoA synthetase
<222> (1)..(561)

<400> 22

```
Met Lys Lys Val Trp Leu Asn Arg Tyr Pro Ala Asp Val Pro Thr Glu
1               5                   10                  15

Ile Asn Pro Asp Arg Tyr Gln Ser Leu Val Asp Met Phe Glu Gln Ser
            20                  25                  30

Val Ala Arg Tyr Ala Asp Gln Pro Ala Phe Val Asn Met Gly Glu Val
        35                  40                  45

Met Thr Phe Arg Lys Leu Glu Glu Arg Ser Arg Ala Phe Ala Ala Tyr
    50                  55                  60

Leu Gln Gln Gly Leu Gly Leu Lys Lys Gly Asp Arg Val Ala Leu Met
65                  70                  75                  80

Met Pro Asn Leu Leu Gln Tyr Pro Val Ala Leu Phe Gly Ile Leu Arg
                85                  90                  95

Ala Gly Met Ile Val Val Asn Val Asn Pro Leu Tyr Thr Pro Arg Glu
            100                 105                 110

Leu Glu His Gln Leu Asn Asp Ser Gly Ala Ser Ala Ile Val Ile Val
        115                 120                 125

Ser Asn Phe Ala His Thr Leu Glu Lys Val Val Asp Lys Thr Ala Val
    130                 135                 140

Gln His Val Ile Leu Thr Arg Met Gly Asp Gln Leu Ser Thr Ala Lys
145                 150                 155                 160

Gly Thr Val Val Asn Phe Val Val Lys Tyr Ile Lys Arg Leu Val Pro
                165                 170                 175

Lys Tyr His Leu Pro Asp Ala Ile Ser Phe Arg Ser Ala Leu His Asn
            180                 185                 190

Gly Tyr Arg Met Gln Tyr Val Lys Pro Glu Leu Val Pro Glu Asp Leu
        195                 200                 205

Ala Phe Leu Gln Tyr Thr Gly Gly Thr Thr Gly Val Ala Lys Gly Ala
    210                 215                 220
```

Met Leu Thr His Arg Asn Met Leu Ala Asn Leu Glu Gln Val Asn Ala
225                 230                 235                 240

Thr Tyr Gly Pro Leu Leu His Pro Gly Lys Glu Leu Val Val Thr Ala
                245                 250                 255

Leu Pro Leu Tyr His Ile Phe Ala Leu Thr Ile Asn Cys Leu Leu Phe
            260                 265                 270

Ile Glu Leu Gly Gly Gln Asn Leu Leu Ile Thr Asn Pro Arg Asp Ile
        275                 280                 285

Pro Gly Leu Val Lys Glu Leu Ala Lys Tyr Pro Phe Thr Ala Ile Thr
    290                 295                 300

Gly Val Asn Thr Leu Phe Asn Ala Leu Leu Asn Asn Lys Glu Phe Gln
305                 310                 315                 320

Gln Leu Asp Phe Ser Ser Leu His Leu Ser Ala Gly Gly Gly Met Pro
                325                 330                 335

Val Gln Gln Val Val Ala Glu Arg Trp Val Lys Leu Thr Gly Gln Tyr
            340                 345                 350

Leu Leu Glu Gly Tyr Gly Leu Thr Glu Cys Ala Pro Leu Val Ser Val
        355                 360                 365

Asn Pro Tyr Asp Ile Asp Tyr His Ser Gly Ser Ile Gly Leu Pro Val
    370                 375                 380

Pro Ser Thr Glu Ala Lys Leu Val Asp Asp Asp Asp Asn Glu Val Pro
385                 390                 395                 400

Pro Gly Gln Pro Gly Glu Leu Cys Val Lys Gly Pro Gln Val Met Leu
                405                 410                 415

Gly Tyr Trp Gln Arg Pro Asp Ala Thr Asp Glu Ile Ile Lys Asn Gly
            420                 425                 430

Trp Leu His Thr Gly Asp Ile Ala Val Met Asp Glu Glu Gly Phe Leu
        435                 440                 445

Arg Ile Val Asp Arg Lys Lys Asp Met Ile Leu Val Ser Gly Phe Asn
    450                 455                 460

Val Tyr Pro Asn Glu Ile Glu Asp Val Val Met Gln His Pro Gly Val
465                 470                 475                 480

Gln Glu Val Ala Ala Val Gly Val Pro Ser Gly Ser Ser Gly Glu Ala
                485                 490                 495

Val Lys Ile Phe Val Val Lys Lys Asp Pro Ser Leu Thr Glu Glu Ser
            500                 505                 510

Leu Val Thr Phe Cys Arg Arg Gln Leu Thr Gly Tyr Lys Val Pro Lys
        515                 520                 525

Leu Val Glu Phe Arg Asp Glu Leu Pro Lys Ser Asn Val Gly Lys Ile
    530                 535                 540

Leu Arg Arg Glu Leu Arg Asp Glu Ala Arg Gly Lys Val Asp Asn Lys
545                 550                 555                 560

Ala

<210>  23
<211>  400
<212>  PRT
<213>  Escherichia coli

<220>
<221>  fatty acyl kinase
<222>  (1)..(400)

<400>  23

Met Ser Ser Lys Leu Val Leu Val Leu Asn Cys Gly Ser Ser Ser Leu
1               5                   10                  15

Lys Phe Ala Ile Ile Asp Ala Val Asn Gly Glu Glu Tyr Leu Ser Gly
            20                  25                  30

Leu Ala Glu Cys Phe His Leu Pro Glu Ala Arg Ile Lys Trp Lys Met
        35                  40                  45

Asp Gly Asn Lys Gln Glu Ala Ala Leu Gly Ala Gly Ala Ala His Ser
    50                  55                  60

Glu Ala Leu Asn Phe Ile Val Asn Thr Ile Leu Ala Gln Lys Pro Glu
65                  70                  75                  80

Leu Ser Ala Gln Leu Thr Ala Ile Gly His Arg Ile Val His Gly Gly
                85                  90                  95

Glu Lys Tyr Thr Ser Ser Val Val Ile Asp Glu Ser Val Ile Gln Gly

```
            100                 105                 110

Ile Lys Asp Ala Ala Ser Phe Ala Pro Leu His Asn Pro Ala His Leu
        115                 120                 125

Ile Gly Ile Glu Glu Ala Leu Lys Ser Phe Pro Gln Leu Lys Asp Lys
    130                 135                 140

Asn Val Ala Val Phe Asp Thr Ala Phe His Gln Thr Met Pro Glu Glu
145                 150                 155                 160

Ser Tyr Leu Tyr Ala Leu Pro Tyr Asn Leu Tyr Lys Glu His Gly Ile
                165                 170                 175

Arg Arg Tyr Gly Ala His Gly Thr Ser His Phe Tyr Val Thr Gln Glu
            180                 185                 190

Ala Ala Lys Met Leu Asn Lys Pro Val Glu Glu Leu Asn Ile Ile Thr
        195                 200                 205

Cys His Leu Gly Asn Gly Gly Ser Val Ser Ala Ile Arg Asn Gly Lys
    210                 215                 220

Cys Val Asp Thr Ser Met Gly Leu Thr Pro Leu Glu Gly Leu Val Met
225                 230                 235                 240

Gly Thr Arg Ser Gly Asp Ile Asp Pro Ala Ile Ile Phe His Leu His
                245                 250                 255

Asp Thr Leu Gly Met Ser Val Asp Ala Ile Asn Lys Leu Leu Thr Lys
            260                 265                 270

Glu Ser Gly Leu Leu Gly Leu Thr Glu Val Thr Ser Asp Cys Arg Tyr
        275                 280                 285

Val Glu Asp Asn Tyr Ala Thr Lys Glu Asp Ala Lys Arg Ala Met Asp
    290                 295                 300

Val Tyr Cys His Arg Leu Ala Lys Tyr Ile Gly Ala Tyr Thr Ala Leu
305                 310                 315                 320

Met Asp Gly Arg Leu Asp Ala Val Val Phe Thr Gly Gly Ile Gly Glu
                325                 330                 335

Asn Ala Ala Met Val Arg Glu Leu Ser Leu Gly Lys Leu Gly Val Leu
            340                 345                 350
```

Gly Phe Glu Val Asp His Glu Arg Asn Leu Ala Ala Arg Phe Gly Lys
355 360 365

Ser Gly Phe Ile Asn Lys Glu Gly Thr Arg Pro Ala Val Val Ile Pro
370 375 380

Thr Asn Glu Glu Leu Val Ile Ala Gln Asp Ala Ser Arg Leu Thr Ala
385 390 395 400

<210> 24
<211> 301
<212> PRT
<213> Clostridium acetobutylicum

<220>
<221> phosphotransacylase
<222> (1)..(301)

<400> 24

Met Ile Lys Ser Phe Asn Glu Ile Ile Met Lys Val Lys Ser Lys Glu
1 5 10 15

Met Lys Lys Val Ala Val Ala Val Ala Gln Asp Glu Pro Val Leu Glu
20 25 30

Ala Val Arg Asp Ala Lys Lys Asn Gly Ile Ala Asp Ala Ile Leu Val
35 40 45

Gly Asp His Asp Glu Ile Val Ser Ile Ala Leu Lys Ile Gly Met Asp
50 55 60

Val Asn Asp Phe Glu Ile Val Asn Glu Pro Asn Val Lys Lys Ala Ala
65 70 75 80

Leu Lys Ala Val Glu Leu Val Ser Thr Gly Lys Ala Asp Met Val Met
85 90 95

Lys Gly Leu Val Asn Thr Ala Thr Phe Leu Arg Ser Val Leu Asn Lys
100 105 110

Glu Val Gly Leu Arg Thr Gly Lys Thr Met Ser His Val Ala Val Phe
115 120 125

Glu Thr Glu Lys Phe Asp Arg Leu Leu Phe Leu Thr Asp Val Ala Phe
130 135 140

Asn Thr Tyr Pro Glu Leu Lys Glu Lys Ile Asp Ile Val Asn Asn Ser
145 150 155 160

Val Lys Val Ala His Ala Ile Gly Ile Glu Asn Pro Lys Val Ala Pro
165 170 175

Ile Cys Ala Val Glu Val Ile Asn Pro Lys Met Pro Ser Thr Leu Asp
180 185 190

Ala Ala Met Leu Ser Lys Met Ser Asp Arg Gly Gln Ile Lys Gly Cys
195 200 205

Val Val Asp Gly Pro Leu Ala Leu Asp Ile Ala Leu Ser Glu Glu Ala
210 215 220

Ala His His Lys Gly Val Thr Gly Glu Val Ala Gly Lys Ala Asp Ile
225 230 235 240

Phe Leu Met Pro Asn Ile Glu Thr Gly Asn Val Met Tyr Lys Thr Leu
245 250 255

Thr Tyr Thr Thr Asp Ser Lys Asn Gly Gly Ile Leu Val Gly Thr Ser
260 265 270

Ala Pro Val Val Leu Thr Ser Arg Ala Asp Ser His Glu Thr Lys Met
275 280 285

Asn Ser Ile Ala Leu Ala Ala Leu Val Ala Gly Asn Lys
290 295 300

<210> 25
<211> 355
<212> PRT
<213> Clostridium acetobutylicum

<220>
<221> butyrate kinase
<222> (1)..(355)

<400> 25

Met Tyr Arg Leu Leu Ile Ile Asn Pro Gly Ser Thr Ser Thr Lys Ile
1 5 10 15

Gly Ile Tyr Asp Asp Glu Lys Glu Ile Phe Glu Lys Thr Leu Arg His
20 25 30

Ser Ala Glu Glu Ile Glu Lys Tyr Asn Thr Ile Phe Asp Gln Phe Gln
35 40 45

Phe Arg Lys Asn Val Ile Leu Asp Ala Leu Lys Glu Ala Asn Ile Glu
50 55 60

Val Ser Ser Leu Asn Ala Val Val Gly Arg Gly Gly Leu Leu Lys Pro
65 70 75 80

Ile Val Ser Gly Thr Tyr Ala Val Asn Gln Lys Met Leu Glu Asp Leu
85 90 95

Lys Val Gly Val Gln Gly Gln His Ala Ser Asn Leu Gly Gly Ile Ile
100 105 110

Ala Asn Glu Ile Ala Lys Glu Ile Asn Val Pro Ala Tyr Ile Val Asp
115 120 125

Pro Val Val Val Asp Glu Leu Asp Glu Val Ser Arg Ile Ser Gly Met
130 135 140

Ala Asp Ile Pro Arg Lys Ser Ile Phe His Ala Leu Asn Gln Lys Ala
145 150 155 160

Val Ala Arg Arg Tyr Ala Lys Glu Val Gly Lys Lys Tyr Glu Asp Leu
165 170 175

Asn Leu Ile Val Val His Met Gly Gly Gly Thr Ser Val Gly Thr His
180 185 190

Lys Asp Gly Arg Val Ile Glu Val Asn Asn Thr Leu Asp Gly Glu Gly
195 200 205

Pro Phe Ser Pro Glu Arg Ser Gly Gly Val Pro Ile Gly Asp Leu Val
210 215 220

Arg Leu Cys Phe Ser Asn Lys Tyr Thr Tyr Glu Glu Val Met Lys Lys
225 230 235 240

Ile Asn Gly Lys Gly Gly Val Val Ser Tyr Leu Asn Thr Ile Asp Phe
245 250 255

Lys Ala Val Val Asp Lys Ala Leu Glu Gly Asp Lys Lys Cys Ala Leu
260 265 270

Ile Tyr Glu Ala Phe Thr Phe Gln Val Ala Lys Glu Ile Gly Lys Cys
275 280 285

Ser Thr Val Leu Lys Gly Asn Val Asp Ala Ile Ile Leu Thr Gly Gly
290 295 300

Ile Ala Tyr Asn Glu His Val Cys Asn Ala Ile Glu Asp Arg Val Lys

305 310 315 320

Phe Ile Ala Pro Val Val Arg Tyr Gly Gly Glu Asp Glu Leu Leu Ala
325 330 335

Leu Ala Glu Gly Gly Leu Arg Val Leu Arg Gly Glu Glu Lys Ala Lys
340 345 350

Glu Tyr Lys
355

<210> 26
<211> 400
<212> PRT
<213> Escherichia coli

<220>
<221> propionate kinase
<222> (1)..(400)

<400> 26

Met Ser Ser Lys Leu Val Leu Val Leu Asn Cys Gly Ser Ser Ser Leu
1 5 10 15

Lys Phe Ala Ile Ile Asp Ala Val Asn Gly Glu Glu Tyr Leu Ser Gly
20 25 30

Leu Ala Glu Cys Phe His Leu Pro Glu Ala Arg Ile Lys Trp Lys Met
35 40 45

Asp Gly Asn Lys Gln Glu Ala Ala Leu Gly Ala Gly Ala Ala His Ser
50 55 60

Glu Ala Leu Asn Phe Ile Val Asn Thr Ile Leu Ala Gln Lys Pro Glu
65 70 75 80

Leu Ser Ala Gln Leu Thr Ala Ile Gly His Arg Ile Val His Gly Gly
85 90 95

Glu Lys Tyr Thr Ser Ser Val Val Ile Asp Glu Ser Val Ile Gln Gly
100 105 110

Ile Lys Asp Ala Ala Ser Phe Ala Pro Leu His Asn Pro Ala His Leu
115 120 125

Ile Gly Ile Glu Glu Ala Leu Lys Ser Phe Pro Gln Leu Lys Asp Lys
130 135 140

Asn Val Ala Val Phe Asp Thr Ala Phe His Gln Thr Met Pro Glu Glu
145 150 155 160

Ser Tyr Leu Tyr Ala Leu Pro Tyr Asn Leu Tyr Lys Glu His Gly Ile
165 170 175

Arg Arg Tyr Gly Ala His Gly Thr Ser His Phe Tyr Val Thr Gln Glu
180 185 190

Ala Ala Lys Met Leu Asn Lys Pro Val Glu Glu Leu Asn Ile Ile Thr
195 200 205

Cys His Leu Gly Asn Gly Gly Ser Val Ser Ala Ile Arg Asn Gly Lys
210 215 220

Cys Val Asp Thr Ser Met Gly Leu Thr Pro Leu Glu Gly Leu Val Met
225 230 235 240

Gly Thr Arg Ser Gly Asp Ile Asp Pro Ala Ile Ile Phe His Leu His
245 250 255

Asp Thr Leu Gly Met Ser Val Asp Ala Ile Asn Lys Leu Leu Thr Lys
260 265 270

Glu Ser Gly Leu Leu Gly Leu Thr Glu Val Thr Ser Asp Cys Arg Tyr
275 280 285

Val Glu Asp Asn Tyr Ala Thr Lys Glu Asp Ala Lys Arg Ala Met Asp
290 295 300

Val Tyr Cys His Arg Leu Ala Lys Tyr Ile Gly Ala Tyr Thr Ala Leu
305 310 315 320

Met Asp Gly Arg Leu Asp Ala Val Val Phe Thr Gly Gly Ile Gly Glu
325 330 335

Asn Ala Ala Met Val Arg Glu Leu Ser Leu Gly Lys Leu Gly Val Leu
340 345 350

Gly Phe Glu Val Asp His Glu Arg Asn Leu Ala Ala Arg Phe Gly Lys
355 360 365

Ser Gly Phe Ile Asn Lys Glu Gly Thr Arg Pro Ala Val Val Ile Pro
370 375 380

Thr Asn Glu Glu Leu Val Ile Ala Gln Asp Ala Ser Arg Leu Thr Ala
385 390 395 400

<210> 27
<211> 865
<212> PRT
<213> Escherichia coli

<400> 27

Met Leu Glu Glu Tyr Arg Lys His Val Ala Glu Arg Ala Ala Glu Gly
1 5 10 15

Ile Ala Pro Lys Pro Leu Asp Ala Asn Gln Met Ala Ala Leu Val Glu
20 25 30

Leu Leu Lys Asn Pro Pro Ala Gly Glu Glu Glu Phe Leu Leu Asp Leu
35 40 45

Leu Thr Asn Arg Val Pro Pro Gly Val Asp Glu Ala Ala Tyr Val Lys
50 55 60

Ala Gly Phe Leu Ala Ala Ile Ala Lys Gly Glu Ala Lys Ser Pro Leu
65 70 75 80

Leu Thr Pro Glu Lys Ala Ile Glu Leu Leu Gly Thr Met Gln Gly Gly
85 90 95

Tyr Asn Ile His Pro Leu Ile Asp Ala Leu Asp Asp Ala Lys Leu Ala
100 105 110

Pro Ile Ala Ala Lys Ala Leu Ser His Thr Leu Leu Met Phe Asp Asn
115 120 125

Phe Tyr Asp Val Glu Glu Lys Ala Lys Ala Gly Asn Glu Tyr Ala Lys
130 135 140

Gln Val Met Gln Ser Trp Ala Asp Ala Glu Trp Phe Leu Asn Arg Pro
145 150 155 160

Ala Leu Ala Glu Lys Leu Thr Val Thr Val Phe Lys Val Thr Gly Glu
165 170 175

Thr Asn Thr Asp Asp Leu Ser Pro Ala Pro Asp Ala Trp Ser Arg Pro
180 185 190

Asp Ile Pro Leu His Ala Leu Ala Met Leu Lys Asn Ala Arg Glu Gly
195 200 205

Ile Glu Pro Asp Gln Pro Gly Val Val Gly Pro Ile Lys Gln Ile Glu
210 215 220

```
Ala Leu Gln Gln Lys Gly Phe Pro Leu Ala Tyr Val Gly Asp Val Val
225                 230                 235                 240

Gly Thr Gly Ser Ser Arg Lys Ser Ala Thr Asn Ser Val Leu Trp Phe
                245                 250                 255

Met Gly Asp Asp Ile Pro His Val Pro Asn Lys Arg Gly Gly Gly Leu
            260                 265                 270

Cys Leu Gly Gly Lys Ile Ala Pro Ile Phe Phe Asn Thr Met Glu Asp
        275                 280                 285

Ala Gly Ala Leu Pro Ile Glu Val Asp Val Ser Asn Leu Asn Met Gly
    290                 295                 300

Asp Val Ile Asp Val Tyr Pro Tyr Lys Gly Glu Val Arg Asn His Glu
305                 310                 315                 320

Thr Gly Glu Leu Leu Ala Thr Phe Glu Leu Lys Thr Asp Val Leu Ile
                325                 330                 335

Asp Glu Val Arg Ala Gly Gly Arg Ile Pro Leu Ile Ile Gly Arg Gly
            340                 345                 350

Leu Thr Thr Lys Ala Arg Glu Ala Leu Gly Leu Pro His Ser Asp Val
        355                 360                 365

Phe Arg Gln Ala Lys Asp Val Ala Glu Ser Asp Arg Gly Phe Ser Leu
    370                 375                 380

Ala Gln Lys Met Val Gly Arg Ala Cys Gly Val Lys Gly Ile Arg Pro
385                 390                 395                 400

Gly Ala Tyr Cys Glu Pro Lys Met Thr Ser Val Gly Ser Gln Asp Thr
                405                 410                 415

Thr Gly Pro Met Thr Arg Asp Glu Leu Lys Asp Leu Ala Cys Leu Gly
            420                 425                 430

Phe Ser Ala Asp Leu Val Met Gln Ser Phe Cys His Thr Ala Ala Tyr
        435                 440                 445

Pro Lys Pro Val Asp Val Asn Thr His His Thr Leu Pro Asp Phe Ile
    450                 455                 460

Met Asn Arg Gly Gly Val Ser Leu Arg Pro Gly Asp Gly Val Ile His
465                 470                 475                 480
```

Ser Trp Leu Asn Arg Met Leu Leu Pro Asp Thr Val Gly Thr Gly Gly
485 490 495

Asp Ser His Thr Arg Phe Pro Ile Gly Ile Ser Phe Pro Ala Gly Ser
500 505 510

Gly Leu Val Ala Phe Ala Ala Ala Thr Gly Val Met Pro Leu Asp Met
515 520 525

Pro Glu Ser Val Leu Val Arg Phe Lys Gly Lys Met Gln Pro Gly Ile
530 535 540

Thr Leu Arg Asp Leu Val His Ala Ile Pro Leu Tyr Ala Ile Lys Gln
545 550 555 560

Gly Leu Leu Thr Val Glu Lys Lys Gly Lys Lys Asn Ile Phe Ser Gly
565 570 575

Arg Ile Leu Glu Ile Glu Gly Leu Pro Asp Leu Lys Val Glu Gln Ala
580 585 590

Phe Glu Leu Thr Asp Ala Ser Ala Glu Arg Ser Ala Ala Gly Cys Thr
595 600 605

Ile Lys Leu Asn Lys Glu Pro Ile Ile Glu Tyr Leu Asn Ser Asn Ile
610 615 620

Val Leu Leu Lys Trp Met Ile Ala Glu Gly Tyr Gly Asp Arg Arg Thr
625 630 635 640

Leu Glu Arg Arg Ile Gln Gly Met Glu Lys Trp Leu Ala Asn Pro Glu
645 650 655

Leu Leu Glu Ala Asp Ala Asp Ala Glu Tyr Ala Ala Val Ile Asp Ile
660 665 670

Asp Leu Ala Asp Ile Lys Glu Pro Ile Leu Cys Ala Pro Asn Asp Pro
675 680 685

Asp Asp Ala Arg Pro Leu Ser Ala Val Gln Gly Glu Lys Ile Asp Glu
690 695 700

Val Phe Ile Gly Ser Cys Met Thr Asn Ile Gly His Phe Arg Ala Ala
705 710 715 720

Gly Lys Leu Leu Asp Ala His Lys Gly Gln Leu Pro Thr Arg Leu Trp

```
                    725                 730                 735

Val Ala Pro Pro Thr Arg Met Asp Ala Ala Gln Leu Thr Glu Glu Gly
            740                 745                 750

Tyr Tyr Ser Val Phe Gly Lys Ser Gly Ala Arg Ile Glu Ile Pro Gly
        755                 760                 765

Cys Ser Leu Cys Met Gly Asn Gln Ala Arg Val Ala Asp Gly Ala Thr
    770                 775                 780

Val Val Ser Thr Ser Thr Arg Asn Phe Pro Asn Arg Leu Gly Thr Gly
785                 790                 795                 800

Ala Asn Val Phe Leu Ala Ser Ala Glu Leu Ala Ala Val Ala Ala Leu
                805                 810                 815

Ile Gly Lys Leu Pro Thr Pro Glu Glu Tyr Gln Thr Tyr Val Ala Gln
            820                 825                 830

Val Asp Lys Thr Ala Val Asp Thr Tyr Arg Tyr Leu Asn Phe Asn Gln
        835                 840                 845

Leu Ser Gln Tyr Thr Glu Lys Ala Asp Gly Val Ile Phe Gln Thr Ala
    850                 855                 860

Val
865


<210>  28
<211>  305
<212>  PRT
<213>  Corynebacterium glutamicum

<400>  28

Met Asn Leu Phe Ser Asn Gly Val Asp Val Gly Arg Arg Arg Gln Ala
1               5                   10                  15

Phe Lys Ala Ala Leu Ala Ala Pro His Ile Ala Arg Leu Pro Gly Ala
            20                  25                  30

Phe Ser Pro Leu Ile Ala Arg Ser Ile Glu Glu Ala Gly Phe Glu Gly
        35                  40                  45

Val Tyr Val Ser Gly Ala Val Ile Ala Ala Asp Leu Ala Leu Pro Asp
    50                  55                  60

Ile Gly Leu Thr Thr Leu Thr Glu Val Ala His Arg Ala Arg Gln Ile
```

```
65                  70                  75                  80

Ala Arg Val Thr Asp Leu Gly Val Leu Val Asp Ala Asp Thr Gly Phe
                85                  90                  95

Gly Glu Pro Met Ser Ala Ala Arg Thr Val Ala Glu Leu Glu Asp Ala
            100                 105                 110

Gly Val Ala Gly Cys His Leu Glu Asp Gln Val Asn Pro Lys Arg Cys
        115                 120                 125

Gly His Leu Asp Gly Lys Glu Val Val Arg Thr Asp Val Met Val Arg
    130                 135                 140

Arg Ile Ala Ala Ala Val Ser Ala Arg Arg Asp Pro Asn Phe Val Ile
145                 150                 155                 160

Cys Ala Arg Thr Asp Ala Ala Gly Val Glu Gly Ile Asp Ala Ala Ile
                165                 170                 175

Glu Arg Ala Lys Ala Tyr Leu Asp Ala Gly Ala Asp Met Ile Phe Thr
            180                 185                 190

Glu Ala Leu His Ser Glu Ala Asp Phe Arg Tyr Phe Arg His Ala Ile
        195                 200                 205

Pro Asp Ala Leu Leu Leu Ala Asn Met Thr Glu Phe Gly Lys Thr Thr
    210                 215                 220

Leu Leu Ser Ala Asp Val Leu Glu Glu Ile Gly Tyr Asn Ala Val Ile
225                 230                 235                 240

Tyr Pro Val Thr Thr Leu Arg Ile Ala Met Gly Gln Val Glu Gln Ala
                245                 250                 255

Leu Ala Glu Ile Lys Glu His Gly Thr Gln Glu Gly Trp Leu Asp Arg
            260                 265                 270

Met Gln His Arg Ser Arg Leu Tyr Glu Leu Leu Arg Tyr Glu Asp Tyr
        275                 280                 285

Asn Val Phe Asp Gln His Ile Phe Thr Tyr Arg Lys Gly Glu Asn Asn
    290                 295                 300

Glu
305
```

<210> 29
<211> 865
<212> PRT
<213> Escherichia coli

<400> 29

```
Met Leu Glu Glu Tyr Arg Lys His Val Ala Glu Arg Ala Ala Glu Gly
1               5                   10                  15

Ile Ala Pro Lys Pro Leu Asp Ala Asn Gln Met Ala Ala Leu Val Glu
            20                  25                  30

Leu Leu Lys Asn Pro Pro Ala Gly Glu Glu Glu Phe Leu Leu Asp Leu
        35                  40                  45

Leu Thr Asn Arg Val Pro Pro Gly Val Asp Glu Ala Ala Tyr Val Lys
    50                  55                  60

Ala Gly Phe Leu Ala Ala Ile Ala Lys Gly Glu Ala Lys Ser Pro Leu
65                  70                  75                  80

Leu Thr Pro Glu Lys Ala Ile Glu Leu Leu Gly Thr Met Gln Gly Gly
                85                  90                  95

Tyr Asn Ile His Pro Leu Ile Asp Ala Leu Asp Asp Ala Lys Leu Ala
            100                 105                 110

Pro Ile Ala Ala Lys Ala Leu Ser His Thr Leu Leu Met Phe Asp Asn
        115                 120                 125

Phe Tyr Asp Val Glu Glu Lys Ala Lys Ala Gly Asn Glu Tyr Ala Lys
    130                 135                 140

Gln Val Met Gln Ser Trp Ala Asp Ala Glu Trp Phe Leu Asn Arg Pro
145                 150                 155                 160

Ala Leu Ala Glu Lys Leu Thr Val Thr Val Phe Lys Val Thr Gly Glu
                165                 170                 175

Thr Asn Thr Asp Asp Leu Ser Pro Ala Pro Asp Ala Trp Ser Arg Pro
            180                 185                 190

Asp Ile Pro Leu His Ala Leu Ala Met Leu Lys Asn Ala Arg Glu Gly
        195                 200                 205

Ile Glu Pro Asp Gln Pro Gly Val Val Gly Pro Ile Lys Gln Ile Glu
    210                 215                 220
```

Ala Leu Gln Gln Lys Gly Phe Pro Leu Ala Tyr Val Gly Asp Val Val
225                 230                 235                 240

Gly Thr Gly Ser Ser Arg Lys Ser Ala Thr Asn Ser Val Leu Trp Phe
                245                 250                 255

Met Gly Asp Asp Ile Pro His Val Pro Asn Lys Arg Gly Gly Gly Leu
            260                 265                 270

Cys Leu Gly Gly Lys Ile Ala Pro Ile Phe Phe Asn Thr Met Glu Asp
        275                 280                 285

Ala Gly Ala Leu Pro Ile Glu Val Asp Val Ser Asn Leu Asn Met Gly
    290                 295                 300

Asp Val Ile Asp Val Tyr Pro Tyr Lys Gly Glu Val Arg Asn His Glu
305                 310                 315                 320

Thr Gly Glu Leu Leu Ala Thr Phe Glu Leu Lys Thr Asp Val Leu Ile
                325                 330                 335

Asp Glu Val Arg Ala Gly Gly Arg Ile Pro Leu Ile Ile Gly Arg Gly
            340                 345                 350

Leu Thr Thr Lys Ala Arg Glu Ala Leu Gly Leu Pro His Ser Asp Val
        355                 360                 365

Phe Arg Gln Ala Lys Asp Val Ala Glu Ser Asp Arg Gly Phe Ser Leu
    370                 375                 380

Ala Gln Lys Met Val Gly Arg Ala Cys Gly Val Lys Gly Ile Arg Pro
385                 390                 395                 400

Gly Ala Tyr Cys Glu Pro Lys Met Thr Ser Val Gly Ser Gln Asp Thr
                405                 410                 415

Thr Gly Pro Met Thr Arg Asp Glu Leu Lys Asp Leu Ala Cys Leu Gly
            420                 425                 430

Phe Ser Ala Asp Leu Val Met Gln Ser Phe Cys His Thr Ala Ala Tyr
        435                 440                 445

Pro Lys Pro Val Asp Val Asn Thr His His Thr Leu Pro Asp Phe Ile
    450                 455                 460

Met Asn Arg Gly Gly Val Ser Leu Arg Pro Gly Asp Gly Val Ile His
465                 470                 475                 480

Ser Trp Leu Asn Arg Met Leu Leu Pro Asp Thr Val Gly Thr Gly Gly
485 490 495

Asp Ser His Thr Arg Phe Pro Ile Gly Ile Ser Phe Pro Ala Gly Ser
500 505 510

Gly Leu Val Ala Phe Ala Ala Ala Thr Gly Val Met Pro Leu Asp Met
515 520 525

Pro Glu Ser Val Leu Val Arg Phe Lys Gly Lys Met Gln Pro Gly Ile
530 535 540

Thr Leu Arg Asp Leu Val His Ala Ile Pro Leu Tyr Ala Ile Lys Gln
545 550 555 560

Gly Leu Leu Thr Val Glu Lys Lys Gly Lys Lys Asn Ile Phe Ser Gly
565 570 575

Arg Ile Leu Glu Ile Glu Gly Leu Pro Asp Leu Lys Val Glu Gln Ala
580 585 590

Phe Glu Leu Thr Asp Ala Ser Ala Glu Arg Ser Ala Ala Gly Cys Thr
595 600 605

Ile Lys Leu Asn Lys Glu Pro Ile Ile Glu Tyr Leu Asn Ser Asn Ile
610 615 620

Val Leu Leu Lys Trp Met Ile Ala Glu Gly Tyr Gly Asp Arg Arg Thr
625 630 635 640

Leu Glu Arg Arg Ile Gln Gly Met Glu Lys Trp Leu Ala Asn Pro Glu
645 650 655

Leu Leu Glu Ala Asp Ala Asp Ala Glu Tyr Ala Ala Val Ile Asp Ile
660 665 670

Asp Leu Ala Asp Ile Lys Glu Pro Ile Leu Cys Ala Pro Asn Asp Pro
675 680 685

Asp Asp Ala Arg Pro Leu Ser Ala Val Gln Gly Glu Lys Ile Asp Glu
690 695 700

Val Phe Ile Gly Ser Cys Met Thr Asn Ile Gly His Phe Arg Ala Ala
705 710 715 720

Gly Lys Leu Leu Asp Ala His Lys Gly Gln Leu Pro Thr Arg Leu Trp
725 730 735

Val Ala Pro Pro Thr Arg Met Asp Ala Ala Gln Leu Thr Glu Glu Gly
740 745 750

Tyr Tyr Ser Val Phe Gly Lys Ser Gly Ala Arg Ile Glu Ile Pro Gly
755 760 765

Cys Ser Leu Cys Met Gly Asn Gln Ala Arg Val Ala Asp Gly Ala Thr
770 775 780

Val Val Ser Thr Ser Thr Arg Asn Phe Pro Asn Arg Leu Gly Thr Gly
785 790 795 800

Ala Asn Val Phe Leu Ala Ser Ala Glu Leu Ala Ala Val Ala Ala Leu
805 810 815

Ile Gly Lys Leu Pro Thr Pro Glu Glu Tyr Gln Thr Tyr Val Ala Gln
820 825 830

Val Asp Lys Thr Ala Val Asp Thr Tyr Arg Tyr Leu Asn Phe Asn Gln
835 840 845

Leu Ser Gln Tyr Thr Glu Lys Ala Asp Gly Val Ile Phe Gln Thr Ala
850 855 860

Val
865

<210> 30
<211> 389
<212> PRT
<213> Escherichia coli

<400> 30

Met Ser Asp Thr Thr Ile Leu Gln Asn Ser Thr His Val Ile Lys Pro
1 5 10 15

Lys Lys Ser Val Ala Leu Ser Gly Val Pro Ala Gly Asn Thr Ala Leu
20 25 30

Cys Thr Val Gly Lys Ser Gly Asn Asp Leu His Tyr Arg Gly Tyr Asp
35 40 45

Ile Leu Asp Leu Ala Lys His Cys Glu Phe Glu Glu Val Ala His Leu
50 55 60

Leu Ile His Gly Lys Leu Pro Thr Arg Asp Glu Leu Ala Ala Tyr Lys
65 70 75 80

```
Thr Lys Leu Lys Ala Leu Arg Gly Leu Pro Ala Asn Val Arg Thr Val
                85                  90                  95

Leu Glu Ala Leu Pro Ala Ala Ser His Pro Met Asp Val Met Arg Thr
            100                 105                 110

Gly Val Ser Ala Leu Gly Cys Thr Leu Pro Glu Lys Glu Gly His Thr
        115                 120                 125

Val Ser Gly Ala Arg Asp Ile Ala Asp Lys Leu Leu Ala Ser Leu Ser
    130                 135                 140

Ser Ile Leu Leu Tyr Trp Tyr His Tyr Ser His Asn Gly Glu Arg Ile
145                 150                 155                 160

Gln Pro Glu Thr Asp Asp Asp Ser Ile Gly Gly His Phe Leu His Leu
                165                 170                 175

Leu His Gly Glu Lys Pro Ser Gln Ser Trp Glu Lys Ala Met His Ile
            180                 185                 190

Ser Leu Val Leu Tyr Ala Glu His Glu Phe Asn Ala Ser Thr Phe Thr
        195                 200                 205

Ser Arg Val Ile Ala Gly Thr Gly Ser Asp Met Tyr Ser Ala Ile Ile
    210                 215                 220

Gly Ala Ile Gly Ala Leu Arg Gly Pro Lys His Gly Gly Ala Asn Glu
225                 230                 235                 240

Val Ser Leu Glu Ile Gln Gln Arg Tyr Glu Thr Pro Asp Glu Ala Glu
                245                 250                 255

Ala Asp Ile Arg Lys Arg Val Glu Asn Lys Glu Val Val Ile Gly Phe
            260                 265                 270

Gly His Pro Val Tyr Thr Ile Ala Asp Pro Arg His Gln Val Ile Lys
        275                 280                 285

Arg Val Ala Lys Gln Leu Ser Gln Glu Gly Gly Ser Leu Lys Met Tyr
    290                 295                 300

Asn Ile Ala Asp Arg Leu Glu Thr Val Met Trp Glu Ser Lys Lys Met
305                 310                 315                 320

Phe Pro Asn Leu Asp Trp Phe Ser Ala Val Ser Tyr Asn Met Met Gly
```

325 330 335

Val Pro Thr Glu Met Phe Thr Pro Leu Phe Val Ile Ala Arg Val Thr
340 345 350

Gly Trp Ala Ala His Ile Ile Glu Gln Arg Gln Asp Asn Lys Ile Ile
355 360 365

Arg Pro Ser Ala Asn Tyr Val Gly Pro Glu Asp Arg Pro Phe Val Ala
370 375 380

Leu Asp Lys Arg Gln
385

<210> 31
<211> 461
<212> PRT
<213> Corynebacterium glutamicum

<400> 31

Met Ser Asp Thr Pro Thr Ser Ala Leu Ile Thr Thr Val Asn Arg Ser
1 5 10 15

Phe Asp Gly Phe Asp Leu Glu Glu Val Ala Ala Asp Leu Gly Val Arg
20 25 30

Leu Thr Tyr Leu Pro Asp Glu Glu Leu Glu Val Ser Lys Val Leu Ala
35 40 45

Ala Asp Leu Leu Ala Glu Gly Pro Ala Leu Ile Ile Gly Val Gly Asn
50 55 60

Thr Phe Phe Asp Ala Gln Val Ala Ala Ala Leu Gly Val Pro Val Leu
65 70 75 80

Leu Leu Val Asp Lys Gln Gly Lys His Val Ala Leu Ala Arg Thr Gln
85 90 95

Val Asn Asn Ala Gly Ala Val Val Ala Ala Ala Phe Thr Ala Glu Gln
100 105 110

Glu Pro Met Pro Asp Lys Leu Arg Lys Ala Val Arg Asn His Ser Asn
115 120 125

Leu Glu Pro Val Met Ser Ala Glu Leu Phe Glu Asn Trp Leu Leu Lys
130 135 140

Arg Ala Arg Ala Glu His Ser His Ile Val Leu Pro Glu Gly Asp Asp

145 150 155 160

Asp Arg Ile Leu Met Ala Ala His Gln Leu Leu Asp Gln Asp Ile Cys
165 170 175

Asp Ile Thr Ile Leu Gly Asp Pro Val Lys Ile Lys Glu Arg Ala Thr
180 185 190

Glu Leu Gly Leu His Leu Asn Thr Ala Tyr Leu Val Asn Pro Leu Thr
195 200 205

Asp Pro Arg Leu Glu Glu Phe Ala Glu Gln Phe Ala Glu Leu Arg Lys
210 215 220

Ser Lys Ser Val Thr Ile Asp Glu Ala Arg Glu Ile Met Lys Asp Ile
225 230 235 240

Ser Tyr Phe Gly Thr Met Met Val His Asn Gly Asp Ala Asp Gly Met
245 250 255

Val Ser Gly Ala Ala Asn Thr Thr Ala His Thr Ile Lys Pro Ser Phe
260 265 270

Gln Ile Ile Lys Thr Val Pro Glu Ala Ser Val Val Ser Ser Ile Phe
275 280 285

Leu Met Val Leu Arg Gly Arg Leu Trp Ala Phe Gly Asp Cys Ala Val
290 295 300

Asn Pro Asn Pro Thr Ala Glu Gln Leu Gly Glu Ile Ala Val Val Ser
305 310 315 320

Ala Lys Thr Ala Ala Gln Phe Gly Ile Asp Pro Arg Val Ala Ile Leu
325 330 335

Ser Tyr Ser Thr Gly Asn Ser Gly Gly Gly Ser Asp Val Asp Arg Ala
340 345 350

Ile Asp Ala Leu Ala Glu Ala Arg Arg Leu Asn Pro Glu Leu Cys Val
355 360 365

Asp Gly Pro Leu Gln Phe Asp Ala Ala Val Asp Pro Gly Val Ala Arg
370 375 380

Lys Lys Met Pro Asp Ser Asp Val Ala Gly Gln Ala Asn Val Phe Ile
385 390 395 400

Phe Pro Asp Leu Glu Ala Gly Asn Ile Gly Tyr Lys Thr Ala Gln Arg
405 410 415

Thr Gly His Ala Leu Ala Val Gly Pro Ile Leu Gln Gly Leu Asn Lys
420 425 430

Pro Val Asn Asp Leu Ser Arg Gly Ala Thr Val Pro Asp Ile Val Asn
435 440 445

Thr Val Ala Ile Thr Ala Ile Gln Ala Gly Gly Arg Ser
450 455 460

<210> 32
<211> 628
<212> PRT
<213> Escherichia coli

<400> 32

Met Ser Phe Ser Glu Phe Tyr Gln Arg Ser Ile Asn Glu Pro Glu Gln
1 5 10 15

Phe Trp Ala Glu Gln Ala Arg Arg Ile Asp Trp Gln Thr Pro Phe Thr
20 25 30

Gln Thr Leu Asp His Ser Asn Pro Pro Phe Ala Arg Trp Phe Cys Glu
35 40 45

Gly Arg Thr Asn Leu Cys His Asn Ala Ile Asp Arg Trp Leu Glu Lys
50 55 60

Gln Pro Glu Ala Leu Ala Leu Ile Ala Val Ser Ser Glu Thr Glu Glu
65 70 75 80

Glu Arg Thr Phe Thr Phe Arg Gln Leu His Asp Glu Val Asn Ala Val
85 90 95

Ala Ser Met Leu Arg Ser Leu Gly Val Gln Arg Gly Asp Arg Val Leu
100 105 110

Val Tyr Met Pro Met Ile Ala Glu Ala His Ile Thr Leu Leu Ala Cys
115 120 125

Ala Arg Ile Gly Ala Ile His Ser Val Val Phe Gly Gly Phe Ala Ser
130 135 140

His Ser Val Ala Ala Arg Ile Asp Asp Ala Lys Pro Val Leu Ile Val
145 150 155 160

```
Ser Ala Asp Ala Gly Ala Arg Gly Gly Lys Ile Ile Pro Tyr Lys Lys
                165                 170                 175

Leu Leu Asp Asp Ala Ile Ser Gln Ala Gln His Gln Pro Arg His Val
            180                 185                 190

Leu Leu Val Asp Arg Gly Leu Ala Lys Met Ala Arg Val Ser Gly Arg
        195                 200                 205

Asp Val Asp Phe Ala Ser Leu Arg His Gln His Ile Gly Ala Arg Val
    210                 215                 220

Pro Val Ala Trp Leu Glu Ser Asn Glu Thr Ser Cys Ile Leu Tyr Thr
225                 230                 235                 240

Ser Gly Thr Thr Gly Lys Pro Lys Gly Val Gln Arg Asp Val Gly Gly
                245                 250                 255

Tyr Ala Val Ala Leu Ala Thr Ser Met Asp Thr Ile Phe Gly Gly Lys
            260                 265                 270

Ala Gly Ser Val Phe Phe Cys Ala Ser Asp Ile Gly Trp Val Val Gly
        275                 280                 285

His Ser Tyr Ile Val Tyr Ala Pro Leu Leu Ala Gly Met Ala Thr Ile
    290                 295                 300

Val Tyr Glu Gly Leu Pro Thr Trp Pro Asp Cys Gly Val Trp Trp Thr
305                 310                 315                 320

Ile Val Glu Lys Tyr Gln Val Ser Arg Met Phe Ser Ala Pro Thr Ala
                325                 330                 335

Ile Arg Val Leu Lys Lys Phe Pro Thr Ala Glu Ile Arg Lys His Asp
            340                 345                 350

Leu Ser Ser Leu Glu Val Leu Tyr Leu Ala Gly Glu Pro Leu Asp Glu
        355                 360                 365

Pro Thr Ala Ser Trp Val Ser Asn Thr Leu Asp Val Pro Val Ile Asp
    370                 375                 380

Asn Tyr Trp Gln Thr Glu Ser Gly Trp Pro Ile Met Ala Ile Ala Arg
385                 390                 395                 400

Gly Leu Asp Asp Arg Pro Thr Arg Leu Gly Ser Pro Gly Val Pro Met
                405                 410                 415
```

Tyr Gly Tyr Asn Val Gln Leu Leu Asn Glu Val Thr Gly Glu Pro Cys
420 425 430

Gly Val Asn Glu Lys Gly Met Leu Val Val Glu Gly Pro Leu Pro Pro
435 440 445

Gly Cys Ile Gln Thr Ile Trp Gly Asp Asp Gly Arg Phe Val Lys Thr
450 455 460

Tyr Trp Ser Leu Phe Ser Arg Pro Val Tyr Ala Thr Phe Asp Trp Gly
465 470 475 480

Ile Arg Asp Ala Asp Gly Tyr His Phe Ile Leu Gly Arg Thr Asp Asp
485 490 495

Val Ile Asn Val Ala Gly His Arg Leu Gly Thr Arg Glu Ile Glu Glu
500 505 510

Ser Ile Ser Ser His Pro Gly Val Ala Glu Val Ala Val Val Gly Val
515 520 525

Lys Asp Ala Leu Lys Gly Gln Val Ala Val Ala Phe Val Ile Pro Lys
530 535 540

Glu Ser Asp Ser Leu Glu Asp Arg Asp Val Ala His Ser Gln Glu Lys
545 550 555 560

Ala Ile Met Ala Leu Val Asp Ser Gln Ile Gly Asn Phe Gly Arg Pro
565 570 575

Ala His Val Trp Phe Val Ser Gln Leu Pro Lys Thr Arg Ser Gly Lys
580 585 590

Met Leu Arg Arg Thr Ile Gln Ala Ile Cys Glu Gly Arg Asp Pro Gly
595 600 605

Asp Leu Thr Thr Ile Asp Asp Pro Ala Ser Leu Asp Gln Ile Arg Gln
610 615 620

Ala Met Glu Glu
625

<210> 33
<211> 1350
<212> DNA
<213> Escherichia coli

<400> 33

atgtctgaaa ttgttgtctc caaatttggc ggtaccagcg tagctgattt tgacgccatg 60

aaccgcagcg ctgatattgt gctttctgat gccaacgtgc gtttagttgt cctctcggct 120

tctgctggta tcactaatct gctggtcgct ttagctgaag gactggaacc tggcgagcga 180

ttcgaaaaac tcgacgctat ccgcaacatc cagtttgcca ttctggaacg tctgcgttac 240

ccgaacgtta tccgtgaaga gattgaacgt ctgctggaga acattactgt tctggcagaa 300

gcggcggcgc tggcaacgtc tccggcgctg acagatgagc tggtcagcca cggcgagctg 360

atgtcgaccc tgctgtttgt tgagatcctg cgcgaacgcg atgttcaggc acagtggttt 420

gatgtacgta aagtgatgcg taccaacgac cgatttggtc gtgcagagcc agatatagcc 480

gcgctggcgg aactggccgc gctgcagctg ctcccacgtc tcaatgaagg cttagtgatc 540

acccagggat ttatcggtag cgaaaataaa ggtcgtacaa cgacgcttgg ccgtggaggc 600

agcgattata cggcagcctt gctggcggag gctttacacg catctcgtgt tgatatctgg 660

accgacgtcc cgggcatcta caccaccgat ccacgcgtag tttccgcagc aaaacgcatt 720

gatgaaatcg cgtttgccga agcggcagag atggcaactt ttggtgcaaa agtactgcat 780

ccggcaacgt tgctacccgc agtacgcagc gatatcccgg tctttgtcgg ctccagcaaa 840

gacccacgcg caggtggtac gctggtgtgc aataaaactg aaaatccgcc gctgttccgc 900

gctctggcgc ttcgtcgcaa tcagactctg ctcactttgc acagcctgaa tatgctgcat 960

tctcgcggtt tcctcgcgga agttttcggc atcctcgcgc ggcataatat ttcggtagac 1020

ttaatcacca cgtcagaagt gagcgtggca ttaatccttg ataccaccgg ttcaacctcc 1080

actggcgata cgttgctgac gcaatctctg ctgatggagc tttccgcact gtgtcgggtg 1140

gaggtggaag aaggtctggc gctggtcgcg ttgattggca atgacctgtc aaaagcctgc 1200

ggcgttggca aagaggtatt cggcgtactg gaaccgttca acattcgcat gatttgttat 1260

ggcgcatcca gccataacct gtgcttcctg gtgcccggcg aagatgccga gcaggtggtg 1320

caaaaactgc atagtaattt gtttgagtaa 1350

<210> 34
<211> 2466
<212> DNA
<213> Escherichia coli

<400> 34
atgcgagtgt tgaagttcgg cggtacatca gtggcaaatg cagaacgttt tctgcgtgtt 60

gccgatattc tggaaagcaa tgccaggcag gggcaggtgg ccaccgtcct ctctgccccc 120

gccaaaatca ccaaccacct ggtggcgatg attgaaaaaa ccattagcgg ccaggatgct 180

ttacccaata tcagcgatgc cgaacgtatt tttgccgaac ttttgacggg actcgccgcc 240

gcccagccgg ggttcccgct ggcgcaattg aaaactttcg tcgatcagga atttgcccaa 300

```
ataaaacatg tcctgcatgg cattagtttg ttggggcagt gcccggatag catcaacgct      360
gcgctgattt gccgtggcga gaaaatgtcg atcgccatta tggccggcgt attagaagcg      420
cgcggtcaca acgttactgt tatcgatccg gtcgaaaaac tgctggcagt ggggcattac      480
ctcgaatcta ccgtcgatat tgctgagtcc acccgccgta ttgcggcaag ccgcattccg      540
gctgatcaca tggtgctgat ggcaggtttc accgccggta atgaaaaagg cgaactggtg      600
gtgcttggac gcaacggttc cgactactct gctgcggtgc tggctgcctg tttacgcgcc      660
gattgttgcg agatttggac ggacgttgac ggggtctata cctgcgaccc gcgtcaggtg      720
cccgatgcga ggttgttgaa gtcgatgtcc taccaggaag cgatggagct ttcctacttc      780
ggcgctaaag ttcttcaccc ccgcaccatt acccccatcg cccagttcca gatcccttgc      840
ctgattaaaa ataccggaaa tcctcaagca ccaggtacgc tcattggtgc cagccgtgat      900
gaagacgaat taccggtcaa gggcatttcc aatctgaata acatggcaat gttcagcgtt      960
tctggtccgg ggatgaaagg gatggtcggc atggcggcgc gcgtctttgc agcgatgtca     1020
cgcgcccgta ttttcgtggt gctgattacg caatcatctt ccgaatacag catcagtttc     1080
tgcgttccac aaagcgactg tgtgcgagct gaacgggcaa tgcaggaaga gttctacctg     1140
gaactgaaag aaggcttact ggagccgctg gcagtgacgg aacggctggc cattatctcg     1200
gtggtaggtg atggtatgcg caccttgcgt gggatctcgg cgaaattctt tgccgcactg     1260
gcccgcgcca atatcaacat tgtcgccatt gctcagggat cttctgaacg ctcaatctct     1320
gtcgtggtaa ataacgatga tgcgaccact ggcgtgcgcg ttactcatca gatgctgttc     1380
aataccgatc aggttatcga agtgtttgtg attggcgtcg gtggcgttgg cggtgcgctg     1440
ctggagcaac tgaagcgtca gcaaagctgg ctgaagaata aacatatcga cttacgtgtc     1500
tgcggtgttg ccaactcgaa ggctctgctc accaatgtac atggccttaa tctggaaaac     1560
tggcaggaag aactggcgca agccaaagag ccgtttaatc tcgggcgctt aattcgcctc     1620
gtgaaagaat atcatctgct gaacccggtc attgttgact gcacttccag ccaggcagtg     1680
gcggatcaat atgccgactt cctgcgcgaa ggtttccacg ttgtcacgcc gaacaaaaag     1740
gccaacacct cgtcgatgga ttactaccat cagttgcgtt atgcggcgga aaaatcgcgg     1800
cgtaaattcc tctatgacac caacgttggg gctggattac cggttattga gaacctgcaa     1860
aatctgctca atgcaggtga tgaattgatg aagttctccg gcattctttc tggttcgctt     1920
tcttatatct tcggcaagtt agacgaaggc atgagtttct ccgaggcgac cacgctggcg     1980
cgggaaatgg gttataccga accggacccg cgagatgatc tttctggtat ggatgtggcg     2040
cgtaaactat tgattctcgc tcgtgaaacg ggacgtgaac tggagctggc ggatattgaa     2100
attgaacctg tgctgcccgc agagtttaac gccgagggtg atgttgccgc ttttatggcg     2160
aatctgtcac aactcgacga tctctttgcc gcgcgcgtgg cgaaggcccg tgatgaagga     2220
```

aaagttttgc gctatgttgg caatattgat gaagatggcg tctgccgcgt gaagattgcc 2280

gaagtggatg gtaatgatcc gctgttcaaa gtgaaaaatg gcgaaaacgc cctggccttc 2340

tatagccact attatcagcc gctgccgttg gtactgcgcg gatatggtgc gggcaatgac 2400

gttacagctg ccggtgtctt tgctgatctg ctacgtaccc tctcatggaa gttaggagtc 2460

tgataa 2466

<210> 35
<211> 7849
<212> DNA
<213> Thauera butanivorans

<400> 35
atgtcagcaa atatggcagt caaacaggcc ttgaaggcca atccggtccc gagttccgtg 60

gatcctcagg aagtccacaa atggcttcag gatttcactt gggatttcaa gggcaagacc 120

gcgaagtatc cgaccaagta tgagatggac gtcaatacgc gcgagcagtt caagctgact 180

gccaaggagt acgcgcgcat ggagtcgatc aaggaagagc gccagtacgg caccctgctc 240

gatggtctcg accgtctgga tgcgggcaac aaggtgcatc cgaaatgggg cgaggtgatg 300

aagctggtct ccaacttcct cgagaccggc gaatacggcg caatcgccgg ttctgctctg 360

ctgtgggaca cggcccagtc accggagcag cgcaacggtt acctcgctca ggtgatcgac 420

gaaatccgcc atgtgaacca gaccgcgtac gtgaattact actacggcaa gcactactac 480

gaccccgccg gccacaccaa catgcgccag cttcgggcga tcaacccccct gtaccccggt 540

gtcaagcgcg ccttcgggga gggctttctc gcgggcgatg ccgtcgagtc ctccatcaac 600

ctccaattgg tcggcgaagc ctgcttcacc aatccgctca tcgtttcgct taccgaatgg 660

gcggccgcga acggcgatga aatcacgccg accgtgttcc tgtcgatcga aaccgatgaa 720

ctgcgccata tggccaacgg ctatcagacc atcgtgtcga tcatgaacaa tccggagacg 780

atgaagtacc tgcaaacgga cctcgataac gcattctgga cgcagcacaa gttcctgacc 840

cccttcgtcg gggtggcgct cgaatatggt tccaagtaca aggtcgagcc gtgggccaag 900

tcctggaacc ggtgggtgta cgaagactgg gctggcatct ggctgggccg actgcagcag 960

ttcggcgtca aaacgccaaa gtgcctgccc gacgccaaga aagacgccgt ttgggcacac 1020

cacgatctcg cgctgctggc ccttgccctg tggccgctga ccggcatccg catggaactc 1080

ccggatagcc tggcgatgga gtggttcgag gctaattacc ccggttggta caaccattac 1140

ggcaagatct acgaggagtg gcgcgctgcc ggcttcgagg atccgaagag cggcttctgt 1200

ggtgcgctct ggctgatgga gcgtggtcac ggcattttcg tcgaccatgc ctcgggtttg 1260

ccgttctgcc ccagcctcgc caaaagctct atcaagccgc ggttcactga atacaacggc 1320

aagcgctacg ccttcgccga gccgtatggc gagcgccagt ggctgctcga gcccgagcgc 1380

tacgagttcc agaacttctt cgagcagttc gaaggctggg aactctccga tctcgtcaag 1440

gcggcaggcg gcgtgcgcag cgatggcaaa acgctgatcg cgcagccgca tcttcgcgac 1500

accgacatgt ggacgcttga cgatctgaag cggatcaacc tgacgatccc cgacccgatg 1560

aagatcctca actggcaacc cgtcgcccag tgagggttgg gccggcgcac gccgccaggc 1620

gagagatgcg ccggcgggga agacttgccg cccgcctacg agcgggcgga cgtgccaacg 1680

aacgaacagc actatatgga gtagataaat atgtcaacaa acatctttac gcgcgggatg 1740

gtagacccgg agcgccaagc ctgcattcag gaggtcgtac ccaaggcgcc gctggaaacc 1800

aagcgtgacc atattccttt cgccaaacgc ggctggcgcc gactcaccga gtatgaagcg 1860

gtgatgttgc atgcgcagaa ttcactggac gccgtcccgg gcagccagga ggtgggtgaa 1920

gtcgtacaga agtggccggg tggcaggccg aactacggcg tcgagtcgac cgcggccctc 1980

tccagcaact ggttccattt ccgcgacccg tcgaagcgct ggttcatgcc ctacgtgaag 2040

cagaagaacg aggaagggca gacagccgaa cgcgcgatga agagttgggc cgagggcggc 2100

gacgcggaaa tgatgaacgc cgcgtggcgg gagcacatcc tggcccggca ctacggcgcg 2160

ttcgtctata acgagtacgg tctgttcagc gcccattcaa caacagttta cggtggcctt 2220

tctgatctga tcaaaacctg gattgccgag gccgcgttcg acaagaacga cgccggtcag 2280

atgatccaga tgcagcgcgt gctgttgagc aaggtgttcc ctggcttcga cgccgacctg 2340

gccgaagcca agcaggcatg gactgaggac aagtcatgga aacccgcccg cgagttcgtc 2400

gagcacatct gggccgagac ctacgactgg gtcgagcagc tttgggcgat ccacgccgtc 2460

tacgaccata ttttcgggca atttgtccgg cgcgaattct tccagcggct cggcggcatc 2520

catggagaca cgctgacgcc cttcattcag aaccaggcgc tgacgtatca cctgcaagcc 2580

cgcgacggcg taacggcact gtgcttcaaa tttctgatcg aagacgagcc ggtatacgcc 2640

caacacaaca ggcgctacct gcgggcatgg acagggcgct atctcccgca agtgggccgc 2700

gcattgaagg ccttcctggc aatctacaag gaggttccgg taaagatcga tggggtgact 2760

tgccgggagg gcgtgcgcgc cagtgtcgaa cgcgtggtgg acgactgggc ggcgcggttc 2820

gctgaaccga tcaacttcaa gttcaaccgc gcggcgttca tcgacgatgt gctgagtggc 2880

tactaattca ggggcagacc gaatgtcaaa cgtaaatgca taccacgctg gcaccaatgg 2940

taaagaaggc caggacttca tcgatgactt tttgagcgaa gagaacagtg ctctacccac 3000

gagcgaagcc gtggttctgg ccctgatgaa gaccgaagag atcgacgcgg tcgtcgacga 3060

gatgatcaag ccgcagatgg aggacaaccc gaccatcgcc gtcgaggacc gcggtggata 3120

ctggtggatc aaggccaacg gaaagatcgt catcgattgt gacgaagcca ccgaactgct 3180

gggcaagaag tatacggtgt acgatttgct ggtcaacgtc agcaccaccg ttgggcgggc 3240

gatgaccctc ggcaatcagt tcatcatcac caacgagctc cttggtctcg aaaccaaggt 3300
cgaaagcgtt tactgaggag gatagacgac atgtcgaaac aggtttggta caacacaccg 3360
gtgcgcgacg agtggatcga gaagatcact gcgatcagga cagcgcgcga aggcaccgac 3420
atgctcgccc gcttccgcgc ggagcatacg gggccggatc gcacgacgta cgatctcaag 3480
aaggaataca attggatcga gtcgcgcatc gagatgcggg tcagccagct tcatgccgag 3540
gccacggcct cggacgaaga cctcctcacc aagacgatcg acggccgctg cgcaaaggaa 3600
gtcgcagcgg agtggctgaa aaaggctgcg gacatcgact gtcactacga gatggagcgg 3660
ctttgtgtcg ccttccgcaa ggcgtgcaaa ccgccgatga tgcccatcaa cttcttcgcg 3720
ccggcagaga aggagttggt ggcgaagctc atgaagctga gggcgcccac gtatctgact 3780
acctccctcg acgagttgcg cgaggcacgg ggtgtaacga tgatttccgt gcagtaagcg 3840
gccggacgtt ctgagccgga tcctccccga caacggggag gactggtcaa aatagccatc 3900
aagactgaaa ggagcaggga atgccgaaat acatcatcga acgttcgatt ccgggggcag 3960
ggaagctgac ggagcgggac ctcgcgtcgg tgtcgcagaa atcctgtgga atcctccgct 4020
ccatggggcc acaaatccaa tgggtcaaaa gctacgtcac cgacgacaag ctctactgca 4080
tctatatcgc tcccgatgag gcctccatcc gcgagcatgc aagatcggga gactttcccg 4140
ccgacaaggt gtcccgcata catcggatga tcgacccgac ttgcgcggaa tcggcataat 4200
cgagccgtcc tcggaggacc acgacttccc gagggcctcc ggacgatgga tcgcggcgga 4260
cggaaacctg cgtggccgcg gacgcggggt ggccacaaga tcgtaacgta aagtactcta 4320
atccgagagc gcggtcgctt tcacccgatc gaggtggaag tgactgggcg aggggggagat 4380
atgaaagaag cgccggcaat acccgatctg cccggtttgc ccgagactgt cggcgaaccg 4440
acgctggtcc tggaagaaga tggcttccgg gttttcgcca cagagctgac gatcatgtgg 4500
cgatgggaca tctacaacgg cgatgcgcac gttcataccg gatgcgccca gcacccggag 4560
agctgcgtcg tcgcggcgag atccaaaatt cgattcttac ggcgccccac tgtggccatg 4620
ctactggcgg gtgaaggtca atgagagggg gttcgagacg ccaactgatg gacgcgacac 4680
cctagtagga gactgaaggt atatgctgat gcaacaatat aaaatagtcg cccgcttcga 4740
agacggcgtg acatacgagt acgattgcgg cgaggacgaa aacctgctcg ccgcggcgtt 4800
gcgccagaac gtccgtttgc tgtgtcaatg cagaaaggcg ttttgcggca gttgcaaggc 4860
cttgtgcagc gagggcgact atgaactggg cgatcatatc aacgttcagg tcttgccgcc 4920
tgacgaagaa gaggacgggg tggtggtcac ctgcgatacc ttcccacgca gcgatctggt 4980
ccttgagttt ccgtatacca gcgaccgtct cggtaccgtt acggctaccg aggcgaagac 5040
gagcgtcgtc agcgtggaga gactctctag caccgtttac cgtctggtgc tgcaggcact 5100
cgacgccgaa ggaatgcctg cacggttcga cttcgttccc gggcagtatg tcgaaatcag 5160

tacagccgat tcgctcgaga ccagggcgtt ttcgctggcg aaccttccaa acgacgccgg 5220
attgctcgag ttcttgatcc gactcgtccc cggcggatac tatgcggcct atctggagca 5280
acgcgcagcg gcagggcaga cgatcaacgt gaagggaccg ttcggcgagt tcgtgctgcg 5340
tgaacacgag ttggtggagg acttcacgct gccagcggac agcccagcga gaggtgggac 5400
gattgcgttc ctggcgggta gcacggggct ggcgcctctc gcgagcatgc tgcgcgagct 5460
cgggcggcga ggattcaacg gcgagtgtca cctcttcttc ggcatgcagg acaccgcaac 5520
gatgttctac gagaaggaac tccgggacat caagcgaacg cttccggggc tcacgctgca 5580
tctcgccctg atggtccctt ctgccgaatg ggaaggctac cgtggtaacg ccgtagctgc 5640
gttcaaagaa catttcgccg ccagtagcca gatacccgag aacgtttacc tgtgcgggcc 5700
cggaccgatg atcgcggccg cgctcggcgc ttgtcgcgag cttggaattc ccgacaatag 5760
agttcacagg gaagaattcg tagcgagcgg cggttgaacg gcgcttgaag aaaccggggc 5820
gcgacgggca gcgtttacga gaccatgagt caagaagaaa gagattttct ggccggtgca 5880
gcggcaacac tcggcgagag cgtcggacaa ctggagcgcc ttgcccaaga attaagtgaa 5940
aagctgggcg atgagcgtgt caacattgtt cggggtttgc tcgaacaggt ttatgaccgc 6000
gacgacgaag cggaagtgcg tgcatccctc tgctatacgg agagaaagct gttgtgggcg 6060
tgggtccgtt tgaagagact gaagggactc aggctcagta ttggtcgtgg ttcgatgaga 6120
aacattatat gagaagcaga acgtgatcag tttaaattgc aagaagacaa ccacagggtt 6180
aactgcgcac ttggccctgg tccgcggaat gaaggcgttg gcggaattgg ttggaacaac 6240
gctggggccc cagggccgcc acgtcatgct tgcgcatcgg gccggattgg ctccgcacgt 6300
cagcaaggac ggcgtcgagg tcgcacgtca cctgtcgttg ccggacagcg aggaagaact 6360
tggtgtccgg ttgctgcgca acgcagccgt cgcagtgtcc gagtcctttg gcgacggcac 6420
ctccactgca accgttttta cggcggatct ggccgtgcgg gcgctcaaac tgatcggtgc 6480
cggggcggat accctggagg tccgtcgagg tctgggtttg gctgcctatg ccgcgttggt 6540
cgccctgaac gacatggccc ggcgcgccga ccggggaatg ctcaccgccg ttgcccaaac 6600
cgctgccaac ggcgaccgtc gcgtggcgga tctcctcgtg gaggccttcg aaagggtcgg 6660
ggctgaaggc acgatcgaag tagaaatggg taattcggtc gaggacgtcc ttgaagtcgc 6720
gcagggcagt tatttcgaca cggtgccgct cgtcacggcc ttgttgccgc caacagggca 6780
ggtcgagttc gcccggccac ttatcctttt ccattgcgac gcgatcgaga cggcggacga 6840
gattcttccg gcgctggagc tagctcgcag ttcacggaga cccttgctga ttctggccga 6900
ctcggtgggt atcgacgtcg agacgctgct cgtccggaat caaaatgaag gcaccttggc 6960
tgttgcggtc gtcagggcgc caatgtatgg cgatacgcgt cgcgaggccc tgcttgacct 7020

gacgagcaaa ttcgggggga cggcgtttgg aagggaggga ttcgtcgaat tcgcactcag 7080

gtctttggga tcactgtcgg aaggtgactt ggggcaggcg gacgaagcaa tcctagaggc 7140

ggatggtgtg actctgagag gcgccggaaa caatccgtct gcattggaag accgaatcgc 7200

gctggtgcgt gctgaactcg atcgcggcga cgtttccgtg ggcgactccc catcggcaaa 7260

gctcgactac atcgagaagc gaaaggagcg gctgaagttg ctggctgcag gtagcgccaa 7320

gctgcatatc ggggggccga cggacgttga gatcaagacg cgtttgccgc tcgccgagaa 7380

cgctcatcgg gcgctcttgg cggccgcgaa gtcgggagtg ctgcccgggg gcggcgtcgc 7440

aatgattcgt gcggctgaaa aggtgcagca agagatgggg cgacttgaag gcgacgttgc 7500

ttctggggca tccattttcc tgcagtcgct tgacacaccc atccggtgga ttgcacgaaa 7560

tgcggggctg cgcccggatg aagtgctcgc tcgaaccctg gcgaacgagt cggactttta 7620

cggactcaac gccatgactg gccgatatgg cgacttggct gaagacgggg ttctcgacgc 7680

cctcgatatg gtcaccgacg tgatacgcgt cgcagtcagc gtcgtcgggt cgatgttggg 7740

cgtcggcgcc ctggtgactc gcgcatcgcc caagcccgcg ccagagcgct tcaagggcac 7800

ggagcgggta cacgacaagt tgatgcgcga gggcgggttc gatgaatga 7849

<210> 36
<211> 1038
<212> DNA
<213> Corynebacterium glutamicum

<400> 36
atgaccactg ctgcacccca agaatttacc gctgctgttg ttgaaaaatt cggtcatgac 60

gtgaccgtga aggatattga ccttccaaag ccagggccac accaggcatt ggtgaaggta 120

ctcacctccg gcatctgcca caccgacctc cacgccttgg agggcgattg gccagtaaag 180

ccggaaccac cattcgtacc aggacacgaa ggtgtaggtg aagttgttga gctcggacca 240

ggtgaacacg atgtgaaggt cggcgatatt gtcggcaatg cgtggctctg gtcagcgtgt 300

ggcacctgcg aatactgcat caccggcagg gaaactcagt gcaacgaagc tgagtatggt 360

ggctacaccc aaaatggatc cttcggccag tacatgctgg tggatacccg ttacgccgct 420

cgcatcccag acggcgtgga ctacctcgaa gcagcaccaa ttctgtgtgc aggcgtgact 480

gtctacaagg cactcaaagt ctctgaaacc cgcccgggcc aattcatggt gatctccggt 540

gtcggcggac ttggccacat cgcagtccaa tacgcagcgg cgatgggcat gcgtgtcatt 600

gcggtagata ttgccgatga caagctggaa cttgcccgta agcacggtgc ggaatttacc 660

gtgaatgcgc gtaatgaaga ttcaggcgaa gctgtacaga agtacaccaa cggtggcgca 720

cacggcgtgc ttgtgactgc agttcacgag gcagcattcg gccaggcact ggatatggct 780

cgacgtgcag gaacaattgt gttcaacggt ctgccaccgg gagagttccc agcatccgtg 840

ttcaacatcg tattcaaggg cctgaccatc cgtggatccc tcgtgggaac ccgccaagac 900

ttggccgaag cgctcgattt ctttgcacgc ggactaatca agccaaccgt gagtgagtgc 960

tccctcgatg aggtcaatgg tgtgcttgac cgcatgcgaa acggcaagat cgatggtcgt 1020

gtggcgattc gtttctaa 1038

<210> 37
<211> 1521
<212> DNA
<213> Corynebacterium glutamicum

<400> 37
atgactgtct acgcaaatcc aggaaccgaa ggctcgatcg ttaactatga aaagcgctac 60

gagaactaca ttggtggcaa gtgggttcca ccggtagagg gccagtacct tgagaacatt 120

tcacctgtca ctggtgaagt tttctgtgag gtcgcacgtg gcaccgcagc ggacgtggag 180

cttgcactgg atgctgcaca tgcagccgct gatgcgtggg gcaagacttc tgtcgctgaa 240

cgtgctctga tcctgcaccg cattgcggac cgcatggaag agcacctgga agaaatcgca 300

gttgcagaaa cctgggagaa cggcaaggca gtccgtgaga ctcttgctgc agatatccca 360

ctggcaatcg accacttccg ctactttgct ggcgcgatcc gtgctcagga agatcgttcc 420

tcacagatcg accacaacac tgttgcttac cacttcaacg agccaatcgg tgttgttggt 480

cagatcattc cttggaactt cccaatcctc atggctacct ggaagctcgc accggcactt 540

gctgcaggta acgcgatcgt catgaagcca gctgagcaga ccccagcatc cattttgtat 600

ctgattaaca tcatcggcga tctcatccca gagggcgtcc tcaacatcgt caacggactc 660

ggcggtgaag caggcgctgc actgtccggc tctaatcgga ttggcaagat tgctttcacc 720

ggttccaccg aggtcggcaa gctgatcaac cgcgctgcat ccgacaagat cattcctgtc 780

accctggagc tcggcggtaa gtccccatcc atcttcttct ccgatgttct gtcacaggat 840

gacgccttcg cagagaaggc agttgaaggc ttcgcgatgt tcgccctcaa tcagggtgaa 900

gtttgtacct gtccttcccg tgcacttgtt catgagtcca tcgctgatga attcctcgag 960

cttggcgtga agcgagttca gaacatcaag ctgggtaacc cacttgatac tgaaaccatg 1020

atgggtgctc aggcgtccca ggagcagatg gacaagatct cctcctacct gaagatcggc 1080

ccagaagaag gcgctcaaac cctcactggt ggcaaggtca acaaggttga tggcatggag 1140

aacggttact acattgagcc aaccgttttc cgcggcacca acgacatgag gatcttccgc 1200

gaggaaatct tcggaccagt cctttctgtt gctaccttca gcgacttcga tgaggccatc 1260

cgtattgcaa acgacaccaa ctacggcctc ggcgctggtg tctggagccg tgaccaaaac 1320

accatttatc gtgcaggtcg cgcaatccag gctggtcgag tttgggtcaa ccagtaccac 1380

aactacccag cgcactccgc tttcggtgga tacaaggagt ccggcatcgg ccgtgagaac 1440

cacctcatga tgctgaacca ctaccagcag accaagaacc tgttggtctc ctacgatcca 1500

aacccaaccg gactgttctg a 1521

<210> 38
<211> 1134
<212> DNA
<213> Corynebacterium glutamicum

<400> 38
atgcccaccc tcgcgccttc aggtcaactt gaaatccaag cgatcggtga tgtctccacc 60

gaagccggag caatcattac aaacgctgaa atcgcctatc accgctgggg tgaataccgc 120

gtagataaag aaggacgcag caatgtcgtt ctcatcgaac acgccctcac tggagattcc 180

aacgcagccg attggtgggc tgacttgctc ggtcccggca aagccatcaa cactgatatt 240

tactgcgtga tctgtaccaa cgtcatcggt ggttgcaacg gttccaccgg acctggctcc 300

atgcatccag atggaaattt ctggggtaat cgcttccccg ccacgtccat tcgtgatcag 360

gtaaacgccg aaaaacaatt cctcgacgca ctcggcatca ccacggtcgc cgcagtactt 420

ggtggttcca tgggtggtgc ccgcacccta gagtgggccg caatgtaccc agaaactgtt 480

ggcgcagctg ctgttcttgc agtttctgca cgcgccagcg cctggcaaat cggcattcaa 540

tccgcccaaa ttaaggcgat tgaaaacgac caccactggc acgaaggcaa ctactacgaa 600

tccggctgca acccagccac cggactcggc gccgcccgac gcatcgccca cctcacctac 660

cgtggcgaac tagaaatcga cgaacgcttc ggcaccaaag cccaaaagaa cgaaaaccca 720

ctcggtccct accgcaagcc cgaccagcgc ttcgccgtgg aatcctactt ggactaccaa 780

gcagacaagc tagtacagcg tttcgacgcc ggctcctacg tcttgctcac cgacgccctc 840

aaccgccacg acattggtcg cgaccgcgga ggcctcaaca aggcactcga atccatcaaa 900

gttccagtcc ttgtcgcagg cgtagatacc gatattttgt acccctacca ccagcaagaa 960

cacctctcca gaaacctggg aaatctactg gcaatggcaa aaatcgtatc ccctgtcggc 1020

cacgatgctt tcctcaccga aagccgccaa atggatcgca tcgtgaggaa cttcttcagc 1080

ctcatctccc cagacgaaga caacccttcg acctacatcg agttctacat ctaa 1134

<210> 39
<211> 6686
<212> DNA
<213> Artificial Sequence

<220>
<223> vector

<400> 39
cctttcgtct tcgaataaat acctgtgacg gaagatcact tcgcagaata aataaatcct 60

ggtgtccctg ttgataccgg gaagccctgg gccaactttt ggcgaaaatg agacgttgat 120

cggcacgtaa gaggttccaa ctttcaccat aatgaaataa gatcactacc gggcgtattt 180
tttgagttat cgagattttc aggagctaag gaagctaaaa tggagaaaaa aatcactgga 240
tataccaccg ttgatatatc ccaatggcat cgtaaagaac attttgaggc atttcagtca 300
gttgctcaat gtacctataa ccagaccgtt cagctggata ttacggcctt tttaaagacc 360
gtaaagaaaa ataagcacaa gttttatccg gcctttattc acattcttgc ccgcctgatg 420
aatgctcatc cggaattccg tatggcaatg aaagacggtg agctggtgat atgggatagt 480
gttcaccctt gttacaccgt tttccatgag caaactgaaa cgttttcatc gctctggagt 540
gaataccacg acgatttccg gcagtttcta cacatatatt cgcaagatgt ggcgtgttac 600
ggtgaaaacc tggcctattt ccctaaaggg tttattgaga atatgttttt cgtctcagcc 660
aatccctggg tgagtttcac cagttttgat ttaaacgtgg ccaatatgga caacttcttc 720
gcccccgttt tcaccatggg caaatattat acgcaaggcg acaaggtgct gatgccgctg 780
gcgattcagg ttcatcatgc cgtttgtgat ggcttccatg tcggcagaat gcttaatgaa 840
ttacaacagt actgcgatga gtggcagggc ggggcgtaat ttttttaagg cagttattgg 900
tgcccttaaa cgcctggttg ctacgcctga ataagtgata ataagcggat gaatggcaga 960
aattcgaaag caaattcgac ccggtcgtcg gttcagggca gggtcgttaa atagccgctt 1020
atgtctattg ctggtctcgg tacccgggga tcgccattcc ggctgatcac atggtgctga 1080
tggcaggttt caccgccggt aatgaaaaag gcgaactggt ggtgcttgga cgcaacggtt 1140
ccgactactc tgctgcggtg ctggctgcct gtttacgcgc cgattgttgc gagatttgga 1200
cggacgttga cggggtctat acctgcgacc cgcgtcaggt gcccgatgcg aggttgttga 1260
agtcgatgtc ctaccaggaa gcgatggagc tttcctactt cggcgctaaa gttcttcacc 1320
cccgcaccat taccccatc gcccagttcc agatcccttg cctgattaaa aataccggaa 1380
atcctcaagc accaggtacg ctcattggtg ccagccgtga tgaagacgaa ttaccggtca 1440
agggcatttc caatctgaat aacatggcaa tgttcagcgt ttctggtccg ggatgaaag 1500
ggatggtcgg catggcggcg cgcgtctttg cagcgatgtc acgcgcccgt attttcgtgg 1560
tgctgattac gcaatcatct tccgaataca gcatcagttt ctgcgttcca caaagcgact 1620
gtgtgcgagc tgaacggcca atgcaggaag agttctacct ggaactgaaa gaaggcttac 1680
tggagccgct ggcagtgacg gaacggctgg ccattatctc ggtggtaggt gatggtatgc 1740
gcaccttgcg tgggatctcg gcgaaattct ttgccgcact ggcccgcgcc aatatcaaca 1800
ttgtcgccat tgctcaggga tcttctgaac gctcaatctc tgtcgtggta aataacgatg 1860
atgcgaccac tggcgtgcgc gttactcatc agatgctgtt caataccgat caggttatcg 1920
aagtgtttgt gattggcgtc ggtggcgttg gcggtgcgct gctggagcaa ctgaagcgtc 1980
agcaaagctg gctgaagaat aaacatatcg acttacgtgt ctgcggtgtt gccaactcga 2040

aggctctgct caccaatgta catggcctta atctggaaaa ctggcaggaa gaactggcgc 2100

aagcgatcct ctagagtcga ccggtggcga atgggacgcg ccctgtagcg gcgcattaag 2160

cgcggcgggt gtggtggtta cgcgcagcgt gaccgctaca cttgccagcg ccctagcgcc 2220

cgctcctttc gctttcttcc cttcctttct cgccacgttc gccggctttc cccgtcaagc 2280

tctaaatcgg gggctccctt tagggttccg atttagtgct ttacggcacc tcgaccccaa 2340

aaaacttgat tagggtgatg gttcacgtag tgggccatcg ccctgataga cggtttttcg 2400

ccctttgacg ttggagtcca cgttctttaa tagtggactc ttgttccaaa ctggaacaac 2460

actcaaccct atctcggtct attcttttga tttataaggg attttgccga tttcggccta 2520

ttggttaaaa aatgagctga tttaacaaaa atttaacgcg aattttaaca aaatattaac 2580

gcttacaatt taggtggcac ttttcgggga aatgtgcgcg gaacccctat ttgtttattt 2640

ttctaaatac attcaaatat gtatccgctc accgcgatcc tttttaaccc atcacatata 2700

cctgccgttc actattattt agtgaaatga gatattatga tattttctga attgtgatta 2760

aaaaggcaac tttatgccca tgcaacagaa actataaaaa atacagagaa tgaaaagaaa 2820

cagatagatt ttttagttct ttaggcccgt agtctgcaaa tccttttatg attttctatc 2880

aaacaaaaga ggaaaataga ccagttgcaa tccaaacgag agtctaatag aatgaggtcg 2940

aaaagtaaat cgcgcgggtt tgttactgat aaagcaggca agacctaaaa tgtgtaaagg 3000

gcaaagtgta tactttggcg tcacccctta catattttag gtcttttttt attgtgcgta 3060

actaacttgc catcttcaaa caggagggct ggaagaagca gaccgctaac acagtacata 3120

aaaaaggaga catgaacgat gaacatcaaa aagtttgcaa aacaagcaac agtattaacc 3180

tttactaccg cactgctggc aggaggcgca actcaagcgt ttgcgaaaga aacgaaccaa 3240

aagccatata aggaaacata cggcatttcc catattacac gccatgatat gctgcaaatc 3300

cctgaacagc aaaaaaatga aaaatatcaa gttcctgagt tcgattcgtc cacaattaaa 3360

aatatctctt ctgcaaaagg cctggacgtt tgggacagct ggccattaca aaacgctgac 3420

ggcactgtcg caaactatca cggctaccac atcgtctttg cattagccgg agatcctaaa 3480

aatgcggatg acacatcgat ttacatgttc tatcaaaaag tcggcgaaac ttctattgac 3540

agctggaaaa acgctggccg cgtctttaaa gacagcgaca aattcgatgc aaatgattct 3600

atcctaaaag accaaacaca agaatggtca ggttcagcca catttacatc tgacggaaaa 3660

atccgtttat tctacactga tttctccggt aaacattacg gcaaacaaac actgacaact 3720

gcacaagtta acgtatcagc atcagacagc tctttgaaca tcaacggtgt agaggattat 3780

aaatcaatct ttgacggtga cggaaaaacg tatcaaaatg tacagcagtt catcgatgaa 3840

ggcaactaca gctcaggcga caaccatacg ctgagagatc ctcactacgt agaagataaa 3900

ggccacaaat acttagtatt tgaagcaaac actggaactg aagatggcta ccaaggcgaa 3960
gaatctttat ttaacaaagc atactatggc aaaagcacat cattcttccg tcaagaaagt 4020
caaaaacttc tgcaaagcga taaaaaacgc acggctgagt tagcaaacgg cgctctcggt 4080
atgattgagc taaacgatga ttacacactg aaaaaagtga tgaaaccgct gattgcatct 4140
aacacagtaa cagatgaaat tgaacgcgcg aacgtcttta aaatgaacgg caaatggtac 4200
ctgttcactg actcccgcgg atcaaaaatg acgattgacg gcattacgtc taacgatatt 4260
tacatgcttg gttatgtttc taattcttta actggcccat acaagccgct gaacaaaact 4320
ggccttgtgt taaaaatgga tcttgatcct aacgatgtaa cctttactta ctcacacttc 4380
gctgtacctc aagcgaaagg aaacaatgtc gtgattacaa gctatatgac aaacagagga 4440
ttctacgcag acaaacaatc aacgtttgcg ccaagcttcc tgctgaacat caaaggcaag 4500
aaaacatctg ttgtcaaaga cagcatcctt gaacaaggac aattaacagt taacaaataa 4560
aaacgcaaaa gaaaatgccg atattgacta ccggaagcag tgtgaccgtg tgcttctcaa 4620
atgcctgatt caggctgtct atgtgtgact gttgagctgt aacaagttgt ctcaggtgtt 4680
caatttcatg ttctagttgc tttgttttac tggtttcacc tgttctatta ggtgttacat 4740
gctgttcatc tgttacattg tcgatctgtt catggtgaac agctttaaat gcaccaaaaa 4800
ctcgtaaaag ctctgatgta tctatctttt ttacaccgtt ttcatctgtg catatggaca 4860
gttttccctt tgatatgtaa cggtgaacag ttgttctact tttgtttgtt agtcttgatg 4920
cttcactgat agatacaaga gccataagaa cctcagatcc ttccgtattt agccagtatg 4980
ttctctagtg tggttcgttg tttttgcgtg agccatgaga acgaaccatt gagatcatac 5040
ttactttgca tgtcactcaa aaattttgcc tcaaaactgg tgagctgaat ttttgcagtt 5100
aaagcatcgt gtagtgtttt tcttagtccg ttatgtaggt aggaatctga tgtaatggtt 5160
gttggtattt tgtcaccatt cattttatc tggttgttct caagttcggt tacgagatcc 5220
atttgtctat ctagttcaac ttggaaaatc aacgtatcag tcgggcggcc tcgcttatca 5280
accaccaatt tcatattgct gtaagtgttt aaatctttac ttattggttt caaaacccat 5340
tggttaagcc ttttaaactc atggtagtta ttttcaagca ttaacatgaa cttaaattca 5400
tcaaggctaa tctctatatt tgccttgtga gttttctttt gtgttagttc ttttaataac 5460
cactcataaa tcctcataga gtatttgttt tcaaaagact taacatgttc cagattatat 5520
tttatgaatt tttttaactg gaaaagataa ggcaatatct cttcactaaa aactaattct 5580
aatttttcgc ttgagaactt ggcatagttt gtccactgga aaatctcaaa gcctttaacc 5640
aaaggattcc tgatttccac agttctcgtc atcagctctc tggttgcttt agctaataca 5700
ccataagcat ttccctact gatgttcatc atctgaacgt attggttata agtgaacgat 5760
accgtccgtt ctttccttgt agggttttca atcgtggggt tgagtagtgc cacacagcat 5820

aaaattagct tggtttcatg ctccgttaag tcatagcgac taatcgctag ttcatttgct 5880

ttgaaaacaa ctaattcaga catacatctc aattggtcta ggtgatttta atcactatac 5940

caattgagat gggctagtca atgataatta ctagtccttt tcctttgagt tgtgggtatc 6000

tgtaaattct gctagacctt tgctggaaaa cttgtaaatt ctgctagacc ctctgtaaat 6060

tccgctagac ctttgtgtgt tttttttgtt tatattcaag tggttataat ttatagaata 6120

aagaaagaat aaaaaaagat aaaaagaata gatcccagcc ctgtgtataa ctcactactt 6180

tagtcagttc cgcagtatta caaaaggatg tcgcaaacgc tgtttgctcc tctacaaaac 6240

agaccttaaa accctaaagg cttaagtagc accctcgcaa gctcgggcaa atcgctgaat 6300

attccttttg tctccgacca tcaggcacct gagtcgctgt ctttttcgtg acattcagtt 6360

cgctgcgctc acggctctgg cagtgaatgg gggtaaatgg cactacaggc gccttttatg 6420

gattcatgca aggaaactac ccataataca agaaaagccc gtcacgggct tctcagggcg 6480

ttttatggcg ggtctgctat gtggtgctat ctgacttttt gctgttcagc agttcctgcc 6540

ctctgatttt ccagtctgac cacttcggat tatcccgtga caggtcattc agactggcta 6600

atgcacccag taaggcagcg gtatcatcaa caggcttacc cgtcttactg tcggggatcg 6660

acgctctccc ttatgcgact cctgca 6686

<210> 40
<211> 6665
<212> DNA
<213> Artificial Sequence

<220>
<223> vector

<400> 40
cctttcgtct tcgaataaat acctgtgacg gaagatcact tcgcagaata aataaatcct 60

ggtgtccctg ttgataccgg gaagccctgg gccaactttt ggcgaaaatg agacgttgat 120

cggcacgtaa gaggttccaa ctttcaccat aatgaaataa gatcactacc gggcgtattt 180

tttgagttat cgagattttc aggagctaag gaagctaaaa tggagaaaaa aatcactgga 240

tataccaccg ttgatatatc ccaatggcat cgtaaagaac attttgaggc atttcagtca 300

gttgctcaat gtacctataa ccagaccgtt cagctggata ttacggcctt tttaaagacc 360

gtaaagaaaa ataagcacaa gttttatccg gcctttattc acattcttgc ccgcctgatg 420

aatgctcatc cggaattccg tatggcaatg aaagacggtg agctggtgat atgggatagt 480

gttcaccctt gttacaccgt tttccatgag caaactgaaa cgttttcatc gctctggagt 540

gaataccacg acgatttccg gcagtttcta cacatatatt cgcaagatgt ggcgtgttac 600

ggtgaaaacc tggcctattt ccctaaaggg tttattgaga atatgttttt cgtctcagcc 660

aatccctggg tgagtttcac cagttttgat ttaaacgtgg ccaatatgga caacttcttc 720
gcccccgttt tcaccatggg caaatattat acgcaaggcg acaaggtgct gatgccgctg 780
gcgattcagg ttcatcatgc cgtttgtgat ggcttccatg tcggcagaat gcttaatgaa 840
ttacaacagt actgcgatga gtggcagggc ggggcgtaat ttttttaagg cagttattgg 900
tgcccttaaa cgcctggttg ctacgcctga ataagtgata ataagcggat gaatggcaga 960
aattcgaaag caaattcgac ccggtcgtcg gttcagggca gggtcgttaa atagccgctt 1020
atgtctattg ctggtctcgg tacccgggga tcgctcaccc agggatttat cggtagcgaa 1080
aataaaggtc gtacaacgac gcttggccgt ggaggcagcg attatacggc agccttgctg 1140
gcggaggctt tacacgcatc tcgtgttgat atctggaccg acgtcccggg catctacacc 1200
accgatccac gcgtagtttc cgcagcaaaa cgcattgatg aaatcgcgtt tgccgaagcg 1260
gcagagatgg caacttttgg tgcaaaagta ctgcatccgg caacgttgct acccgcagta 1320
cgcagcgata tcccggtctt tgtcggctcc agcaaagacc cacgcgcagg tggtacgctg 1380
gtgtgcaata aaactgaaaa tccgccgctg ttccgcgctc tggcgcttcg tcgcaatcag 1440
actctgctca ctttgcacag cctgaatatg ctgcattctc gcggtttcct cgcggaagtt 1500
ttcggcatcc tcgcgcggca taatatttcg gtagacttaa tcaccacgtc agaagtgagc 1560
gtggcattaa tccttgatac caccggttca acctccactg gcgatacgtt gctgacgcaa 1620
tctctgctga tggagctttc cgcactgtgt cgggtggagg tggaagaagg tctggcgctg 1680
gtcgcgttga ttggcaatga cctgtcaaaa gcctgcggcg ttggcaaaga ggtattcggc 1740
gtactggaac cgttcaacat tcgcatgatt tgttatggcg catccagcca taacctgtgc 1800
ttcctggtgc ccggcgaaga tgccgagcag gtggtgcaaa aactgcatag taatttgttt 1860
gagtaaatac tgtatggcct ggaagctata tttcgggccg tattgatttt cttgtcacta 1920
tgctcatcaa taaacgagcc tgtactctgt taaccagcgt ctttatcgga gaataattgc 1980
ctttaatttt tttatctgca tctctaatta attatcgaaa gagataaata gttaagagaa 2040
ggcaaaatga atattatcag ttctgctcgc aaacgaattc cgcgatcctc tagagtcgac 2100
cggtggcgaa tgggacgcgc cctgtagcgg cgcattaagc gcggcgggtg tggtggttac 2160
gcgcagcgtg accgctacac ttgccagcgc cctagcgccc gctcctttcg ctttcttccc 2220
ttcctttctc gccacgttcg ccggctttcc ccgtcaagct ctaaatcggg ggctcccttt 2280
agggttccga tttagtgctt tacggcacct cgaccccaaa aaacttgatt agggtgatgg 2340
ttcacgtagt gggccatcgc cctgatagac ggtttttcgc cctttgacgt tggagtccac 2400
gttctttaat agtggactct tgttccaaac tggaacaaca ctcaacccta tctcggtcta 2460
ttcttttgat ttataaggga ttttgccgat ttcggcctat tggttaaaaa atgagctgat 2520
ttaacaaaaa tttaacgcga attttaacaa aatattaacg cttacaattt aggtggcact 2580

tttcggggaa atgtgcgcgg aacccctatt tgtttatttt tctaaataca ttcaaatatg 2640

tatccgctca ccgcgatcct ttttaaccca tcacatatac ctgccgttca ctattattta 2700

gtgaaatgag atattatgat attttctgaa ttgtgattaa aaaggcaact ttatgcccat 2760

gcaacagaaa ctataaaaaa tacagagaat gaaaagaaac agatagattt tttagttctt 2820

taggcccgta gtctgcaaat ccttttatga ttttctatca aacaaaagag gaaaatagac 2880

cagttgcaat ccaaacgaga gtctaataga atgaggtcga aaagtaaatc gcgcgggttt 2940

gttactgata aagcaggcaa gacctaaaat gtgtaaaggg caaagtgtat actttggcgt 3000

caccccttac atattttagg tcttttttta ttgtgcgtaa ctaacttgcc atcttcaaac 3060

aggagggctg gaagaagcag accgctaaca cagtacataa aaaaggagac atgaacgatg 3120

aacatcaaaa agtttgcaaa acaagcaaca gtattaacct ttactaccgc actgctggca 3180

ggaggcgcaa ctcaagcgtt tgcgaaagaa acgaaccaaa agccatataa ggaaacatac 3240

ggcatttccc atattacacg ccatgatatg ctgcaaatcc ctgaacagca aaaaaatgaa 3300

aaatatcaag ttcctgagtt cgattcgtcc acaattaaaa atatctcttc tgcaaaaggc 3360

ctggacgttt gggacagctg gccattacaa aacgctgacg gcactgtcgc aaactatcac 3420

ggctaccaca tcgtctttgc attagccgga gatcctaaaa atgcggatga cacatcgatt 3480

tacatgttct atcaaaaagt cggcgaaact tctattgaca gctggaaaaa cgctggccgc 3540

gtctttaaag acagcgacaa attcgatgca aatgattcta tcctaaaaga ccaaacacaa 3600

gaatggtcag gttcagccac atttacatct gacggaaaaa tccgtttatt ctacactgat 3660

ttctccggta aacattacgg caaacaaaca ctgacaactg cacaagttaa cgtatcagca 3720

tcagacagct ctttgaacat caacggtgta gaggattata aatcaatctt tgacggtgac 3780

ggaaaaacgt atcaaaatgt acagcagttc atcgatgaag gcaactacag ctcaggcgac 3840

aaccatacgc tgagagatcc tcactacgta gaagataaag gccacaaata cttagtattt 3900

gaagcaaaca ctggaactga agatggctac caaggcgaag aatctttatt taacaaagca 3960

tactatggca aaagcacatc attcttccgt caagaaagtc aaaaacttct gcaaagcgat 4020

aaaaaacgca cggctgagtt agcaaacggc gctctcggta tgattgagct aaacgatgat 4080

tacacactga aaaaagtgat gaaaccgctg attgcatcta acacagtaac agatgaaatt 4140

gaacgcgcga acgtctttaa aatgaacggc aaatggtacc tgttcactga ctcccgcgga 4200

tcaaaaatga cgattgacgg cattacgtct aacgatattt acatgcttgg ttatgtttct 4260

aattctttaa ctggcccata caagccgctg aacaaaactg gccttgtgtt aaaaatggat 4320

cttgatccta acgatgtaac ctttacttac tcacacttcg ctgtacctca agcgaaagga 4380

aacaatgtcg tgattacaag ctatatgaca aacagaggat tctacgcaga caaacaatca 4440

acgtttgcgc caagcttcct gctgaacatc aaaggcaaga aaacatctgt tgtcaaagac 4500

agcatccttg aacaaggaca attaacagtt aacaaataaa aacgcaaaag aaaatgccga 4560

tattgactac cggaagcagt gtgaccgtgt gcttctcaaa tgcctgattc aggctgtcta 4620

tgtgtgactg ttgagctgta acaagttgtc tcaggtgttc aatttcatgt tctagttgct 4680

ttgttttact ggtttcacct gttctattag gtgttacatg ctgttcatct gttacattgt 4740

cgatctgttc atggtgaaca gctttaaatg caccaaaaac tcgtaaaagc tctgatgtat 4800

ctatcttttt tacaccgttt tcatctgtgc atatggacag ttttcccttt gatatgtaac 4860

ggtgaacagt tgttctactt ttgtttgtta gtcttgatgc ttcactgata gatacaagag 4920

ccataagaac ctcagatcct tccgtattta gccagtatgt tctctagtgt ggttcgttgt 4980

ttttgcgtga gccatgagaa cgaaccattg agatcatact tactttgcat gtcactcaaa 5040

aattttgcct caaaactggt gagctgaatt tttgcagtta aagcatcgtg tagtgttttt 5100

cttagtccgt tatgtaggta ggaatctgat gtaatggttg ttggtatttt gtcaccattc 5160

atttttatct ggttgttctc aagttcggtt acgagatcca tttgtctatc tagttcaact 5220

tggaaaatca acgtatcagt cgggcggcct cgcttatcaa ccaccaattt catattgctg 5280

taagtgttta aatctttact tattggtttc aaaacccatt ggttaagcct tttaaactca 5340

tggtagttat tttcaagcat taacatgaac ttaaattcat caaggctaat ctctatattt 5400

gccttgtgag ttttcttttg tgttagttct tttaataacc actcataaat cctcatagag 5460

tatttgtttt caaaagactt aacatgttcc agattatatt ttatgaattt ttttaactgg 5520

aaaagataag gcaatatctc ttcactaaaa actaattcta atttttcgct tgagaacttg 5580

gcatagtttg tccactggaa aatctcaaag cctttaacca aaggattcct gatttccaca 5640

gttctcgtca tcagctctct ggttgcttta gctaatacac cataagcatt ttccctactg 5700

atgttcatca tctgaacgta ttggttataa gtgaacgata ccgtccgttc tttccttgta 5760

gggttttcaa tcgtggggtt gagtagtgcc acacagcata aaattagctt ggtttcatgc 5820

tccgttaagt catagcgact aatcgctagt tcatttgctt tgaaaacaac taattcagac 5880

atacatctca attggtctag gtgattttaa tcactatacc aattgagatg ggctagtcaa 5940

tgataattac tagtcctttt cctttgagtt gtgggtatct gtaaattctg ctagaccttt 6000

gctggaaaac ttgtaaattc tgctagaccc tctgtaaatt ccgctagacc tttgtgtgtt 6060

ttttttgttt atattcaagt ggttataatt tatagaataa agaaagaata aaaaaagata 6120

aaaagaatag atcccagccc tgtgtataac tcactacttt agtcagttcc gcagtattac 6180

aaaaggatgt cgcaaacgct gtttgctcct ctacaaaaca gaccttaaaa ccctaaaggc 6240

ttaagtagca ccctcgcaag ctcgggcaaa tcgctgaata ttccttttgt ctccgaccat 6300

caggcacctg agtcgctgtc tttttcgtga cattcagttc gctgcgctca cggctctggc 6360

agtgaatggg ggtaaatggc actacaggcg ccttttatgg attcatgcaa ggaaactacc 6420

cataatacaa gaaaagcccg tcacgggctt ctcagggcgt tttatggcgg gtctgctatg 6480

tggtgctatc tgactttttg ctgttcagca gttcctgccc tctgattttc cagtctgacc 6540

acttcggatt atcccgtgac aggtcattca gactggctaa tgcacccagt aaggcagcgg 6600

tatcatcaac aggcttaccc gtcttactgt cggggatcga cgctctccct tatgcgactc 6660

ctgca 6665

<210> 41
<211> 15214
<212> DNA
<213> Artificial Sequence

<220>
<223> Plasmid HM-p-25

<400> 41
aaataatttt gtttaacttt aagaaggaga tatacgatgt cagcaaatat ggcagtcaaa 60

caggccttga aggccaatcc ggtcccgagt tccgtggatc ctcaggaagt ccacaaatgg 120

cttcaggatt tcacttggga tttcaagggc aagaccgcga agtatccgac caagtatgag 180

atggacgtca atacgcgcga gcagttcaag ctgactgcca aggagtacgc gcgcatggag 240

tcgatcaagg aagagcgcca gtacggcacc ctgctcgatg gtctcgaccg tctggatgcg 300

ggcaacaagg tgcatccgaa atggggcgag gtgatgaagc tggtctccaa cttcctcgag 360

accggcgaat acggcgcaat cgccggttct gctctgctgt gggacacggc ccagtcaccg 420

gagcagcgca acggttacct cgctcaggtg atcgacgaaa tccgccatgt gaaccagacc 480

gcgtacgtga attactacta cggcaagcac tactacgacc ccgccggcca caccaacatg 540

cgccagcttc gggcgatcaa ccccctgtac cccggtgtca agcgcgcctt cggggagggc 600

tttctcgcgg gcgatgccgt cgagtcctcc atcaacctcc aattggtcgg cgaagcctgc 660

ttcaccaatc cgctcatcgt ttcgcttacc gaatgggcgg ccgcgaacgg cgatgaaatc 720

acgccgaccg tgttcctgtc gatcgaaacc gatgaactgc gccatatggc caacggctat 780

cagaccatcg tgtcgatcat gaacaatccg gagacgatga agtacctgca aacggacctc 840

gataacgcat tctggacgca gcacaagttc ctgaccccct tcgtcggggt ggcgctcgaa 900

tatggttcca agtacaaggt cgagccgtgg gccaagtcct ggaaccggtg ggtgtacgaa 960

gactgggctg gcatctggct gggccgactg cagcagttcg gcgtcaaaac gccaaagtgc 1020

ctgcccgacg ccaagaaaga cgccgtttgg gcacaccacg atctcgcgct gctggccctt 1080

gccctgtggc cgctgaccgg catccgcatg gaactcccgg atagcctggc gatggagtgg 1140

ttcgaggcta attaccccgg ttggtacaac cattacggca agatctacga ggagtggcgc 1200

gctgccggct tcgaggatcc gaagagcggc ttctgtggtg cgctctggct gatggagcgt 1260
ggtcacggca ttttcgtcga ccatgcctcg ggtttgccgt tctgccccag cctcgccaaa 1320
agctctatca agccgcggtt cactgaatac aacggcaagc gctacgcctt cgccgagccg 1380
tatggcgagc gccagtggct gctcgagccc gagcgctacg agttccagaa cttcttcgag 1440
cagttcgaag gctgggaact ctccgatctc gtcaaggcgg caggcggcgt gcgcagcgat 1500
ggcaaaacgc tgatcgcgca gccgcatctt cgcgacaccg acatgtggac gcttgacgat 1560
ctgaagcgga tcaacctgac gatccccgac ccgatgaaga tcctcaactg gcaacccgtc 1620
gcccagtgag ggttgggccg gcgcacgccg ccaggcgaga gatgcgccgg cggggaagac 1680
ttgccgcccg cctacgagcg ggcggacgtg ccaacgaacg aacagcacta tatggagtag 1740
ataaatatgt caacaaacat ctttacgcgc gggatggtag acccggagcg ccaagcctgc 1800
attcaggagg tcgtacccaa ggcgccgctg gaaaccaagc gtgaccatat tcctttcgcc 1860
aaacgcggct ggcgccgact caccgagtat gaagcggtga tgttgcatgc gcagaattca 1920
ctggacgccg tcccgggcag ccaggaggtg ggtgaagtcg tacagaagtg gccgggtggc 1980
aggccgaact acggcgtcga gtcgaccgcg gccctctcca gcaactggtt ccatttccgc 2040
gacccgtcga agcgctggtt catgccctac gtgaagcaga agaacgagga agggcagaca 2100
gccgaacgcg cgatgaagag ttgggccgag ggcggcgacg cggaaatgat gaacgccgcg 2160
tggcgggagc acatcctggc ccggcactac ggcgcgttcg tctataacga gtacggtctg 2220
ttcagcgccc attcaacaac agtttacggt ggcctttctg atctgatcaa aacctggatt 2280
gccgaggccg cgttcgacaa gaacgacgcc ggtcagatga tccagatgca gcgcgtgctg 2340
ttgagcaagg tgttccctgg cttcgacgcc gacctggccg aagccaagca ggcatggact 2400
gaggacaagt catggaaacc cgcccgcgag ttcgtcgagc acatctgggc cgagacctac 2460
gactgggtcg agcagctttg ggcgatccac gccgtctacg accatatttt cgggcaattt 2520
gtccggcgcg aattcttcca gcggctcggc ggcatccatg gagacacgct gacgcccttc 2580
attcagaacc aggcgctgac gtatcacctg caagcccgcg acggcgtaac ggcactgtgc 2640
ttcaaatttc tgatcgaaga cgagccggta tacgcccaac acaacaggcg ctacctgcgg 2700
gcatggacag ggcgctatct cccgcaagtg ggccgcgcat tgaaggcctt cctggcaatc 2760
tacaaggagg ttccggtaaa gatcgatggg gtgacttgcc gggagggcgt gcgcgccagt 2820
gtcgaacgcg tggtggacga ctgggcggcg cggttcgctg aaccgatcaa cttcaagttc 2880
aaccgcgcgg cgttcatcga cgatgtgctg agtggctact aattcagggg cagaccgaat 2940
gtcaaacgta aatgcatacc acgctggcac caatggtaaa gaaggccagg acttcatcga 3000
tgacttttg agcgaagaga acagtgctct acccacgagc gaagccgtgg ttctggccct 3060
gatgaagacc gaagagatcg acgcggtcgt cgacgagatg atcaagccgc agatggagga 3120

caacccgacc atcgccgtcg aggaccgcgg tggatactgg tggatcaagg ccaacggaaa 3180
gatcgtcatc gattgtgacg aagccaccga actgctgggc aagaagtata cggtgtacga 3240
tttgctggtc aacgtcagca ccaccgttgg gcgggcgatg accctcggca atcagttcat 3300
catcaccaac gagctccttg gtctcgaaac caaggtcgaa agcgtttact gaggaggata 3360
gacgacatgt cgaaacaggt ttggtacaac acaccggtgc gcgacgagtg gatcgagaag 3420
atcactgcga tcaggacagc gcgcgaaggc accgacatgc tcgcccgctt ccgcgcggag 3480
catacggggc cggatcgcac gacgtacgat ctcaagaagg aatacaattg gatcgagtcg 3540
cgcatcgaga tgcgggtcag ccagcttcat gccgaggcca cggcctcgga cgaagacctc 3600
ctcaccaaga cgatcgacgg ccgctgcgca aaggaagtcg cagcggagtg gctgaaaaag 3660
gctgcggaca tcgactgtca ctacgagatg gagcggcttt gtgtcgcctt ccgcaaggcg 3720
tgcaaaccgc cgatgatgcc catcaacttc ttcgcgccgg cagagaagga gttggtggcg 3780
aagctcatga agctgagggc gcccacgtat ctgactacct ccctcgacga gttgcgcgag 3840
gcacggggtg taacgatgat ttccgtgcag taagcggccg gacgttctga gccggatcct 3900
ccccgacaac ggggaggact ggtcaaaata gccatcaaga ctgaaaggag cagggaatgc 3960
cgaaatacat catcgaacgt tcgattccgg gggcagggaa gctgacggag cgggacctcg 4020
cgtcggtgtc gcagaaatcc tgtggaatcc tccgctccat ggggccacaa atccaatggg 4080
tcaaaagcta cgtcaccgac gacaagctct actgcatcta tatcgctccc gatgaggcct 4140
ccatccgcga gcatgcaaga tcgggagact ttcccgccga caaggtgtcc cgcatacatc 4200
ggatgatcga cccgacttgc gcggaatcgg cataatcgag ccgtcctcgg aggaccacga 4260
cttcccgagg gcctccggac gatggatcgc ggcggacgga aacctgcgtg gccgcggacg 4320
cggggtggcc acaagatcgt aacgtaaagt actctaatcc gagagcgcgg tcgctttcac 4380
ccgatcgagg tggaagtgac tgggcgaggg ggagatatga aagaagcgcc ggcaataccc 4440
gatctgcccg gtttgcccga gactgtcggc gaaccgacgc tggtcctgga agaagatggc 4500
ttccgggttt tcgccacaga gctgacgatc atgtggcgat gggacatcta caacggcgat 4560
gcgcacgttc ataccggatg cgcccagcac ccggagagct gcgtcgtcgc ggcgagatcc 4620
aaaattcgat tcttacggcg ccccactgtg gccatgctac tgggcggtga aggtcaatga 4680
gaggggggttc gagacgccaa ctgatggacg cgacacccta gtaggagact gaaggtatat 4740
gctgatgcaa caatataaaa tagtcgcccg cttcgaagac ggcgtgacat acgagtacga 4800
ttgcggcgag gacgaaaacc tgctcgccgc ggcgttgcgc cagaacgtcc gtttgctgtg 4860
tcaatgcaga aaggcgtttt gcggcagttg caaggccttg tgcagcgagg gcgactatga 4920
actgggcgat catatcaacg ttcaggtctt gccgcctgac gaagaagagg acggggtggt 4980

ggtcacctgc gataccttcc cacgcagcga tctggtcctt gagtttccgt ataccagcga 5040
ccgtctcggt accgttacgg ctaccgaggc gaagacgagc gtcgtcagcg tggagagact 5100
ctctagcacc gtttaccgtc tggtgctgca ggcactcgac gccgaaggaa tgcctgcacg 5160
gttcgacttc gttcccgggc agtatgtcga aatcagtaca gccgattcgc tcgagaccag 5220
ggcgttttcg ctggcgaacc ttccaaacga cgccggattg ctcgagttct tgatccgact 5280
cgtccccggc ggatactatg cggcctatct ggagcaacgc gcagcggcag ggcagacgat 5340
caacgtgaag ggaccgttcg gcgagttcgt gctgcgtgaa cacgagttgg tggaggactt 5400
cacgctgcca gcggacagcc cagcgagagg tgggacgatt gcgttcctgg cgggtagcac 5460
ggggctggcg cctctcgcga gcatgctgcg cgagctcggg cggcgaggat tcaacggcga 5520
gtgtcacctc ttcttcggca tgcaggacac cgcaacgatg ttctacgaga aggaactccg 5580
ggacatcaag cgaacgcttc cggggctcac gctgcatctc gccctgatgg tcccttctgc 5640
cgaatgggaa ggctaccgtg gtaacgccgt agctgcgttc aaagaacatt tcgccgccag 5700
tagccagata cccgagaacg tttacctgtg cgggcccgga ccgatgatcg cggccgcgct 5760
cggcgcttgt cgcgagcttg gaattcccga caatagagtt cacagggaag aattcgtagc 5820
gagcggcggt tgaacggcgc ttgaagaaac cggggcgcga cgggcagcgt ttacgagacc 5880
atgagtcaag aagaaagaga ttttctggcc ggtgcagcgg caacactcgg cgagagcgtc 5940
ggacaactgg agcgccttgc ccaagaatta agtgaaaagc tgggcgatga gcgtgtcaac 6000
attgttcggg gtttgctcga acaggtttat gaccgcgacg acgaagcgga agtgcgtgca 6060
tccctctgct atacggagag aaagctgttg tgggcgtggg tccgtttgaa gagactgaag 6120
ggactcaggc tcagtattgg tcgtggttcg atgagaaaca ttatatgaga agcagaacgt 6180
gatcagttta aattgcaaga agacaaccac agggttaact gcgcacttgg ccctggtccg 6240
cggaatgaag gcgttggcgg aattggttgg aacaacgctg gggccccagg gccgccacgt 6300
catgcttgcg catcgggccg gattggctcc gcacgtcagc aaggacggcg tcgaggtcgc 6360
acgtcacctg tcgttgccgg acagcgagga agaacttggt gtccggttgc tgcgcaacgc 6420
agccgtcgca gtgtccgagt cctttggcga cggcacctcc actgcaaccg tttttacggc 6480
ggatctggcc gtgcgggcgc tcaaactgat cggtgccggg gcggataccc tggaggtccg 6540
tcgaggtctg ggtttggctg cctatgccgc gttggtcgcc ctgaacgaca tggcccggcg 6600
cgccgaccgg ggaatgctca ccgccgttgc ccaaaccgct gccaacggcg accgtcgcgt 6660
ggcggatctc ctcgtggagg ccttcgaaag ggtcggggct gaaggcacga tcgaagtaga 6720
aatgggtaat tcggtcgagg acgtccttga agtcgcgcag ggcagttatt tcgacacggt 6780
gccgctcgtc acggccttgt tgccgccaac agggcaggtc gagttcgccc ggccacttat 6840
ccttttccat tgcgacgcga tcgagacggc ggacgagatt cttccggcgc tggagctagc 6900

```
tcgcagttca cggagaccct tgctgattct ggccgactcg gtgggtatcg acgtcgagac      6960
gctgctcgtc cggaatcaaa atgaaggcac cttggctgtt gcggtcgtca gggcgccaat      7020
gtatggcgat acgcgtcgcg aggccctgct tgacctgacg agcaaattcg gggggacggc      7080
gtttggaagg gagggattcg tcgaattcgc actcaggtct ttgggatcac tgtcggaagg      7140
tgacttgggg caggcggacg aagcaatcct agaggcggat ggtgtgactc tgagaggcgc      7200
cggaaacaat ccgtctgcat tggaagaccg aatcgcgctg gtgcgtgctg aactcgatcg      7260
cggcgacgtt tccgtgggcg actccccatc ggcaaagctc gactacatcg agaagcgaaa      7320
ggagcggctg aagttgctgg ctgcaggtag cgccaagctg catatcgggg ggccgacgga      7380
cgttgagatc aagacgcgtt tgccgctcgc cgagaacgct catcgggcgc tcttggcggc      7440
cgcgaagtcg ggagtgctgc ccggggggcgg cgtcgcaatg attcgtgcgg ctgaaaaggt      7500
gcagcaagag atggggcgac ttgaaggcga cgttgcttct ggggcatcca ttttcctgca      7560
gtcgcttgac acacccatcc ggtggattgc acgaaatgcg gggctgcgcc cggatgaagt      7620
gctcgctcga accctggcga acgagtcgga cttttacgga ctcaacgcca tgactggccg      7680
atatggcgac ttggctgaag acggggttct cgacgccctc gatatggtca ccgacgtgat      7740
acgcgtcgca gtcagcgtcg tcgggtcgat gttgggcgtc ggcgccctgg tgactcgcgc      7800
atcgcccaag cccgcgccag agcgcttcaa gggcacggag cgggtacacg acaagttgat      7860
gcgcgagggc gggttcgatg aatgaaattt cagtgcaatt tatctcttca aatgtagcac      7920
ctgaagtcag ccccatacga tataagttgt aattctcatg tttgacagct tatcatcgat      7980
aagctttaat gcggtagttt atcacagtta aattgctaac gcagtcaggc accgtgtatg      8040
aaatctaaca atgcgctcat cgtcatcctc ggcaccgtca ccctggatgc tgtaggcata      8100
ggcttggtta tgccggtact gccgggcctc ttgcgggata tcgtccattc cgacagcatc      8160
gccagtcact atggcgtgct gctagcgcta tatgcgttga tgcaatttct atgcgcaccc      8220
gttctcggag cactgtccga ccgctttggc cgccgcccag tcctgctcgc ttcgctactt      8280
ggagccacta tcgactacgc gatcatggcg accacacccg tcctgtggat cctctacgcc      8340
ggacgcatcg tggccggcat caccggcgcc acaggtgcgg ttgctggcgc ctatatcgcc      8400
gacatcaccg atggggaaga tcgggctcgc cacttcgggc tcatgagcgc ttgtttcggc      8460
gtgggtatgg tggcaggccc cgtggccggg ggactgttgg gcgccatctc cttgcatgca      8520
ccattccttg cggcggcggt gctcaacggc ctcaacctac tactgggctg cttcctaatg      8580
caggagtcgc ataagggaga gcgtcgaccg atgcccttga gagccttcaa cccagtcagc      8640
tccttccggt gggcgcgggg catgactatc gtcgccgcac ttatgactgt cttctttatc      8700
atgcaactcg taggacaggt gccggcagcg ctctgggtca ttttcggcga ggaccgcttt      8760
```

cgctggagcg cgacgatgat cggcctgtcg cttgcggtat tcggaatctt gcacgccctc 8820
gctcaagcct tcgtcactgg tcccgccacc aaacgtttcg gcgagaagca ggccattatc 8880
gccggcatgg cggccgacgc gctgggctac gtcttgctgg cgttcgcgac gcgaggctgg 8940
atggccttcc ccattatgat tcttctcgct tccggcggca tcgggatgcc cgcgttgcag 9000
gccatgctgt ccaggcaggt agatgacgac catcagggac agcttcaagg atcgctcgcg 9060
gctcttacca gcctaacttc gatcattgga ccgctgatcg tcacggcgat ttatgccgcc 9120
tcggcgagca catggaacgg gttggcatgg attgtaggcg ccgccctata ccttgtctgc 9180
ctccccgcgt tgcgtcgcgg tgcatggagc cgggccacct cgacctgaat ggaagccggc 9240
ggcacctcgc taacggattc accactccaa gaattggagc caatcaattc ttgcggagaa 9300
ctgtgaatgc gcaaaccaac ccttggcaga acatatccat cgcgtccgcc atctccagca 9360
gccgcacgcg gcgcatctcg ggcagcgttg ggtcctggcc acgggtgcgc atgatcgtgc 9420
tcctgtcgtt gaggacccgg ctaggctggc ggggttgcct tactggttag cagaatgaat 9480
caccgatacg cgagcgaacg tgaagcgact gctgctgcaa aacgtctgcg acctgagcaa 9540
caacatgaat ggtcttcggt ttccgtgttt cgtaaagtct ggaaacgcgg aagtccccta 9600
cgtgctgctg aagttgcccg caacagagag tggaaccaac cggtgatacc acgatactat 9660
gactgagagt caacgccatg agcggcctca tttcttattc tgagttacaa cagtccgcac 9720
cgctgccggt agctccttcc ggtgggcgcg gggcatgact atcgtcgccg cacttatgac 9780
tgtcttcttt atcatgcaac tcgtaggaca ggtgccggca gcgcccaaca gtcccccggc 9840
cacggggcct gccaccatac ccacgccgaa acaagcgccc tgcaccatta tgttccggat 9900
ctgcatcgca ggatgctgct ggctaccctg tggaacacct acatctgtat taacgaagcg 9960
ctaaccgttt ttatcaggct ctgggaggca gaataaatga tcatatcgtc aattattacc 10020
tccacgggga gagcctgagc aaactggcct caggcatttg agaagcacac ggtcacactg 10080
cttccggtag tcaataaacc ggtaaaccag caatagacat aagcggctat ttaacgaccc 10140
tgccctgaac cgacgaccgg gtcgaatttg ctttcgaatt tctgccattc atccgcttat 10200
tatcacttat tcaggcgtag caaccaggcg tttaagggca ccaataactg ccttaaaaaa 10260
attacgcccc gccctgccac tcatcgcagt actgttgtaa ttcattaagc attctgccga 10320
catggaagcc atcacaaacg gcatgatgaa cctgaatcgc cagcggcatc agcaccttgt 10380
cgccttgcgt ataatatttg cccatggtga aaacgggggc gaagaagttg tccatattgg 10440
ccacgtttaa atcaaaactg gtgaaactca cccagggatt ggctgagacg aaaaacatat 10500
tctcaataaa ccctttaggg aaataggcca ggttttcacc gtaacacgcc acatcttgcg 10560
aatatatgtg tagaaactgc cggaaatcgt cgtggtattc actccagagc gatgaaaacg 10620
tttcagtttg ctcatggaaa acggtgtaac aagggtgaac actatcccat atcaccagct 10680

caccgtcttt cattgccata cggaattccg gatgagcatt catcaggcgg gcaagaatgt 10740
gaataaaggc cggataaaac ttgtgcttat ttttctttac ggtctttaaa aaggccgtaa 10800
tatccagctg aacggtctgg ttataggtac attgagcaac tgactgaaat gcctcaaaat 10860
gttctttacg atgccattgg gatatatcaa cggtggtata tccagtgatt tttttctcca 10920
ttttagcttc cttagctcct gaaaatctcg ataactcaaa aaatacgccc ggtagtgatc 10980
ttatttcatt atggtgaaag ttggaacctc ttacgtgccg atcaacgtct cattttcgcc 11040
aaaagttggc ccagggcttc ccggtatcaa cagggacacc aggatttatt tattctgcga 11100
agtgatcttc cgtcacaggt atttattcgg cgcaaagtgc gtcgggtgat gctgccaact 11160
tactgattta gtgtatgatg gtgtttttga ggtgctccag tggcttctgt ttctatcagc 11220
tgtccctcct gttcagctac tgacggggtg gtgcgtaacg gcaaaagcac cgccggacat 11280
cagcgctagc ggagtgtata ctggcttact atgttggcac tgatgagggt gtcagtgaag 11340
tgcttcatgt ggcaggagaa aaaaggctgc accggtgcgt cagcagaata tgtgatacag 11400
gatatattcc gcttcctcgc tcactgactc gctacgctcg gtcgttcgac tgcggcgagc 11460
ggaaatggct tacgaacggg gcggagattt cctggaagat gccaggaaga tacttaacag 11520
ggaagtgaga gggccgcggc aaagccgttt ttccataggc tccgcccccc tgacaagcat 11580
cacgaaatct gacgctcaaa tcagtggtgg cgaaacccga caggactata aagataccag 11640
gcgtttcccc ctggcggctc cctcgtgcgc tctcctgttc ctgcctttcg gtttaccggt 11700
gtcattccgc tgttatggcc gcgtttgtct cattccacgc ctgacactca gttccgggta 11760
ggcagttcgc tccaagctgg actgtatgca cgaacccccc gttcagtccg accgctgcgc 11820
cttatccggt aactatcgtc ttgagtccaa cccggaaaga catgcaaaag caccactggc 11880
agcagccact ggtaattgat ttagaggagt tagtcttgaa gtcatgcgcc ggttaaggct 11940
aaactgaaag gacaagtttt ggtgactgcg ctcctccaag ccagttacct cggttcaaag 12000
agttggtagc tcagagaacc ttcgaaaaac cgccctgcaa ggcggttttt tcgttttcag 12060
agcaagagat tacgcgcaga ccaaaacgat ctcaagaaga tcatcttatt aatcagataa 12120
aatatttatt agataattcc ttgacgctca gcttcaattg ttgcttgcgt gcgattcact 12180
acattcaagg tggcaaatat tttcctcata tgccacttta tagcatcttc ggtgacatgc 12240
atatttgttg ctatttgttt gtttgagcac ccctctttta caagcctcaa gacagcaatc 12300
tgcttccgtg tcaataaagc gtcagcttta ttctctgcgg actttccaat ctcaactatt 12360
cgcggaagac taaaagcccc aatcgcttga tctaaattaa ctgctgtgaa ggcttcacat 12420
gaagccggta ttattcgctc aattaaacat acttcatcaa gaactgtttg aaagcattga 12480
agctgttttg ctatctccac tgcataaaca atgttaagct gagccttttt taaatcaccg 12540

gcacctgcct gcgctccggc caaacacaat aatccacgga cttccagctg gcccgcgtta 12600
attttacggg cttgctgaat agccaataac gctctgtgcg cggcactatg aaagttccga 12660
tctcgggaaa gcactagtga ttgaacaagc agcaggcgtg cttttagggg ggctgagtgc 12720
tgtccggaga aaatcttatg atcttcaaga gtttttaaat tatttatgcc cgttatgcct 12780
tgacagacta agcgctgata gatctcaatt tggctcataa cttccaatct tggtagattt 12840
ttttcaaccg catgcgcctt cgcccactcc aatatctcaa tggagccatt taggtcactc 12900
cttccaagcc gccaagctga cacagcacgg catacggaaa aaaacacgtc tgtcaccccg 12960
tgattggaaa tgaactctaa aattttggag agcttttctt ctgaggtgtc caagcagcgc 13020
aattcataat gtaactcaag ctctagagcg tcaaacattt tcgaagtaaa ctcggattcc 13080
atcatctgcg cgcgactgtc tgtgcgtgct tgagttataa tctgcctcgc ccagcccatt 13140
tttccgcttg ctagggcttg ttgaaacctc gcgacataca gccaaccaaa agcaaaattt 13200
tgttttgcaa atttattcac ggcttgggcc tgagccagca ccttctccaa ctctgcaaat 13260
ctatactcac tggcaaaaat aaaagccaaa caggttagcg cggccccttt tccaactgcg 13320
tttgaatccc caaataaact aatccactta ttacagagct cctcactcga aagcatttca 13380
tctttcgttg ctttacctat tgcaagcaca agctgcagcc attccttttc ttgccattta 13440
ttttttttat cggattgtga agataggtct ttaattaact tctctgctcg cgcgccttgc 13500
tgactgaaat acaataccca cgcgtaacta ataagcacta tgggtttttt gtgccaggcc 13560
tgcttcggca gctctaacag ccactgtctc agcgcatcta tttcgccctg acgaaatgac 13620
aaatctaaaa ttattctctc agacatgctg actgcccagc gacagtcatt cgcccgtagg 13680
gatattcgta ttgcatactg gtattcacct ctacgccaat gccagaaagc tgcacgctta 13740
agcaggtagg atcttttagc aggattttca gtccaagtaa tttctcgtag aaaattacgc 13800
agtactggat gcagtgtaaa ctgcgctggc tcaccgctca catggcgaag caacatgtaa 13860
ttagtgctta aatacttaat acatgagacc ccattgacgc atttgaatac ataattgtat 13920
tgatcaggcg tcacgaaatc gagcaatgaa gaatttgcaa gaaaaacacg atagcgctcg 13980
ggaatcgcct caaatatttc atccctaaag taattgtcta cttcaactac tgctgaaata 14040
tgcttggccg gcaactcacg ctttaacaaa aaaactacaa gagcaggcca cccctcaact 14100
tcttgcacca aggtctctat ctgttcttca ggaactccaa gaacagactc tgcctccgct 14160
aacgccaccg cctcttctgc gctaaaggcc aagtctttct cggtgtactc ccgcatagcg 14220
cctgcaagtt taagctgcga gaaccctttt attgtattgc ctgcaactgc aaacctgata 14280
ttttttggtg tatttaacat aaactccata agtgcgtgca acaacggcaa gtctaagtca 14340
tgattaatat tatccaaaca aactagcgtt tctatctcgt tattcgaggt gctctgccaa 14400
agactagatg caaggtctcg caagagcgca ggcttgctca caccctctct cacacggctg 14460

aattttacca tttcgaaagt ttcaagctgc tcaataatct ctgcgcagat atcaaattca 14520

ctgtaagaac tggctcttaa agaaagccac actgcaggac gtccggctgt tctgtggcgt 14580

agccactcga acgcaagagc aacggttttc ccatatccag gtggggctct gtaaaggcat 14640

actctgggag cggctccatc cgcgatactc aatcttggcc gatatatgca actatgaact 14700

ttggcactta ctagagtcgt aatttgatcc gctccgacct tagcgaccgg gaaatcatta 14760

tttattatta ttttcattat gctattctcg cgccagctga ctggaaattt tcaccatagg 14820

ttacggtgtt aaatattaaa actacactta agtgtagtcg gcatgatcgg tggtgcaaaa 14880

tatttactag ggaaggtctg aagtaggccg ctatttctgg ccgacttcgg ccttcgccga 14940

ttttgaagac gggcaccggg tcaaaatcga ccagatagct cgctcatttc ggtgctttca 15000

gccgtcgcga gtagctcgcg gtacctggca tgcttgcggc cagctcgtgt tttccagca 15060

gacgacggag caaaaactac ccgtaggtgt agttggcgca agcgtccgat tagctcaggt 15120

ttaagatgtc gagagtgaga gtgggcggct taactttctc agttaggcat aaaattacgt 15180

cttaaatctc gtagcgacta atttaataaa aatt 15214

<210> 42
<211> 506
<212> PRT
<213> Corynebacterium glutamicum

<400> 42

Met Thr Val Tyr Ala Asn Pro Gly Thr Glu Gly Ser Ile Val Asn Tyr
1 5 10 15

Glu Lys Arg Tyr Glu Asn Tyr Ile Gly Gly Lys Trp Val Pro Pro Val
20 25 30

Glu Gly Gln Tyr Leu Glu Asn Ile Ser Pro Val Thr Gly Glu Val Phe
35 40 45

Cys Glu Val Ala Arg Gly Thr Ala Ala Asp Val Glu Leu Ala Leu Asp
50 55 60

Ala Ala His Ala Ala Ala Asp Ala Trp Gly Lys Thr Ser Val Ala Glu
65 70 75 80

Arg Ala Leu Ile Leu His Arg Ile Ala Asp Arg Met Glu Glu His Leu
85 90 95

Glu Glu Ile Ala Val Ala Glu Thr Trp Glu Asn Gly Lys Ala Val Arg
100 105 110

```
Glu Thr Leu Ala Ala Asp Ile Pro Leu Ala Ile Asp His Phe Arg Tyr
        115                 120                 125

Phe Ala Gly Ala Ile Arg Ala Gln Glu Asp Arg Ser Ser Gln Ile Asp
    130                 135                 140

His Asn Thr Val Ala Tyr His Phe Asn Glu Pro Ile Gly Val Val Gly
145                 150                 155                 160

Gln Ile Ile Pro Trp Asn Phe Pro Ile Leu Met Ala Thr Trp Lys Leu
                165                 170                 175

Ala Pro Ala Leu Ala Ala Gly Asn Ala Ile Val Met Lys Pro Ala Glu
            180                 185                 190

Gln Thr Pro Ala Ser Ile Leu Tyr Leu Ile Asn Ile Ile Gly Asp Leu
        195                 200                 205

Ile Pro Glu Gly Val Leu Asn Ile Val Asn Gly Leu Gly Gly Glu Ala
    210                 215                 220

Gly Ala Ala Leu Ser Gly Ser Asn Arg Ile Gly Lys Ile Ala Phe Thr
225                 230                 235                 240

Gly Ser Thr Glu Val Gly Lys Leu Ile Asn Arg Ala Ala Ser Asp Lys
                245                 250                 255

Ile Ile Pro Val Thr Leu Glu Leu Gly Gly Lys Ser Pro Ser Ile Phe
            260                 265                 270

Phe Ser Asp Val Leu Ser Gln Asp Asp Ala Phe Ala Glu Lys Ala Val
        275                 280                 285

Glu Gly Phe Ala Met Phe Ala Leu Asn Gln Gly Glu Val Cys Thr Cys
    290                 295                 300

Pro Ser Arg Ala Leu Val His Glu Ser Ile Ala Asp Glu Phe Leu Glu
305                 310                 315                 320

Leu Gly Val Lys Arg Val Gln Asn Ile Lys Leu Gly Asn Pro Leu Asp
                325                 330                 335

Thr Glu Thr Met Met Gly Ala Gln Ala Ser Gln Glu Gln Met Asp Lys
            340                 345                 350

Ile Ser Ser Tyr Leu Lys Ile Gly Pro Glu Glu Gly Ala Gln Thr Leu
        355                 360                 365
```

```
Thr Gly Gly Lys Val Asn Lys Val Asp Gly Met Glu Asn Gly Tyr Tyr
    370                 375                 380

Ile Glu Pro Thr Val Phe Arg Gly Thr Asn Asp Met Arg Ile Phe Arg
385                 390                 395                 400

Glu Glu Ile Phe Gly Pro Val Leu Ser Val Ala Thr Phe Ser Asp Phe
                405                 410                 415

Asp Glu Ala Ile Arg Ile Ala Asn Asp Thr Asn Tyr Gly Leu Gly Ala
            420                 425                 430

Gly Val Trp Ser Arg Asp Gln Asn Thr Ile Tyr Arg Ala Gly Arg Ala
        435                 440                 445

Ile Gln Ala Gly Arg Val Trp Val Asn Gln Tyr His Asn Tyr Pro Ala
    450                 455                 460

His Ser Ala Phe Gly Gly Tyr Lys Glu Ser Gly Ile Gly Arg Glu Asn
465                 470                 475                 480

His Leu Met Met Leu Asn His Tyr Gln Gln Thr Lys Asn Leu Leu Val
                485                 490                 495

Ser Tyr Asp Pro Asn Pro Thr Gly Leu Phe
            500                 505


<210>  43
<211>  42
<212>  DNA
<213>  Artificial Sequence

<220>
<223>  primer_MW_15_66_f

<400>  43
tctcggtacc cggggatcgc tcacccaggg atttatcggt ag                        42


<210>  44
<211>  22
<212>  DNA
<213>  Artificial Sequence

<220>
<223>  primer_MW_15_67_r

<400>  44
caaggattaa tgccacgctc ac                                              22


<210>  45
<211>  22
```

<212> DNA
<213> Artificial Sequence

<220>
<223> primer_MW_15_68_f

<400> 45
gtgagcgtgg cattaatcct tg 22

<210> 46
<211> 42
<212> DNA
<213> Artificial Sequence

<220>
<223> primer_MW_15_69_r

<400> 46
ggtcgactct agaggatcgc ggaattcgtt tgcgagcaga ac 42

<210> 47
<211> 45
<212> DNA
<213> Artificial Sequence

<220>
<223> primer_JC-15-009_fw

<400> 47
gctggtctcg gtacccgggg atcgccattc cggctgatca catgg 45

<210> 48
<211> 25
<212> DNA
<213> Artificial Sequence

<220>
<223> primer_JC-15-006_rv

<400> 48
gtaatcagca ccacgaaaat acggg 25

<210> 49
<211> 25
<212> DNA
<213> Artificial Sequence

<220>
<223> primer_JC-15-007_fw

<400> 49
cccgtatttt cgtggtgctg attac 25

<210> 50
<211> 45
<212> DNA
<213> Artificial Sequence

<220>
<223> primer_JC-15-010_rv

<400> 50
ccaccggtcg actctagagg atcgcttgcg ccagttcttc ctgcc 45

<210> 51
<211> 820
<212> PRT
<213> Escherichia coli

<400> 51

Met Arg Val Leu Lys Phe Gly Gly Thr Ser Val Ala Asn Ala Glu Arg
1 5 10 15

Phe Leu Arg Val Ala Asp Ile Leu Glu Ser Asn Ala Arg Gln Gly Gln
20 25 30

Val Ala Thr Val Leu Ser Ala Pro Ala Lys Ile Thr Asn His Leu Val
35 40 45

Ala Met Ile Glu Lys Thr Ile Ser Gly Gln Asp Ala Leu Pro Asn Ile
50 55 60

Ser Asp Ala Glu Arg Ile Phe Ala Glu Leu Leu Thr Gly Leu Ala Ala
65 70 75 80

Ala Gln Pro Gly Phe Pro Leu Ala Gln Leu Lys Thr Phe Val Asp Gln
85 90 95

Glu Phe Ala Gln Ile Lys His Val Leu His Gly Ile Ser Leu Leu Gly
100 105 110

Gln Cys Pro Asp Ser Ile Asn Ala Ala Leu Ile Cys Arg Gly Glu Lys
115 120 125

Met Ser Ile Ala Ile Met Ala Gly Val Leu Glu Ala Arg Gly His Asn
130 135 140

Val Thr Val Ile Asp Pro Val Glu Lys Leu Leu Ala Val Gly His Tyr
145 150 155 160

Leu Glu Ser Thr Val Asp Ile Ala Glu Ser Thr Arg Arg Ile Ala Ala
165 170 175

Ser Arg Ile Pro Ala Asp His Met Val Leu Met Ala Gly Phe Thr Ala
180 185 190

Gly Asn Glu Lys Gly Glu Leu Val Val Leu Gly Arg Asn Gly Ser Asp

195 200 205

Tyr Ser Ala Ala Val Leu Ala Ala Cys Leu Arg Ala Asp Cys Cys Glu
210 215 220

Ile Trp Thr Asp Val Asp Gly Val Tyr Thr Cys Asp Pro Arg Gln Val
225 230 235 240

Pro Asp Ala Arg Leu Leu Lys Ser Met Ser Tyr Gln Glu Ala Met Glu
245 250 255

Leu Ser Tyr Phe Gly Ala Lys Val Leu His Pro Arg Thr Ile Thr Pro
260 265 270

Ile Ala Gln Phe Gln Ile Pro Cys Leu Ile Lys Asn Thr Gly Asn Pro
275 280 285

Gln Ala Pro Gly Thr Leu Ile Gly Ala Ser Arg Asp Glu Asp Glu Leu
290 295 300

Pro Val Lys Gly Ile Ser Asn Leu Asn Asn Met Ala Met Phe Ser Val
305 310 315 320

Ser Gly Pro Gly Met Lys Gly Met Val Gly Met Ala Ala Arg Val Phe
325 330 335

Ala Ala Met Ser Arg Ala Arg Ile Ser Val Val Leu Ile Thr Gln Ser
340 345 350

Ser Ser Glu Tyr Ser Ile Ser Phe Cys Val Pro Gln Ser Asp Cys Val
355 360 365

Arg Ala Glu Arg Ala Met Gln Glu Glu Phe Tyr Leu Glu Leu Lys Glu
370 375 380

Gly Leu Leu Glu Pro Leu Ala Val Thr Glu Arg Leu Ala Ile Ile Ser
385 390 395 400

Val Val Gly Asp Gly Met Arg Thr Leu Arg Gly Ile Ser Ala Lys Phe
405 410 415

Phe Ala Ala Leu Ala Arg Ala Asn Ile Asn Ile Val Ala Ile Ala Gln
420 425 430

Gly Ser Ser Glu Arg Ser Ile Ser Val Val Val Asn Asn Asp Asp Ala
435 440 445

Thr Thr Gly Val Arg Val Thr His Gln Met Leu Phe Asn Thr Asp Gln
    450                 455                 460

Val Ile Glu Val Phe Val Ile Gly Val Gly Gly Val Gly Gly Ala Leu
465                 470                 475                 480

Leu Glu Gln Leu Lys Arg Gln Gln Ser Trp Leu Lys Asn Lys His Ile
                485                 490                 495

Asp Leu Arg Val Cys Gly Val Ala Asn Ser Lys Ala Leu Leu Thr Asn
            500                 505                 510

Val His Gly Leu Asn Leu Glu Asn Trp Gln Glu Glu Leu Ala Gln Ala
        515                 520                 525

Lys Glu Pro Phe Asn Leu Gly Arg Leu Ile Arg Leu Val Lys Glu Tyr
    530                 535                 540

His Leu Leu Asn Pro Val Ile Val Asp Cys Thr Ser Ser Gln Ala Val
545                 550                 555                 560

Ala Asp Gln Tyr Ala Asp Phe Leu Arg Glu Gly Phe His Val Val Thr
                565                 570                 575

Pro Asn Lys Lys Ala Asn Thr Ser Ser Met Asp Tyr Tyr His Gln Leu
            580                 585                 590

Arg Tyr Ala Ala Glu Lys Ser Arg Arg Lys Phe Leu Tyr Asp Thr Asn
        595                 600                 605

Val Gly Ala Gly Leu Pro Val Ile Glu Asn Leu Gln Asn Leu Leu Asn
    610                 615                 620

Ala Gly Asp Glu Leu Met Lys Phe Ser Gly Ile Leu Ser Gly Ser Leu
625                 630                 635                 640

Ser Tyr Ile Phe Gly Lys Leu Asp Glu Gly Met Ser Phe Ser Glu Ala
                645                 650                 655

Thr Thr Leu Ala Arg Glu Met Gly Tyr Thr Glu Pro Asp Pro Arg Asp
            660                 665                 670

Asp Leu Ser Gly Met Asp Val Ala Arg Lys Leu Leu Ile Leu Ala Arg
        675                 680                 685

Glu Thr Gly Arg Glu Leu Glu Leu Ala Asp Ile Glu Ile Glu Pro Val
    690                 695                 700

```
Leu Pro Ala Glu Phe Asn Ala Glu Gly Asp Val Ala Ala Phe Met Ala
705                 710                 715                 720

Asn Leu Ser Gln Leu Asp Asp Leu Phe Ala Ala Arg Val Ala Lys Ala
                725                 730                 735

Arg Asp Glu Gly Lys Val Leu Arg Tyr Val Gly Asn Ile Asp Glu Asp
            740                 745                 750

Gly Val Cys Arg Val Lys Ile Ala Glu Val Asp Gly Asn Asp Pro Leu
        755                 760                 765

Phe Lys Val Lys Asn Gly Glu Asn Ala Leu Ala Phe Tyr Ser His Tyr
    770                 775                 780

Tyr Gln Pro Leu Pro Leu Val Leu Arg Gly Tyr Gly Ala Gly Asn Asp
785                 790                 795                 800

Val Thr Ala Ala Gly Val Phe Ala Asp Leu Leu Arg Thr Leu Ser Trp
                805                 810                 815

Lys Leu Gly Val
            820


<210>  52
<211>  1134
<212>  DNA
<213>  Corynebacterium glutamicum

<400>  52
atgcccaccc tcgcgccttc aggtcaactt gaaatccaag cgatcggtga tgtctccacc      60

gaagccggag caatcattac aaacgctgaa atcgcctatc accgctgggg tgaataccgc     120

gtagataaag aaggacgcag caatgtcgtt ctcatcgaac acgccctcac tggagattcc     180

aacgcagccg attggtgggc tgacttgctc ggtcccggca aagccatcaa cactgatatt     240

tactgcgtga tctgtaccaa cgtcatcggt ggttgcaacg gttccaccgg acctggctcc     300

atgcatccag atggaaattt ctggggtaat cgcttccccg ccacgtccat tcgtgatcag     360

gtaaacgccg aaaaacaatt cctcgacgca ctcggcatca ccacggtcgc cgcagtactt     420

ggtggttcca tgggtggtgc ccgcacccta gagtgggccg caatgtaccc agaaactgtt     480

ggcgcagctg ctgttcttgc agtttctgca cgcgccagcg cctggcaaat cggcattcaa     540

tccgcccaaa ttaaggcgat tgaaaacgac caccactggc acgaaggcaa ctactacgaa     600

tccggctgca acccagccac cggactcggc gccgcccgac gcatcgccca cctcacctac     660

cgtggcgaac tagaaatcga cgaacgcttc ggcaccaaag cccaaaagaa cgaaaaccca     720
```

ctcggtccct accgcaagcc cgaccagcgc ttcgccgtgg aatcctactt ggactaccaa 780

gcagacaagc tagtacagcg tttcgacgcc ggctcctacg tcttgctcac cgacgccctc 840

aaccgccacg acattggtcg cgaccgcgga ggcctcaaca aggcactcga atccatcaaa 900

gttccagtcc ttgtcgcagg cgtagatacc gatattttgt acccctacca ccagcaagaa 960

cacctctcca gaaacctggg aaatctactg gcaatggcaa aaatcgtatc cctgtcggc 1020

cacgatgctt tcctcaccga aagccgccaa atggatcgca tcgtgaggaa cttcttcagc 1080

ctcatctccc cagacgaaga caacccttcg acctacatcg agttctacat ctaa 1134

<210> 53
<211> 167
<212> DNA
<213> Artificial Sequence

<220>
<223> tac promoter

<400> 53
atgttttacc tcctgttaaa caaaattatt tctagaggga aaccgttgtg gaattgtgag 60

cgctcacaat tccacatcca cacattatac gagccgatga ttaattgtca acagcgtcga 120

tcactgtgca tgaagctcgt aattgttatc cgctcacaat tggatcc 167

<210> 54
<211> 43
<212> DNA
<213> Artificial Sequence

<220>
<223> primer_metX_Cg

<400> 54
ggatctagga accaaggaga gtggcatgcc caccctcgcg cct 43

<210> 55
<211> 34
<212> DNA
<213> Artificial Sequence

<220>
<223> primer_metX_Cg

<400> 55
caattggatc cgtttatccg gagggttgcc tgtg 34

<210> 56
<211> 38
<212> DNA
<213> Artificial Sequence

<220>
<223> primer_tac promotor

<400> 56
accctccgga taaacggatc caattgtgag cggataac 38

<210> 57
<211> 31
<212> DNA
<213> Artificial Sequence

<220>
<223> primer_tac promotor

<400> 57
acactcgcat atgttttacc tcctgttaaa c 31

<210> 58
<211> 35
<212> DNA
<213> Artificial Sequence

<220>
<223> primer_thrA part 1

<400> 58
caggaggtaa aacatatgcg agtgttgaag ttcgg 35

<210> 59
<211> 25
<212> DNA
<213> Artificial Sequence

<220>
<223> primer_thrA part 1

<400> 59
gtaatcagca ccacgaaaat acggg 25

<210> 60
<211> 25
<212> DNA
<213> Artificial Sequence

<220>
<223> primer_thrA part 2

<400> 60
cccgtatttt cgtggtgctg attac 25

<210> 61
<211> 50
<212> DNA
<213> Artificial Sequence

<220>
<223> primer_thrA part 2

<400> 61
ggtgcgccag gagagttgtt gatttatcag actcctaact tccatgagag 50

<210> 62
<211> 7052
<212> DNA
<213> Artificial Sequence

<220>
<223> plasmid_pJ281_alaT_C.gl._TA_C.v.(Ct)

<400> 62
gtataggaac ttctgaagtg gggggatccg gccggcccaa aaaggccggg aaatacccag 60
cctcgctttg taacggagta gagacgaaag tgattgcgcc tacccggata ttatcgtgag 120
gatgcgtcat cgccattaat tcactgatca gtgataagct gtcaaacatg agaattaatt 180
ccggcgatcc gtcgacttgc agcaattccc gaggctgtag ccgacgatgg tgcgccagga 240
gagttgttga tctagattat taggcgaggc cacgcgcttt gagggtctgt tcaaattctt 300
cgaggcaacg ttccgccacg gcgagcattt catccacttc cgcacgggtc ataacgagcg 360
gcggtgcaga cacaatgtga tcgccgcacg cacgcataat gaggttgttg cgaaaaaaaa 420
tatcgcggca gagggtgcca atttcgccaa aatccggaaa gagttcacgt ttcgctttgt 480
ttttcacgag ggtaaacgcc tgcaccatgc ccacgccacg cacatcatcc acatgttcaa 540
aacggctaaa ggtttcacgc caacgtttct gcatatacgg gccaatatca tctttcacac 600
gctgcacaat gccttcatca cggagcgccg caacattcgc atgcgccacc gccgcacaca 660
ccggatggcc gctataggta aagccatggt taaaatcacc gcccgcaatg agaccttccg 720
caacacgttt gcccacaaac accgcgccaa tcgggagata gccgctgctg aggcctttcg 780
ccgcggtaaa gaggtccggc tgaaagccaa aatgctgatg gccaaaccat tcgccggtac 840
ggccaaagcc gcaaatcact tcatccgcaa cgaggagcac atcatatttg cggcaaatac 900
gttcaatttc cggccaatag gttgccggcg gaacaatcac accgcccgca ccctgaatcg 960
gttcgcccac aaacgccgcc actttatccg cgccgatttc gagaattttt tcttcgagcc 1020
aacgcgccgc aaccacgcca aattcatccg gggtcatatc tttgccatgt ttataccacc 1080
acggctgttc aatatgcgcc atgcccggaa tcgggagatc gccctgttca tgcatatatt 1140
tcatgccgcc gaggctcgca ccgccaatgg tgctgccgtg atagccgttc caacggccaa 1200
tgagggtttt tttttccggt ttgccctgca catcccaata acgacgcacc atacgaatca 1260
tggtatccac gctttcgctg ccgctgttgg tataaaacac acgatcaaaa cctgccgggg 1320
taacttcggc gaggaggctg ctgagttcca ccaccgccgg atgggtggtt ttaaagaagg 1380
tgttataaaa cgggagttct tccatctgac gacgcgccgc ttccgcaaaa tctttacggc 1440
catagcccac gttcacgcac cagaggcccg ccatgccatc aataattttg ttgccttcgc 1500
tatcccagag atacacgcct tcgccacggg tcatcacacg cgcacccgcc tgattgaggc 1560
tcgcggtatc ggtaaacgga tggagatgat gcgccgcatc gagttcacgc cactgagagg 1620

tggtacgctg tttctgcata ttatatctcc ttaaagcttt tactactgct tgtaagtgga 1680

caggaagtta cccaggcgct caattgcgtt ttccaactgg gatgcccatg gcagggtgac 1740

cactcggaag tgatcgtgat gtggccagtt gaagccagtg ccctgaacca tgaggatttt 1800

ctcggcacgg agaagatcca gcatgagttg ggtgtcgtcg tggatttcgt acacgttggg 1860

gtcgagcttg gggaacgcgt atagagctcc cattggtttc acacagctga cacctgggat 1920

ttcgttgagt ttcgtccatg ccatgttgcg ctgttccagg agtcggccgt gttcgccagt 1980

gaggtcgtag atggactggc gtccaccgag agctacctga atagcgtgct gagctgggac 2040

atttgggcag agtcgagtgc ctgcgaggag ttcgaggccc tcaataaatc cacgtgcgta 2100

ttgctttggt ccagtcaata ccatccagcc agctcggtat cctgcgacgc ggtatgcctt 2160

ggatagaccg ttgtatgtga tgcaaaggag atctggtgca agggttgcca ggctgatgtg 2220

ctcggcatca tcgtagagaa tgcggtcgta gatttcatcg gccaaaatca gcaggtcatg 2280

ctcgcgtgca atctcgacga tttgttccaa cacccggcgc gggtagacag ctcccgtggg 2340

gttgttgggg ttgatcacca caatagcttt ggttttctct gagattttgg acttgatgtc 2400

ttcgatggat gggttccagt catcttcctc atcacagagg tagtgcacag gcttaccacc 2460

agccagggag gttgcggcag tccacagtgg gtagtccggt gcggggataa gaacttcatc 2520

gccgtcgttg aggagtgctt gggtggtcat ggtgattagt tctgagacac cgttgcctaa 2580

gaacacatca tcaacatcga agtgggggaa tccgggcaca acttcgtagc gggtgaccac 2640

tgctcgccgg gccggaataa tgcctttgga ggtggaatac ccttgggaag ttggaaggtt 2700

ggcgatcatg tcacgcataa tcacgtcggg ggcatcgaat ccgaacacgg ctggatttcc 2760

cgtgttgagc tttaagatgt tatgcccatc aagctccatg cgttccgcct ccgcggccac 2820

cgggccacgg atctcgtaca gcacgtcctt catcttctcc gactgatcga agatgcggcg 2880

agttgttcgc cgagtgggac gcgctgcctg atccgcgccc acagccttgt tggcggtgtc 2940

ggtggtctta gaggttttgc gcttgtctgt agtcatatgc cactctcctt ggttcctaga 3000

tcctgtgtga aattgttatt gttatccgct cacaattcca cacattatac gagccggaag 3060

cataaagtgt caagcctggg gtgcctaatg agtgagctaa cttacattaa ttgcgttgcg 3120

ctcactgccc gcatatctat gagccgggct gaatgatcga ccgagacagg ccctgcgggg 3180

ctgcaggccg gccggatcca aaatgaagtg aagttcctat acttactaga gaataggaac 3240

ttctatagtg agtcgaataa gggcgacaca aaatttattc taaatgcata ataaatactg 3300

ataacatctt atagtttgta ttatattttg tattatcgtt gacatgtata attttgatat 3360

caaaaactga ttttcccttt attattttcg agatttattt tcttaattct ctttaacaaa 3420

ctagaaatat tgtatataca aaaaatcata aataatagat gaatagttta attataggtg 3480

ttcatcaatc gaaaaagcaa cgtatcttat ttaaagtgcg ttgctttttt ctcatttata 3540

aggttaaata attctcatat atcaagcaaa gtgacaggcg cccttaaata ttctgacaaa 3600

tgctctttcc ctaaactccc cccataaaaa aacccgccga agcgggtttt tacgttattt 3660

gcggattaac gattactcgt tatcagaacc gcccaggggg cccgagctta agactggccg 3720

tcgttttaca acacagaaag agtttgtaga aacgcaaaaa ggccatccgt caggggcctt 3780

ctgcttagtt tgatgcctgg cagttcccta ctctcgcctt ccgcttcctc gctcactgac 3840

tcgctgcgct cggtcgttcg gctgcggcga gcggtatcag ctcactcaaa ggcggtaata 3900

cggttatcca cagaatcagg ggataacgca ggaaagaaca tgtgagcaaa aggccagcaa 3960

aaggccagga accgtaaaaa ggccgcgttg ctggcgtttt tccataggct ccgcccccct 4020

gacgagcatc acaaaaatcg acgctcaagt cagaggtggc gaaacccgac aggactataa 4080

agataccagg cgtttccccc tggaagctcc ctcgtgcgct ctcctgttcc gaccctgccg 4140

cttaccggat acctgtccgc ctttctccct tcgggaagcg tggcgctttc tcatagctca 4200

cgctgtaggt atctcagttc ggtgtaggtc gttcgctcca agctgggctg tgtgcacgaa 4260

ccccccgttc agcccgaccg ctgcgcctta tccggtaact atcgtcttga gtccaacccg 4320

gtaagacacg acttatcgcc actggcagca gccactggta acaggattag cagagcgagg 4380

tatgtaggcg gtgctacaga gttcttgaag tggtgggcta actacggcta cactagaaga 4440

acagtatttg gtatctgcgc tctgctgaag ccagttacct tcggaaaaag agttggtagc 4500

tcttgatccg gcaaacaaac caccgctggt agcggtggtt tttttgtttg caagcagcag 4560

attacgcgca gaaaaaaagg atctcaagaa gatcctttga tcttttctac ggggtctgac 4620

gctcagtgga acgacgcgcg cgtaactcac gttaagggat tttggtcatg agtcactgcc 4680

cgctttccag tcgggaaacc tgtcgtgcca gctgcattaa tgaatcggcc aacgcgcggg 4740

gagaggcggt ttgcgtattg ggcgccaggg tggtttttct tttcaccagt gagactggca 4800

acagctgatt gcccttcacc gcctggccct gagagagttg cagcaagcgg tccacgctgg 4860

tttgccccag caggcgaaaa tcctgtttga tggtggttaa cggcgggata taacatgagc 4920

tatcttcggt atcgtcgtat cccactaccg agatatccgc accaacgcgc agcccggact 4980

cggtaatggc gcgcattgcg cccagcgcca tctgatcgtt ggcaaccagc atcgcagtgg 5040

gaacgatgcc ctcattcagc atttgcatgg tttgttgaaa accggacatg gcactccagt 5100

cgccttcccg ttccgctatc ggctgaattt gattgcgagt gagatattta tgccagccag 5160

ccagacgcag acgcgccgag acagaactta atgggcccgc taacagcgcg atttgctggt 5220

gacccaatgc gaccagatgc tccacgccca gtcgcgtacc gtcctcatgg gagaaaataa 5280

tactgttgat gggtgtctgg tcagagacat caagaaataa cgccggaaca ttagtgcagg 5340

cagcttccac agcaatggca tcctggtcat ccagcggata gttaatgatc agcccactga 5400

cgcgttgcgc gagaagattg tgcaccgccg ctttacaggc ttcgacgccg cttcgttcta 5460

ccatcgacac caccacgctg gcacccagtt gatcggcgcg agatttaatc gccgcgacaa 5520

tttgcgacgg cgcgtgcagg gccagactgg aggtggcaac gccaatcagc aacgactgtt 5580

tgcccgccag ttgttgtgcc acgcggttgg gaatgtaatt cagctccgcc atcgccgctt 5640

ccactttttc ccgcgttttc gcagaaacgt ggctggcctg gttcaccacg cgggaaacgg 5700

tctgataaga gacaccggca tactctgcga catcgtataa cgttactggt ttcatattca 5760

ccaccctgaa ttgactctct tccgggcgct atcatgccat accgcgaaag gttttgcgcc 5820

attcgatggc gcgccgcttt tagaaaaact catcgagcat caaatgaaac tgcaatttat 5880

tcatatcagg attatcaata ccatattttt gaaaaagccg tttctgtaat gaaggagaaa 5940

actcaccgag gcagttccat aggatggcaa gatcctggta tcggtctgcg attccgactc 6000

gtccaacatc aatacaacct attaatttcc cctcgtcaaa aataaggtta tcaagtgaga 6060

aatcaccatg agtgacgact gaatccggtg agaatggcaa aagtttatgc atttctttcc 6120

agacttgttc aacaggccag ccattacgct cgtcatcaaa atcactcgca tcaaccaaac 6180

cgttattcat tcgtgattgc gcctgagcga ggcgaaatac gcgatcgctg ttaaaaggac 6240

aattacaaac aggaatcgag tgcaaccggc gcaggaacac tgccagcgca tcaacaatat 6300

tttcacctga atcaggatat tcttctaata cctggaacgc tgtttttccg gggatcgcag 6360

tggtgagtaa ccatgcatca tcaggagtac ggataaaatg cttgatggtc ggaagtggca 6420

taaattccgt cagccagttt agtctgacca tctcatctgt aacatcattg gcaacgctac 6480

ctttgccatg tttcagaaac aactctggcg catcgggctt cccatacaag cgatagattg 6540

tcgcacctga ttgcccgaca ttatcgcgag cccatttata cccatataaa tcagcatcca 6600

tgttggaatt taatcgcggc ctcgacgttt cccgttgaat atggctcata ttcttccttt 6660

ttcaatatta ttgaagcatt tatcagggtt attgtctcat gagcggatac atatttgaat 6720

gtatttagaa aaataaacaa ataggggtca gtgttacaac caattaacca attctgaaca 6780

ttatcgcgag cccatttata cctgaatatg gctcataaca ccccttgttt gcctggcggc 6840

agtagcgcgg tggtcccacc tgaccccatg ccgaactcag aagtgaaacg ccgtagcgcc 6900

gatggtagtg tggggactcc ccatgcgaga gtagggaact gccaggcatc aaataaaacg 6960

aaaggctcag tcgaaagact gggcctttcg cccgggctaa ttagggggtg tcgcccttat 7020

tcgactctat agtgaagttc ctattctcta gt 7052

<210> 63
<211> 8155
<212> DNA
<213> Artificial Sequence

<220>
<223> plasmid_pJAG-4-48

<400> 63
actagagaat aggaacttca ctatagagtc gaataagggc gacacccccct aattagcccg 60

ggcgaaaggc ccagtctttc gactgagcct ttcgttttat ttgatgcctg gcagttccct 120

actctcgcat ggggagtccc cacactacca tcggcgctac ggcgtttcac ttctgagttc 180

ggcatggggt caggtgggac caccgcgcta ctgccgccag gcaaacaagg ggtgttatga 240

gccatattca ggtataaatg ggctcgcgat aatgttcaga attggttaat tggttgtaac 300

actgacccct atttgtttat ttttctaaat acattcaaat atgtatccgc tcatgagaca 360

ataaccctga taaatgcttc aataatattg aaaaaggaag aatatgagcc atattcaacg 420

ggaaacgtcg aggccgcgat taaattccaa catggatgct gatttatatg ggtataaatg 480

ggctcgcgat aatgtcgggc aatcaggtgc gacaatctat cgcttgtatg ggaagcccga 540

tgcgccagag ttgtttctga aacatggcaa aggtagcgtt gccaatgatg ttacagatga 600

gatggtcaga ctaaactggc tgacggaatt tatgccactt ccgaccatca agcattttat 660

ccgtactcct gatgatgcat ggttactcac cactgcgatc cccggaaaaa cagcgttcca 720

ggtattagaa gaatatcctg attcaggtga aaatattgtt gatgcgctgg cagtgttcct 780

gcgccggttg cactcgattc ctgtttgtaa ttgtcctttt aacagcgatc gcgtatttcg 840

cctcgctcag gcgcaatcac gaatgaataa cggtttggtt gatgcgagtg attttgatga 900

cgagcgtaat ggctggcctg ttgaacaagt ctggaaagaa atgcataaac ttttgccatt 960

ctcaccggat tcagtcgtca ctcatggtga tttctcactt gataacctta tttttgacga 1020

ggggaaatta ataggttgta ttgatgttgg acgagtcgga atcgcagacc gataccagga 1080

tcttgccatc ctatggaact gcctcggtga gttttctcct tcattacaga aacggctttt 1140

tcaaaaatat ggtattgata atcctgatat gaataaattg cagtttcatt tgatgctcga 1200

tgagtttttc taaaagcggc gcgccatcga atggcgcaaa acctttcgcg gtatggcatg 1260

atagcgcccg gaagagagtc aattcagggt ggtgaatatg aaaccagtaa cgttatacga 1320

tgtcgcagag tatgccggtg tctcttatca gaccgtttcc cgcgtggtga accaggccag 1380

ccacgtttct gcgaaaacgc gggaaaaagt ggaagcggcg atggcggagc tgaattacat 1440

tcccaaccgc gtggcacaac aactggcggg caaacagtcg ttgctgattg gcgttgccac 1500

ctccagtctg gccctgcacg cgccgtcgca aattgtcgcg gcgattaaat ctcgcgccga 1560

tcaactgggt gccagcgtgg tggtgtcgat ggtagaacga agcggcgtcg aagcctgtaa 1620

agcggcggtg cacaatcttc tcgcgcaacg cgtcagtggg ctgatcatta actatccgct 1680

ggatgaccag gatgccattg ctgtggaagc tgcctgcact aatgttccgg cgttatttct 1740

tgatgtctct gaccagacac ccatcaacag tattattttc tcccatgagg acggtacgcg 1800

actgggcgtg gagcatctgg tcgcattggg tcaccagcaa atcgcgctgt tagcgggccc 1860
attaagttct gtctcggcgc gtctgcgtct ggctggctgg cataaatatc tcactcgcaa 1920
tcaaattcag ccgatagcgg aacgggaagg cgactggagt gccatgtccg gttttcaaca 1980
aaccatgcaa atgctgaatg agggcatcgt tcccactgcg atgctggttg ccaacgatca 2040
gatggcgctg ggcgcaatgc gcgccattac cgagtccggg ctgcgcgttg gtgcggatat 2100
ctcggtagtg ggatacgacg ataccgaaga tagctcatgt tatatcccgc cgttaaccac 2160
catcaaacag gattttcgcc tgctggggca aaccagcgtg gaccgcttgc tgcaactctc 2220
tcagggccag gcggtgaagg gcaatcagct gttgccagtc tcactggtga aaagaaaaac 2280
caccctggcg cccaatacgc aaaccgcctc tccccgcgcg ttggccgatt cattaatgca 2340
gctggcacga caggtttccc gactggaaag cgggcagtga ctcatgacca aaatccctta 2400
acgtgagtta cgcgcgcgtc gttccactga gcgtcagacc ccgtagaaaa gatcaaagga 2460
tcttcttgag atcctttttt tctgcgcgta atctgctgct tgcaaacaaa aaaaccaccg 2520
ctaccagcgg tggtttgttt gccggatcaa gagctaccaa ctctttttcc gaaggtaact 2580
ggcttcagca gagcgcagat accaaatact gttcttctag tgtagccgta gttagcccac 2640
cacttcaaga actctgtagc accgcctaca tacctcgctc tgctaatcct gttaccagtg 2700
gctgctgcca gtggcgataa gtcgtgtctt accgggttgg actcaagacg atagttaccg 2760
gataaggcgc agcggtcggg ctgaacgggg ggttcgtgca cacagcccag cttggagcga 2820
acgacctaca ccgaactgag atacctacag cgtgagctat gagaaagcgc cacgcttccc 2880
gaagggagaa aggcggacag gtatccggta agcggcaggg tcggaacagg agagcgcacg 2940
agggagcttc cagggggaaa cgcctggtat ctttatagtc ctgtcgggtt tcgccacctc 3000
tgacttgagc gtcgattttt gtgatgctcg tcaggggggc ggagcctatg gaaaaacgcc 3060
agcaacgcgg cctttttacg gttcctggcc ttttgctggc cttttgctca catgttcttt 3120
cctgcgttat cccctgattc tgtggataac cgtattaccg cctttgagtg agctgatacc 3180
gctcgccgca gccgaacgac cgagcgcagc gagtcagtga gcgaggaagc ggaaggcgag 3240
agtagggaac tgccaggcat caaactaagc agaaggcccc tgacggatgg cctttttgcg 3300
tttctacaaa ctctttctgt gttgtaaaac gacggccagt cttaagctcg ggccccctgg 3360
gcggttctga taacgagtaa tcgttaatcc gcaaataacg taaaaacccg cttcggcggg 3420
tttttttatg gggggagttt agggaaagag catttgtcag aatatttaag ggcgcctgtc 3480
actttgcttg atatatgaga attatttaac cttataaatg agaaaaaagc aacgcacttt 3540
aaataagata cgttgctttt tcgattgatg aacacctata attaaactat tcatctatta 3600
tttatgattt tttgtatata caatatttct agtttgttaa agagaattaa gaaaataaat 3660

ctcgaaaata ataaagggaa aatcagtttt tgatatcaaa attatacatg tcaacgataa 3720
tacaaaatat aatacaaact ataagatgtt atcagtattt attatgcatt tagaataaat 3780
tttgtgtcgc ccttattcga ctcactatag aagttcctat tctctagtaa gtataggaac 3840
ttcacttcat tttggatccg gccggcctgc agccccgcag ggcctgtctc ggtcgatcat 3900
tcagcccggc tcatagatat gcgggcagtg agcgcaacgc aattaatgta agttagctca 3960
ctcattaggc accccaggct tgacacttta tgcttccggc tcgtataatg tgtggaattg 4020
tgagcggata acaataacaa tttcacacag gatctaggaa ccaaggagag tggcatgccc 4080
accctcgcgc cttcaggtca acttgaaatc caagcgatcg gtgatgtctc caccgaagcc 4140
ggagcaatca ttacaaacgc tgaaatcgcc tatcaccgct ggggtgaata ccgcgtagat 4200
aaagaaggac gcagcaatgt cgttctcatc gaacacgccc tcactggaga ttccaacgca 4260
gccgattggt gggctgactt gctcggtccc ggcaaagcca tcaacactga tatttactgc 4320
gtgatctgta ccaacgtcat cggtggttgc aacggttcca ccggacctgg ctccatgcat 4380
ccagatggaa atttctgggg taatcgcttc cccgccacgt ccattcgtga tcaggtaaac 4440
gccgaaaaac aattcctcga cgcactcggc atcaccacgg tcgccgcagt acttggtggt 4500
tccatgggtg gtgcccgcac cctagagtgg gccgcaatgt acccagaaac tgttggcgca 4560
gctgctgttc ttgcagtttc tgcacgcgcc agcgcctggc aaatcggcat tcaatccgcc 4620
caaattaagg cgattgaaaa cgaccaccac tggcacgaag gcaactacta cgaatccggc 4680
tgcaacccag ccaccggact cggcgccgcc cgacgcatcg cccacctcac ctaccgtggc 4740
gaactagaaa tcgacgaacg cttcggcacc aaagcccaaa agaacgaaaa cccactcggt 4800
ccctaccgca agcccgacca gcgcttcgcc gtggaatcct acttggacta ccaagcagac 4860
aagctagtac agcgtttcga cgccggctcc tacgtcttgc tcaccgacgc cctcaaccgc 4920
cacgacattg gtcgcgaccg cggaggcctc aacaaggcac tcgaatccat caaagttcca 4980
gtccttgtcg caggcgtaga taccgatatt ttgtacccct accaccagca agaacacctc 5040
tccagaaacc tgggaaatct actggcaatg gcaaaaatcg tatcccctgt cggccacgat 5100
gctttcctca ccgaaagccg ccaaatggat cgcatcgtga ggaacttctt cagcctcatc 5160
tccccagacg aagacaaccc ttcgacctac atcgagttct acatctaata ggtatttacg 5220
acaaatagac agggatctct aaacaactca caggcaaccc tccggataaa cggatccaat 5280
tgtgagcgga taacaattac gagcttcatg cacagtgatc gacgctgttg acaattaatc 5340
atcggctcgt ataatgtgtg gatgtggaat tgtgagcgct cacaattcca caacggtttc 5400
cctctagaaa taattttgtt taacaggagg taaaacatat gcgagtgttg aagttcggcg 5460
gtacatcagt ggcaaatgca gaacgttttc tgcgtgttgc cgatattctg gaaagcaatg 5520
ccaggcaggg gcaggtggcc accgtcctct ctgcccccgc caaaatcacc aaccacctgg 5580

tggcgatgat tgaaaaaacc attagcggcc aggatgcttt acccaatatc agcgatgccg 5640

aacgtatttt tgccgaactt ttgacgggac tcgccgccgc ccagccgggg ttcccgctgg 5700

cgcaattgaa aactttcgtc gatcaggaat ttgcccaaat aaaacatgtc ctgcatggca 5760

ttagtttgtt ggggcagtgc ccggatagca tcaacgctgc gctgatttgc cgtggcgaga 5820

aaatgtcgat cgccattatg gccggcgtat tagaagcgcg cggtcacaac gttactgtta 5880

tcgatccggt cgaaaaactg ctggcagtgg ggcattacct cgaatctacc gtcgatattg 5940

ctgagtccac ccgccgtatt gcggcaagcc gcattccggc tgatcacatg gtgctgatgg 6000

caggtttcac cgccggtaat gaaaaaggcg aactggtggt gcttggacgc aacggttccg 6060

actactctgc tgcggtgctg gctgcctgtt tacgcgccga ttgttgcgag atttggacgg 6120

acgttgacgg ggtctatacc tgcgacccgc gtcaggtgcc cgatgcgagg ttgttgaagt 6180

cgatgtccta ccaggaagcg atggagcttt cctacttcgg cgctaaagtt cttcaccccc 6240

gcaccattac ccccatcgcc cagttccaga tcccttgcct gattaaaaat accggaaatc 6300

ctcaagcacc aggtacgctc attggtgcca gccgtgatga agacgaatta ccggtcaagg 6360

gcatttccaa tctgaataac atggcaatgt tcagcgtttc tggtccgggg atgaaaggga 6420

tggtcggcat ggcggcgcgc gtctttgcag cgatgtcacg cgcccgtatt ttcgtggtgc 6480

tgattacgca atcatcttcc gaatacagca tcagtttctg cgttccacaa agcgactgtg 6540

tgcgagctga acgggcaatg caggaagagt tctacctgga actgaaagaa ggcttactgg 6600

agccgctggc agtgacggaa cggctggcca ttatctcggt ggtaggtgat ggtatgcgca 6660

ccttgcgtgg gatctcggcg aaattctttg ccgcactggc ccgcgccaat atcaacattg 6720

tcgccattgc tcagggatct tctgaacgct caatctctgt cgtggtaaat aacgatgatg 6780

cgaccactgg cgtgcgcgtt actcatcaga tgctgttcaa taccgatcag gttatcgaag 6840

tgtttgtgat tggcgtcggt ggcgttggcg gtgcgctgct ggagcaactg aagcgtcagc 6900

aaagctggct gaagaataaa catatcgact tacgtgtctg cggtgttgcc aactcgaagg 6960

ctctgctcac caatgtacat ggccttaatc tggaaaactg gcaggaagaa ctggcgcaag 7020

ccaaagagcc gtttaatctc gggcgcttaa ttcgcctcgt gaaagaatat catctgctga 7080

acccggtcat tgttgactgc acttccagcc aggcagtggc ggatcaatat gccgacttcc 7140

tgcgcgaagg tttccacgtt gtcacgccga acaaaaaggc caacacctcg tcgatggatt 7200

actaccatca gttgcgttat gcggcggaaa aatcgcggcg taaattcctc tatgacacca 7260

acgttggggc tggattaccg gttattgaga acctgcaaaa tctgctcaat gcaggtgatg 7320

aattgatgaa gttctccggc attctttctg gttcgctttc ttatatcttc ggcaagttag 7380

acgaaggcat gagtttctcc gaggcgacca cgctggcgcg ggaaatgggt tataccgaac 7440

cggacccgcg agatgatctt tctggtatgg atgtggcgcg taaactattg attctcgctc 7500

gtgaaacggg acgtgaactg gagctggcgg atattgaaat tgaacctgtg ctgcccgcag 7560

agtttaacgc cgagggtgat gttgccgctt ttatggcgaa tctgtcacaa ctcgacgatc 7620

tctttgccgc gcgcgtggcg aaggcccgtg atgaaggaaa agttttgcgc tatgttggca 7680

atattgatga agatggcgtc tgccgcgtga agattgccga agtggatggt aatgatccgc 7740

tgttcaaagt gaaaaatggc gaaaacgccc tggccttcta tagccactat tatcagccgc 7800

tgccgttggt actgcgcgga tatggtgcgg gcaatgacgt tacagctgcc ggtgtctttg 7860

ctgatctgct acgtaccctc tcatggaagt taggagtctg ataaatcaac aactctcctg 7920

gcgcaccatc gtcggctaca gcctcgggaa ttgctgcaag tcgacggatc gccggaatta 7980

attctcatgt ttgacagctt atcactgatc agtgaattaa tggcgatgac gcatcctcac 8040

gataatatcc gggtaggcgc aatcactttc gtctctactc cgttacaaag cgaggctggg 8100

tatttcccgg cctttttggg ccggccggat ccccccactt cagaagttcc tatac 8155

<210> 64
<211> 3089
<212> DNA
<213> Artificial Sequence

<220>
<223> DCPK induction system

<400> 64
tccaattttt attaaattag tcgctacgag atttaagacg taattttatg cctaactgag 60

aaagttaagc cgcccactct cactctcgac atcttaaacc tgagctaatc ggacgcttgc 120

gccaactaca cctacgggta gtttttgctc cgtcgtctgc tggaaaaaca cgagctggcc 180

gcaagcatgc caggtaccgc gagctactcg cgacggctga aagcaccgaa atgagcgagc 240

tatctggtcg attttgaccc ggtgcccgtc ttcaaaatcg gcgaaggccg aagtcggcca 300

gaaatagcgg cctacttcag accttcccta gtaaatattt tgcaccaccg atcatgccga 360

ctacacttaa gtgtagtttt aatatttaac accgtaacct atggtgaaaa tttccagtca 420

gctggcgcga gaatagcata atgaaaataa taataaataa tgatttcccg gtcgctaagg 480

tcggagcgga tcaaattacg actctagtaa gtgccaaagt tcatagttgc atatatcggc 540

caagattgag tatcgcggat ggagccgctc ccagagtatg cctttacaga gccccacctg 600

gatatgggaa aaccgttgct cttgcgttcg agtggctacg ccacagaaca gccggacgtc 660

ctgcagtgtg gctttcttta agagccagtt cttacagtga atttgatatc tgcgcagaga 720

ttattgagca gcttgaaact ttcgaaatgg taaaattcag ccgtgtgaga gagggtgtga 780

gcaagcctgc gctcttgcga gaccttgcat ctagtctttg gcagagcacc tcgaataacg 840

agatagaaac gctagtttgt ttggataata ttaatcatga cttagacttg ccgttgttgc 900

acgcacttat ggagtttatg ttaaatacac caaaaaatat caggtttgca gttgcaggca 960
atacaataaa agggttctcg cagcttaaac ttgcaggcgc tatgcgggag tacaccgaga 1020
aagacttggc ctttagcgca gaagaggcgg tggcgttagc ggaggcagag tctgttcttg 1080
gagttcctga agaacagata gagaccttgg tgcaagaagt tgaggggtgg cctgctcttg 1140
tagttttttt gttaaagcgt gagttgccgg ccaagcatat ttcagcagta gttgaagtag 1200
acaattactt tagggatgaa atatttgagg cgattcccga gcgctatcgt gtttttcttg 1260
caaattcttc attgctcgat ttcgtgacgc ctgatcaata caattatgta ttcaaatgcg 1320
tcaatggggt ctcatgtatt aagtatttaa gcactaatta catgttgctt cgccatgtga 1380
gcggtgagcc agcgcagttt acactgcatc cagtactgcg taattttcta cgagaaatta 1440
cttggactga aaatcctgct aaaagatcct acctgcttaa gcgtgcagct ttctggcatt 1500
ggcgtagagg tgaataccag tatgcaatac gaatatccct acgggcgaat gactgtcgct 1560
gggcagtcag catgtctgag agaataattt tagatttgtc atttcgtcag ggcgaaatag 1620
atgcgctgag acagtggctg ttagagctgc cgaagcaggc ctggcacaaa aaacccatag 1680
tgcttattag ttacgcgtgg gtattgtatt tcagtcagca aggcgcgcga gcagagaagt 1740
taattaaaga cctatcttca caatccgata aaaaaaataa atggcaagaa aaggaatggc 1800
tgcagcttgt gcttgcaata ggtaaagcaa cgaaagatga aatgctttcg agtgaggagc 1860
tctgtaataa gtggattagt ttatttgggg attcaaacgc agttggaaaa ggggccgcgc 1920
taacctgttt ggcttttatt tttgccagtg agtatagatt tgcagagttg gagaaggtgc 1980
tggctcaggc ccaagccgtg aataaatttg caaaacaaaa ttttgctttt ggttggctgt 2040
atgtcgcgag gtttcaacaa gccctagcaa gcggaaaaat gggctgggcg aggcagatta 2100
taactcaagc acgcacagac agtcgcgcgc agatgatgga atccgagttt acttcgaaaa 2160
tgtttgacgc tctagagctt gagttacatt atgaattgcg ctgcttggac acctcagaag 2220
aaaagctctc caaaatttta gagttcattt ccaatcacgg ggtgacagac gtgttttttt 2280
ccgtatgccg tgctgtgtca gcttggcggc ttggaaggag tgacctaaat ggctccattg 2340
agatattgga gtgggcgaag gcgcatgcgg ttgaaaaaaa tctaccaaga ttggaagtta 2400
tgagccaaat tgagatctat cagcgcttag tctgtcaagg cataacgggc ataaataatt 2460
taaaaactct tgaagatcat aagattttct ccggacagca ctcagccccc ctaaaagcac 2520
gcctgctgct tgttcaatca ctagtgcttt cccgagatcg gaactttcat agtgccgcgc 2580
acagagcgtt attggctatt cagcaagccc gtaaaattaa cgcgggccag ctggaagtcc 2640
gtggattatt gtgtttggcc ggagcgcagg caggtgccgg tgatttaaaa aaggctcagc 2700
ttaacattgt ttatgcagtg gagatagcaa aacagcttca atgctttcaa acagttcttg 2760

atgaagtatg tttaattgag cgaataatac cggcttcatg tgaagccttc acagcagtta 2820

atttagatca agcgattggg gcttttagtc ttccgcgaat agttgagatt ggaaagtccg 2880

cagagaataa agctgacgct ttattgacac ggaagcagat tgctgtcttg aggcttgtaa 2940

aagaggggtg ctcaaacaaa caaatagcaa caaatatgca tgtcaccgaa gatgctataa 3000

agtggcatat gaggaaaata tttgccacct tgaatgtagt gaatcgcacg caagcaacaa 3060

ttgaagctga gcgtcaagga attatctaa 3089

<210> 65
<211> 48
<212> DNA
<213> Artificial Sequence

<220>
<223> primer_BF_MD 27

<400> 65
cgtagcgact aatttaataa aaattggaag caatttgcca agtaatcc 48

<210> 66
<211> 33
<212> DNA
<213> Artificial Sequence

<220>
<223> primer_BF_MD 28

<400> 66
acctgagcac tacttagaaa cgaatcgcca cac 33

<210> 67
<211> 40
<212> DNA
<213> Artificial Sequence

<220>
<223> primer_BF_MD 29

<400> 67
attcgtttct aagtagtgct caggtggagg tggcccaaag 40

<210> 68
<211> 47
<212> DNA
<213> Artificial Sequence

<220>
<223> primer_BF_MD 30

<400> 68
gcagattgta ctgagagtgc accaaaaaag ccgccgcaca ctggagg 47

<210> 69
<211> 9451

<212> DNA
<213> Artificial Sequence

<220>
<223> plasmid_AH-p-125

<400> 69
gcatgatcgt gctcctgtcg ttgaggaccc ggctaggctg gcggggttgc cttactggtt 60

agcagaatga atcaccgata cgcgagcgaa cgtgaagcga ctgctgctgc aaaacgtctg 120

cgacctgagc aacaacatga atggtcttcg gtttccgtgt ttcgtaaagt ctggaaacgc 180

ggaagtcagc gccctgcacc attatgttcc ggatctgcat cgcaggatgc tgctggctac 240

cctgtggaac acctacatct gtattaacga agcgctggca ttgaccctga gtgatttttc 300

tctggtcccg ccgcatccat accgccagtt gtttaccctc acaacgttcc agtaaccggg 360

catgttcatc atcagtaacc cgtatcgtga gcatcctctc tcgtttcatc ggtatcatta 420

cccccatgaa cagaaatccc ccttacacgg aggcatcagt gaccaaacag gaaaaaaccg 480

cccttaacat ggcccgcttt atcagaagcc agacattaac gcttctggag aaactcaacg 540

agctggacgc ggatgaacag gcagacatct gtgaatcgct tcacgaccac gctgatgagc 600

tttaccgcag ctgcctcgcg cgtttcggtg atgacggtga aaacctctga cacatgcagc 660

tcccggagac ggtcacagct tgtctgtaag cggatgccgg gagcagacaa gcccgtcagg 720

gcgcgtcagc gggtgttggc gggtgtcggg gcgcagccat gacccagtca cgtagcgata 780

gcggagtgta tactggctta actatgcggc atcagagcag attgtactga gagtgcacca 840

aaaaagccgc cgcacactgg aggatgtgtg aggcagcttt tctaggactt aacgcttaga 900

tcagaacagt ccggttgggt ttggatcgta ggagaccaac aggttcttgg tctgctggta 960

gtggttcagc atcatgaggt ggttctcacg gccgatgccg gactccttgt atccaccgaa 1020

agcggagtgc gctgggtagt tgtggtactg gttgacccaa actcgaccag cctggattgc 1080

gcgacctgca cgataaatgg tgttttggtc acggctccag acaccagcgc cgaggccgta 1140

gttggtgtcg tttgcaatac ggatggcctc atcgaagtcg ctgaaggtag caacagaaag 1200

gactggtccg aagatttcct cgcggaagat cctcatgtcg ttggtgccgc ggaaaacggt 1260

tggctcaatg tagtaaccgt tctccatgcc atcaaccttg ttgaccttgc caccagtgag 1320

ggtttgagcg ccttcttctg ggccgatctt caggtaggag gagatcttgt ccatctgctc 1380

ctgggacgcc tgagcaccca tcatggtttc agtatcaagt gggttaccca gcttgatgtt 1440

ctgaactcgc ttcacgccaa gctcgaggaa ttcatcagcg atggactcat gaacaagtgc 1500

acgggaagga caggtacaaa cttcaccctg attgagggcg aacatcgcga agccttcaac 1560

tgccttctct gcgaaggcgt catcctgtga cagaacatcg gagaagaaga tggatgggga 1620

cttaccgccg agctccaggg tgacaggaat gatcttgtcg gatgcagcgc ggttgatcag 1680

cttgccgacc tcggtggaac cggtgaaagc aatcttgcca atccgattag agccggacag 1740
tgcagcgcct gcttcaccgc cgagtccgtt gacgatgttg aggacgccct ctgggatgag 1800
atcgccgatg atgttaatca gatacaaaat ggatgctggg gtctgctcag ctggcttcat 1860
gacgatcgcg ttacctgcag caagtgccgg tgcgagcttc caggtagcca tgaggattgg 1920
gaagttccaa ggaatgatct gaccaacaac accgattggc tcgttgaagt ggtaagcaac 1980
agtgttgtgg tcgatctgtg aggaacgatc ttcctgagca cggatcgcgc cagcaaagta 2040
gcggaagtgg tcgattgcca gtgggatatc tgcagcaaga gtctcacgga ctgccttgcc 2100
gttctcccag gtttctgcaa ctgcgatttc ttccaggtgc tcttccatgc ggtccgcaat 2160
gcggtgcagg atcagagcac gttcagcgac agaagtcttg ccccacgcat cagcggctgc 2220
atgtgcagca tccagtgcaa gctccacgtc cgctgcggtg ccacgtgcga cctcacagaa 2280
aacttcacca gtgacaggtg aaatgttctc aaggtactgg ccctctaccg gtggaaccca 2340
cttgccacca atgtagttct cgtagcgctt ttcatagtta acgatcgagc cttcggttcc 2400
tggatttgcg tagacagtca ttgggtctcc tttgggccac ctccacctga gcactactta 2460
gaaacgaatc gccacacgac catcgatctt gccgtttcgc atgcggtcaa gcacaccatt 2520
gacctcatcg agggagcact cactcacggt tggcttgatt agtccgcgtg caaagaaatc 2580
gagcgcttcg gccaagtctt ggcgggttcc cacgagggat ccacggatgg tcaggccctt 2640
gaatacgatg ttgaacacgg atgctgggaa ctctcccggt ggcagaccgt tgaacacaat 2700
tgttcctgca cgtcgagcca tatccagtgc ctggccgaat gctgcctcgt gaactgcagt 2760
cacaagcacg ccgtgtgcgc caccgttggt gtacttctgt acagcttcgc ctgaatcttc 2820
attacgcgca ttcacggtaa attccgcacc gtgcttacgg gcaagttcca gcttgtcatc 2880
ggcaatatct accgcaatga cacgcatgcc catcgccgct gcgtattgga ctgcgatgtg 2940
gccaagtccg ccgacaccgg agatcaccat gaattggccc gggcgggttt cagagacttt 3000
gagtgccttg tagacagtca cgcctgcaca cagaattggt gctgcttcga ggtagtccac 3060
gccgtctggg atgcgagcgg cgtaacgggt atccaccagc atgtactggc cgaaggatcc 3120
attttgggtg tagccaccat actcagcttc gttgcactga gtttccctgc cggtgatgca 3180
gtattcgcag gtgccacacg ctgaccagag ccacgcattg ccgacaatat cgccgacctt 3240
cacatcgtgt tcacctggtc cgagctcaac aacttcacct acaccttcgt gtcctggtac 3300
gaatggtggt tccggcttta ctggccaatc gccctccaag gcgtggaggt cggtgtggca 3360
gatgccggag gtgagtacct tcaccaatgc ctggtgtggc cctggctttg gaaggtcaat 3420
atccttcacg gtcacgtcat gaccgaattt ttcaacaaca gcagcggtaa attcttgggg 3480
tgcagcagtg gtcataaaac tcactccttc tcgcttggat tacttggcaa attgcttcca 3540
atttttatta aattagtcgc tacgagattt aagacgtaat tttatgccta actgagaaag 3600

ttaagccgcc cactctcact ctcgacatct taaacctgag ctaatcggac gcttgcgcca 3660

actacaccta cgggtagttt ttgctccgtc gtctgctgga aaaacacgag ctggccgcaa 3720

gcatgccagg taccgcgagc tactcgcgac ggctgaaagc accgaaatga gcgagctatc 3780

tggtcgattt tgacccggtg cccgtcttca aaatcggcga aggccgaagt cggccagaaa 3840

tagcggccta cttcagacct tccctagtaa atattttgca ccaccgatca tgccgactac 3900

acttaagtgt agttttaata tttaacaccg taacctatgg tgaaaatttc cagtcagctg 3960

gcgcgagaat agcataatga aaataataat aaataatgat ttcccggtcg ctaaggtcgg 4020

agcggatcaa attacgactc tagtaagtgc caaagttcat agttgcatat atcggccaag 4080

attgagtatc gcggatggag ccgctcccag agtatgcctt tacagagccc cacctggata 4140

tgggaaaacc gttgctcttg cgttcgagtg gctacgccac agaacagccg gacgtcctgc 4200

agtgtggctt tctttaagag ccagttctta cagtgaattt gatatctgcg cagagattat 4260

tgagcagctt gaaactttcg aaatggtaaa attcagccgt gtgagagagg gtgtgagcaa 4320

gcctgcgctc ttgcgagacc ttgcatctag tctttggcag agcacctcga ataacgagat 4380

agaaacgcta gtttgtttgg ataatattaa tcatgactta gacttgccgt tgttgcacgc 4440

acttatggag tttatgttaa atacaccaaa aaatatcagg tttgcagttg caggcaatac 4500

aataaaaggg ttctcgcagc ttaaacttgc aggcgctatg cgggagtaca ccgagaaaga 4560

cttggccttt agcgcagaag aggcggtggc gttagcggag gcagagtctg ttcttggagt 4620

tcctgaagaa cagatagaga ccttggtgca agaagttgag gggtggcctg ctcttgtagt 4680

ttttttgtta aagcgtgagt tgccggccaa gcatatttca gcagtagttg aagtagacaa 4740

ttactttagg gatgaaatat ttgaggcgat tcccgagcgc tatcgtgttt ttcttgcaaa 4800

ttcttcattg ctcgatttcg tgacgcctga tcaatacaat tatgtattca aatgcgtcaa 4860

tggggtctca tgtattaagt atttaagcac taattacatg ttgcttcgcc atgtgagcgg 4920

tgagccagcg cagtttacac tgcatccagt actgcgtaat tttctacgag aaattacttg 4980

gactgaaaat cctgctaaaa gatcctacct gcttaagcgt gcagctttct ggcattggcg 5040

tagaggtgaa taccagtatg caatacgaat atccctacgg gcgaatgact gtcgctgggc 5100

agtcagcatg tctgagagaa taattttaga tttgtcattt cgtcagggcg aaatagatgc 5160

gctgagacag tggctgttag agctgccgaa gcaggcctgg cacaaaaaac ccatagtgct 5220

tattagttac gcgtgggtat tgtatttcag tcagcaaggc gcgcgagcag agaagttaat 5280

taaagaccta tcttcacaat ccgataaaaa aaataaatgg caagaaaagg aatggctgca 5340

gcttgtgctt gcaataggta aagcaacgaa agatgaaatg ctttcgagtg aggagctctg 5400

taataagtgg attagtttat ttggggattc aaacgcagtt ggaaaagggg ccgcgctaac 5460

ctgtttggct tttatttttg ccagtgagta tagatttgca gagttggaga aggtgctggc 5520
tcaggcccaa gccgtgaata aatttgcaaa acaaaatttt gcttttggtt ggctgtatgt 5580
cgcgaggttt caacaagccc tagcaagcgg aaaaatgggc tgggcgaggc agattataac 5640
tcaagcacgc acagacagtc gcgcgcagat gatggaatcc gagtttactt cgaaaatgtt 5700
tgacgctcta gagcttgagt tacattatga attgcgctgc ttggacacct cagaagaaaa 5760
gctctccaaa attttagagt tcatttccaa tcacggggtg acagacgtgt ttttttccgt 5820
atgccgtgct gtgtcagctt ggcggcttgg aaggagtgac ctaaatggct ccattgagat 5880
attggagtgg gcgaaggcgc atgcggttga aaaaaatcta ccaagattgg aagttatgag 5940
ccaaattgag atctatcagc gcttagtctg tcaaggcata acgggcataa ataatttaaa 6000
aactcttgaa gatcataaga ttttctccgg acagcactca gcccccctaa aagcacgcct 6060
gctgcttgtt caatcactag tgctttcccg agatcggaac tttcatagtg ccgcgcacag 6120
agcgttattg gctattcagc aagcccgtaa aattaacgcg ggccagctgg aagtccgtgg 6180
attattgtgt ttggccggag cgcaggcagg tgccggtgat ttaaaaaagg ctcagcttaa 6240
cattgtttat gcagtggaga tagcaaaaca gcttcaatgc tttcaaacag ttcttgatga 6300
agtatgttta attgagcgaa taataccggc ttcatgtgaa gccttcacag cagttaattt 6360
agatcaagcg attggggctt ttagtcttcc gcgaatagtt gagattggaa agtccgcaga 6420
gaataaagct gacgctttat tgacacggaa gcagattgct gtcttgaggc ttgtaaaaga 6480
ggggtgctca aacaaacaaa tagcaacaaa tatgcatgtc accgaagatg ctataaagtg 6540
gcatatgagg aaaatatttg ccaccttgaa tgtagtgaat cgcacgcaag caacaattga 6600
agctgagcgt caaggaatta tctaatgcag gtcgactcta gaggatcccc gggtaccgag 6660
ctcgaattca ctggccgtcg ttttacagcc aagcttggct gttttggcgg atgagagaag 6720
attttcagcc tgatacagat taaatcagaa cgcagaagcg gtctgataaa acagaatttg 6780
cctggcggca gtagcgcggt ggtcccacct gaccccatgc cgaactcaga agtgaaacgc 6840
cgtagcgccg atggtagtgt ggggtctccc catgcgagag tagggaactg ccaggcatca 6900
aataaaacga aaggctcagt cgaaagactg ggcctttcgt tttatctgtt gtttgtcggt 6960
gaacgctctc ctgagtagga caaatccgcc gggagcggat ttgaacgttg cgaagcaacg 7020
gcccggaggg tggcgggcag gacgcccgcc ataaactgcc aggcatcaaa ttaagcagaa 7080
ggccatcctg acggatggcc tttttgcgtt tctacaaact cttttgtgcg gtgtgaaata 7140
ccgcacagat gcgtaaggag aaaataccgc atcaggcgct cttccgcttc ctcgctcact 7200
gactcgctgc gctcggtcgt tcggctgcgg cgagcggtat cagctcactc aaaggcggta 7260
atacggttat ccacagaatc aggggataac gcaggaaaga acatgtgagc aaaaggccag 7320
caaaaggcca ggaaccgtaa aaaggccgcg ttgctggcgt ttttccatag gctccgcccc 7380

cctgacgagc atcacaaaaa tcgacgctca agtcagaggt ggcgaaaccc gacaggacta 7440

taaagatacc aggcgtttcc ccctggaagc tccctcgtgc gctctcctgt tccgaccctg 7500

ccgcttaccg gatacctgtc cgcctttctc ccttcgggaa gcgtggcgct ttctcatagc 7560

tcacgctgta ggtatctcag ttcggtgtag gtcgttcgct ccaagctggg ctgtgtgcac 7620

gaaccccccg ttcagcccga ccgctgcgcc ttatccggta actatcgtct tgagtccaac 7680

ccggtaagac acgacttatc gccactggca gcagccactg gtaacaggat tagcagagcg 7740

aggtatgtag gcggtgctac agagttcttg aagtggtggc ctaactacgg ctacactaga 7800

aggacagtat ttggtatctg cgctctgctg aagccagtta ccttcggaaa aagagttggt 7860

agctcttgat ccggcaaaca aaccaccgct ggtagcggtg gtttttttgt ttgcaagcag 7920

cagattacgc gcagaaaaaa aggatctcaa gaagatcctt tgatcttttc tacggggtct 7980

gacgctcagt ggaacgaaaa ctcacgttaa gggattttgg tcatgagatt atcaaaaagg 8040

atcttcacct agatcctttt aaattaaaaa tgaagtttta aatcaatcta aagtatatat 8100

gagtaaactt ggtctgacag ttaccaatgc ttaatcagtg aggcacctat ctcagcgatc 8160

tgtctatttc gttcatccat agttgcctga ctccccgtcg tgtagataac tacgatacgg 8220

gagggcttac catctggccc cagtgctgca atgataccgc gagacccacg ctcaccggct 8280

ccagatttat cagcaataaa ccagccagcc ggaagggccg agcgcagaag tggtcctgca 8340

actttatccg cctccatcca gtctattaat tgttgccggg aagctagagt aagtagttcg 8400

ccagttaata gtttgcgcaa cgttgttgcc attgctgcag gcatcgtggt gtcacgctcg 8460

tcgtttggta tggcttcatt cagctccggt tcccaacgat caaggcgagt tacatgatcc 8520

cccatgttgt gcaaaaaagc ggttagctcc ttcggtcctc cgatcgttgt cagaagtaag 8580

ttggccgcag tgttatcact catggttatg gcagcactgc ataattctct tactgtcatg 8640

ccatccgtaa gatgcttttc tgtgactggt gagtactcaa ccaagtcatt ctgagaatag 8700

tgtatgcggc gaccgagttg ctcttgcccg gcgtcaacac gggataatac cgcgccacat 8760

agcagaactt taaaagtgct catcattgga aaacgttctt cggggcgaaa actctcaagg 8820

atcttaccgc tgttgagatc cagttcgatg taacccactc gtgcacccaa ctgatcttca 8880

gcatctttta ctttcaccag cgtttctggg tgagcaaaaa caggaaggca aaatgccgca 8940

aaaaagggaa taagggcgac acggaaatgt tgaatactca tactcttcct ttttcaatat 9000

tattgaagca tttatcaggg ttattgtctc atgagcggat acatatttga atgtatttag 9060

aaaaataaac aaataggggt tccgcgcaca tttccccgaa aagtgccacc tgacgtctaa 9120

gaaaccatta ttatcatgac attaacctat aaaaataggc gtatcacgag gccctttcgt 9180

cttcaagaat tctcatgttt gacagcttat catcgataag ctttaatgcg gtagtttatc 9240

acagttaaat tgctaacgca gtcaggcacc gtgtatgaaa tctaacaatg cgctcatcgt 9300

catcctcggc accgtcaccc tggatgctgt aggcataggc ttggttatgc cggtactgcc 9360

gggcctcttg cgggatatcg tccattccga cagcatcgcc agtcactatg gcgtgctgct 9420

agcgctatat gcgttgatgc aatttctatg c 9451

<210> 70
<211> 5040
<212> DNA
<213> Artificial Sequence

<220>
<223> plasmid 4-52

<400> 70
ttctcatgtt tgacagctta tcatcgataa gctttaatgc ggtagtttat cacagttaaa 60

ttgctaacgc agtcaggcac cgtgtatgaa atctaacaat gcgctcatcg tcatcctcgg 120

caccgtcacc ctggatgctg taggcatagg cttggttatg ccggtactgc cgggcctctt 180

gcgggatatc gtccattccg acagcatcgc cagtcactat ggcgtgctgc tagcgctata 240

tgcgttgatg caatttctat gcgcacccgt tctcggagca ctgtccgacc gctttggccg 300

ccgcccagtc ctgctcgctt cgctacttgg agccactatc gactacgcga tcatggcgac 360

cacacccgtc ctgtggatcc tctacgccgg acgcatcgtg gccggcatca ccggcgccac 420

aggtgcggtt gctggcgcct atatcgccga catcaccgat ggggaagatc gggctcgcca 480

cttcgggctc atgagcgctt gtttcggcgt gggtatggtg gcaggccccg tggccggggg 540

actgttgggc gccatctcct tgcatgcacc attccttgcg gcggcggtgc tcaacggcct 600

caacctacta ctgggctgct tcctaatgca ggagtcgcat aagggagagc gtcgaccgat 660

gcccttgaga gccttcaacc cagtcagctc cttccggtgg gcgcggggca tgactatcgt 720

cgccgcactt atgactgtct tctttatcat gcaactcgta ggacaggtgc cggcagcgct 780

ctgggtcatt ttcggcgagg accgctttcg ctggagcgcg acgatgatcg gcctgtcgct 840

tgcggtattc ggaatcttgc acgccctcgc tcaagccttc gtcactggtc ccgccaccaa 900

acgtttcggc gagaagcagg ccattatcgc cggcatggcg gccgacgcgc tgggctacgt 960

cttgctggcg ttcgcgacgc gaggctggat ggccttcccc attatgattc ttctcgcttc 1020

cggcggcatc gggatgcccg cgttgcaggc catgctgtcc aggcaggtag atgacgacca 1080

tcagggacag cttcaaggat cgctcgcggc tcttaccagc ctaacttcga tcattggacc 1140

gctgatcgtc acggcgattt atgccgcctc ggcgagcaca tggaacgggt tggcatggat 1200

tgtaggcgcc gccctatacc ttgtctgcct ccccgcgttg cgtcgcggtg catggagccg 1260

ggccacctcg acctgaatgg aagccggcgg cacctcgcta acggattcac cactccaaga 1320

attggagcca atcaattctt gcggagaact gtgaatgcgc aaaccaaccc ttggcagaac 1380

atatccatcg cgtccgccat ctccagcagc cgcacgcggc gcatctcggg cagcgttggg 1440
tcctggccac gggtgcgcat gatcgtgctc ctgtcgttga ggacccggct aggctggcgg 1500
ggttgcctta ctggttagca gaatgaatca ccgatacgcg agcgaacgtg aagcgactgc 1560
tgctgcaaaa cgtctgcgac ctgagcaaca acatgaatgg tcttcggttt ccgtgtttcg 1620
taaagtctgg aaacgcggaa gtcccctacg tgctgctgaa gttgcccgca acagagagtg 1680
gaaccaaccg gtgataccac gatactatga ctgagagtca acgccatgag cggcctcatt 1740
tcttattctg agttacaaca gtccgcaccg ctgccggtag ctattgacta tccggctgca 1800
ctagccctgc gtcagatggc tctgatccaa ggcaaactgc caaaatatct gctggcaccg 1860
gaagtcagcg ccctgcacca ttatgttccg gatctgcatc gcaggatgct gctggctacc 1920
ctgtggaaca cctacatctg tattaacgaa gcgctggcat tgaccctgag tgatttttct 1980
ctggtgccgc cctatccctt tgtgcagctt gccacgctca aggggtttg aggtccaacc 2040
gtacgaaaac gtacggtaag aggaaaatta tcgtctgaaa aatcgattag tagacaagaa 2100
agtccgttaa gtgccaattt tcgattaaaa agacaccgtt ttgatggcgt tttccaatgt 2160
acattatgtt tcgatatatc agacagttac ttcactaacg tacgttttcg ttctattggc 2220
cttcagaccc catatcctta atgtccttta tttgctgggg ttatcagatc cccccgacac 2280
gtttaattaa tgctttctcc gccggagatc gacgcacagc gttctgtgct ctatgatgtt 2340
atttcttaat aatcatccag gtattctctt tatcaccata cgtagtgcga gtgtccacct 2400
taacgcaggg ctttccgtca cagcgcgata tgtcagccag cggggctttc ttttgccaga 2460
ccgcttccat cctctgcatt tcagcaatct ggctataccc gtcattcata aaccacgtaa 2520
atgccgtcac gcaggaagcc aggacgaaga atatcgtcag tacaagataa atcgcggatt 2580
tccacgtata gcgtgacatc tcacgacgca tttcatggat catcgctttc gccgtatcgg 2640
cagcctgatt cagcgcttct gtcgccggtt tctgctgtgc taatccggct tgtttcagtt 2700
ctttctcaac ctgagtgagc gcggaactca ccgatttcct gacggtgtca gtcatattac 2760
cggacgcgct gtccagctca cgaatgaccc tgctcagcgt ttcactttgc tgctgtaatt 2820
gtgatgaggc ggcctgaaac tgttctgtca gagaagtaac acgcttttcc agcgcctgat 2880
gatgcccgat aagggcggca atttgtttaa tttcgtcgct catacaaaat cctgcctatc 2940
gtgagaatga ccagccttta tccggcttct gtcgtatctg ttcggcgagt cgctgtcgtt 3000
ctttctcctg ctgacgctgt ttttccgcca gacgttcgcg ctctctctgc ctttccatct 3060
cctgatgtat cccctggaac tccgccatcg catcgttaac aagggactga agatcgattt 3120
cttcctgtat atccttcatg gcatcactga ccagtgcgtt cagcttgtca ggctcttttt 3180
caaaatcaaa cgttctgccg gaatgggatt cctgctcagg ctctgacttc agctcctgtt 3240

ttagcgtcag agtatccctc tcgctgaggg cttcccgtaa cgaggtagtc acgtcaatta 3300
cgctgtcacg ttcatcacgg gactgctgca cctgcctttc agcctccctg cgctcaagaa 3360
tggcctgtag ctgctcagta tcgaatcgct gaacctgacc cgcgcccaga tgccgctcag 3420
gctcacggtc aatgccctgc gccttcaggg aacgggaatc aacccggtca gcgtgctgat 3480
accgttcaag gtgcttattc tggaggtcag cccagcgttc cctctgggca acaaggtatt 3540
ctttgcgttc ggtcggtgtt tccccgaaac gtgccttttt tgcgccaccg cgctccggct 3600
ctttggtgtt agcccgttta aaatactgct cagggtcacg gtgaataccg tcattaatgc 3660
gttcagagaa catgatatgg gcgtggggct gctcgccacc ggctatcgct gctttcggat 3720
tatggatagc gaactgatag gcatggcggt cgccaatttc ctgttggaca aaatcgcgga 3780
caagctcaag acgttgttcg ggttttaact cacgcggcag ggcaatctcg atttcacggt 3840
aggtacagcc gttggcacgt tcagacgtgt cagcggcttt ccagaactcg gacggtttat 3900
gcgctgccca cgccggcata ttgccggact ccttgtgctc aaggtcggag tctttttcac 3960
gggcatactt tccctcacgc gcaatataat cggcatgagg agaggcactg ccttttccgc 4020
cggtttttac gctgagatga taggatgcca tcgtgtttta tcccgctgaa ggcgcgcacc 4080
gtttctgaac gaagtgaaga aacgtctaag tgcgccctga taaataaaag agttatcagg 4140
gattgtagtg ggatttgacc tcctctgcca tcactgagca taatcattcc gttagcattc 4200
aggaggtaaa cagcatgaat aaaagcgaaa aacaggaaca atgggcagca gaaagagtgc 4260
agtatattcg cggcttaaag tcgccgaatg agcaacagaa acttatgctg atactgacgg 4320
ataaagcaga taaaacagca caggatatca aaacgctgtc cctgctgatg aaggctgaac 4380
aggcagcaga gaaagcgcag gaagccagag cgaaagtcat gaacctgata caggcagaaa 4440
agcgagccga agccagagcc gcccgtaaag cccgtgacca tgctctgtac cagtctgccg 4500
gattgcttat cctggcgggt ctggttgaca gtaagacggg taagcctgtt gatgataccg 4560
ctgccttact gggtgcatta gccagtctga atgacctgtc acgggataat ccgaagtggt 4620
cagactggaa aatcagaggg caggaactgc tgaacagcaa aaagtcagat agcaccacat 4680
agcagacccg ccataaaacg ccctgagaag cccgtgacgg gcttttcttg tattatgggt 4740
agtttccttg catgaatcca taaaaggcgc ctgtagtgcc atttaccccc attcactgcc 4800
agagccgtga gcgcagcgaa ctgaatgtca cgaaaaagac agcgactcag gtgcctgatg 4860
gtcggagaca aaaggaatat tcagcgattt gcccgagctt gcgagggtgc tacttaagcc 4920
tttagggttt taaggtctgt tttgtagagg agcaaacagc gtttgcgaca tccttttgta 4980
atactgcgga actgactaaa gtagtgagtt atacacaggg ctgggatcta ttctttttat 5040

<210> 71
<211> 58

<212> DNA
<213> Artificial Sequence

<220>
<223> primer_o-HM-51

<400> 71
ctcggtaccc ggggatcctc tagagttatc agactcctaa cttccatgag agggtacg 58

<210> 72
<211> 43
<212> DNA
<213> Artificial Sequence

<220>
<223> primer_o-HM-52

<400> 72
gcgtcaagga attatctaat gcaggtcgcg gcatctccgg cca 43

<210> 73
<211> 14946
<212> DNA
<213> Artificial Sequence

<220>
<223> plasmid_HM-p-50

<400> 73
gcatgatcgt gctcctgtcg ttgaggaccc ggctaggctg gcggggttgc cttactggtt 60

agcagaatga atcaccgata cgcgagcgaa cgtgaagcga ctgctgctgc aaaacgtctg 120

cgacctgagc aacaacatga atggtcttcg gtttccgtgt ttcgtaaagt ctggaaacgc 180

ggaagtcagc gccctgcacc attatgttcc ggatctgcat cgcaggatgc tgctggctac 240

cctgtggaac acctacatct gtattaacga agcgctggca ttgaccctga gtgatttttc 300

tctggtcccg ccgcatccat accgccagtt gtttaccctc acaacgttcc agtaaccggg 360

catgttcatc atcagtaacc cgtatcgtga gcatcctctc tcgtttcatc ggtatcatta 420

cccccatgaa cagaaatccc ccttacacgg aggcatcagt gaccaaacag gaaaaaaccg 480

cccttaacat ggcccgcttt atcagaagcc agacattaac gcttctggag aaactcaacg 540

agctggacgc ggatgaacag gcagacatct gtgaatcgct tcacgaccac gctgatgagc 600

tttaccgcag ctgcctcgcg cgtttcggtg atgacggtga aaacctctga cacatgcagc 660

tcccggagac ggtcacagct tgtctgtaag cggatgccgg gagcagacaa gcccgtcagg 720

gcgcgtcagc gggtgttggc gggtgtcggg gcgcagccat gacccagtca cgtagcgata 780

gcggagtgta tactggctta actatgcggc atcagagcag attgtactga gagtgcacca 840

aaaaagccgc cgcacactgg aggatgtgtg aggcagcttt tctaggactt aacgcttaga 900

tcagaacagt ccggttgggt ttggatcgta ggagaccaac aggttcttgg tctgctggta 960

gtggttcagc atcatgaggt ggttctcacg gccgatgccg gactccttgt atccaccgaa 1020
agcggagtgc gctgggtagt tgtggtactg gttgacccaa actcgaccag cctggattgc 1080
gcgacctgca cgataaatgg tgttttggtc acggctccag acaccagcgc cgaggccgta 1140
gttggtgtcg tttgcaatac ggatggcctc atcgaagtcg ctgaaggtag caacagaaag 1200
gactggtccg aagatttcct cgcggaagat cctcatgtcg ttggtgccgc ggaaaacggt 1260
tggctcaatg tagtaaccgt tctccatgcc atcaaccttg ttgaccttgc caccagtgag 1320
ggtttgagcg ccttcttctg ggccgatctt caggtaggag gagatcttgt ccatctgctc 1380
ctgggacgcc tgagcaccca tcatggtttc agtatcaagt gggttaccca gcttgatgtt 1440
ctgaactcgc ttcacgccaa gctcgaggaa ttcatcagcg atggactcat gaacaagtgc 1500
acgggaagga caggtacaaa cttcaccctg attgagggcg aacatcgcga agccttcaac 1560
tgccttctct gcgaaggcgt catcctgtga cagaacatcg gagaagaaga tggatgggga 1620
cttaccgccg agctccaggg tgacaggaat gatcttgtcg gatgcagcgc ggttgatcag 1680
cttgccgacc tcggtggaac cggtgaaagc aatcttgcca atccgattag agccggacag 1740
tgcagcgcct gcttcaccgc cgagtccgtt gacgatgttg aggacgccct ctgggatgag 1800
atcgccgatg atgttaatca gatacaaaat ggatgctggg gtctgctcag ctggcttcat 1860
gacgatcgcg ttacctgcag caagtgccgg tgcgagcttc caggtagcca tgaggattgg 1920
gaagttccaa ggaatgatct gaccaacaac accgattggc tcgttgaagt ggtaagcaac 1980
agtgttgtgg tcgatctgtg aggaacgatc ttcctgagca cggatcgcgc cagcaaagta 2040
gcggaagtgg tcgattgcca gtgggatatc tgcagcaaga gtctcacgga ctgccttgcc 2100
gttctcccag gtttctgcaa ctgcgatttc ttccaggtgc tcttccatgc ggtccgcaat 2160
gcggtgcagg atcagagcac gttcagcgac agaagtcttg ccccacgcat cagcggctgc 2220
atgtgcagca tccagtgcaa gctccacgtc cgctgcggtg ccacgtgcga cctcacagaa 2280
aacttcacca gtgacaggtg aaatgttctc aaggtactgg ccctctaccg gtggaaccca 2340
cttgccacca atgtagttct cgtagcgctt ttcatagtta acgatcgagc cttcggttcc 2400
tggatttgcg tagacagtca ttgggtctcc tttgggccac ctccacctga gcactactta 2460
gaaacgaatc gccacacgac catcgatctt gccgtttcgc atgcggtcaa gcacaccatt 2520
gacctcatcg agggagcact cactcacggt tggcttgatt agtccgcgtg caaagaaatc 2580
gagcgcttcg gccaagtctt ggcgggttcc cacgagggat ccacggatgg tcaggccctt 2640
gaatacgatg ttgaacacgg atgctgggaa ctctcccggt ggcagaccgt tgaacacaat 2700
tgttcctgca cgtcgagcca tatccagtgc ctggccgaat gctgcctcgt gaactgcagt 2760
cacaagcacg ccgtgtgcgc caccgttggt gtacttctgt acagcttcgc ctgaatcttc 2820
attacgcgca ttcacggtaa attccgcacc gtgcttacgg gcaagttcca gcttgtcatc 2880

```
ggcaatatct accgcaatga cacgcatgcc catcgccgct gcgtattgga ctgcgatgtg     2940
gccaagtccg ccgacaccgg agatcaccat gaattggccc gggcgggttt cagagacttt     3000
gagtgccttg tagacagtca cgcctgcaca cagaattggt gctgcttcga ggtagtccac     3060
gccgtctggg atgcgagcgg cgtaacgggt atccaccagc atgtactggc cgaaggatcc     3120
attttgggtg tagccaccat actcagcttc gttgcactga gtttccctgc cggtgatgca     3180
gtattcgcag gtgccacacg ctgaccagag ccacgcattg ccgacaatat cgccgacctt     3240
cacatcgtgt tcacctggtc cgagctcaac aacttcacct acaccttcgt gtcctggtac     3300
gaatggtggt tccggcttta ctggccaatc gccctccaag gcgtggaggt cggtgtggca     3360
gatgccggag gtgagtacct tcaccaatgc ctggtgtggc cctggctttg gaaggtcaat     3420
atccttcacg gtcacgtcat gaccgaattt ttcaacaaca gcagcggtaa attcttgggg     3480
tgcagcagtg gtcataaaac tcactccttc tcgcttggat tacttggcaa attgcttcca     3540
atttttatta aattagtcgc tacgagattt aagacgtaat tttatgccta actgagaaag     3600
ttaagccgcc cactctcact ctcgacatct taaacctgag ctaatcggac gcttgcgcca     3660
actacaccta cgggtagttt ttgctccgtc gtctgctgga aaaacacgag ctggccgcaa     3720
gcatgccagg taccgcgagc tactcgcgac ggctgaaagc accgaaatga gcgagctatc     3780
tggtcgattt tgacccggtg cccgtcttca aaatcggcga aggccgaagt cggccagaaa     3840
tagcggccta cttcagacct tccctagtaa atattttgca ccaccgatca tgccgactac     3900
acttaagtgt agttttaata tttaacaccg taacctatgg tgaaaatttc cagtcagctg     3960
gcgcgagaat agcataatga aaataataat aaataatgat ttcccggtcg ctaaggtcgg     4020
agcggatcaa attacgactc tagtaagtgc caaagttcat agttgcatat atcggccaag     4080
attgagtatc gcggatggag ccgctcccag agtatgcctt tacagagccc cacctggata     4140
tgggaaaacc gttgctcttg cgttcgagtg gctacgccac agaacagccg gacgtcctgc     4200
agtgtggctt tctttaagag ccagttctta cagtgaattt gatatctgcg cagagattat     4260
tgagcagctt gaaactttcg aaatggtaaa attcagccgt gtgagagagg gtgtgagcaa     4320
gcctgcgctc ttgcgagacc ttgcatctag tctttggcag agcacctcga ataacgagat     4380
agaaacgcta gtttgtttgg ataatattaa tcatgactta gacttgccgt tgttgcacgc     4440
acttatggag tttatgttaa atacaccaaa aaatatcagg tttgcagttg caggcaatac     4500
aataaaaggg ttctcgcagc ttaaacttgc aggcgctatg cgggagtaca ccgagaaaga     4560
cttggccttt agcgcagaag aggcggtggc gttagcggag gcagagtctg ttcttggagt     4620
tcctgaagaa cagatagaga ccttggtgca agaagttgag ggtggcctg ctcttgtagt     4680
ttttttgtta aagcgtgagt tgccggccaa gcatatttca gcagtagttg aagtagacaa     4740
```

ttactttagg gatgaaatat ttgaggcgat tcccgagcgc tatcgtgttt ttcttgcaaa 4800
ttcttcattg ctcgatttcg tgacgcctga tcaatacaat tatgtattca aatgcgtcaa 4860
tggggtctca tgtattaagt atttaagcac taattacatg ttgcttcgcc atgtgagcgg 4920
tgagccagcg cagtttacac tgcatccagt actgcgtaat tttctacgag aaattacttg 4980
gactgaaaat cctgctaaaa gatcctacct gcttaagcgt gcagctttct ggcattggcg 5040
tagaggtgaa taccagtatg caatacgaat atccctacgg gcgaatgact gtcgctgggc 5100
agtcagcatg tctgagagaa taattttaga tttgtcattt cgtcagggcg aaatagatgc 5160
gctgagacag tggctgttag agctgccgaa gcaggcctgg cacaaaaaac ccatagtgct 5220
tattagttac gcgtgggtat tgtatttcag tcagcaaggc gcgcgagcag agaagttaat 5280
taaagaccta tcttcacaat ccgataaaaa aaataaatgg caagaaaagg aatggctgca 5340
gcttgtgctt gcaataggta aagcaacgaa agatgaaatg ctttcgagtg aggagctctg 5400
taataagtgg attagtttat ttggggattc aaacgcagtt ggaaaagggg ccgcgctaac 5460
ctgtttggct tttatttttg ccagtgagta tagatttgca gagttggaga aggtgctggc 5520
tcaggcccaa gccgtgaata aatttgcaaa acaaaatttt gcttttggtt ggctgtatgt 5580
cgcgaggttt caacaagccc tagcaagcgg aaaaatgggc tgggcgaggc agattataac 5640
tcaagcacgc acagacagtc gcgcgcagat gatggaatcc gagtttactt cgaaaatgtt 5700
tgacgctcta gagcttgagt tacattatga attgcgctgc ttggacacct cagaagaaaa 5760
gctctccaaa attttagagt tcatttccaa tcacggggtg acagacgtgt ttttttccgt 5820
atgccgtgct gtgtcagctt ggcggcttgg aaggagtgac ctaaatggct ccattgagat 5880
attggagtgg gcgaaggcgc atgcggttga aaaaaatcta ccaagattgg aagttatgag 5940
ccaaattgag atctatcagc gcttagtctg tcaaggcata acgggcataa ataatttaaa 6000
aactcttgaa gatcataaga ttttctccgg acagcactca gccccccctaa aagcacgcct 6060
gctgcttgtt caatcactag tgctttcccg agatcggaac tttcatagtg ccgcgcacag 6120
agcgttattg gctattcagc aagcccgtaa aattaacgcg ggccagctgg aagtccgtgg 6180
attattgtgt ttggccggag cgcaggcagg tgccggtgat ttaaaaaagg ctcagcttaa 6240
cattgtttat gcagtggaga tagcaaaaca gcttcaatgc tttcaaacag ttcttgatga 6300
agtatgttta attgagcgaa taataccggc ttcatgtgaa gccttcacag cagttaattt 6360
agatcaagcg attggggctt ttagtcttcc gcgaatagtt gagattggaa agtccgcaga 6420
gaataaagct gacgctttat tgacacggaa gcagattgct gtcttgaggc ttgtaaaaga 6480
ggggtgctca aacaaacaaa tagcaacaaa tatgcatgtc accgaagatg ctataaagtg 6540
gcatatgagg aaaatatttg ccaccttgaa tgtagtgaat cgcacgcaag caacaattga 6600
agctgagcgt caaggaatta tctaatgcag gtcgcggcat ctccggccag gcaaagggaa 6660

ttgcggattg acctagccga actccttggt aaatcgctgc gtgtttatgc gtccccgaga 6720

tagttgcggc cgactctgcg cttaatgggc tcggttggca tgaggggcgg ctggggtcac 6780

tcgaggagtt accttcggaa aaagagttgg tagctcttga tccggcaaac aaaccaccgc 6840

tggtagcggt ggtttttttg tttgcaagca gcagattacg cgcagaaaaa aaggatctca 6900

agaagatcct ttgatctttt ctacggggtc tgacgctcag tggaacgacg cgcgcgtaac 6960

tcacgttaag ggattttggt catgagtcac tgcccgcttt ccagtcggga aacctgtcgt 7020

gccagctgca ttaatgaatc ggccaacgcg cggggagagg cggtttgcgt attgggcgcc 7080

agggtggttt ttcttttcac cagtgagact ggcaacagct gattgccctt caccgcctgg 7140

ccctgagaga gttgcagcaa gcggtccacg ctggtttgcc ccagcaggcg aaaatcctgt 7200

ttgatggtgg ttaacggcgg gatataacat gagctatctt cggtatcgtc gtatcccact 7260

accgagatat ccgcaccaac gcgcagcccg gactcggtaa tggcgcgcat tgcgcccagc 7320

gccatctgat cgttggcaac cagcatcgca gtgggaacga tgccctcatt cagcatttgc 7380

atggtttgtt gaaaaccgga catggcactc cagtcgcctt cccgttccgc tatcggctga 7440

atttgattgc gagtgagata tttatgccag ccagccagac gcagacgcgc cgagacagaa 7500

cttaatgggc ccgctaacag cgcgatttgc tggtgaccca atgcgaccag atgctccacg 7560

cccagtcgcg taccgtcctc atgggagaaa ataatactgt tgatgggtgt ctggtcagag 7620

acatcaagaa ataacgccgg aacattagtg caggcagctt ccacagcaat ggcatcctgg 7680

tcatccagcg gatagttaat gatcagccca ctgacgcgtt gcgcgagaag attgtgcacc 7740

gccgctttac aggcttcgac gccgcttcgt tctaccatcg acaccaccac gctggcaccc 7800

agttgatcgg cgcgagattt aatcgccgcg acaatttgcg acggcgcgtg cagggccaga 7860

ctggaggtgg caacgccaat cagcaacgac tgtttgcccg ccagttgttg tgccacgcgg 7920

ttgggaatgt aattcagctc cgccatcgcc gcttccactt tttcccgcgt tttcgcagaa 7980

acgtggctgg cctggttcac cacgcgggaa acggtctgat aagagacacc ggcatactct 8040

gcgacatcgt ataacgttac tggtttcata ttcaccaccc tgaattgact ctcttccggg 8100

cgctatcatg ccataccgcg aaaggttttg cgccattcga tggcgcgccg gcagtgagcg 8160

caacgcaatt aatgtaagtt agctcactca ttaggcaccc caggcttgac actttatgct 8220

tccggctcgt ataatgtgtg gaattgtgag cggataacaa taacaatttc acacaggatc 8280

taggaaccaa ggagagtggc atgcccaccc tcgcgccttc aggtcaactt gaaatccaag 8340

cgatcggtga tgtctccacc gaagccggag caatcattac aaacgctgaa atcgcctatc 8400

accgctgggg tgaataccgc gtagataaag aaggacgcag caatgtcgtt ctcatcgaac 8460

acgccctcac tggagattcc aacgcagccg attggtgggc tgacttgctc ggtcccggca 8520

aagccatcaa cactgatatt tactgcgtga tctgtaccaa cgtcatcggt ggttgcaacg 8580
gttccaccgg acctggctcc atgcatccag atggaaattt ctggggtaat cgcttccccg 8640
ccacgtccat tcgtgatcag gtaaacgccg aaaaacaatt cctcgacgca ctcggcatca 8700
ccacggtcgc cgcagtactt ggtggttcca tgggtggtgc ccgcacccta gagtgggccg 8760
caatgtaccc agaaactgtt ggcgcagctg ctgttcttgc agtttctgca cgcgccagcg 8820
cctggcaaat cggcattcaa tccgcccaaa ttaaggcgat tgaaaacgac caccactggc 8880
acgaaggcaa ctactacgaa tccggctgca acccagccac cggactcggc gccgcccgac 8940
gcatcgccca cctcacctac cgtggcgaac tagaaatcga cgaacgcttc ggcaccaaag 9000
cccaaaagaa cgaaaaccca ctcggtccct accgcaagcc cgaccagcgc ttcgccgtgg 9060
aatcctactt ggactaccaa gcagacaagc tagtacagcg tttcgacgcc ggctcctacg 9120
tcttgctcac cgacgccctc aaccgccacg acattggtcg cgaccgcgga ggcctcaaca 9180
aggcactcga atccatcaaa gttccagtcc ttgtcgcagg cgtagatacc gatattttgt 9240
acccctacca ccagcaagaa cacctctcca gaaacctggg aaatctactg gcaatggcaa 9300
aaatcgtatc ccctgtcggc cacgatgctt tcctcaccga aagccgccaa atggatcgca 9360
tcgtgaggaa cttcttcagc ctcatctccc cagacgaaga caaccccttcg acctacatcg 9420
agttctacat ctaataggta tttacgacaa atagacaggg atctctaaac aactcacagg 9480
caaccctccg gataaacgga tccaattgtg agcggataac aattacgagc ttcatgcaca 9540
gtgatcgacg ctgttgacaa ttaatcatcg gctcgtataa tgtgtggatg tggaattgtg 9600
agcgctcaca attccacaac ggtttccctc tagaaataat tttgtttaac aggaggtaaa 9660
acatatgcga gtgttgaagt tcggcggtac atcagtggca aatgcagaac gttttctgcg 9720
tgttgccgat attctggaaa gcaatgccag gcaggggcag gtggccaccg tcctctctgc 9780
ccccgccaaa atcaccaacc acctggtggc gatgattgaa aaaaccatta gcggccagga 9840
tgctttaccc aatatcagcg atgccgaacg tatttttgcc gaacttttga cgggactcgc 9900
cgccgcccag ccggggttcc cgctggcgca attgaaaact ttcgtcgatc aggaatttgt 9960
ccaaataaaa catgtcctgc atggcattag tttgttgggg cagtgcccgg atagcatcaa 10020
cgctgcgctg atttgccgtg gcgagaaaat gtcgatcgcc attatggccg gcgtattaga 10080
agcgcgcggt cacaacgtta ctgttatcga tccggtcgaa aaactgctgg cagtggggca 10140
ttacctcgaa tctaccgtcg atattgctga gtccacccgc cgtattgcgg caagccgcat 10200
tccggctgat cacatggtgc tgatggcagg tttcaccgcc ggtaatgaaa aaggcgaact 10260
ggtggtgctt ggacgcaacg gttccgacta ctctgctgcg gtgctggctg cctgtttacg 10320
cgccgattgt tgcgagattt ggacggacgt tgacggggtc tatacctgcg acccgcgtca 10380
ggtgcccgat gcgaggttgt tgaagtcgat gtcctaccag gaagcgatgg agctttccta 10440

cttcggcgct aaagttcttc acccccgcac cattaccccc atcgcccagt tccagatccc 10500
ttgcctgatt aaaaataccg gaaatcctca agcaccaggt acgctcattg gtgccagccg 10560
tgatgaagac gaattaccgg tcaagggcat ttccaatctg aataacatgg caatgttcag 10620
cgtttctggt ccggggatga aagggatggt cggcatggcg gcgcgcgtct ttgcagcgat 10680
gtcacgcgcc cgtattttcg tggtgctgat tacgcaatca tcttccgaat acagcatcag 10740
tttctgcgtt ccacaaagcg actgtgtgcg agctgaacgg gcaatgcagg aagagttcta 10800
cctggaactg aaagaaggct tactggagcc gctggcagtg acggaacggc tggccattat 10860
ctcggtggta ggtgatggta tgcgcacctt gcgtgggatc tcggcgaaat tctttgccgc 10920
actggcccgc gccaatatca acattgtcgc cattgctcag ggatcttctg aacgctcaat 10980
ctctgtcgtg gtaaataacg atgatgcgac cactggcgtg cgcgttactc atcagatgct 11040
gttcaatacc gatcaggtta tcgaagtgtt tgtgattggc gtcggtggcg ttggcggtgc 11100
gctgctggag caactgaagc gtcagcaaag ctggctgaag aataaacata tcgacttacg 11160
tgtctgcggt gttgccaact cgaaggctct gctcaccaat gtacatggcc ttaatctgga 11220
aaactggcag gaagaactgg cgcaagccaa agagccgttt aatctcgggc gcttaattcg 11280
cctcgtgaaa gaatatcatc tgctgaaccc ggtcattgtt gactgcactt ccagccaggc 11340
agtggcggat caatatgccg acttcctgcg cgaaggtttc cacgttgtca cgccgaacaa 11400
aaaggccaac acctcgtcga tggattacta ccatcagttg cgttatgcgg cggaaaaatc 11460
gcggcgtaaa ttcctctatg acaccaacgt tggggctgga ttaccggtta ttgagaacct 11520
gcaaaatctg ctcaatgcag gtgatgaatt gatgaagttc tccggcattc tttctggttc 11580
gctttcttat atcttcggca agttagacga aggcatgagt ttctccgagg cgaccacgct 11640
ggcgcgggaa atgggttata ccgaaccgga cccgcgagat gatctttctg gtatggatgt 11700
ggcgcgtaaa ctattgattc tcgctcgtga aacgggacgt gaactggagc tggcggatat 11760
tgaaattgaa cctgtgctgc ccgcagagtt taacgccgag ggtgatgttg ccgcttttat 11820
ggcgaatctg tcacaactcg acgatctctt tgccgcgcgc gtggcgaagg cccgtgatga 11880
aggaaaagtt ttgcgctatg ttggcaatat tgatgaagat ggcgtctgcc gcgtgaagat 11940
tgccgaagtg gatggtaatg atccgctgtt caaagtgaaa aatggcgaaa acgccctggc 12000
cttctatagc cactattatc agccgctgcc gttggtactg cgcggatatg gtgcgggcaa 12060
tgacgttaca gctgccggtg tctttgctga tctgctacgt accctctcat ggaagttagg 12120
agtctgataa ctctagagga tccccgggta ccgagctcga attcactggc cgtcgtttta 12180
cagccaagct tggctgtttt ggcggatgag agaagatttt cagcctgata cagattaaat 12240
cagaacgcag aagcggtctg ataaaacaga atttgcctgg cggcagtagc gcggtggtcc 12300

cacctgaccc catgccgaac tcagaagtga aacgccgtag cgccgatggt agtgtggggt 12360
ctccccatgc gagagtaggg aactgccagg catcaaataa aacgaaaggc tcagtcgaaa 12420
gactgggcct ttcgttttat ctgttgtttg tcggtgaacg ctctcctgag taggacaaat 12480
ccgccgggag cggatttgaa cgttgcgaag caacggcccg gagggtggcg ggcaggacgc 12540
ccgccataaa ctgccaggca tcaaattaag cagaaggcca tcctgacgga tggccttttt 12600
gcgtttctac aaactctttt gtgcggtgtg aaataccgca cagatgcgta aggagaaaat 12660
accgcatcag gcgctcttcc gcttcctcgc tcactgactc gctgcgctcg gtcgttcggc 12720
tgcggcgagc ggtatcagct cactcaaagg cggtaatacg gttatccaca gaatcagggg 12780
ataacgcagg aaagaacatg tgagcaaaag gccagcaaaa ggccaggaac cgtaaaaagg 12840
ccgcgttgct ggcgtttttc cataggctcc gcccccctga cgagcatcac aaaaatcgac 12900
gctcaagtca gaggtggcga aacccgacag gactataaag ataccaggcg tttccccctg 12960
gaagctccct cgtgcgctct cctgttccga ccctgccgct taccggatac ctgtccgcct 13020
ttctcccttc gggaagcgtg gcgctttctc atagctcacg ctgtaggtat ctcagttcgg 13080
tgtaggtcgt tcgctccaag ctgggctgtg tgcacgaacc ccccgttcag cccgaccgct 13140
gcgccttatc cggtaactat cgtcttgagt ccaacccggt aagacacgac ttatcgccac 13200
tggcagcagc cactggtaac aggattagca gagcgaggta tgtaggcggt gctacagagt 13260
tcttgaagtg gtggcctaac tacggctaca ctagaaggac agtatttggt atctgcgctc 13320
tgctgaagcc agttaccttc ggaaaaagag ttggtagctc ttgatccggc aaacaaacca 13380
ccgctggtag cggtggtttt tttgtttgca agcagcagat tacgcgcaga aaaaaaggat 13440
ctcaagaaga tcctttgatc ttttctacgg ggtctgacgc tcagtggaac gaaaactcac 13500
gttaagggat tttggtcatg agattatcaa aaaggatctt cacctagatc cttttaaatt 13560
aaaaatgaag ttttaaatca atctaaagta tatatgagta aacttggtct gacagttacc 13620
aatgcttaat cagtgaggca cctatctcag cgatctgtct atttcgttca tccatagttg 13680
cctgactccc cgtcgtgtag ataactacga tacgggaggg cttaccatct ggccccagtg 13740
ctgcaatgat accgcgagac ccacgctcac cggctccaga tttatcagca ataaaccagc 13800
cagccggaag ggccgagcgc agaagtggtc ctgcaacttt atccgcctcc atccagtcta 13860
ttaattgttg ccgggaagct agagtaagta gttcgccagt taatagtttg cgcaacgttg 13920
ttgccattgc tgcaggcatc gtggtgtcac gctcgtcgtt tggtatggct tcattcagct 13980
ccggttccca acgatcaagg cgagttacat gatcccccat gttgtgcaaa aaagcggtta 14040
gctccttcgg tcctccgatc gttgtcagaa gtaagttggc cgcagtgtta tcactcatgg 14100
ttatggcagc actgcataat tctcttactg tcatgccatc cgtaagatgc ttttctgtga 14160
ctggtgagta ctcaaccaag tcattctgag aatagtgtat gcggcgaccg agttgctctt 14220

gcccggcgtc aacacgggat aataccgcgc cacatagcag aactttaaaa gtgctcatca 14280

ttggaaaacg ttcttcgggg cgaaaactct caaggatctt accgctgttg agatccagtt 14340

cgatgtaacc cactcgtgca cccaactgat cttcagcatc ttttactttc accagcgttt 14400

ctgggtgagc aaaaacagga aggcaaaatg ccgcaaaaaa gggaataagg gcgacacgga 14460

aatgttgaat actcatactc ttcctttttc aatattattg aagcatttat cagggttatt 14520

gtctcatgag cggatacata tttgaatgta tttagaaaaa taaacaaata ggggttccgc 14580

gcacatttcc ccgaaaagtg ccacctgacg tctaagaaac cattattatc atgacattaa 14640

cctataaaaa taggcgtatc acgaggccct ttcgtcttca agaattctca tgtttgacag 14700

cttatcatcg ataagcttta atgcggtagt ttatcacagt taaattgcta acgcagtcag 14760

gcaccgtgta tgaaatctaa caatgcgctc atcgtcatcc tcggcaccgt caccctggat 14820

gctgtaggca taggcttggt tatgccggta ctgccgggcc tcttgcggga tatcgtccat 14880

tccgacagca tcgccagtca ctatggcgtg ctgctagcgc tatatgcgtt gatgcaattt 14940

ctatgc 14946

<210> 74
<211> 4174
<212> DNA
<213> Artificial Sequence

<220>
<223> plasmid_HM-p-54

<400> 74
ttctcatgtt tgacagctta tcatcgatag gcagtgagcg caacgcaatt aatgtaagtt 60

agctcactca ttaggcaccc caggcttgac actttatgct tccggctcgt ataatgtgtg 120

gaattgtgag cggataacaa tgcgcaattt gccaagtaat ccaagcgaga aggagtgagt 180

tttatgttca cgggaagtat tgtcgcgatt gttactccga tggatgaaaa aggtaatgtc 240

tgtcgggcta gcttgaaaaa actgattgat tatcatgtcg ccagcggtac ttcggcgatc 300

gtttctgttg gcaccactgg cgagtccgct accttaaatc atgacgaaca tgctgatgtg 360

gtgatgatga cgctggagct ggctgacggg cgcattccgg tgattgccgg gactggtgct 420

aacgctactg cgacagccat tagcctgacg cagcgcttca atgacagtgg tatcgtcggc 480

tgcctgacgg taacccctta ctacaatcgt ccgtcgcaag aaggtttgta tcagcatttc 540

aaagccatcg ctgagcatac tgacctgccg caaattctgt ataatgtgcc gtcccgtact 600

ggctgcgatc tgctcccgga aacggtgggc cgtctggcga aagtaaaaaa tattatcgga 660

atcaaagagg caacagggaa cttaacgcgt gtaaaccaga tcaaagagct ggtttcagat 720

gattttgttc tgctgagcgg cgatgatgcg agcgcgctgg acttcatgca attaggcggt 780

```
catggggtta tttccgttac ggctaacgtc gcagcgcgtg atatggccca gatgtgcaaa      840
ctggcagcag aagggcattt tgccgaggca cgcgttatta atcagcgtct gatgccatta      900
cacaacaaac tatttgtcga acccaatcca atcccggtga aatgggcatg taaggaactg      960
ggtcttgtgg cgaccgatac gctgcgcctg ccaatgacac caatcaccga cagtggtcgt     1020
gagacggtca gagcggcgct taagcatgcc ggtttgctgt aataccggtg tcgacatcga     1080
taaggctagc tcgagcgcgg ccgcgcgatc gcacctggtg tttaaacggc cggcccctgc     1140
aggggcgcgc cccgggggg cccccgcgga tctctagagt cccaaggcag aacatatcca      1200
tcgcgtccgc catctccagc agccgcacgc ggcgcatctc gggcagcgtt gggtcctggc     1260
cacgggtgcg catgatcgtg ctcctgtcgt tgaggacccg gctaggctgg cggggttgcc     1320
ttactggtta gcagaatgaa tcaccgatac gcgagcgaac gtgaagcgac tgctgctgca     1380
aaacgtctgc gacctgagca acaacatgaa tggtcttcgg tttccgtgtt tcgtaaagtc     1440
tggaaacgcg gaagtcagcg ccctgcacca ttatgttccg gatctgcatc gcaggatgct     1500
gctggctacc ctgtggaaca cctacatctg tattaacgaa gcgctggcat tgaccctgag     1560
tgatttttct ctggtcccgc cgcatccata ccgccagttg tttaccctca caacgttcca     1620
gtaaccgggc atgttcatca tcagtaaccc gtatcgtgag catcctctct cgtttcatcg     1680
gtatcattac ccccatgaac agaaatcccc cttacacgga ggcatcagtg accaaacagg     1740
aaaaaaccgc ccttaacatg gcccgcttta tcagaagcca gacattaacg cttctggaga     1800
aactcaacga gctggacgcg gatgaacagg cagacatctg tgaatcgctt cacgaccacg     1860
ctgatgagct ttaccgcagc tgcctcgcgc gtttcggtga tgacggtgaa aacctctgac     1920
acatgcagct cccggagacg gtcacagctt gtctgtaagc ggatgccggg agcagacaag     1980
cccgtcaggg cgcgtcagcg ggtgttggcg ggtgtcgggg cgcagccatg acccagtcac     2040
gtagcgatag cggagtgtat actggcttaa ctatgcggca tcagagcaga ttgtactgag     2100
agtgcaccat atgcggtgtg aaataccgca cagatgcgta aggagaaaat accgcatcag     2160
gcgctcttcc gcttcctcgc tcactgactc gctgcgctcg gtcgttcggc tgcggcgagc     2220
ggtatcagct cactcaaagg cggtaatacg gttatccaca gaatcagggg ataacgcagg     2280
aaagaacatg tgagcaaaag gccagcaaaa ggccaggaac cgtaaaaagg ccgcgttgct     2340
ggcgtttttc cataggctcc gcccccctga cgagcatcac aaaaatcgac gctcaagtca     2400
gaggtggcga aacccgacag gactataaag ataccaggcg tttccccctg gaagctccct     2460
cgtgcgctct cctgttccga ccctgccgct taccggatac ctgtccgcct ttctcccttc     2520
gggaagcgtg gcgctttctc atagctcacg ctgtaggtat ctcagttcgg tgtaggtcgt     2580
tcgctccaag ctgggctgtg tgcacgaacc ccccgttcag cccgaccgct gcgccttatc     2640
cggtaactat cgtcttgagt ccaacccggt aagacacgac ttatcgccac tggcagcagc     2700
```

cactggtaac aggattagca gagcgaggta tgtaggcggt gctacagagt tcttgaagtg 2760

gtggcctaac tacggctaca ctagaaggac agtatttggt atctgcgctc tgctgaagcc 2820

agttaccttc ggaaaaagag ttggtagctc ttgatccggc aaacaaacca ccgctggtag 2880

cggtggtttt tttgtttgca agcagcagat tacgcgcaga aaaaaaggat ctcaagaaga 2940

tcctttgatc ttttctacgg ggtctgacgc tcagtggaac gaaaactcac gttaagggat 3000

tttggtcatg agattatcaa aaaggatctt cacctagatc cttttaaatt aaaaatgaag 3060

ttttaaatca atctaaagta tatatgagta aacttggtct gacagttacc aatgcttaat 3120

cagtgaggca cctatctcag cgatctgtct atttcgttca tccatagttg cctgactccc 3180

cgtcgtgtag ataactacga tacgggaggg cttaccatct ggccccagtg ctgcaatgat 3240

accgcgagac ccacgctcac cggctccaga tttatcagca ataaaccagc cagccggaag 3300

ggccgagcgc agaagtggtc ctgcaacttt atccgcctcc atccagtcta ttaattgttg 3360

ccgggaagct agagtaagta gttcgccagt taatagtttg cgcaacgttg ttgccattgc 3420

tgcaggcatc gtggtgtcac gctcgtcgtt tggtatggct tcattcagct ccggttccca 3480

acgatcaagg cgagttacat gatcccccat gttgtgcaaa aaagcggtta gctccttcgg 3540

tcctccgatc gttgtcagaa gtaagttggc cgcagtgtta tcactcatgg ttatggcagc 3600

actgcataat tctcttactg tcatgccatc cgtaagatgc ttttctgtga ctggtgagta 3660

ctcaaccaag tcattctgag aatagtgtat gcggcgaccg agttgctctt gcccggcgtc 3720

aacacgggat aataccgcgc cacatagcag aactttaaaa gtgctcatca ttggaaaacg 3780

ttcttcgggg cgaaaactct caaggatctt accgctgttg agatccagtt cgatgtaacc 3840

cactcgtgca cccaactgat cttcagcatc ttttactttc accagcgttt ctgggtgagc 3900

aaaaacagga aggcaaaatg ccgcaaaaaa gggaataagg gcgacacgga aatgttgaat 3960

actcatactc ttcctttttc aatattattg aagcatttat cagggttatt gtctcatgag 4020

cggatacata tttgaatgta tttagaaaaa taaacaaata ggggttccgc gcacatttcc 4080

ccgaaaagtg ccacctgacg tctaagaaac cattattatc atgacattaa cctataaaaa 4140

taggcgtatc acgaggccct ttcgtcttca agaa 4174

<210> 75
<211> 18060
<212> DNA
<213> Artificial Sequence

<220>
<223> plasmid_HM-p-48

<400> 75
aaataatttt gtttaacttt aagaaggaga tatacgatgt cagcaaatat ggcagtcaaa 60

```
caggccttga aggccaatcc ggtcccgagt tccgtggatc ctcaggaagt ccacaaatgg      120
cttcaggatt tcacttggga tttcaagggc aagaccgcga agtatccgac caagtatgag      180
atggacgtca atacgcgcga gcagttcaag ctgactgcca aggagtacgc gcgcatggag      240
tcgatcaagg aagagcgcca gtacggcacc ctgctcgatg gtctcgaccg tctggatgcg      300
ggcaacaagg tgcatccgaa atggggcgag gtgatgaagc tggtctccaa cttcctcgag      360
accggcgaat acggcgcaat cgccggttct gctctgctgt gggacacggc ccagtcaccg      420
gagcagcgca acggttacct cgctcaggtg atcgacgaaa tccgccatgt gaaccagacc      480
gcgtacgtga attactacta cggcaagcac tactacgacc ccgccggcca caccaacatg      540
cgccagcttc gggcgatcaa ccccctgtac cccggtgtca agcgcgcctt cggggagggc      600
tttctcgcgg gcgatgccgt cgagtcctcc atcaacctcc aattggtcgg cgaagcctgc      660
ttcaccaatc cgctcatcgt ttcgcttacc gaatgggcgg ccgcgaacgg cgatgaaatc      720
acgccgaccg tgttcctgtc gatcgaaacc gatgaactgc gccatatggc caacggctat      780
cagaccatcg tgtcgatcat gaacaatccg gagacgatga agtacctgca aacggacctc      840
gataacgcat tctggacgca gcacaagttc ctgacccccct tcgtcggggt ggcgctcgaa     900
tatggttcca agtacaaggt cgagccgtgg gccaagtcct ggaaccggtg ggtgtacgaa      960
gactgggctg gcatctggct gggccgactg cagcagttcg gcgtcaaaac gccaaagtgc     1020
ctgcccgacg ccaagaaaga cgccgtttgg gcacaccacg atctcgcgct gctggccctt     1080
gccctgtggc cgctgaccgg catccgcatg gaactcccgg atagcctggc gatggagtgg     1140
ttcgaggcta attaccccgg ttggtacaac cattacggca agatctacga ggagtggcgc     1200
gctgccggct tcgaggatcc gaagagcggc ttctgtggtg cgctctggct gatggagcgt     1260
ggtcacggca ttttcgtcga ccatgcctcg ggtttgccgt tctgccccag cctcgccaaa     1320
agctctatca agccgcggtt cactgaatac aacggcaagc gctacgcctt cgccgagccg     1380
tatggcgagc gccagtggct gctcgagccc gagcgctacg agttccagaa cttcttcgag     1440
cagttcgaag gctgggaact ctccgatctc gtcaaggcgg caggcggcgt gcgcagcgat     1500
ggcaaaacgc tgatcgcgca gccgcatctt cgcgacaccg acatgtggac gcttgacgat     1560
ctgaagcgga tcaacctgac gatccccgac ccgatgaaga tcctcaactg gcaacccgtc     1620
gcccagtgag ggttgggccg gcgcacgccg ccaggcgaga gatgcgccgg cggggaagac     1680
ttgccgcccg cctacgagcg ggcggacgtg ccaacgaacg aacagcacta tatggagtag     1740
ataaatatgt caacaaacat ctttacgcgc gggatggtag acccggagcg ccaagcctgc     1800
attcaggagg tcgtacccaa ggcgccgctg gaaaccaagc gtgaccatat tcctttcgcc     1860
aaacgcggct ggcgccgact caccgagtat gaagcggtga tgttgcatgc gcagaattca     1920
ctggacgccg tcccgggcag ccaggaggtg ggtgaagtcg tacagaagtg gccgggtggc     1980
```

aggccgaact acggcgtcga gtcgaccgcg gccctctcca gcaactggtt ccatttccgc 2040
gacccgtcga agcgctggtt catgccctac gtgaagcaga agaacgagga agggcagaca 2100
gccgaacgcg cgatgaagag ttgggccgag ggcggcgacg cggaaatgat gaacgccgcg 2160
tggcgggagc acatcctggc ccggcactac ggcgcgttcg tctataacga gtacggtctg 2220
ttcagcgccc attcaacaac agtttacggt ggcctttctg atctgatcaa aacctggatt 2280
gccgaggccg cgttcgacaa gaacgacgcc ggtcagatga tccagatgca gcgcgtgctg 2340
ttgagcaagg tgttccctgg cttcgacgcc gacctggccg aagccaagca ggcatggact 2400
gaggacaagt catggaaacc cgcccgcgag ttcgtcgagc acatctgggc cgagacctac 2460
gactgggtcg agcagctttg ggcgatccac gccgtctacg accatatttt cgggcaattt 2520
gtccggcgcg aattcttcca gcggctcggc ggcatccatg gagacacgct gacgcccttc 2580
attcagaacc aggcgctgac gtatcacctg caagcccgcg acggcgtaac ggcactgtgc 2640
ttcaaatttc tgatcgaaga cgagccggta tacgcccaac acaacaggcg ctacctgcgg 2700
gcatggacag ggcgctatct cccgcaagtg ggccgcgcat tgaaggcctt cctggcaatc 2760
tacaaggagg ttccggtaaa gatcgatggg gtgacttgcc gggagggcgt gcgcgccagt 2820
gtcgaacgcg tggtggacga ctgggcggcg cggttcgctg aaccgatcaa cttcaagttc 2880
aaccgcgcgg cgttcatcga cgatgtgctg agtggctact aattcagggg cagaccgaat 2940
gtcaaacgta aatgcatacc acgctggcac caatggtaaa gaaggccagg acttcatcga 3000
tgactttttg agcgaagaga acagtgctct acccacgagc gaagccgtgg ttctggccct 3060
gatgaagacc gaagagatcg acgcggtcgt cgacgagatg atcaagccgc agatggagga 3120
caacccgacc atcgccgtcg aggaccgcgg tggatactgg tggatcaagg ccaacggaaa 3180
gatcgtcatc gattgtgacg aagccaccga actgctgggc aagaagtata cggtgtacga 3240
tttgctggtc aacgtcagca ccaccgttgg gcgggcgatg accctcggca atcagttcat 3300
catcaccaac gagctccttg gtctcgaaac caaggtcgaa agcgtttact gaggaggata 3360
gacgacatgt cgaaacaggt ttggtacaac acaccggtgc gcgacgagtg gatcgagaag 3420
atcactgcga tcaggacagc gcgcgaaggc accgacatgc tcgcccgctt ccgcgcggag 3480
catacggggc cggatcgcac gacgtacgat ctcaagaagg aatacaattg gatcgagtcg 3540
cgcatcgaga tgcgggtcag ccagcttcat gccgaggcca cggcctcgga cgaagacctc 3600
ctcaccaaga cgatcgacgg ccgctgcgca aaggaagtcg cagcggagtg gctgaaaaag 3660
gctgcggaca tcgactgtca ctacgagatg gagcggcttt gtgtcgcctt ccgcaaggcg 3720
tgcaaaccgc cgatgatgcc catcaacttc ttcgcgccgg cagagaagga gttggtggcg 3780
aagctcatga agctgagggc gcccacgtat ctgactacct ccctcgacga gttgcgcgag 3840

gcacggggtg taacgatgat ttccgtgcag taagcggccg gacgttctga gccggatcct 3900
ccccgacaac ggggaggact ggtcaaaata gccatcaaga ctgaaaggag cagggaatgc 3960
cgaaatacat catcgaacgt tcgattccgg gggcagggaa gctgacggag cgggacctcg 4020
cgtcggtgtc gcagaaatcc tgtggaatcc tccgctccat ggggccacaa atccaatggg 4080
tcaaaagcta cgtcaccgac gacaagctct actgcatcta tatcgctccc gatgaggcct 4140
ccatccgcga gcatgcaaga tcgggagact ttcccgccga caaggtgtcc cgcatacatc 4200
ggatgatcga cccgacttgc gcggaatcgg cataatcgag ccgtcctcgg aggaccacga 4260
cttcccgagg gcctccggac gatggatcgc ggcggacgga aacctgcgtg gccgcggacg 4320
cggggtggcc acaagatcgt aacgtaaagt actctaatcc gagagcgcgg tcgctttcac 4380
ccgatcgagg tggaagtgac tgggcgaggg ggagatatga aagaagcgcc ggcaataccc 4440
gatctgcccg gtttgcccga gactgtcggc gaaccgacgc tggtcctgga agaagatggc 4500
ttccgggttt tcgccacaga gctgacgatc atgtggcgat gggacatcta caacggcgat 4560
gcgcacgttc ataccggatg cgcccagcac ccggagagct gcgtcgtcgc ggcgagatcc 4620
aaaattcgat tcttacggcg ccccactgtg gccatgctac tgggcggtga aggtcaatga 4680
gagggggttc gagacgccaa ctgatggacg cgacacccta gtaggagact gaaggtatat 4740
gctgatgcaa caatataaaa tagtcgcccg cttcgaagac ggcgtgacat acgagtacga 4800
ttgcggcgag gacgaaaacc tgctcgccgc ggcgttgcgc cagaacgtcc gtttgctgtg 4860
tcaatgcaga aaggcgtttt gcggcagttg caaggccttg tgcagcgagg gcgactatga 4920
actgggcgat catatcaacg ttcaggtctt gccgcctgac gaagaagagg acggggtggt 4980
ggtcacctgc gataccttcc cacgcagcga tctggtcctt gagtttccgt ataccagcga 5040
ccgtctcggt accgttacgg ctaccgaggc gaagacgagc gtcgtcagcg tggagagact 5100
ctctagcacc gtttaccgtc tggtgctgca ggcactcgac gccgaaggaa tgcctgcacg 5160
gttcgacttc gttcccgggc agtatgtcga aatcagtaca gccgattcgc tcgagaccag 5220
ggcgttttcg ctggcgaacc ttccaaacga cgccggattg ctcgagttct tgatccgact 5280
cgtccccggc ggatactatg cggcctatct ggagcaacgc gcagcggcag ggcagacgat 5340
caacgtgaag ggaccgttcg gcgagttcgt gctgcgtgaa cacgagttgg tggaggactt 5400
cacgctgcca gcggacagcc cagcgagagg tgggacgatt gcgttcctgg cgggtagcac 5460
ggggctggcg cctctcgcga gcatgctgcg cgagctcggg cggcgaggat tcaacggcga 5520
gtgtcacctc ttcttcggca tgcaggacac cgcaacgatg ttctacgaga aggaactccg 5580
ggacatcaag cgaacgcttc cggggctcac gctgcatctc gccctgatgg tcccttctgc 5640
cgaatgggaa ggctaccgtg gtaacgccgt agctgcgttc aaagaacatt tcgccgccag 5700
tagccagata cccgagaacg tttacctgtg cgggcccgga ccgatgatcg cggccgcgct 5760

cggcgcttgt cgcgagcttg gaattcccga caatagagtt cacagggaag aattcgtagc 5820
gagcggcggt tgaacggcgc ttgaagaaac cggggcgcga cgggcagcgt ttacgagacc 5880
atgagtcaag aagaaagaga ttttctggcc ggtgcagcgg caacactcgg cgagagcgtc 5940
ggacaactgg agcgccttgc ccaagaatta agtgaaaagc tgggcgatga gcgtgtcaac 6000
attgttcggg gtttgctcga acaggtttat gaccgcgacg acgaagcgga agtgcgtgca 6060
tccctctgct atacggagag aaagctgttg tgggcgtggg tccgtttgaa gagactgaag 6120
ggactcaggc tcagtattgg tcgtggttcg atgagaaaca ttatatgaga agcagaacgt 6180
gatcagttta aattgcaaga agacaaccac agggttaact gcgcacttgg ccctggtccg 6240
cggaatgaag gcgttggcgg aattggttgg aacaacgctg ggccccagg gccgccacgt 6300
catgcttgcg catcgggccg gattggctcc gcacgtcagc aaggacggcg tcgaggtcgc 6360
acgtcacctg tcgttgccgg acagcgagga agaacttggt gtccggttgc tgcgcaacgc 6420
agccgtcgca gtgtccgagt cctttggcga cggcacctcc actgcaaccg tttttacggc 6480
ggatctggcc gtgcgggcgc tcaaactgat cggtgccggg gcggataccc tggaggtccg 6540
tcgaggtctg ggtttggctg cctatgccgc gttggtcgcc ctgaacgaca tggcccggcg 6600
cgccgaccgg ggaatgctca ccgccgttgc ccaaaccgct gccaacggcg accgtcgcgt 6660
ggcggatctc ctcgtggagg ccttcgaaag ggtcggggct gaaggcacga tcgaagtaga 6720
aatgggtaat tcggtcgagg acgtccttga agtcgcgcag ggcagttatt tcgacacggt 6780
gccgctcgtc acggccttgt tgccgccaac agggcaggtc gagttcgccc ggccacttat 6840
ccttttccat tgcgacgcga tcgagacggc ggacgagatt cttccggcgc tggagctagc 6900
tcgcagttca cggagaccct tgctgattct ggccgactcg gtgggtatcg acgtcgagac 6960
gctgctcgtc cggaatcaaa atgaaggcac cttggctgtt gcggtcgtca gggcgccaat 7020
gtatggcgat acgcgtcgcg aggccctgct tgacctgacg agcaaattcg gggggacggc 7080
gtttggaagg gagggattcg tcgaattcgc actcaggtct ttgggatcac tgtcggaagg 7140
tgacttgggg caggcggacg aagcaatcct agaggcggat ggtgtgactc tgagaggcgc 7200
cggaaacaat ccgtctgcat tggaagaccg aatcgcgctg gtgcgtgctg aactcgatcg 7260
cggcgacgtt tccgtgggcg actccccatc ggcaaagctc gactacatcg agaagcgaaa 7320
ggagcggctg aagttgctgg ctgcaggtag cgccaagctg catatcgggg ggccgacgga 7380
cgttgagatc aagacgcgtt tgccgctcgc cgagaacgct catcgggcgc tcttggcggc 7440
cgcgaagtcg ggagtgctgc ccgggggcgg cgtcgcaatg attcgtgcgg ctgaaaaggt 7500
gcagcaagag atggggcgac ttgaaggcga cgttgcttct ggggcatcca ttttcctgca 7560
gtcgcttgac acacccatcc ggtggattgc acgaaatgcg gggctgcgcc cggatgaagt 7620

gctcgctcga accctggcga acgagtcgga cttttacgga ctcaacgcca tgactggccg 7680

atatggcgac ttggctgaag acggggttct cgacgccctc gatatggtca ccgacgtgat 7740

acgcgtcgca gtcagcgtcg tcgggtcgat gttgggcgtc ggcgccctgg tgactcgcgc 7800

atcgcccaag cccgcgccag agcgcttcaa gggcacggag cgggtacacg acaagttgat 7860

gcgcgagggc gggttcgatg aatgaaattt cagtgcaatt tatctcttca aatgtagcac 7920

ctgaagtcag ccccatacga tataagttgt aattctcatg tttgacagct tatcatcgat 7980

aagctttaat gcggtagttt atcacagtta aattgctaac gcagtcaggc accgtgtatg 8040

aaatctaaca atgcgctcat cgtcatcctc ggcaccgtca ccctggatgc tgtaggcata 8100

ggcttggtta tgccggtact gccgggcctc ttgcgggata tcgtccattc cgacagcatc 8160

gccagtcact atggcgtgct gctagcgcta tatgcgttga tgcaatttct atgcgcaccc 8220

gttctcggag cactgtccga ccgctttggc cgccgcccag tcctgctcgc ttcgctactt 8280

ggagccacta tcgactacgc gatcatggcg accacacccg tcctgtggat cctctacgcc 8340

ggacgcatcg tggccggcat caccggcgcc acaggtgcgg ttgctggcgc ctatatcgcc 8400

gacatcaccg atggggaaga tcgggctcgc cacttcgggc tcatgagcgc ttgtttcggc 8460

gtgggtatgg tggcaggccc cgtggccggg ggactgttgg gcgccatctc cttgcatgca 8520

ccattccttg cggcggcggt gctcaacggc ctcaacctac tactgggctg cttcctaatg 8580

caggagtcgc ataagggaga gcgtcgaccg atgcccttga gagccttcaa cccagtcagc 8640

tccttccggt gggcgcgggg catgactatc gtcgccgcac ttatgactgt cttctttatc 8700

atgcaactcg taggacaggt gccggcagcg ctctgggtca ttttcggcga ggaccgcttt 8760

cgctggagcg cgacgatgat cggcctgtcg cttgcggtat tcggaatctt gcacgccctc 8820

gctcaagcct tcgtcactgg tcccgccacc aaacgtttcg gcgagaagca ggccattatc 8880

gccggcatgg cggccgacgc gctgggctac gtcttgctgg cgttcgcgac gcgaggctgg 8940

atggccttcc ccattatgat tcttctcgct tccggcggca tcgggatgcc cgcgttgcag 9000

gccatgctgt ccaggcaggt agatgacgac catcagggac agcttcaagg atcgctcgcg 9060

gctcttacca gcctaacttc gatcattgga ccgctgatcg tcacggcgat ttatgccgcc 9120

tcggcgagca catggaacgg gttggcatgg attgtaggcg ccgccctata ccttgtctgc 9180

ctccccgcgt tgcgtcgcgg tgcatggagc cgggccacct cgacctgaat ggaagccggc 9240

ggcacctcgc taacggattc accactccaa gaattggagc caatcaattc ttgcggagaa 9300

ctgtgaatgc gcaaaccaac ccttggcaga acatatccat cgcgtccgcc atctccagca 9360

gccgcacgcg gcgcatctcg ggcagcgttg ggtcctggcc acgggtgcgc atgatcgtgc 9420

tcctgtcgtt gaggacccgg ctaggctggc ggggttgcct tactggttag cagaatgaat 9480

caccgatacg cgagcgaacg tgaagcgact gctgctgcaa aacgtctgcg acctgagcaa 9540

caacatgaat ggtcttcggt ttccgtgttt cgtaaagtct ggaaacgcgg aagtccccta 9600
cgtgctgctg aagttgcccg caacagagag tggaaccaac cggtgatacc acgatactat 9660
gactgagagt caacgccatg agcggcctca tttcttattc tgagttacaa cagtccgcac 9720
cgctgccggt agctccttcc ggtgggcgcg gggcatgact atcgtcgccg cacttatgac 9780
tgtcttcttt atcatgcaac tcgtaggaca ggtgccggca gcgcccaaca gtcccccggc 9840
cacggggcct gccaccatac ccacgccgaa acaagcgccc tgcaccatta tgttccggat 9900
ctgcatcgca ggatgctgct ggctaccctg tggaacacct acatctgtat taacgaagcg 9960
ctaaccgttt ttatcaggct ctgggaggca gaataaatga tcatatcgtc aattattacc 10020
tccacgggga gagcctgagc aaactggcct caggcatttg agaagcacac ggtcacactg 10080
cttccggtag tcaataaacc ggtaaaccag caatagacat aagcggctat ttaacgaccc 10140
tgccctgaac cgacgaccgg gtcgaatttg ctttcgaatt tctgccattc atccgcttat 10200
tatcacttat tcaggcgtag caaccaggcg tttaagggca ccaataactg ccttaaaaaa 10260
attacgcccc gccctgccac tcatcgcagt actgttgtaa ttcattaagc attctgccga 10320
catggaagcc atcacaaacg gcatgatgaa cctgaatcgc cagcggcatc agcaccttgt 10380
cgccttgcgt ataatatttg cccatggtga aaacgggggc gaagaagttg tccatattgg 10440
ccacgtttaa atcaaaactg gtgaaactca cccagggatt ggctgagacg aaaaacatat 10500
tctcaataaa ccctttaggg aaataggcca ggttttcacc gtaacacgcc acatcttgcg 10560
aatatatgtg tagaaactgc cggaaatcgt cgtggtattc actccagagc gatgaaaacg 10620
tttcagtttg ctcatggaaa acggtgtaac aagggtgaac actatcccat atcaccagct 10680
caccgtcttt cattgccata cggaattccg gatgagcatt catcaggcgg gcaagaatgt 10740
gaataaaggc cggataaaac ttgtgcttat ttttctttac ggtctttaaa aaggccgtaa 10800
tatccagctg aacggtctgg ttataggtac attgagcaac tgactgaaat gcctcaaaat 10860
gttctttacg atgccattgg gatatatcaa cggtggtata tccagtgatt tttttctcca 10920
ttttagcttc cttagctcct gaaaatctcg ataactcaaa aaatacgccc ggtagtgatc 10980
ttatttcatt atggtgaaag ttggaacctc ttacgtgccg atcaacgtct cattttcgcc 11040
aaaagttggc ccagggcttc ccggtatcaa cagggacacc aggatttatt tattctgcga 11100
agtgatcttc cgtcacaggt atttattcgg cgcaaagtgc gtcgggtgat gctgccaact 11160
tactgattta gtgtatgatg gtgtttttga ggtgctccag tggcttctgt ttctatcagc 11220
tgtccctcct gttcagctac tgacggggtg gtgcgtaacg gcaaaagcac cgccggacat 11280
cagcgctagc ggagtgtata ctggcttact atgttggcac tgatgagggt gtcagtgaag 11340
tgcttcatgt ggcaggagaa aaaaggctgc accggtgcgt cagcagaata tgtgatacag 11400

gatatattcc gcttcctcgc tcactgactc gctacgctcg gtcgttcgac tgcggcgagc 11460
ggaaatggct tacgaacggg gcggagattt cctggaagat gccaggaaga tacttaacag 11520
ggaagtgaga gggccgcggc aaagccgttt ttccataggc tccgcccccc tgacaagcat 11580
cacgaaatct gacgctcaaa tcagtggtgg cgaaacccga caggactata aagataccag 11640
gcgtttcccc ctggcggctc cctcgtgcgc tctcctgttc ctgcctttcg gtttaccggt 11700
gtcattccgc tgttatggcc gcgtttgtct cattccacgc ctgacactca gttccgggta 11760
ggcagttcgc tccaagctgg actgtatgca cgaacccccc gttcagtccg accgctgcgc 11820
cttatccggt aactatcgtc ttgagtccaa cccggaaaga catgcaaaag caccactggc 11880
agcagccact ggtaattgat ttagaggagt tagtcttgaa gtcatgcgcc ggttaaggct 11940
aaactgaaag gacaagtttt ggtgactgcg ctcctccaag ccagttacct cggttcaaag 12000
agttggtagc tcagagaacc ttcgaaaaac cgccctgcaa ggcggttttt tcgttttcag 12060
agcaagagat tacgcgcaga ccaaaacgat ctcaagaaga tcatcttatt aatcagataa 12120
aatatttatt agataattcc ttgacgcgtt taaacggcag tgagcgcaac gcaattaatg 12180
taagttagct cactcattag gcaccccagg cttgacactt tatgcttccg gctcgtataa 12240
tgtgtggaat tgtgagcgga taacaatgcg caatttgcca agtaatccaa gcgagaagga 12300
gtgagtttta tgaccactgc tgcaccccaa gaatttaccg ctgctgttgt tgaaaaattc 12360
ggtcatgacg tgaccgtgaa ggatattgac cttccaaagc cagggccaca ccaggcattg 12420
gtgaaggtac tcacctccgg catctgccac accgacctcc acgccttgga gggcgattgg 12480
ccagtaaagc cggaaccacc attcgtacca ggacacgaag gtgtaggtga agttgttgag 12540
ctcggaccag gtgaacacga tgtgaaggtc ggcgatattg tcggcaatgc gtggctctgg 12600
tcagcgtgtg gcacctgcga atactgcatc accggcaggg aaactcagtg caacgaagct 12660
gagtatggtg gctacaccca aaatggatcc ttcggccagt acatgctggt ggatacccgt 12720
tacgccgctc gcatcccaga cggcgtggac tacctcgaag cagcaccaat tctgtgtgca 12780
ggcgtgactg tctacaaggc actcaaagtc tctgaaaccc gcccgggcca attcatggtg 12840
atctccggtg tcggcggact tggccacatc gcagtccaat acgcagcggc gatgggcatg 12900
cgtgtcattg cggtagatat tgccgatgac aagctggaac ttgcccgtaa gcacggtgcg 12960
gaatttaccg tgaatgcgcg taatgaagat tcaggcgaag ctgtacagaa gtacaccaac 13020
ggtggcgcac acggcgtgct tgtgactgca gttcacgagg cagcattcgg ccaggcactg 13080
gatatggctc gacgtgcagg aacaattgtg ttcaacggtc tgccaccggg agagttccca 13140
gcatccgtgt tcaacatcgt attcaagggc ctgaccatcc gtggatccct cgtgggaacc 13200
cgccaagact tggccgaagc gctcgatttc tttgcacgcg gactaatcaa gccaaccgtg 13260
agtgagtgct ccctcgatga ggtcaatggt gtgcttgacc gcatgcgaaa cggcaagatc 13320

gatggtcgtg tggcgattcg tttctaagta gtgctcaggt ggaggtggcc caaaggagac 13380

ccaatgactg tctacgcaaa tccaggaacc gaaggctcga tcgttaacta tgaaaagcgc 13440

tacgagaact acattggtgg caagtgggtt ccaccggtag agggccagta ccttgagaac 13500

atttcacctg tcactggtga agttttctgt gaggtcgcac gtggcaccgc agcggacgtg 13560

gagcttgcac tggatgctgc acatgcagcc gctgatgcgt ggggcaagac ttctgtcgct 13620

gaacgtgctc tgatcctgca ccgcattgcg gaccgcatgg aagagcacct ggaagaaatc 13680

gcagttgcag aaacctggga gaacggcaag gcagtccgtg agactcttgc tgcagatatc 13740

ccactggcaa tcgaccactt ccgctacttt gctggcgcga tccgtgctca ggaagatcgt 13800

tcctcacaga tcgaccacaa cactgttgct taccacttca acgagccaat cggtgttgtt 13860

ggtcagatca ttccttggaa cttcccaatc ctcatggcta cctggaagct cgcaccggca 13920

cttgctgcag gtaacgcgat cgtcatgaag ccagctgagc agaccccagc atccattttg 13980

tatctgatta acatcatcgg cgatctcatc ccagagggcg tcctcaacat cgtcaacgga 14040

ctcggcggtg aagcaggcgc tgcactgtcc ggctctaatc ggattggcaa gattgctttc 14100

accggttcca ccgaggtcgg caagctgatc aaccgcgctg catccgacaa gatcattcct 14160

gtcaccctgg agctcggcgg taagtcccca tccatcttct tctccgatgt tctgtcacag 14220

gatgacgcct tcgcagagaa ggcagttgaa ggcttcgcga tgttcgccct caatcagggt 14280

gaagtttgta cctgtccttc ccgtgcactt gttcatgagt ccatcgctga tgaattcctc 14340

gagcttggcg tgaagcgagt tcagaacatc aagctgggta acccacttga tactgaaacc 14400

atgatgggtg ctcaggcgtc ccaggagcag atggacaaga tctcctccta cctgaagatc 14460

ggcccagaag aaggcgctca aaccctcact ggtggcaagg tcaacaaggt tgatggcatg 14520

gagaacggtt actacattga gccaaccgtt ttccgcggca ccaacgacat gaggatcttc 14580

cgcgaggaaa tcttcggacc agtcctttct gttgctacct tcagcgactt cgatgaggcc 14640

atccgtattg caaacgacac caactacggc ctcggcgctg gtgtctggag ccgtgaccaa 14700

aacaccattt atcgtgcagg tcgcgcaatc caggctggtc gagtttgggt caaccagtac 14760

cacaactacc cagcgcactc cgctttcggt ggatacaagg agtccggcat cggccgtgag 14820

aaccacctca tgatgctgaa ccactaccag cagaccaaga acctgttggt ctcctacgat 14880

ccaaacccaa ccggactgtt ctgatctaag cgttaagtcc tagaaaagct gcctcacaca 14940

tcctccagtg tgcggcggct tttgctattt aaatttagat aattccttga cgctcagctt 15000

caattgttgc ttgcgtgcga ttcactacat tcaaggtggc aaatattttc ctcatatgcc 15060

actttatagc atcttcggtg acatgcatat ttgttgctat ttgtttgttt gagcacccct 15120

cttttacaag cctcaagaca gcaatctgct tccgtgtcaa taaagcgtca gctttattct 15180

ctgcggactt tccaatctca actattcgcg gaagactaaa agccccaatc gcttgatcta 15240
aattaactgc tgtgaaggct tcacatgaag ccggtattat tcgctcaatt aaacatactt 15300
catcaagaac tgtttgaaag cattgaagct gttttgctat ctccactgca taaacaatgt 15360
taagctgagc ctttttttaaa tcaccggcac ctgcctgcgc tccggccaaa cacaataatc 15420
cacggacttc cagctggccc gcgttaattt tacgggcttg ctgaatagcc aataacgctc 15480
tgtgcgcggc actatgaaag ttccgatctc gggaaagcac tagtgattga acaagcagca 15540
ggcgtgcttt tagggggggct gagtgctgtc cggagaaaat cttatgatct tcaagagttt 15600
ttaaattatt tatgcccgtt atgccttgac agactaagcg ctgatagatc tcaatttggc 15660
tcataacttc caatcttggt agattttttt caaccgcatg cgccttcgcc cactccaata 15720
tctcaatgga gccatttagg tcactccttc caagccgcca agctgacaca gcacggcata 15780
cggaaaaaaa cacgtctgtc accccgtgat tggaaatgaa ctctaaaatt ttggagagct 15840
tttcttctga ggtgtccaag cagcgcaatt cataatgtaa ctcaagctct agagcgtcaa 15900
acattttcga agtaaactcg gattccatca tctgcgcgcg actgtctgtg cgtgcttgag 15960
ttataatctg cctcgcccag cccatttttc cgcttgctag ggcttgttga aacctcgcga 16020
catacagcca accaaaagca aaattttgtt ttgcaaattt attcacggct tgggcctgag 16080
ccagcacctt ctccaactct gcaaatctat actcactggc aaaaataaaa gccaaacagg 16140
ttagcgcggc ccttttcca actgcgtttg aatccccaaa taaactaatc cacttattac 16200
agagctcctc actcgaaagc atttcatctt tcgttgcttt acctattgca agcacaagct 16260
gcagccattc cttttcttgc catttatttt ttttatcgga ttgtgaagat aggtctttaa 16320
ttaacttctc tgctcgcgcg ccttgctgac tgaaatacaa tacccacgcg taactaataa 16380
gcactatggg ttttttgtgc caggcctgct tcggcagctc taacagccac tgtctcagcg 16440
catctatttc gccctgacga aatgacaaat ctaaaattat tctctcagac atgctgactg 16500
cccagcgaca gtcattcgcc cgtagggata ttcgtattgc atactggtat tcacctctac 16560
gccaatgcca gaaagctgca cgcttaagca ggtaggatct tttagcagga ttttcagtcc 16620
aagtaatttc tcgtagaaaa ttacgcagta ctggatgcag tgtaaactgc gctggctcac 16680
cgctcacatg gcgaagcaac atgtaattag tgcttaaata cttaatacat gagaccccat 16740
tgacgcattt gaatacataa ttgtattgat caggcgtcac gaaatcgagc aatgaagaat 16800
ttgcaagaaa aacacgatag cgctcgggaa tcgcctcaaa tatttcatcc ctaaagtaat 16860
tgtctacttc aactactgct gaaatatgct tggccggcaa ctcacgcttt aacaaaaaaa 16920
ctacaagagc aggccacccc tcaacttctt gcaccaaggt ctctatctgt tcttcaggaa 16980
ctccaagaac agactctgcc tccgctaacg ccaccgcctc ttctgcgcta aaggccaagt 17040
ctttctcggt gtactcccgc atagcgcctg caagtttaag ctgcgagaac ccttttattg 17100

tattgcctgc aactgcaaac ctgatatttt ttggtgtatt taacataaac tccataagtg 17160

cgtgcaacaa cggcaagtct aagtcatgat taatattatc caaacaaact agcgtttcta 17220

tctcgttatt cgaggtgctc tgccaaagac tagatgcaag gtctcgcaag agcgcaggct 17280

tgctcacacc ctctctcaca cggctgaatt ttaccatttc gaaagtttca agctgctcaa 17340

taatctctgc gcagatatca aattcactgt aagaactggc tcttaaagaa agccacactg 17400

caggacgtcc ggctgttctg tggcgtagcc actcgaacgc aagagcaacg gttttcccat 17460

atccaggtgg ggctctgtaa aggcatactc tgggagcggc tccatccgcg atactcaatc 17520

ttggccgata tatgcaacta tgaactttgg cacttactag agtcgtaatt tgatccgctc 17580

cgaccttagc gaccgggaaa tcattattta ttattatttt cattatgcta ttctcgcgcc 17640

agctgactgg aaattttcac cataggttac ggtgttaaat attaaaacta cacttaagtg 17700

tagtcggcat gatcggtggt gcaaaatatt tactagggaa ggtctgaagt aggccgctat 17760

ttctggccga cttcggcctt cgccgatttt gaagacgggc accgggtcaa aatcgaccag 17820

atagctcgct catttcggtg ctttcagccg tcgcgagtag ctcgcggtac ctggcatgct 17880

tgcggccagc tcgtgttttt ccagcagacg acggagcaaa aactacccgt aggtgtagtt 17940

ggcgcaagcg tccgattagc tcaggtttaa gatgtcgaga gtgagagtgg gcggcttaac 18000

tttctcagtt aggcataaaa ttacgtctta aatctcgtag cgactaattt aataaaaatt 18060

<210> 76
<211> 879
<212> DNA
<213> Escherichia coli

<400> 76
atgttcacgg gaagtattgt cgcgattgtt actccgatgg atgaaaaagg taatgtctgt 60

cgggctagct tgaaaaaact gattgattat catgtcgcca gcggtacttc ggcgatcgtt 120

tctgttggca ccactggcga gtccgctacc ttaaatcatg acgaacatgc tgatgtggtg 180

atgatgacgc tggatctggc tgatgggcgc attccggtaa ttgccgggac cggcgctaac 240

gctactgcgg aagccattag cctgacgcag cgcttcaatg acagtggtat cgtcggctgc 300

ctgacggtaa ccccttacta caatcgtccg tcgcaagaag gtttgtatca gcatttcaaa 360

gccatcgctg agcatactga cctgccgcaa attctgtata atgtgccgtc ccgtactggc 420

tgcgatctgc tcccggaaac ggtgggccgt ctggcgaaag taaaaaatat tatcggaatc 480

aaagaggcaa cagggaactt aacgcgtgta aaccagatca aagagctggt ttcagatgat 540

tttgttctgc tgagcggcga tgatgcgagc gcgctggact tcatgcaatt gggcggtcat 600

ggggttattt ccgttacggc taacgtcgca gcgcgtgata tggcccagat gtgcaaactg 660

gcagcagaag ggcattttgc cgaggcacgc gttattaatc agcgtctgat gccattacac 720

aacaaactat ttgtcgaacc caatccaatc ccggtgaaat gggcatgtaa ggaactgggt 780

cttgtggcga ccgatacgct gcgcctgcca atgacaccaa tcaccgacag tggtcgtgag 840

acggtcagag cggcgcttaa gcatgccggt ttgctgtaa 879

<210> 77
<211> 2749
<212> DNA
<213> Artificial Sequence

<220>
<223> lacUV5 promotor region

<400> 77
ggcagtgagc gcaacgcaat taatgtaagt tagctcactc attaggcacc ccaggcttga 60

cactttatgc ttccggctcg tataatgtgt ggaattgtga gcggataaca atgcgcaatt 120

tgccaagtaa tccaagcgag aaggagtgag ttttatgacc actgctgcac cccaagaatt 180

taccgctgct gttgttgaaa aattcggtca tgacgtgacc gtgaaggata ttgaccttcc 240

aaagccaggg ccacaccagg cattggtgaa ggtactcacc tccggcatct gccacaccga 300

cctccacgcc ttggagggcg attggccagt aaagccggaa ccaccattcg taccaggaca 360

cgaaggtgta ggtgaagttg ttgagctcgg accaggtgaa cacgatgtga aggtcggcga 420

tattgtcggc aatgcgtggc tctggtcagc gtgtggcacc tgcgaatact gcatcaccgg 480

cagggaaact cagtgcaacg aagctgagta tggtggctac acccaaaatg gatccttcgg 540

ccagtacatg ctggtggata cccgttacgc cgctcgcatc ccagacggcg tggactacct 600

cgaagcagca ccaattctgt gtgcaggcgt gactgtctac aaggcactca aagtctctga 660

aacccgcccg ggccaattca tggtgatctc cggtgtcggc ggacttggcc acatcgcagt 720

ccaatacgca gcggcgatgg gcatgcgtgt cattgcggta gatattgccg atgacaagct 780

ggaacttgcc cgtaagcacg gtgcggaatt taccgtgaat gcgcgtaatg aagattcagg 840

cgaagctgta cagaagtaca ccaacggtgg cgcacacggc gtgcttgtga ctgcagttca 900

cgaggcagca ttcggccagg cactggatat ggctcgacgt gcaggaacaa ttgtgttcaa 960

cggtctgcca ccgggagagt tcccagcatc cgtgttcaac atcgtattca agggcctgac 1020

catccgtgga tccctcgtgg gaacccgcca agacttggcc gaagcgctcg atttctttgc 1080

acgcggacta atcaagccaa ccgtgagtga gtgctccctc gatgaggtca atggtgtgct 1140

tgaccgcatg cgaaacggca agatcgatgg tcgtgtggcg attcgtttct aagtagtgct 1200

caggtggagg tggcccaaag gagacccaat gactgtctac gcaaatccag gaaccgaagg 1260

ctcgatcgtt aactatgaaa agcgctacga gaactacatt ggtggcaagt gggttccacc 1320

ggtagagggc cagtaccttg agaacatttc acctgtcact ggtgaagttt tctgtgaggt 1380

cgcacgtggc accgcagcgg acgtggagct tgcactggat gctgcacatg cagccgctga 1440

tgcgtggggc aagacttctg tcgctgaacg tgctctgatc ctgcaccgca ttgcggaccg 1500

catggaagag cacctggaag aaatcgcagt tgcagaaacc tgggagaacg gcaaggcagt 1560

ccgtgagact cttgctgcag atatcccact ggcaatcgac cacttccgct actttgctgg 1620

cgcgatccgt gctcaggaag atcgttcctc acagatcgac cacaacactg ttgcttacca 1680

cttcaacgag ccaatcggtg ttgttggtca gatcattcct tggaacttcc caatcctcat 1740

ggctacctgg aagctcgcac cggcacttgc tgcaggtaac gcgatcgtca tgaagccagc 1800

tgagcagacc ccagcatcca ttttgtatct gattaacatc atcggcgatc tcatcccaga 1860

gggcgtcctc aacatcgtca acggactcgg cggtgaagca ggcgctgcac tgtccggctc 1920

taatcggatt ggcaagattg ctttcaccgg ttccaccgag gtcggcaagc tgatcaaccg 1980

cgctgcatcc gacaagatca ttcctgtcac cctggagctc ggcggtaagt ccccatccat 2040

cttcttctcc gatgttctgt cacaggatga cgccttcgca gagaaggcag ttgaaggctt 2100

cgcgatgttc gccctcaatc agggtgaagt ttgtacctgt ccttcccgtg cacttgttca 2160

tgagtccatc gctgatgaat tcctcgagct tggcgtgaag cgagttcaga acatcaagct 2220

gggtaaccca cttgatactg aaaccatgat gggtgctcag gcgtcccagg agcagatgga 2280

caagatctcc tcctacctga agatcggccc agaagaaggc gctcaaaccc tcactggtgg 2340

caaggtcaac aaggttgatg gcatggagaa cggttactac attgagccaa ccgttttccg 2400

cggcaccaac gacatgagga tcttccgcga ggaaatcttc ggaccagtcc tttctgttgc 2460

taccttcagc gacttcgatg aggccatccg tattgcaaac gacaccaact acggcctcgg 2520

cgctggtgtc tggagccgtg accaaaacac catttatcgt gcaggtcgcg caatccaggc 2580

tggtcgagtt tgggtcaacc agtaccacaa ctacccagcg cactccgctt tcggtggata 2640

caaggagtcc ggcatcggcc gtgagaacca cctcatgatg ctgaaccact accagcagac 2700

caagaacctg ttggtctcct acgatccaaa cccaaccgga ctgttctga 2749

<210> 78
<211> 309
<212> PRT
<213> Escherichia coli

<400> 78

Met Pro Ile Arg Val Pro Asp Glu Leu Pro Ala Val Asn Phe Leu Arg
1 5 10 15

Glu Glu Asn Val Phe Val Met Thr Thr Ser Arg Ala Ser Gly Gln Glu
20 25 30

Ile Arg Pro Leu Lys Val Leu Ile Leu Asn Leu Met Pro Lys Lys Ile

35 40 45

Glu Thr Glu Asn Gln Phe Leu Arg Leu Leu Ser Asn Ser Pro Leu Gln
50 55 60

Val Asp Ile Gln Leu Leu Arg Ile Asp Ser Arg Glu Ser Arg Asn Thr
65 70 75 80

Pro Ala Glu His Leu Asn Asn Phe Tyr Cys Asn Phe Glu Asp Ile Gln
85 90 95

Asp Gln Asn Phe Asp Gly Leu Ile Val Thr Gly Ala Pro Leu Gly Leu
100 105 110

Val Glu Phe Asn Asp Val Ala Tyr Trp Pro Gln Ile Lys Gln Val Leu
115 120 125

Glu Trp Ser Lys Asp His Val Thr Ser Thr Leu Phe Val Cys Trp Ala
130 135 140

Val Gln Ala Ala Leu Asn Ile Leu Tyr Gly Ile Pro Lys Gln Thr Arg
145 150 155 160

Thr Glu Lys Leu Ser Gly Val Tyr Glu His His Ile Leu His Pro His
165 170 175

Ala Leu Leu Thr Arg Gly Phe Asp Asp Ser Phe Leu Ala Pro His Ser
180 185 190

Arg Tyr Ala Asp Phe Pro Ala Ala Leu Ile Arg Asp Tyr Thr Asp Leu
195 200 205

Glu Ile Leu Ala Glu Thr Glu Glu Gly Asp Ala Tyr Leu Phe Ala Ser
210 215 220

Lys Asp Lys Arg Ile Ala Phe Val Thr Gly His Pro Glu Tyr Asp Ala
225 230 235 240

Gln Thr Leu Ala Gln Glu Phe Phe Arg Asp Val Glu Ala Gly Leu Asp
245 250 255

Pro Asp Val Pro Tyr Asn Tyr Phe Pro His Asn Asp Pro Gln Asn Thr
260 265 270

Pro Arg Ala Ser Trp Arg Ser His Gly Asn Leu Leu Phe Thr Asn Trp
275 280 285

```
Leu Asn Tyr Tyr Val Tyr Gln Ile Thr Pro Tyr Asp Leu Arg His Met
    290                 295                 300

Asn Pro Thr Leu Asp
305

<210>  79
<211>  421
<212>  PRT
<213>  Corynebacterium glutamicum

<400>  79

Met Ala Leu Val Val Gln Lys Tyr Gly Gly Ser Ser Leu Glu Ser Ala
1               5                   10                  15

Glu Arg Ile Arg Asn Val Ala Glu Arg Ile Val Ala Thr Lys Lys Ala
            20                  25                  30

Gly Asn Asp Val Val Val Val Cys Ser Ala Met Gly Asp Thr Thr Asp
        35                  40                  45

Glu Leu Leu Glu Leu Ala Ala Ala Val Asn Pro Val Pro Pro Ala Arg
    50                  55                  60

Glu Met Asp Met Leu Leu Thr Ala Gly Glu Arg Ile Ser Asn Ala Leu
65                  70                  75                  80

Val Ala Met Ala Ile Glu Ser Leu Gly Ala Glu Ala Gln Ser Phe Thr
                85                  90                  95

Gly Ser Gln Ala Gly Val Leu Thr Thr Glu Arg His Gly Asn Ala Arg
            100                 105                 110

Ile Val Asp Val Thr Pro Gly Arg Val Arg Glu Ala Leu Asp Glu Gly
        115                 120                 125

Lys Ile Cys Ile Val Ala Gly Phe Gln Gly Val Asn Lys Glu Thr Arg
    130                 135                 140

Asp Val Thr Thr Leu Gly Arg Gly Gly Ser Asp Thr Thr Ala Val Ala
145                 150                 155                 160

Leu Ala Ala Ala Leu Asn Ala Asp Val Cys Glu Ile Tyr Ser Asp Val
                165                 170                 175

Asp Gly Val Tyr Thr Ala Asp Pro Arg Ile Val Pro Asn Ala Gln Lys
            180                 185                 190
```

```
Leu Glu Lys Leu Ser Phe Glu Glu Met Leu Glu Leu Ala Ala Val Gly
        195                 200                 205

Ser Lys Ile Leu Val Leu Arg Ser Val Glu Tyr Ala Arg Ala Phe Asn
    210                 215                 220

Val Pro Leu Arg Val Arg Ser Ser Tyr Ser Asn Asp Pro Gly Thr Leu
225                 230                 235                 240

Ile Ala Gly Ser Met Glu Asp Ile Pro Val Glu Glu Ala Val Leu Thr
                245                 250                 255

Gly Val Ala Thr Asp Lys Ser Glu Ala Lys Val Thr Val Leu Gly Ile
            260                 265                 270

Ser Asp Lys Pro Gly Glu Ala Ala Lys Val Phe Arg Ala Leu Ala Asp
        275                 280                 285

Ala Glu Ile Asn Ile Asp Met Val Leu Gln Asn Val Ser Ser Val Glu
    290                 295                 300

Asp Gly Thr Thr Asp Ile Thr Phe Thr Cys Pro Arg Ser Asp Gly Arg
305                 310                 315                 320

Arg Ala Met Glu Ile Leu Lys Lys Leu Gln Val Gln Gly Asn Trp Thr
                325                 330                 335

Asn Val Leu Tyr Asp Asp Gln Val Gly Lys Val Ser Leu Val Gly Ala
            340                 345                 350

Gly Met Lys Ser His Pro Gly Val Thr Ala Glu Phe Met Glu Ala Leu
        355                 360                 365

Arg Asp Val Asn Val Asn Ile Glu Leu Ile Ser Thr Ser Glu Ile Arg
    370                 375                 380

Ile Ser Val Leu Ile Arg Glu Asp Asp Leu Asp Ala Ala Ala Arg Ala
385                 390                 395                 400

Leu His Glu Gln Phe Gln Leu Gly Gly Glu Asp Glu Ala Val Val Tyr
                405                 410                 415

Ala Gly Thr Gly Arg
            420
```

<210> 80
<211> 810
<212> PRT

<213> Escherichia coli

<400> 80

Met Ser Val Ile Ala Gln Ala Gly Ala Lys Gly Arg Gln Leu His Lys
1 5 10 15

Phe Gly Gly Ser Ser Leu Ala Asp Val Lys Cys Tyr Leu Arg Val Ala
20 25 30

Gly Ile Met Ala Glu Tyr Ser Gln Pro Asp Asp Met Met Val Val Ser
35 40 45

Ala Ala Gly Ser Thr Thr Asn Gln Leu Ile Asn Trp Leu Lys Leu Ser
50 55 60

Gln Thr Asp Arg Leu Ser Ala His Gln Val Gln Gln Thr Leu Arg Arg
65 70 75 80

Tyr Gln Cys Asp Leu Ile Ser Gly Leu Leu Pro Ala Glu Glu Ala Asp
85 90 95

Ser Leu Ile Ser Ala Phe Val Ser Asp Leu Glu Arg Leu Ala Ala Leu
100 105 110

Leu Asp Ser Gly Ile Asn Asp Ala Val Tyr Ala Glu Val Val Gly His
115 120 125

Gly Glu Val Trp Ser Ala Arg Leu Met Ser Ala Val Leu Asn Gln Gln
130 135 140

Gly Leu Pro Ala Ala Trp Leu Asp Ala Arg Glu Phe Leu Arg Ala Glu
145 150 155 160

Arg Ala Ala Gln Pro Gln Val Asp Glu Gly Leu Ser Tyr Pro Leu Leu
165 170 175

Gln Gln Leu Leu Val Gln His Pro Gly Lys Arg Leu Val Val Thr Gly
180 185 190

Phe Ile Ser Arg Asn Asn Ala Gly Glu Thr Val Leu Leu Gly Arg Asn
195 200 205

Gly Ser Asp Tyr Ser Ala Thr Gln Ile Gly Ala Leu Ala Gly Val Ser
210 215 220

Arg Val Thr Ile Trp Ser Asp Val Ala Gly Val Tyr Ser Ala Asp Pro
225 230 235 240

```
Arg Lys Val Lys Asp Ala Cys Leu Leu Pro Leu Leu Arg Leu Asp Glu
                245                 250                 255

Ala Ser Glu Leu Ala Arg Leu Ala Ala Pro Val Leu His Ala Arg Thr
            260                 265                 270

Leu Gln Pro Val Ser Gly Ser Glu Ile Asp Leu Gln Leu Arg Cys Ser
        275                 280                 285

Tyr Thr Pro Asp Gln Gly Ser Thr Arg Ile Glu Arg Val Leu Ala Ser
    290                 295                 300

Gly Thr Gly Ala Arg Ile Val Thr Ser His Asp Asp Val Cys Leu Ile
305                 310                 315                 320

Glu Phe Gln Val Pro Ala Ser Gln Asp Phe Lys Leu Ala His Lys Glu
                325                 330                 335

Ile Asp Gln Ile Leu Lys Arg Ala Gln Val Arg Pro Leu Ala Val Gly
            340                 345                 350

Val His Asn Asp Arg Gln Leu Leu Gln Phe Cys Tyr Thr Ser Glu Val
        355                 360                 365

Ala Asp Ser Ala Leu Lys Ile Leu Asp Glu Ala Gly Leu Pro Gly Glu
    370                 375                 380

Leu Arg Leu Arg Gln Gly Leu Ala Leu Val Ala Met Val Gly Ala Gly
385                 390                 395                 400

Val Thr Arg Asn Pro Leu His Cys His Arg Phe Trp Gln Gln Leu Lys
                405                 410                 415

Gly Gln Pro Val Glu Phe Thr Trp Gln Ser Asp Asp Gly Ile Ser Leu
            420                 425                 430

Val Ala Val Leu Arg Thr Gly Pro Thr Glu Ser Leu Ile Gln Gly Leu
        435                 440                 445

His Gln Ser Val Phe Arg Ala Glu Lys Arg Ile Gly Leu Val Leu Phe
    450                 455                 460

Gly Lys Gly Asn Ile Gly Ser Arg Trp Leu Glu Leu Phe Ala Arg Glu
465                 470                 475                 480

Gln Ser Thr Leu Ser Ala Arg Thr Gly Phe Glu Phe Val Leu Ala Gly
                485                 490                 495
```

Val Val Asp Ser Arg Arg Ser Leu Leu Ser Tyr Asp Gly Leu Asp Ala
500 505 510

Ser Arg Ala Leu Ala Phe Phe Asn Asp Glu Ala Val Glu Gln Asp Glu
515 520 525

Glu Ser Leu Phe Leu Trp Met Arg Ala His Pro Tyr Asp Asp Leu Val
530 535 540

Val Leu Asp Val Thr Ala Ser Gln Gln Leu Ala Asp Gln Tyr Leu Asp
545 550 555 560

Phe Ala Ser His Gly Phe His Val Ile Ser Ala Asn Lys Leu Ala Gly
565 570 575

Ala Ser Asp Ser Asn Lys Tyr Arg Gln Ile His Asp Ala Phe Glu Lys
580 585 590

Thr Gly Arg His Trp Leu Tyr Asn Ala Thr Val Gly Ala Gly Leu Pro
595 600 605

Ile Asn His Thr Val Arg Asp Leu Ile Asp Ser Gly Asp Thr Ile Leu
610 615 620

Ser Ile Ser Gly Ile Phe Ser Gly Thr Leu Ser Trp Leu Phe Leu Gln
625 630 635 640

Phe Asp Gly Ser Val Pro Phe Thr Glu Leu Val Asp Gln Ala Trp Gln
645 650 655

Gln Gly Leu Thr Glu Pro Asp Pro Arg Asp Asp Leu Ser Gly Lys Asp
660 665 670

Val Met Arg Lys Leu Val Ile Leu Ala Arg Glu Ala Gly Tyr Asn Ile
675 680 685

Glu Pro Asp Gln Val Arg Val Glu Ser Leu Val Pro Ala His Cys Glu
690 695 700

Gly Gly Ser Ile Asp His Phe Phe Glu Asn Gly Asp Glu Leu Asn Glu
705 710 715 720

Gln Met Val Gln Arg Leu Glu Ala Ala Arg Glu Met Gly Leu Val Leu
725 730 735

Arg Tyr Val Ala Arg Phe Asp Ala Asn Gly Lys Ala Arg Val Gly Val

```
            740                 745                 750

Glu Ala Val Arg Glu Asp His Pro Leu Ala Ser Leu Leu Pro Cys Asp
        755                 760                 765

Asn Val Phe Ala Ile Glu Ser Arg Trp Tyr Arg Asp Asn Pro Leu Val
    770                 775                 780

Ile Arg Gly Pro Gly Ala Gly Arg Asp Val Thr Ala Gly Ala Ile Gln
785                 790                 795                 800

Ser Asp Ile Asn Arg Leu Ala Gln Leu Leu
                805                 810
```

<210> 81
<211> 309
<212> PRT
<213> Corynebacterium glutamicum

<400> 81

```
Met Ala Ile Glu Leu Asn Val Gly Arg Lys Val Thr Val Thr Val Pro
1               5                   10                  15

Gly Ser Ser Ala Asn Leu Gly Pro Gly Phe Asp Thr Leu Gly Leu Ala
            20                  25                  30

Leu Ser Val Tyr Asp Thr Val Glu Val Glu Ile Ile Pro Ser Gly Leu
        35                  40                  45

Glu Val Glu Val Phe Gly Glu Gly Gln Gly Glu Val Pro Leu Asp Gly
    50                  55                  60

Ser His Leu Val Val Lys Ala Ile Arg Ala Gly Leu Lys Ala Ala Asp
65                  70                  75                  80

Ala Glu Val Pro Gly Leu Arg Val Val Cys His Asn Asn Ile Pro Gln
                85                  90                  95

Ser Arg Gly Leu Gly Ser Ser Ala Ala Ala Ala Val Ala Gly Val Ala
            100                 105                 110

Ala Ala Asn Gly Leu Ala Asp Phe Pro Leu Thr Gln Glu Gln Ile Val
        115                 120                 125

Gln Leu Ser Ser Ala Phe Glu Gly His Pro Asp Asn Ala Ala Ala Ser
    130                 135                 140

Val Leu Gly Gly Ala Val Val Ser Trp Thr Asn Leu Ser Ile Asp Gly
```

145 150 155 160

Lys Ser Gln Pro Gln Tyr Ala Ala Val Pro Leu Glu Val Gln Asp Asn
165 170 175

Ile Arg Ala Thr Ala Leu Val Pro Asn Phe His Ala Ser Thr Glu Ala
180 185 190

Val Arg Arg Val Leu Pro Thr Glu Val Thr His Ile Asp Ala Arg Phe
195 200 205

Asn Val Ser Arg Val Ala Val Met Ile Val Ala Leu Gln Gln Arg Pro
210 215 220

Asp Leu Leu Trp Glu Gly Thr Arg Asp Arg Leu His Gln Pro Tyr Arg
225 230 235 240

Ala Glu Val Leu Pro Ile Thr Ser Glu Trp Val Asn Arg Leu Arg Asn
245 250 255

Arg Gly Tyr Ala Ala Tyr Leu Ser Gly Ala Gly Pro Thr Ala Met Val
260 265 270

Leu Ser Thr Glu Pro Ile Pro Asp Lys Val Leu Glu Asp Ala Arg Glu
275 280 285

Ser Gly Ile Lys Val Leu Glu Leu Glu Val Ala Gly Pro Val Lys Val
290 295 300

Glu Val Asn Gln Pro
305

<210> 82
<211> 344
<212> PRT
<213> Corynebacterium glutamicum

<400> 82

Met Thr Thr Ile Ala Val Val Gly Ala Thr Gly Gln Val Gly Gln Val
1 5 10 15

Met Arg Thr Leu Leu Glu Glu Arg Asn Phe Pro Ala Asp Thr Val Arg
20 25 30

Phe Phe Ala Ser Pro Arg Ser Ala Gly Arg Lys Ile Glu Phe Arg Gly
35 40 45

Thr Glu Ile Glu Val Glu Asp Ile Thr Gln Ala Thr Glu Glu Ser Leu

50 55 60

Lys Asp Ile Asp Val Ala Leu Phe Ser Ala Gly Gly Thr Ala Ser Lys
65 70 75 80

Gln Tyr Ala Pro Leu Phe Ala Ala Ala Gly Ala Thr Val Val Asp Asn
85 90 95

Ser Ser Ala Trp Arg Lys Asp Asp Glu Val Pro Leu Ile Val Ser Glu
100 105 110

Val Asn Pro Ser Asp Lys Asp Ser Leu Val Lys Gly Ile Ile Ala Asn
115 120 125

Pro Asn Cys Thr Thr Met Ala Ala Met Pro Val Leu Lys Pro Leu His
130 135 140

Asp Ala Ala Gly Leu Val Lys Leu His Val Ser Ser Tyr Gln Ala Val
145 150 155 160

Ser Gly Ser Gly Leu Ala Gly Val Glu Thr Leu Ala Lys Gln Val Ala
165 170 175

Ala Val Gly Asp His Asn Val Glu Phe Val His Asp Gly Gln Ala Ala
180 185 190

Asp Ala Gly Asp Val Gly Pro Tyr Val Ser Pro Ile Ala Tyr Asn Val
195 200 205

Leu Pro Phe Ala Gly Asn Leu Val Asp Asp Gly Thr Phe Glu Thr Asp
210 215 220

Glu Glu Gln Lys Leu Arg Asn Glu Ser Arg Lys Ile Leu Gly Leu Pro
225 230 235 240

Asp Leu Lys Val Ser Gly Thr Cys Val Arg Val Pro Val Phe Thr Gly
245 250 255

His Thr Leu Thr Ile His Ala Glu Phe Asp Lys Ala Ile Thr Val Asp
260 265 270

Gln Ala Gln Glu Ile Leu Gly Ala Ala Ser Gly Val Lys Leu Val Asp
275 280 285

Val Pro Thr Pro Leu Ala Ala Ala Gly Ile Asp Glu Ser Leu Val Gly
290 295 300

Arg Ile Arg Gln Asp Ser Thr Val Asp Asp Asn Arg Gly Leu Val Leu
305 310 315 320

Val Val Ser Gly Asp Asn Leu Arg Lys Gly Ala Ala Leu Asn Thr Ile
325 330 335

Gln Ile Ala Glu Leu Leu Val Lys
340

<210> 83
<211> 481
<212> PRT
<213> Corynebacterium glutamicum

<400> 83

Met Asp Tyr Ile Ser Thr Arg Asp Ala Ser Arg Thr Pro Ala Arg Phe
1 5 10 15

Ser Asp Ile Leu Leu Gly Gly Leu Ala Pro Asp Gly Gly Leu Tyr Leu
20 25 30

Pro Ala Thr Tyr Pro Gln Leu Asp Asp Ala Gln Leu Ser Lys Trp Arg
35 40 45

Glu Val Leu Ala Asn Glu Gly Tyr Ala Ala Leu Ala Ala Glu Val Ile
50 55 60

Ser Leu Phe Val Asp Asp Ile Pro Val Glu Asp Ile Lys Ala Ile Thr
65 70 75 80

Ala Arg Ala Tyr Thr Tyr Pro Lys Phe Asn Ser Glu Asp Ile Val Pro
85 90 95

Val Thr Glu Leu Glu Asp Asn Ile Tyr Leu Gly His Leu Ser Glu Gly
100 105 110

Pro Thr Ala Ala Phe Lys Asp Met Ala Met Gln Leu Leu Gly Glu Leu
115 120 125

Phe Glu Tyr Glu Leu Arg Arg Arg Asn Glu Thr Ile Asn Ile Leu Gly
130 135 140

Ala Thr Ser Gly Asp Thr Gly Ser Ser Ala Glu Tyr Ala Met Arg Gly
145 150 155 160

Arg Glu Gly Ile Arg Val Phe Met Leu Thr Pro Ala Gly Arg Met Thr
165 170 175

Pro Phe Gln Gln Ala Gln Met Phe Gly Leu Asp Asp Pro Asn Ile Phe
180 185 190

Asn Ile Ala Leu Asp Gly Val Phe Asp Asp Cys Gln Asp Val Val Lys
195 200 205

Ala Val Ser Ala Asp Ala Glu Phe Lys Lys Asp Asn Arg Ile Gly Ala
210 215 220

Val Asn Ser Ile Asn Trp Ala Arg Leu Met Ala Gln Val Val Tyr Tyr
225 230 235 240

Val Ser Ser Trp Ile Arg Thr Thr Thr Ser Asn Asp Gln Lys Val Ser
245 250 255

Phe Ser Val Pro Thr Gly Asn Phe Gly Asp Ile Cys Ala Gly His Ile
260 265 270

Ala Arg Gln Met Gly Leu Pro Ile Asp Arg Leu Ile Val Ala Thr Asn
275 280 285

Glu Asn Asp Val Leu Asp Glu Phe Phe Arg Thr Gly Asp Tyr Arg Val
290 295 300

Arg Ser Ser Ala Asp Thr His Glu Thr Ser Ser Pro Ser Met Asp Ile
305 310 315 320

Ser Arg Ala Ser Asn Phe Glu Arg Phe Ile Phe Asp Leu Leu Gly Arg
325 330 335

Asp Ala Thr Arg Val Asn Asp Leu Phe Gly Thr Gln Val Arg Gln Gly
340 345 350

Gly Phe Ser Leu Ala Asp Asp Ala Asn Phe Glu Lys Ala Ala Ala Glu
355 360 365

Tyr Gly Phe Ala Ser Gly Arg Ser Thr His Ala Asp Arg Val Ala Thr
370 375 380

Ile Ala Asp Val His Ser Arg Leu Asp Val Leu Ile Asp Pro His Thr
385 390 395 400

Ala Asp Gly Val His Val Ala Arg Gln Trp Arg Asp Glu Val Asn Thr
405 410 415

Pro Ile Ile Val Leu Glu Thr Ala Leu Pro Val Lys Phe Ala Asp Thr
420 425 430

Ile Val Glu Ala Ile Gly Glu Ala Pro Gln Thr Pro Glu Arg Phe Ala
435 440 445

Ala Ile Met Asp Ala Pro Phe Lys Val Ser Asp Leu Pro Asn Asp Thr
450 455 460

Asp Ala Val Lys Gln Tyr Ile Val Asp Ala Ile Ala Asn Thr Ser Val
465 470 475 480

Lys

<210> 84
<211> 443
<212> PRT
<213> Escherichia coli

<400> 84

Met Ser Thr Ser Asp Ser Ile Val Ser Ser Gln Thr Lys Gln Ser Ser
1 5 10 15

Trp Arg Lys Ser Asp Thr Thr Trp Thr Leu Gly Leu Phe Gly Thr Ala
20 25 30

Ile Gly Ala Gly Val Leu Phe Phe Pro Ile Arg Ala Gly Phe Gly Gly
35 40 45

Leu Ile Pro Ile Leu Leu Met Leu Val Leu Ala Tyr Pro Ile Ala Phe
50 55 60

Tyr Cys His Arg Ala Leu Ala Arg Leu Cys Leu Ser Gly Ser Asn Pro
65 70 75 80

Ser Gly Asn Ile Thr Glu Thr Val Glu Glu His Phe Gly Lys Thr Gly
85 90 95

Gly Val Val Ile Thr Phe Leu Tyr Phe Phe Ala Ile Cys Pro Leu Leu
100 105 110

Trp Ile Tyr Gly Val Thr Ile Thr Asn Thr Phe Met Thr Phe Trp Glu
115 120 125

Asn Gln Leu Gly Phe Ala Pro Leu Asn Arg Gly Phe Val Ala Leu Phe
130 135 140

Leu Leu Leu Leu Met Ala Phe Val Ile Trp Phe Gly Lys Asp Leu Met
145 150 155 160

Val Lys Val Met Ser Tyr Leu Val Trp Pro Phe Ile Ala Ser Leu Val
165 170 175

Leu Ile Ser Leu Ser Leu Ile Pro Tyr Trp Asn Ser Ala Val Ile Asp
180 185 190

Gln Val Asp Leu Gly Ser Leu Ser Leu Thr Gly His Asp Gly Ile Leu
195 200 205

Ile Thr Val Trp Leu Gly Ile Ser Ile Met Val Phe Ser Phe Asn Phe
210 215 220

Ser Pro Ile Val Ser Ser Phe Val Val Ser Lys Arg Glu Glu Tyr Glu
225 230 235 240

Lys Asp Phe Gly Arg Asp Phe Thr Glu Arg Lys Cys Ser Gln Ile Ile
245 250 255

Ser Arg Ala Ser Met Leu Met Val Ala Val Val Met Phe Phe Ala Phe
260 265 270

Ser Cys Leu Phe Thr Leu Ser Pro Ala Asn Met Ala Glu Ala Lys Ala
275 280 285

Gln Asn Ile Pro Val Leu Ser Tyr Leu Ala Asn His Phe Ala Ser Met
290 295 300

Thr Gly Thr Lys Thr Thr Phe Ala Ile Thr Leu Glu Tyr Ala Ala Ser
305 310 315 320

Ile Ile Ala Leu Val Ala Ile Phe Lys Ser Phe Phe Gly His Tyr Leu
325 330 335

Gly Thr Leu Glu Gly Leu Asn Gly Leu Val Leu Lys Phe Gly Tyr Lys
340 345 350

Gly Asp Lys Thr Lys Val Ser Leu Gly Lys Leu Asn Thr Ile Ser Met
355 360 365

Ile Phe Ile Met Gly Ser Thr Trp Val Val Ala Tyr Ala Asn Pro Asn
370 375 380

Ile Leu Asp Leu Ile Glu Ala Met Gly Ala Pro Ile Ile Ala Ser Leu
385 390 395 400

Leu Cys Leu Leu Pro Met Tyr Ala Ile Arg Lys Ala Pro Ser Leu Ala
405 410 415

Lys Tyr Arg Gly Arg Leu Asp Asn Val Phe Val Thr Val Ile Gly Leu
            420                 425                 430

Leu Thr Ile Leu Asn Ile Val Tyr Lys Leu Phe
        435                 440

<210>  85
<211>  295
<212>  PRT
<213>  Escherichia coli

<400>  85

Met Pro Gly Ser Leu Arg Lys Met Pro Val Trp Leu Pro Ile Val Ile
1               5                   10                  15

Leu Leu Val Ala Met Ala Ser Ile Gln Gly Gly Ala Ser Leu Ala Lys
            20                  25                  30

Ser Leu Phe Pro Leu Val Gly Ala Pro Gly Val Thr Ala Leu Arg Leu
        35                  40                  45

Ala Leu Gly Thr Leu Ile Leu Ile Ala Phe Phe Lys Pro Trp Arg Leu
    50                  55                  60

Arg Phe Ala Lys Glu Gln Arg Leu Pro Leu Leu Phe Tyr Gly Val Ser
65                  70                  75                  80

Leu Gly Gly Met Asn Tyr Leu Phe Tyr Leu Ser Ile Gln Thr Val Pro
                85                  90                  95

Leu Gly Ile Ala Val Ala Leu Glu Phe Thr Gly Pro Leu Ala Val Ala
            100                 105                 110

Leu Phe Ser Ser Arg Arg Pro Val Asp Phe Val Trp Val Val Leu Ala
        115                 120                 125

Val Leu Gly Leu Trp Phe Leu Leu Pro Leu Gly Gln Asp Val Ser His
    130                 135                 140

Val Asp Leu Thr Gly Cys Ala Leu Ala Leu Gly Ala Gly Ala Cys Trp
145                 150                 155                 160

Ala Ile Tyr Ile Leu Ser Gly Gln Arg Ala Gly Ala Glu His Gly Pro
                165                 170                 175

Ala Thr Val Ala Ile Gly Ser Leu Ile Ala Ala Leu Ile Phe Val Pro
            180                 185                 190

Ile Gly Ala Leu Gln Ala Gly Glu Ala Leu Trp His Trp Ser Val Ile
195 200 205

Pro Leu Gly Leu Ala Val Ala Ile Leu Ser Thr Ala Leu Pro Tyr Ser
210 215 220

Leu Glu Met Ile Ala Leu Thr Arg Leu Pro Thr Arg Thr Phe Gly Thr
225 230 235 240

Leu Met Ser Met Glu Pro Ala Leu Ala Ala Val Ser Gly Met Ile Phe
245 250 255

Leu Gly Glu Thr Leu Thr Pro Ile Gln Leu Leu Ala Leu Gly Ala Ile
260 265 270

Ile Ala Ala Ser Met Gly Ser Thr Leu Thr Val Arg Lys Glu Ser Lys
275 280 285

Ile Lys Glu Leu Asp Ile Asn
290 295

<210> 86
<211> 206
<212> PRT
<213> Escherichia coli

<400> 86

Met Thr Leu Glu Trp Trp Phe Ala Tyr Leu Leu Thr Ser Ile Ile Leu
1 5 10 15

Ser Leu Ser Pro Gly Ser Gly Ala Ile Asn Thr Met Thr Thr Ser Leu
20 25 30

Asn His Gly Tyr Arg Gly Ala Val Ala Ser Ile Ala Gly Leu Gln Thr
35 40 45

Gly Leu Ala Ile His Ile Val Leu Val Gly Val Gly Leu Gly Thr Leu
50 55 60

Phe Ser Arg Ser Val Ile Ala Phe Glu Val Leu Lys Trp Ala Gly Ala
65 70 75 80

Ala Tyr Leu Ile Trp Leu Gly Ile Gln Gln Trp Arg Ala Ala Gly Ala
85 90 95

Ile Asp Leu Lys Ser Leu Ala Ser Thr Gln Ser Arg Arg His Leu Phe
100 105 110

Gln Arg Ala Val Phe Val Asn Leu Thr Asn Pro Lys Ser Ile Val Phe
115 120 125

Leu Ala Ala Leu Phe Pro Gln Phe Ile Met Pro Gln Gln Pro Gln Leu
130 135 140

Met Gln Tyr Ile Val Leu Gly Val Thr Thr Ile Val Val Asp Ile Ile
145 150 155 160

Val Met Ile Gly Tyr Ala Thr Leu Ala Gln Arg Ile Ala Leu Trp Ile
165 170 175

Lys Gly Pro Lys Gln Met Lys Ala Leu Asn Lys Ile Phe Gly Ser Leu
180 185 190

Phe Met Leu Val Gly Ala Leu Leu Ala Ser Ala Arg His Ala
195 200 205

<210> 87
<211> 384
<212> PRT
<213> Escherichia coli

<400> 87

Met Ala Lys His Leu Phe Thr Ser Glu Ser Val Ser Glu Gly His Pro
1 5 10 15

Asp Lys Ile Ala Asp Gln Ile Ser Asp Ala Val Leu Asp Ala Ile Leu
20 25 30

Glu Gln Asp Pro Lys Ala Arg Val Ala Cys Glu Thr Tyr Val Lys Thr
35 40 45

Gly Met Val Leu Val Gly Gly Glu Ile Thr Thr Ser Ala Trp Val Asp
50 55 60

Ile Glu Glu Ile Thr Arg Asn Thr Val Arg Glu Ile Gly Tyr Val His
65 70 75 80

Ser Asp Met Gly Phe Asp Ala Asn Ser Cys Ala Val Leu Ser Ala Ile
85 90 95

Gly Lys Gln Ser Pro Asp Ile Asn Gln Gly Val Asp Arg Ala Asp Pro
100 105 110

Leu Glu Gln Gly Ala Gly Asp Gln Gly Leu Met Phe Gly Tyr Ala Thr
115 120 125

```
Asn Glu Thr Asp Val Leu Met Pro Ala Pro Ile Thr Tyr Ala His Arg
    130                 135                 140

Leu Val Gln Arg Gln Ala Glu Val Arg Lys Asn Gly Thr Leu Pro Trp
145                 150                 155                 160

Leu Arg Pro Asp Ala Lys Ser Gln Val Thr Phe Gln Tyr Asp Asp Gly
                165                 170                 175

Lys Ile Val Gly Ile Asp Ala Val Val Leu Ser Thr Gln His Ser Glu
            180                 185                 190

Glu Ile Asp Gln Lys Ser Leu Gln Glu Ala Val Met Glu Glu Ile Ile
        195                 200                 205

Lys Pro Ile Leu Pro Ala Glu Trp Leu Thr Ser Ala Thr Lys Phe Phe
    210                 215                 220

Ile Asn Pro Thr Gly Arg Phe Val Ile Gly Gly Pro Met Gly Asp Cys
225                 230                 235                 240

Gly Leu Thr Gly Arg Lys Ile Ile Val Asp Thr Tyr Gly Gly Met Ala
                245                 250                 255

Arg His Gly Gly Gly Ala Phe Ser Gly Lys Asp Pro Ser Lys Val Asp
            260                 265                 270

Arg Ser Ala Ala Tyr Ala Ala Arg Tyr Val Ala Lys Asn Ile Val Ala
        275                 280                 285

Ala Gly Leu Ala Asp Arg Cys Glu Ile Gln Val Ser Tyr Ala Ile Gly
    290                 295                 300

Val Ala Glu Pro Thr Ser Ile Met Val Glu Thr Phe Gly Thr Glu Lys
305                 310                 315                 320

Val Pro Ser Glu Gln Leu Thr Leu Leu Val Arg Glu Phe Phe Asp Leu
                325                 330                 335

Arg Pro Tyr Gly Leu Ile Gln Met Leu Asp Leu Leu His Pro Ile Tyr
            340                 345                 350

Lys Glu Thr Ala Ala Tyr Gly His Phe Gly Arg Glu His Phe Pro Trp
        355                 360                 365

Glu Lys Thr Asp Lys Ala Gln Leu Leu Arg Asp Ala Ala Gly Leu Lys
```

370 375 380

<210> 88
<211> 652
<212> PRT
<213> Escherichia coli

<400> 88

Met Ser Gln Ile His Lys His Thr Ile Pro Ala Asn Ile Ala Asp Arg
1 5 10 15

Cys Leu Ile Asn Pro Gln Gln Tyr Glu Ala Met Tyr Gln Gln Ser Ile
20 25 30

Asn Val Pro Asp Thr Phe Trp Gly Glu Gln Gly Lys Ile Leu Asp Trp
35 40 45

Ile Lys Pro Tyr Gln Lys Val Lys Asn Thr Ser Phe Ala Pro Gly Asn
50 55 60

Val Ser Ile Lys Trp Tyr Glu Asp Gly Thr Leu Asn Leu Ala Ala Asn
65 70 75 80

Cys Leu Asp Arg His Leu Gln Glu Asn Gly Asp Arg Thr Ala Ile Ile
85 90 95

Trp Glu Gly Asp Asp Ala Ser Gln Ser Lys His Ile Ser Tyr Lys Glu
100 105 110

Leu His Arg Asp Val Cys Arg Phe Ala Asn Thr Leu Leu Glu Leu Gly
115 120 125

Ile Lys Lys Gly Asp Val Val Ala Ile Tyr Met Pro Met Val Pro Glu
130 135 140

Ala Ala Val Ala Met Leu Ala Cys Ala Arg Ile Gly Ala Val His Ser
145 150 155 160

Val Ile Phe Gly Gly Phe Ser Pro Glu Ala Val Ala Gly Arg Ile Ile
165 170 175

Asp Ser Asn Ser Arg Leu Val Ile Thr Ser Asp Glu Gly Val Arg Ala
180 185 190

Gly Arg Ser Ile Pro Leu Lys Lys Asn Val Asp Asp Ala Leu Lys Asn
195 200 205

Pro Asn Val Thr Ser Val Glu His Val Val Val Leu Lys Arg Thr Gly

210 215 220

Gly Lys Ile Asp Trp Gln Glu Gly Arg Asp Leu Trp Trp His Asp Leu
225 230 235 240

Val Glu Gln Ala Ser Asp Gln His Gln Ala Glu Glu Met Asn Ala Glu
245 250 255

Asp Pro Leu Phe Ile Leu Tyr Thr Ser Gly Ser Thr Gly Lys Pro Lys
260 265 270

Gly Val Leu His Thr Thr Gly Gly Tyr Leu Val Tyr Ala Ala Leu Thr
275 280 285

Phe Lys Tyr Val Phe Asp Tyr His Pro Gly Asp Ile Tyr Trp Cys Thr
290 295 300

Ala Asp Val Gly Trp Val Thr Gly His Ser Tyr Leu Leu Tyr Gly Pro
305 310 315 320

Leu Ala Cys Gly Ala Thr Thr Leu Met Phe Glu Gly Val Pro Asn Trp
325 330 335

Pro Thr Pro Ala Arg Met Ala Gln Val Val Asp Lys His Gln Val Asn
340 345 350

Ile Leu Tyr Thr Ala Pro Thr Ala Ile Arg Ala Leu Met Ala Glu Gly
355 360 365

Asp Lys Ala Ile Glu Gly Thr Asp Arg Ser Ser Leu Arg Ile Leu Gly
370 375 380

Ser Val Gly Glu Pro Ile Asn Pro Glu Ala Trp Glu Trp Tyr Trp Lys
385 390 395 400

Lys Ile Gly Asn Glu Lys Cys Pro Val Val Asp Thr Trp Trp Gln Thr
405 410 415

Glu Thr Gly Gly Phe Met Ile Thr Pro Leu Pro Gly Ala Thr Glu Leu
420 425 430

Lys Ala Gly Ser Ala Thr Arg Pro Phe Phe Gly Val Gln Pro Ala Leu
435 440 445

Val Asp Asn Glu Gly Asn Pro Leu Glu Gly Ala Thr Glu Gly Ser Leu
450 455 460

Val Ile Thr Asp Ser Trp Pro Gly Gln Ala Arg Thr Leu Phe Gly Asp
465 470 475 480

His Glu Arg Phe Glu Gln Thr Tyr Phe Ser Thr Phe Lys Asn Met Tyr
485 490 495

Phe Ser Gly Asp Gly Ala Arg Arg Asp Glu Asp Gly Tyr Tyr Trp Ile
500 505 510

Thr Gly Arg Val Asp Asp Val Leu Asn Val Ser Gly His Arg Leu Gly
515 520 525

Thr Ala Glu Ile Glu Ser Ala Leu Val Ala His Pro Lys Ile Ala Glu
530 535 540

Ala Ala Val Val Gly Ile Pro His Asn Ile Lys Gly Gln Ala Ile Tyr
545 550 555 560

Ala Tyr Val Thr Leu Asn His Gly Glu Glu Pro Ser Pro Glu Leu Tyr
565 570 575

Ala Glu Val Arg Asn Trp Val Arg Lys Glu Ile Gly Pro Leu Ala Thr
580 585 590

Pro Asp Val Leu His Trp Thr Asp Ser Leu Pro Lys Thr Arg Ser Gly
595 600 605

Lys Ile Met Arg Arg Ile Leu Arg Lys Ile Ala Ala Gly Asp Thr Ser
610 615 620

Asn Leu Gly Asp Thr Ser Thr Leu Ala Asp Pro Gly Val Val Glu Lys
625 630 635 640

Leu Leu Glu Glu Lys Gln Ala Ile Ala Met Pro Ser
645 650

<210> 89
<211> 400
<212> PRT
<213> Escherichia coli

<400> 89

Met Ser Ser Lys Leu Val Leu Val Leu Asn Cys Gly Ser Ser Ser Leu
1 5 10 15

Lys Phe Ala Ile Ile Asp Ala Val Asn Gly Glu Glu Tyr Leu Ser Gly
20 25 30

```
Leu Ala Glu Cys Phe His Leu Pro Glu Ala Arg Ile Lys Trp Lys Met
        35                  40                  45

Asp Gly Asn Lys Gln Glu Ala Ala Leu Gly Ala Gly Ala Ala His Ser
    50                  55                  60

Glu Ala Leu Asn Phe Ile Val Asn Thr Ile Leu Ala Gln Lys Pro Glu
65                  70                  75                  80

Leu Ser Ala Gln Leu Thr Ala Ile Gly His Arg Ile Val His Gly Gly
                85                  90                  95

Glu Lys Tyr Thr Ser Ser Val Val Ile Asp Glu Ser Val Ile Gln Gly
            100                 105                 110

Ile Lys Asp Ala Ala Ser Phe Ala Pro Leu His Asn Pro Ala His Leu
        115                 120                 125

Ile Gly Ile Glu Glu Ala Leu Lys Ser Phe Pro Gln Leu Lys Asp Lys
    130                 135                 140

Asn Val Ala Val Phe Asp Thr Ala Phe His Gln Thr Met Pro Glu Glu
145                 150                 155                 160

Ser Tyr Leu Tyr Ala Leu Pro Tyr Asn Leu Tyr Lys Glu His Gly Ile
                165                 170                 175

Arg Arg Tyr Gly Ala His Gly Thr Ser His Phe Tyr Val Thr Gln Glu
            180                 185                 190

Ala Ala Lys Met Leu Asn Lys Pro Val Glu Glu Leu Asn Ile Ile Thr
        195                 200                 205

Cys His Leu Gly Asn Gly Gly Ser Val Ser Ala Ile Arg Asn Gly Lys
    210                 215                 220

Cys Val Asp Thr Ser Met Gly Leu Thr Pro Leu Glu Gly Leu Val Met
225                 230                 235                 240

Gly Thr Arg Ser Gly Asp Ile Asp Pro Ala Ile Ile Phe His Leu His
                245                 250                 255

Asp Thr Leu Gly Met Ser Val Asp Ala Ile Asn Lys Leu Leu Thr Lys
            260                 265                 270

Glu Ser Gly Leu Leu Gly Leu Thr Glu Val Thr Ser Asp Cys Arg Tyr
        275                 280                 285
```

Val Glu Asp Asn Tyr Ala Thr Lys Glu Asp Ala Lys Arg Ala Met Asp
    290                 295                 300

Val Tyr Cys His Arg Leu Ala Lys Tyr Ile Gly Ala Tyr Thr Ala Leu
305                 310                 315                 320

Met Asp Gly Arg Leu Asp Ala Val Val Phe Thr Gly Gly Ile Gly Glu
                325                 330                 335

Asn Ala Ala Met Val Arg Glu Leu Ser Leu Gly Lys Leu Gly Val Leu
            340                 345                 350

Gly Phe Glu Val Asp His Glu Arg Asn Leu Ala Ala Arg Phe Gly Lys
        355                 360                 365

Ser Gly Phe Ile Asn Lys Glu Gly Thr Arg Pro Ala Val Val Ile Pro
    370                 375                 380

Thr Asn Glu Glu Leu Val Ile Ala Gln Asp Ala Ser Arg Leu Thr Ala
385                 390                 395                 400

<210> 90
<211> 714
<212> PRT
<213> Escherichia coli

<400> 90

Met Ser Arg Ile Ile Met Leu Ile Pro Thr Gly Thr Ser Val Gly Leu
1               5                   10                  15

Thr Ser Val Ser Leu Gly Val Ile Arg Ala Met Glu Arg Lys Gly Val
            20                  25                  30

Arg Leu Ser Val Phe Lys Pro Ile Ala Gln Pro Arg Thr Gly Gly Asp
        35                  40                  45

Ala Pro Asp Gln Thr Thr Thr Ile Val Arg Ala Asn Ser Ser Thr Thr
    50                  55                  60

Thr Ala Ala Glu Pro Leu Lys Met Ser Tyr Val Glu Gly Leu Leu Ser
65                  70                  75                  80

Ser Asn Gln Lys Asp Val Leu Met Glu Glu Ile Val Ala Asn Tyr His
                85                  90                  95

Ala Asn Thr Lys Asp Ala Glu Val Val Leu Val Glu Gly Leu Val Pro
            100                 105                 110

Thr Arg Lys His Gln Phe Ala Gln Ser Leu Asn Tyr Glu Ile Ala Lys
115 120 125

Thr Leu Asn Ala Glu Ile Val Phe Val Met Ser Gln Gly Thr Asp Thr
130 135 140

Pro Glu Gln Leu Lys Glu Arg Ile Glu Leu Thr Arg Asn Ser Phe Gly
145 150 155 160

Gly Ala Lys Asn Thr Asn Ile Thr Gly Val Ile Val Asn Lys Leu Asn
165 170 175

Ala Pro Val Asp Glu Gln Gly Arg Thr Arg Pro Asp Leu Ser Glu Ile
180 185 190

Phe Asp Asp Ser Ser Lys Ala Lys Val Asn Asn Val Asp Pro Ala Lys
195 200 205

Leu Gln Glu Ser Ser Pro Leu Pro Val Leu Gly Ala Val Pro Trp Ser
210 215 220

Phe Asp Leu Ile Ala Thr Arg Ala Ile Asp Met Ala Arg His Leu Asn
225 230 235 240

Ala Thr Ile Ile Asn Glu Gly Asp Ile Asn Thr Arg Arg Val Lys Ser
245 250 255

Val Thr Phe Cys Ala Arg Ser Ile Pro His Met Leu Glu His Phe Arg
260 265 270

Ala Gly Ser Leu Leu Val Thr Ser Ala Asp Arg Pro Asp Val Leu Val
275 280 285

Ala Ala Cys Leu Ala Ala Met Asn Gly Val Glu Ile Gly Ala Leu Leu
290 295 300

Leu Thr Gly Gly Tyr Glu Met Asp Ala Arg Ile Ser Lys Leu Cys Glu
305 310 315 320

Arg Ala Phe Ala Thr Gly Leu Pro Val Phe Met Val Asn Thr Asn Thr
325 330 335

Trp Gln Thr Ser Leu Ser Leu Gln Ser Phe Asn Leu Glu Val Pro Val
340 345 350

Asp Asp His Glu Arg Ile Glu Lys Val Gln Glu Tyr Val Ala Asn Tyr
355 360 365

Ile Asn Ala Asp Trp Ile Glu Ser Leu Thr Ala Thr Ser Glu Arg Ser
370 375 380

Arg Arg Leu Ser Pro Pro Ala Phe Arg Tyr Gln Leu Thr Glu Leu Ala
385 390 395 400

Arg Lys Ala Gly Lys Arg Ile Val Leu Pro Glu Gly Asp Glu Pro Arg
405 410 415

Thr Val Lys Ala Ala Ala Ile Cys Ala Glu Arg Gly Ile Ala Thr Cys
420 425 430

Val Leu Leu Gly Asn Pro Ala Glu Ile Asn Arg Val Ala Ala Ser Gln
435 440 445

Gly Val Glu Leu Gly Ala Gly Ile Glu Ile Val Asp Pro Glu Val Val
450 455 460

Arg Glu Ser Tyr Val Gly Arg Leu Val Glu Leu Arg Lys Asn Lys Gly
465 470 475 480

Met Thr Glu Thr Val Ala Arg Glu Gln Leu Glu Asp Asn Val Val Leu
485 490 495

Gly Thr Leu Met Leu Glu Gln Asp Glu Val Asp Gly Leu Val Ser Gly
500 505 510

Ala Val His Thr Thr Ala Asn Thr Ile Arg Pro Pro Leu Gln Leu Ile
515 520 525

Lys Thr Ala Pro Gly Ser Ser Leu Val Ser Ser Val Phe Phe Met Leu
530 535 540

Leu Pro Glu Gln Val Tyr Val Tyr Gly Asp Cys Ala Ile Asn Pro Asp
545 550 555 560

Pro Thr Ala Glu Gln Leu Ala Glu Ile Ala Ile Gln Ser Ala Asp Ser
565 570 575

Ala Ala Ala Phe Gly Ile Glu Pro Arg Val Ala Met Leu Ser Tyr Ser
580 585 590

Thr Gly Thr Ser Gly Ala Gly Ser Asp Val Glu Lys Val Arg Glu Ala
595 600 605

Thr Arg Leu Ala Gln Glu Lys Arg Pro Asp Leu Met Ile Asp Gly Pro

610 615 620

Leu Gln Tyr Asp Ala Ala Val Met Ala Asp Val Ala Lys Ser Lys Ala
625 630 635 640

Pro Asn Ser Pro Val Ala Gly Arg Ala Thr Val Phe Ile Phe Pro Asp
645 650 655

Leu Asn Thr Gly Asn Thr Thr Tyr Lys Ala Val Gln Arg Ser Ala Asp
660 665 670

Leu Ile Ser Ile Gly Pro Met Leu Gln Gly Met Arg Lys Pro Val Asn
675 680 685

Asp Leu Ser Arg Gly Ala Leu Val Asp Asp Ile Val Tyr Thr Ile Ala
690 695 700

Leu Thr Ala Ile Gln Ser Ala Gln Gln Gln
705 710

<210> 91
<211> 345
<212> PRT
<213> Corynebacterium glutamicum

<400> 91

Met Thr Thr Ala Ala Pro Gln Glu Phe Thr Ala Ala Val Val Glu Lys
1 5 10 15

Phe Gly His Asp Val Thr Val Lys Asp Ile Asp Leu Pro Lys Pro Gly
20 25 30

Pro His Gln Ala Leu Val Lys Val Leu Thr Ser Gly Ile Cys His Thr
35 40 45

Asp Leu His Ala Leu Glu Gly Asp Trp Pro Val Lys Pro Glu Pro Pro
50 55 60

Phe Val Pro Gly His Glu Gly Val Gly Glu Val Val Glu Leu Gly Pro
65 70 75 80

Gly Glu His Asp Val Lys Val Gly Asp Ile Val Gly Asn Ala Trp Leu
85 90 95

Trp Ser Ala Cys Gly Thr Cys Glu Tyr Cys Ile Thr Gly Arg Glu Thr
100 105 110

Gln Cys Asn Glu Ala Glu Tyr Gly Gly Tyr Thr Gln Asn Gly Ser Phe

115 120 125

Gly Gln Tyr Met Leu Val Asp Thr Arg Tyr Ala Ala Arg Ile Pro Asp
130 135 140

Gly Val Asp Tyr Leu Glu Ala Ala Pro Ile Leu Cys Ala Gly Val Thr
145 150 155 160

Val Tyr Lys Ala Leu Lys Val Ser Glu Thr Arg Pro Gly Gln Phe Met
165 170 175

Val Ile Ser Gly Val Gly Gly Leu Gly His Ile Ala Val Gln Tyr Ala
180 185 190

Ala Ala Met Gly Met Arg Val Ile Ala Val Asp Ile Ala Asp Asp Lys
195 200 205

Leu Glu Leu Ala Arg Lys His Gly Ala Glu Phe Thr Val Asn Ala Arg
210 215 220

Asn Glu Asp Ser Gly Glu Ala Val Gln Lys Tyr Thr Asn Gly Gly Ala
225 230 235 240

His Gly Val Leu Val Thr Ala Val His Glu Ala Ala Phe Gly Gln Ala
245 250 255

Leu Asp Met Ala Arg Arg Ala Gly Thr Ile Val Phe Asn Gly Leu Pro
260 265 270

Pro Gly Glu Phe Pro Ala Ser Val Phe Asn Ile Val Phe Lys Gly Leu
275 280 285

Thr Ile Arg Gly Ser Leu Val Gly Thr Arg Gln Asp Leu Ala Glu Ala
290 295 300

Leu Asp Phe Phe Ala Arg Gly Leu Ile Lys Pro Thr Val Ser Glu Cys
305 310 315 320

Ser Leu Asp Glu Val Asn Gly Val Leu Asp Arg Met Arg Asn Gly Lys
325 330 335

Ile Asp Gly Arg Val Ala Ile Arg Phe
340 345

<210> 92
<211> 610
<212> PRT
<213> Corynebacterium glutamicum

<400> 92

Met Thr Thr Ala Ala Ile Arg Gly Leu Gln Gly Glu Ala Pro Thr Lys
1 5 10 15

Asn Lys Glu Leu Leu Asn Trp Ile Ala Asp Ala Val Glu Leu Phe Gln
20 25 30

Pro Glu Ala Val Val Phe Val Asp Gly Ser Gln Ala Glu Trp Asp Arg
35 40 45

Met Ala Glu Asp Leu Val Glu Ala Gly Thr Leu Ile Lys Leu Asn Glu
50 55 60

Glu Lys Arg Pro Asn Ser Tyr Leu Ala Arg Ser Asn Pro Ser Asp Val
65 70 75 80

Ala Arg Val Glu Ser Arg Thr Phe Ile Cys Ser Glu Lys Glu Glu Asp
85 90 95

Ala Gly Pro Thr Asn Asn Trp Ala Pro Pro Gln Ala Met Lys Asp Glu
100 105 110

Met Ser Lys His Tyr Ala Gly Ser Met Lys Gly Arg Thr Met Tyr Val
115 120 125

Val Pro Phe Cys Met Gly Pro Ile Ser Asp Pro Asp Pro Lys Leu Gly
130 135 140

Val Gln Leu Thr Asp Ser Glu Tyr Val Val Met Ser Met Arg Ile Met
145 150 155 160

Thr Arg Met Gly Ile Glu Ala Leu Asp Lys Ile Gly Ala Asn Gly Ser
165 170 175

Phe Val Arg Cys Leu His Ser Val Gly Ala Pro Leu Glu Pro Gly Gln
180 185 190

Glu Asp Val Ala Trp Pro Cys Asn Asp Thr Lys Tyr Ile Thr Gln Phe
195 200 205

Pro Glu Thr Lys Glu Ile Trp Ser Tyr Gly Ser Gly Tyr Gly Gly Asn
210 215 220

Ala Ile Leu Ala Lys Lys Cys Tyr Ala Leu Arg Ile Ala Ser Val Met
225 230 235 240

```
Ala Arg Glu Glu Gly Trp Met Ala Glu His Met Leu Ile Leu Lys Leu
                245                 250                 255

Ile Asn Pro Glu Gly Lys Ala Tyr His Ile Ala Ala Ala Phe Pro Ser
            260                 265                 270

Ala Cys Gly Lys Thr Asn Leu Ala Met Ile Thr Pro Thr Ile Pro Gly
        275                 280                 285

Trp Thr Ala Gln Val Val Gly Asp Asp Ile Ala Trp Leu Lys Leu Arg
    290                 295                 300

Glu Asp Gly Leu Tyr Ala Val Asn Pro Glu Asn Gly Phe Phe Gly Val
305                 310                 315                 320

Ala Pro Gly Thr Asn Tyr Ala Ser Asn Pro Ile Ala Met Lys Thr Met
                325                 330                 335

Glu Pro Gly Asn Thr Leu Phe Thr Asn Val Ala Leu Thr Asp Asp Gly
            340                 345                 350

Asp Ile Trp Trp Glu Gly Met Asp Gly Asp Ala Pro Ala His Leu Ile
        355                 360                 365

Asp Trp Met Gly Asn Asp Trp Thr Pro Glu Ser Asp Glu Asn Ala Ala
    370                 375                 380

His Pro Asn Ser Arg Tyr Cys Val Ala Ile Asp Gln Ser Pro Ala Ala
385                 390                 395                 400

Ala Pro Glu Phe Asn Asp Trp Glu Gly Val Lys Ile Asp Ala Ile Leu
                405                 410                 415

Phe Gly Gly Arg Arg Ala Asp Thr Val Pro Leu Val Thr Gln Thr Tyr
            420                 425                 430

Asp Trp Glu His Gly Thr Met Val Gly Ala Leu Leu Ala Ser Gly Gln
        435                 440                 445

Thr Ala Ala Ser Ala Glu Ala Lys Val Gly Thr Leu Arg His Asp Pro
    450                 455                 460

Met Ala Met Leu Pro Phe Ile Gly Tyr Asn Ala Gly Glu Tyr Leu Gln
465                 470                 475                 480

Asn Trp Ile Asp Met Gly Asn Lys Gly Gly Asp Lys Met Pro Ser Ile
                485                 490                 495
```

Phe Leu Val Asn Trp Phe Arg Arg Gly Glu Asp Gly Arg Phe Leu Trp
            500                 505                 510

Pro Gly Phe Gly Asp Asn Ser Arg Val Leu Lys Trp Val Ile Asp Arg
        515                 520                 525

Ile Glu Gly His Val Gly Ala Asp Glu Thr Val Val Gly His Thr Ala
    530                 535                 540

Lys Ala Glu Asp Leu Asp Leu Asp Gly Leu Asp Thr Pro Ile Glu Asp
545                 550                 555                 560

Val Lys Glu Ala Leu Thr Ala Pro Ala Glu Gln Trp Ala Asn Asp Val
                565                 570                 575

Glu Asp Asn Ala Glu Tyr Leu Thr Phe Leu Gly Pro Arg Val Pro Ala
            580                 585                 590

Glu Val His Ser Gln Phe Asp Ala Leu Lys Ala Arg Ile Ser Ala Ala
        595                 600                 605

His Ala
    610

<210> 93
<211> 540
<212> PRT
<213> Escherichia coli

<400> 93

Met Arg Val Asn Asn Gly Leu Thr Pro Gln Glu Leu Glu Ala Tyr Gly
1               5                   10                  15

Ile Ser Asp Val His Asp Ile Val Tyr Asn Pro Ser Tyr Asp Leu Leu
            20                  25                  30

Tyr Gln Glu Glu Leu Asp Pro Ser Leu Thr Gly Tyr Glu Arg Gly Val
        35                  40                  45

Leu Thr Asn Leu Gly Ala Val Ala Val Asp Thr Gly Ile Phe Thr Gly
    50                  55                  60

Arg Ser Pro Lys Asp Lys Tyr Ile Val Arg Asp Asp Thr Thr Arg Asp
65                  70                  75                  80

Thr Phe Trp Trp Ala Asp Lys Gly Lys Gly Lys Asn Asp Asn Lys Pro
                85                  90                  95

Leu Ser Pro Glu Thr Trp Gln His Leu Lys Gly Leu Val Thr Arg Gln
100 105 110

Leu Ser Gly Lys Arg Leu Phe Val Val Asp Ala Phe Cys Gly Ala Asn
115 120 125

Pro Asp Thr Arg Leu Ser Val Arg Phe Ile Thr Glu Val Ala Trp Gln
130 135 140

Ala His Phe Val Lys Asn Met Phe Ile Arg Pro Ser Asp Glu Glu Leu
145 150 155 160

Ala Gly Phe Lys Pro Asp Phe Ile Val Met Asn Gly Ala Lys Cys Thr
165 170 175

Asn Pro Gln Trp Lys Glu Gln Gly Leu Asn Ser Glu Asn Phe Val Ala
180 185 190

Phe Asn Leu Thr Glu Arg Met Gln Leu Ile Gly Gly Thr Trp Tyr Gly
195 200 205

Gly Glu Met Lys Lys Gly Met Phe Ser Met Met Asn Tyr Leu Leu Pro
210 215 220

Leu Lys Gly Ile Ala Ser Met His Cys Ser Ala Asn Val Gly Glu Lys
225 230 235 240

Gly Asp Val Ala Val Phe Phe Gly Leu Ser Gly Thr Gly Lys Thr Thr
245 250 255

Leu Ser Thr Asp Pro Lys Arg Arg Leu Ile Gly Asp Asp Glu His Gly
260 265 270

Trp Asp Asp Asp Gly Val Phe Asn Phe Glu Gly Gly Cys Tyr Ala Lys
275 280 285

Thr Ile Lys Leu Ser Lys Glu Ala Glu Pro Glu Ile Tyr Asn Ala Ile
290 295 300

Arg Arg Asp Ala Leu Leu Glu Asn Val Thr Val Arg Glu Asp Gly Thr
305 310 315 320

Ile Asp Phe Asp Asp Gly Ser Lys Thr Glu Asn Thr Arg Val Ser Tyr
325 330 335

Pro Ile Tyr His Ile Asp Asn Ile Val Lys Pro Val Ser Lys Ala Gly
340 345 350

His Ala Thr Lys Val Ile Phe Leu Thr Ala Asp Ala Phe Gly Val Leu
355 360 365

Pro Pro Val Ser Arg Leu Thr Ala Asp Gln Thr Gln Tyr His Phe Leu
370 375 380

Ser Gly Phe Thr Ala Lys Leu Ala Gly Thr Glu Arg Gly Ile Thr Glu
385 390 395 400

Pro Thr Pro Thr Phe Ser Ala Cys Phe Gly Ala Ala Phe Leu Ser Leu
405 410 415

His Pro Thr Gln Tyr Ala Glu Val Leu Val Lys Arg Met Gln Ala Ala
420 425 430

Gly Ala Gln Ala Tyr Leu Val Asn Thr Gly Trp Asn Gly Thr Gly Lys
435 440 445

Arg Ile Ser Ile Lys Asp Thr Arg Ala Ile Ile Asp Ala Ile Leu Asn
450 455 460

Gly Ser Leu Asp Asn Ala Glu Thr Phe Thr Leu Pro Met Phe Asn Leu
465 470 475 480

Ala Ile Pro Thr Glu Leu Pro Gly Val Asp Thr Lys Ile Leu Asp Pro
485 490 495

Arg Asn Thr Tyr Ala Ser Pro Glu Gln Trp Gln Glu Lys Ala Glu Thr
500 505 510

Leu Ala Lys Leu Phe Ile Asp Asn Phe Asp Lys Tyr Thr Asp Thr Pro
515 520 525

Ala Gly Ala Ala Leu Val Ala Ala Gly Pro Lys Leu
530 535 540

<210> 94
<211> 943
<212> PRT
<213> Corynebacterium glutamicum

<400> 94

Met Glu Leu Thr Val Thr Glu Ser Lys Asn Ser Phe Asn Ala Lys Ser
1 5 10 15

Thr Leu Glu Val Gly Asp Lys Ser Tyr Asp Tyr Phe Ala Leu Ser Ala
20 25 30

```
Val Pro Gly Met Glu Lys Leu Pro Tyr Ser Leu Lys Val Leu Gly Glu
        35                  40                  45

Asn Leu Leu Arg Thr Glu Asp Gly Ala Asn Ile Thr Asn Glu His Ile
    50                  55                  60

Glu Ala Ile Ala Asn Trp Asp Ala Ser Ser Asp Pro Ser Ile Glu Ile
65                  70                  75                  80

Gln Phe Thr Pro Ala Arg Val Leu Met Gln Asp Phe Thr Gly Val Pro
                85                  90                  95

Cys Val Val Asp Leu Ala Thr Met Arg Glu Ala Val Ala Ala Leu Gly
            100                 105                 110

Gly Asp Pro Asn Asp Val Asn Pro Leu Asn Pro Ala Glu Met Val Ile
        115                 120                 125

Asp His Ser Val Ile Val Glu Ala Phe Gly Arg Pro Asp Ala Leu Ala
    130                 135                 140

Lys Asn Val Glu Ile Glu Tyr Glu Arg Asn Glu Glu Arg Tyr Gln Phe
145                 150                 155                 160

Leu Arg Trp Gly Ser Glu Ser Phe Ser Asn Phe Arg Val Val Pro Pro
                165                 170                 175

Gly Thr Gly Ile Val His Gln Val Asn Ile Glu Tyr Leu Ala Arg Val
            180                 185                 190

Val Phe Asp Asn Glu Gly Leu Ala Tyr Pro Asp Thr Cys Ile Gly Thr
        195                 200                 205

Asp Ser His Thr Thr Met Glu Asn Gly Leu Gly Ile Leu Gly Trp Gly
    210                 215                 220

Val Gly Gly Ile Glu Ala Glu Ala Ala Met Leu Gly Gln Pro Val Ser
225                 230                 235                 240

Met Leu Ile Pro Arg Val Val Gly Phe Lys Leu Thr Gly Glu Ile Pro
                245                 250                 255

Val Gly Val Thr Ala Thr Asp Val Val Leu Thr Ile Thr Glu Met Leu
            260                 265                 270

Arg Asp His Gly Val Val Gln Lys Phe Val Glu Phe Tyr Gly Ser Gly
```

275 280 285

Val Lys Ala Val Pro Leu Ala Asn Arg Ala Thr Ile Gly Asn Met Ser
290 295 300

Pro Glu Phe Gly Ser Thr Cys Ala Met Phe Pro Ile Asp Glu Glu Thr
305 310 315 320

Thr Lys Tyr Leu Arg Leu Thr Gly Arg Pro Glu Glu Gln Val Ala Leu
325 330 335

Val Glu Ala Tyr Ala Lys Ala Gln Gly Met Trp Leu Asp Glu Asp Thr
340 345 350

Val Glu Ala Glu Tyr Ser Glu Tyr Leu Glu Leu Asp Leu Ser Thr Val
355 360 365

Val Pro Ser Ile Ala Gly Pro Lys Arg Pro Gln Asp Arg Ile Leu Leu
370 375 380

Ser Glu Ala Lys Glu Gln Phe Arg Lys Asp Leu Pro Thr Tyr Thr Asp
385 390 395 400

Asp Ala Val Ser Val Asp Thr Ser Ile Pro Ala Thr Arg Met Val Asn
405 410 415

Glu Gly Gly Gly Gln Pro Glu Gly Gly Val Glu Ala Asp Asn Tyr Asn
420 425 430

Ala Ser Trp Ala Gly Ser Gly Glu Ser Leu Ala Thr Gly Ala Glu Gly
435 440 445

Arg Pro Ser Lys Pro Val Thr Val Ala Ser Pro Gln Gly Gly Glu Tyr
450 455 460

Thr Ile Asp His Gly Met Val Ala Ile Ala Ser Ile Thr Ser Cys Thr
465 470 475 480

Asn Thr Ser Asn Pro Ser Val Met Ile Gly Ala Gly Leu Ile Ala Arg
485 490 495

Lys Ala Ala Glu Lys Gly Leu Lys Ser Lys Pro Trp Val Lys Thr Ile
500 505 510

Cys Ala Pro Gly Ser Gln Val Val Asp Gly Tyr Tyr Gln Arg Ala Asp
515 520 525

```
Leu Trp Lys Asp Leu Glu Ala Met Gly Phe Tyr Leu Ser Gly Phe Gly
    530                 535                 540

Cys Thr Thr Cys Ile Gly Asn Ser Gly Pro Leu Pro Glu Glu Ile Ser
545                 550                 555                 560

Ala Ala Ile Asn Glu His Asp Leu Thr Ala Thr Ala Val Leu Ser Gly
                565                 570                 575

Asn Arg Asn Phe Glu Gly Arg Ile Ser Pro Asp Val Lys Met Asn Tyr
            580                 585                 590

Leu Ala Ser Pro Ile Met Val Ile Ala Tyr Ala Ile Ala Gly Thr Met
        595                 600                 605

Asp Phe Asp Phe Glu Asn Glu Ala Leu Gly Gln Asp Gln Asp Gly Asn
    610                 615                 620

Asp Val Phe Leu Lys Asp Ile Trp Pro Ser Thr Glu Glu Ile Glu Asp
625                 630                 635                 640

Thr Ile Gln Gln Ala Ile Ser Arg Glu Leu Tyr Glu Ala Asp Tyr Ala
                645                 650                 655

Asp Val Phe Lys Gly Asp Lys Gln Trp Gln Glu Leu Asp Val Pro Thr
            660                 665                 670

Gly Asp Thr Phe Glu Trp Asp Glu Asn Ser Thr Tyr Ile Arg Lys Ala
        675                 680                 685

Pro Tyr Phe Asp Gly Met Pro Val Glu Pro Val Ala Val Thr Asp Ile
    690                 695                 700

Gln Gly Ala Arg Val Leu Ala Lys Leu Gly Asp Ser Val Thr Thr Asp
705                 710                 715                 720

His Ile Ser Pro Ala Ser Ser Ile Lys Pro Gly Thr Pro Ala Ala Gln
                725                 730                 735

Tyr Leu Asp Glu His Gly Val Glu Arg His Asp Tyr Asn Ser Leu Gly
            740                 745                 750

Ser Arg Arg Gly Asn His Glu Val Met Met Arg Gly Thr Phe Ala Asn
        755                 760                 765

Ile Arg Leu Gln Asn Gln Leu Val Asp Ile Ala Gly Gly Tyr Thr Arg
    770                 775                 780
```

Asp Phe Thr Gln Glu Gly Ala Pro Gln Ala Phe Ile Tyr Asp Ala Ser
785 790 795 800

Val Asn Tyr Lys Ala Ala Gly Ile Pro Leu Val Val Leu Gly Gly Lys
805 810 815

Glu Tyr Gly Thr Gly Ser Ser Arg Asp Trp Ala Ala Lys Gly Thr Asn
820 825 830

Leu Leu Gly Ile Arg Ala Val Ile Thr Glu Ser Phe Glu Arg Ile His
835 840 845

Arg Ser Asn Leu Ile Gly Met Gly Val Val Pro Leu Gln Phe Pro Ala
850 855 860

Gly Glu Ser His Glu Ser Leu Gly Leu Asp Gly Thr Glu Thr Phe Asp
865 870 875 880

Ile Thr Gly Leu Thr Ala Leu Asn Glu Gly Glu Thr Pro Lys Thr Val
885 890 895

Lys Val Thr Ala Thr Lys Glu Asn Gly Asp Val Val Glu Phe Asp Ala
900 905 910

Val Val Arg Ile Asp Thr Pro Gly Glu Ala Asp Tyr Tyr Arg His Gly
915 920 925

Gly Ile Leu Gln Tyr Val Leu Arg Gln Met Ala Ala Ser Ser Lys
930 935 940

<210> 95
<211> 307
<212> PRT
<213> Corynebacterium glutamicum

<400> 95

Met Ala Gly Leu Phe Ser Ser Ala Val Ala Pro Thr Glu Arg Arg Lys
1 5 10 15

Ala Leu Arg Ala Ala Leu Ala Ala Pro Glu Ile Ala Arg Met Pro Gly
20 25 30

Ala Phe Ser Pro Leu Ala Ala Arg Ala Ile Gln Glu Ala Gly Phe Glu
35 40 45

Gly Val Tyr Val Ser Gly Ala Val Val Ala Ala Asp Leu Ala Leu Pro
50 55 60

```
Asp Ile Gly Leu Thr Thr Leu Thr Glu Val Ala His Arg Ser Arg Gln
65                  70                  75                  80

Ile Ala Arg Val Thr Asp Leu Pro Val Leu Val Asp Ala Asp Thr Gly
                85                  90                  95

Phe Gly Glu Pro Met Ser Ala Ala Arg Thr Val Ser Glu Leu Glu Asp
            100                 105                 110

Ala Gly Val Ala Gly Cys His Leu Glu Asp Gln Val Asn Pro Lys Arg
        115                 120                 125

Cys Gly His Leu Asp Gly Lys Glu Val Val Gly Thr Asp Ile Met Val
    130                 135                 140

Arg Arg Ile Ala Ala Ala Val Asn Glu Arg Arg Asp Glu Gln Phe Val
145                 150                 155                 160

Ile Cys Ala Arg Thr Asp Ala Ala Gly Val Glu Gly Ile Asp Ser Ala
                165                 170                 175

Ile Glu Arg Ala Lys Ala Tyr Ala Asp Ala Gly Ala Asp Met Ile Phe
            180                 185                 190

Thr Glu Ala Leu Tyr Ser Pro Ala Asp Phe Glu Lys Phe Arg Ala Ala
        195                 200                 205

Val Asp Ile Pro Leu Leu Ala Asn Met Thr Glu Phe Gly Lys Thr Glu
    210                 215                 220

Leu Leu Pro Ala Gln Leu Leu Glu Asp Ile Gly Tyr Asn Ala Val Ile
225                 230                 235                 240

Tyr Pro Val Thr Leu Leu Arg Ile Ala Met Gly Gln Val Glu Gln Ala
                245                 250                 255

Leu Gly Asp Ile Ala Asn Thr Gly Ile Gln Thr Asp Trp Val Asp Arg
            260                 265                 270

Met Gln His Arg Ser Arg Leu Tyr Glu Leu Leu Arg Tyr Asn Glu Tyr
        275                 280                 285

Asn Ala Phe Asp Gln Gln Val Phe Thr Tyr Ser Ala Asp Ser Tyr Lys
    290                 295                 300

Pro Ile Phe
305
```

<210> 96
<211> 296
<212> PRT
<213> Escherichia coli

<400> 96

Met Ser Leu His Ser Pro Gly Lys Ala Phe Arg Ala Ala Leu Thr Lys
1 5 10 15

Glu Asn Pro Leu Gln Ile Val Gly Thr Ile Asn Ala Asn His Ala Leu
20 25 30

Leu Ala Gln Arg Ala Gly Tyr Gln Ala Ile Tyr Leu Ser Gly Gly Gly
35 40 45

Val Ala Ala Gly Ser Leu Gly Leu Pro Asp Leu Gly Ile Ser Thr Leu
50 55 60

Asp Asp Val Leu Thr Asp Ile Arg Arg Ile Thr Asp Val Cys Ser Leu
65 70 75 80

Pro Leu Leu Val Asp Ala Asp Ile Gly Phe Gly Ser Ser Ala Phe Asn
85 90 95

Val Ala Arg Thr Val Lys Ser Met Ile Lys Ala Gly Ala Ala Gly Leu
100 105 110

His Ile Glu Asp Gln Val Gly Ala Lys Arg Cys Gly His Arg Pro Asn
115 120 125

Lys Ala Ile Val Ser Lys Glu Glu Met Val Asp Arg Ile Arg Ala Ala
130 135 140

Val Asp Ala Lys Thr Asp Pro Asp Phe Val Ile Met Ala Arg Thr Asp
145 150 155 160

Ala Leu Ala Val Glu Gly Leu Asp Ala Ala Ile Glu Arg Ala Gln Ala
165 170 175

Tyr Val Glu Ala Gly Ala Glu Met Leu Phe Pro Glu Ala Ile Thr Glu
180 185 190

Leu Ala Met Tyr Arg Gln Phe Ala Asp Ala Val Gln Val Pro Ile Leu
195 200 205

Ala Asn Ile Thr Glu Phe Gly Ala Thr Pro Leu Phe Thr Thr Asp Glu
210 215 220

Leu Arg Ser Ala His Val Ala Met Ala Leu Tyr Pro Leu Ser Ala Phe
225 230 235 240

Arg Ala Met Asn Arg Ala Ala Glu His Val Tyr Asn Val Leu Arg Gln
245 250 255

Glu Gly Thr Gln Lys Ser Val Ile Asp Thr Met Gln Thr Arg Asn Glu
260 265 270

Leu Tyr Glu Ser Ile Asn Tyr Tyr Gln Tyr Glu Glu Lys Leu Asp Asn
275 280 285

Leu Phe Ala Arg Ser Gln Val Lys
290 295

<210> 97
<211> 498
<212> PRT
<213> Corynebacterium glutamicum

<400> 97

Met Arg Ile His Asp Val Tyr Thr His Leu Ser Ala Asp Asn Phe Pro
1 5 10 15

Lys Ala Glu His Leu Ala Trp Lys Phe Ser Glu Leu Ala Thr Asp Pro
20 25 30

Val Glu Val Thr Pro Asp Val Ser Glu Met Ile Ile Asn Arg Ile Ile
35 40 45

Asp Asn Ala Ala Val Ser Ala Ala Ser Val Leu Arg Arg Pro Val Thr
50 55 60

Val Ala Arg Gln Gln Ala Gln Ser His Pro Arg Glu Lys Gly Gly Lys
65 70 75 80

Val Phe Gly Ile Ser Gly Ser Tyr Ser Pro Glu Trp Ala Ala Phe Ala
85 90 95

Asn Gly Val Ala Val Arg Glu Leu Asp Phe His Asp Thr Phe Leu Ala
100 105 110

Ala Glu Tyr Ser His Pro Gly Asp Asn Ile Pro Pro Leu Leu Ala Val
115 120 125

Ala Gln Ala Gln Arg Ser Ser Gly Arg Asp Leu Ile Arg Gly Ile Ala
130 135 140

Thr Ala Tyr Glu Val Gln Val Glu Leu Val Arg Gly Ile Cys Leu His
145 150 155 160

Glu His Lys Ile Asp His Val Ala His Leu Gly Pro Ser Ala Ala Ala
165 170 175

Gly Leu Gly Thr Leu Leu His Val Asp Glu Glu Thr Ile Tyr Gln Ala
180 185 190

Ile Gly Gln Ala Leu His Thr Thr Thr Ala Thr Arg Gln Ser Arg Lys
195 200 205

Gly Glu Ile Ser Ser Trp Lys Ala Phe Ala Pro Ala Phe Ala Gly Lys
210 215 220

Met Ala Ile Glu Ala Met Asp Arg Ala Met Arg Gly Glu Gly Ser Pro
225 230 235 240

Ala Pro Ile Trp Glu Gly Glu Asp Gly Val Ile Ala Trp Leu Leu Ser
245 250 255

Gly Lys Asp His Val Tyr His Val Pro Leu Pro Glu His Gly Glu Pro
260 265 270

Lys Leu Gly Ile Leu Glu Thr Tyr Thr Lys Glu His Ser Ala Glu Tyr
275 280 285

Gln Ser Gln Ala Pro Ile Asp Leu Ala Arg Arg Met Lys Pro Leu Val
290 295 300

Asp Ala Ala Gly Gly Thr Glu His Ile Ala Glu Ile Val Leu Arg Thr
305 310 315 320

Ser His His Thr His Tyr Val Ile Gly Thr Gly Ala Asn Asp Pro Gln
325 330 335

Lys Met Asp Pro Gln Ala Ser Arg Glu Thr Leu Asp His Ser Ile Met
340 345 350

Tyr Ile Phe Ala Val Ala Leu Gln Asp Gly Val Trp His His Glu Phe
355 360 365

Ser Tyr Thr Arg Lys Arg Ser Thr Arg Pro Glu Thr Val Glu Leu Trp
370 375 380

His Lys Ile Arg Thr Val Glu Asp Pro Glu Trp Thr Arg Arg Tyr His

385 390 395 400

Ser Asp Asp Pro Ala Lys Lys Ala Phe Gly Ala Lys Ala Val Ile Thr
405 410 415

Met Ala Asp Gly Thr Val Ile Glu Asp Glu Leu Ala Val Ala Asp Ala
420 425 430

His Pro Leu Gly Ala Arg Pro Phe Ala Arg Glu Asn Tyr Ile Glu Lys
435 440 445

Phe Arg Thr Leu Ala Gln Gly Ile Val Ile Asp Ser Glu Gln Glu Arg
450 455 460

Phe Leu His Ala Val Gln Ser Leu Pro Asp Leu Asp Asp Leu Asp Gln
465 470 475 480

Leu Asn Ile Glu Val Asp Ile Ser Asn Gln Ala Ala Thr Lys Ala Gly
485 490 495

Leu Leu

<210> 98
<211> 504
<212> PRT
<213> Corynebacterium glutamicum

<400> 98

Met Ile Asn His Glu Val Arg Thr His Arg Ser Ala Glu Glu Phe Pro
1 5 10 15

Tyr Glu Glu His Leu Ala His Lys Ile Ala Arg Val Ala Ala Asp Pro
20 25 30

Val Glu Val Ala Ala Asp Thr Gln Glu Met Ile Ile Asn Arg Ile Ile
35 40 45

Asp Asn Ala Ser Val Gln Ala Ala Ser Val Leu Arg Arg Pro Val Ser
50 55 60

Ser Ala Arg Ala Met Ala Gln Val Arg Pro Val Thr Asp Gly Arg Gly
65 70 75 80

Ala Ser Val Phe Gly Leu Pro Gly Arg Tyr Ala Ala Glu Trp Ala Ala
85 90 95

Leu Ala Asn Gly Thr Ala Val Arg Glu Leu Asp Phe His Asp Thr Phe

100 105 110

Leu Ala Ala Glu Tyr Ser His Pro Gly Asp Asn Ile Pro Pro Ile Leu
115 120 125

Ala Ala Ala Gln Gln Ala Gly Lys Gly Gly Lys Asp Leu Ile Arg Gly
130 135 140

Ile Ala Thr Gly Tyr Glu Ile Gln Val Asn Leu Val Arg Gly Met Cys
145 150 155 160

Leu His Glu His Lys Ile Asp His Val Ala His Leu Gly Pro Ser Ala
165 170 175

Ala Ala Gly Ile Gly Thr Leu Leu Asp Leu Asp Val Asp Thr Ile Tyr
180 185 190

Gln Ala Ile Gly Gln Ala Leu His Thr Thr Thr Ala Thr Arg Gln Ser
195 200 205

Arg Lys Gly Ala Ile Ser Ser Trp Lys Ala Phe Ala Pro Ala Phe Ala
210 215 220

Gly Lys Met Ser Ile Glu Ala Val Asp Arg Ala Met Arg Gly Glu Gly
225 230 235 240

Ala Pro Ser Pro Ile Trp Glu Gly Glu Asp Gly Val Ile Ala Trp Leu
245 250 255

Leu Ser Gly Leu Asp His Ile Tyr Thr Ile Pro Leu Pro Ala Glu Gly
260 265 270

Glu Ala Lys Arg Ala Ile Leu Asp Thr Tyr Thr Lys Glu His Ser Ala
275 280 285

Glu Tyr Gln Ser Gln Ala Pro Ile Asp Leu Ala Arg Ser Met Gly Glu
290 295 300

Lys Leu Ala Ala Gln Gly Leu Asp Leu Arg Asp Val Asp Ser Ile Val
305 310 315 320

Leu His Thr Ser His His Thr His Tyr Val Ile Gly Thr Gly Ser Asn
325 330 335

Asp Pro Gln Lys Phe Asp Pro Asp Ala Ser Arg Glu Thr Leu Asp His
340 345 350

Ser Ile Met Tyr Ile Phe Ala Val Ala Leu Glu Asp Arg Ala Trp His
355 360 365

His Glu Arg Ser Tyr Ala Pro Glu Arg Ala His Arg Arg Glu Thr Ile
370 375 380

Glu Leu Trp Asn Lys Ile Ser Thr Val Glu Asp Pro Glu Trp Thr Arg
385 390 395 400

Arg Tyr His Ser Val Asp Pro Ala Glu Lys Ala Phe Gly Ala Arg Ala
405 410 415

Val Ile Thr Phe Lys Asp Gly Thr Val Val Glu Asp Glu Leu Ala Val
420 425 430

Ala Asp Ala His Pro Leu Gly Ala Arg Pro Phe Ala Arg Glu Gln Tyr
435 440 445

Ile Gln Lys Phe Arg Thr Leu Ala Glu Gly Val Val Ser Glu Lys Glu
450 455 460

Gln Asp Arg Phe Leu Asp Ala Ala Gln Arg Thr His Glu Leu Glu Asp
465 470 475 480

Leu Ser Glu Leu Asn Ile Glu Leu Asp Ala Asp Ile Leu Ala Lys Ala
485 490 495

Pro Val Ile Pro Glu Gly Leu Phe
500

<210> 99
<211> 383
<212> PRT
<213> Corynebacterium glutamicum

<400> 99

Met Ser Ser Ala Thr Thr Thr Asp Val Arg Lys Gly Leu Tyr Gly Val
1 5 10 15

Ile Ala Asp Tyr Thr Ala Val Ser Lys Val Met Pro Glu Thr Asn Ser
20 25 30

Leu Thr Tyr Arg Gly Tyr Ala Val Glu Asp Leu Val Glu Asn Cys Ser
35 40 45

Phe Glu Glu Val Phe Tyr Leu Leu Trp His Gly Glu Leu Pro Thr Ala
50 55 60

Gln Gln Leu Ala Glu Phe Asn Glu Arg Gly Arg Ser Tyr Arg Ser Leu
65 70 75 80

Asp Ala Gly Leu Ile Ser Leu Ile His Ser Leu Pro Lys Glu Ala His
85 90 95

Pro Met Asp Val Met Arg Thr Ala Val Ser Tyr Met Gly Thr Lys Asp
100 105 110

Ser Glu Tyr Phe Thr Thr Asp Ser Glu His Ile Arg Lys Val Gly His
115 120 125

Thr Leu Leu Ala Gln Leu Pro Met Val Leu Ala Met Asp Ile Arg Arg
130 135 140

Arg Lys Gly Leu Asp Ile Ile Ala Pro Asp Ser Ser Lys Ser Val Ala
145 150 155 160

Glu Asn Leu Leu Ser Met Val Phe Gly Thr Gly Pro Glu Ser Pro Ala
165 170 175

Ser Asn Pro Ala Asp Val Arg Asp Phe Glu Lys Ser Leu Ile Leu Tyr
180 185 190

Ala Glu His Ser Phe Asn Ala Ser Thr Phe Thr Ala Arg Val Ile Thr
195 200 205

Ser Thr Lys Ser Asp Val Tyr Ser Ala Ile Thr Gly Ala Ile Gly Ala
210 215 220

Leu Lys Gly Pro Leu His Gly Gly Ala Asn Glu Phe Val Met His Thr
225 230 235 240

Met Leu Ala Ile Asp Asp Pro Asn Lys Ala Ala Ala Trp Ile Asn Asn
245 250 255

Ala Leu Asp Asn Lys Asn Val Val Met Gly Phe Gly His Arg Val Tyr
260 265 270

Lys Arg Gly Asp Ser Arg Val Pro Ser Met Glu Lys Ser Phe Arg Glu
275 280 285

Leu Ala Ala Arg His Asp Gly Glu Lys Trp Val Ala Met Tyr Glu Asn
290 295 300

Met Arg Asp Ala Met Asp Ala Arg Thr Gly Ile Lys Pro Asn Leu Asp
305 310 315 320

Phe Pro Ala Gly Pro Ala Tyr His Leu Leu Gly Phe Pro Val Asp Phe
325 330 335

Phe Thr Pro Leu Phe Val Ile Ala Arg Val Ala Gly Trp Thr Ala His
340 345 350

Ile Val Glu Gln Tyr Glu Asn Asn Ser Leu Ile Arg Pro Leu Ser Glu
355 360 365

Tyr Asn Gly Glu Glu Gln Arg Glu Val Ala Pro Ile Glu Lys Arg
370 375 380

<210> 100
<211> 498
<212> PRT
<213> Corynebacterium glutamicum

<400> 100

Met Arg Ile His Asp Val Tyr Thr His Leu Ser Ala Asp Asn Phe Pro
1 5 10 15

Lys Ala Glu His Leu Ala Trp Lys Phe Ser Glu Leu Ala Thr Asp Pro
20 25 30

Val Glu Val Thr Pro Asp Val Ser Glu Met Ile Ile Asn Arg Ile Ile
35 40 45

Asp Asn Ala Ala Val Ser Ala Ala Ser Val Leu Arg Arg Pro Val Thr
50 55 60

Val Ala Arg Gln Gln Ala Gln Ser His Pro Arg Glu Lys Gly Gly Lys
65 70 75 80

Val Phe Gly Ile Ser Gly Ser Tyr Ser Pro Glu Trp Ala Ala Phe Ala
85 90 95

Asn Gly Val Ala Val Arg Glu Leu Asp Phe His Asp Thr Phe Leu Ala
100 105 110

Ala Glu Tyr Ser His Pro Gly Asp Asn Ile Pro Pro Leu Leu Ala Val
115 120 125

Ala Gln Ala Gln Arg Ser Ser Gly Arg Asp Leu Ile Arg Gly Ile Ala
130 135 140

Thr Ala Tyr Glu Val Gln Val Glu Leu Val Arg Gly Ile Cys Leu His
145 150 155 160

```
Glu His Lys Ile Asp His Val Ala His Leu Gly Pro Ser Ala Ala Ala
                165                 170                 175

Gly Leu Gly Thr Leu Leu His Val Asp Glu Glu Thr Ile Tyr Gln Ala
            180                 185                 190

Ile Gly Gln Ala Leu His Thr Thr Thr Ala Thr Arg Gln Ser Arg Lys
        195                 200                 205

Gly Glu Ile Ser Ser Trp Lys Ala Phe Ala Pro Ala Phe Ala Gly Lys
    210                 215                 220

Met Ala Ile Glu Ala Met Asp Arg Ala Met Arg Gly Glu Gly Ser Pro
225                 230                 235                 240

Ala Pro Ile Trp Glu Gly Glu Asp Gly Val Ile Ala Trp Leu Leu Ser
                245                 250                 255

Gly Lys Asp His Val Tyr His Val Pro Leu Pro Glu His Gly Glu Pro
            260                 265                 270

Lys Leu Gly Ile Leu Glu Thr Tyr Thr Lys Glu His Ser Ala Glu Tyr
        275                 280                 285

Gln Ser Gln Ala Pro Ile Asp Leu Ala Arg Arg Met Lys Pro Leu Val
    290                 295                 300

Asp Ala Ala Gly Gly Thr Glu His Ile Ala Glu Ile Val Leu Arg Thr
305                 310                 315                 320

Ser His His Thr His Tyr Val Ile Gly Thr Gly Ala Asn Asp Pro Gln
                325                 330                 335

Lys Met Asp Pro Gln Ala Ser Arg Glu Thr Leu Asp His Ser Ile Met
            340                 345                 350

Tyr Ile Phe Ala Val Ala Leu Gln Asp Gly Val Trp His His Glu Phe
        355                 360                 365

Ser Tyr Thr Arg Lys Arg Ser Thr Arg Pro Glu Thr Val Glu Leu Trp
    370                 375                 380

His Lys Ile Arg Thr Val Glu Asp Pro Glu Trp Thr Arg Arg Tyr His
385                 390                 395                 400

Ser Asp Asp Pro Ala Lys Lys Ala Phe Gly Ala Lys Ala Val Ile Thr
                405                 410                 415
```

Met Ala Asp Gly Thr Val Ile Glu Asp Glu Leu Ala Val Ala Asp Ala
420 425 430

His Pro Leu Gly Ala Arg Pro Phe Ala Arg Glu Asn Tyr Ile Glu Lys
435 440 445

Phe Arg Thr Leu Ala Gln Gly Ile Val Ile Asp Ser Glu Gln Glu Arg
450 455 460

Phe Leu His Ala Val Gln Ser Leu Pro Asp Leu Asp Asp Leu Asp Gln
465 470 475 480

Leu Asn Ile Glu Val Asp Ile Ser Asn Gln Ala Ala Thr Lys Ala Gly
485 490 495

Leu Leu

<210> 101
<211> 714
<212> PRT
<213> Escherichia coli

<400> 101

Met Ser Arg Ile Ile Met Leu Ile Pro Thr Gly Thr Ser Val Gly Leu
1 5 10 15

Thr Ser Val Ser Leu Gly Val Ile Arg Ala Met Glu Arg Lys Gly Val
20 25 30

Arg Leu Ser Val Phe Lys Pro Ile Ala Gln Pro Arg Thr Gly Gly Asp
35 40 45

Ala Pro Asp Gln Thr Thr Thr Ile Val Arg Ala Asn Ser Ser Thr Thr
50 55 60

Thr Ala Ala Glu Pro Leu Lys Met Ser Tyr Val Glu Gly Leu Leu Ser
65 70 75 80

Ser Asn Gln Lys Asp Val Leu Met Glu Glu Ile Val Ala Asn Tyr His
85 90 95

Ala Asn Thr Lys Asp Ala Glu Val Val Leu Val Glu Gly Leu Val Pro
100 105 110

Thr Arg Lys His Gln Phe Ala Gln Ser Leu Asn Tyr Glu Ile Ala Lys
115 120 125

Thr Leu Asn Ala Glu Ile Val Phe Val Met Ser Gln Gly Thr Asp Thr
130 135 140

Pro Glu Gln Leu Lys Glu Arg Ile Glu Leu Thr Arg Asn Ser Phe Gly
145 150 155 160

Gly Ala Lys Asn Thr Asn Ile Thr Gly Val Ile Val Asn Lys Leu Asn
165 170 175

Ala Pro Val Asp Glu Gln Gly Arg Thr Arg Pro Asp Leu Ser Glu Ile
180 185 190

Phe Asp Asp Ser Ser Lys Ala Lys Val Asn Asn Val Asp Pro Ala Lys
195 200 205

Leu Gln Glu Ser Ser Pro Leu Pro Val Leu Gly Ala Val Pro Trp Ser
210 215 220

Phe Asp Leu Ile Ala Thr Arg Ala Ile Asp Met Ala Arg His Leu Asn
225 230 235 240

Ala Thr Ile Ile Asn Glu Gly Asp Ile Asn Thr Arg Arg Val Lys Ser
245 250 255

Val Thr Phe Cys Ala Arg Ser Ile Pro His Met Leu Glu His Phe Arg
260 265 270

Ala Gly Ser Leu Leu Val Thr Ser Ala Asp Arg Pro Asp Val Leu Val
275 280 285

Ala Ala Cys Leu Ala Ala Met Asn Gly Val Glu Ile Gly Ala Leu Leu
290 295 300

Leu Thr Gly Gly Tyr Glu Met Asp Ala Arg Ile Ser Lys Leu Cys Glu
305 310 315 320

Arg Ala Phe Ala Thr Gly Leu Pro Val Phe Met Val Asn Thr Asn Thr
325 330 335

Trp Gln Thr Ser Leu Ser Leu Gln Ser Phe Asn Leu Glu Val Pro Val
340 345 350

Asp Asp His Glu Arg Ile Glu Lys Val Gln Glu Tyr Val Ala Asn Tyr
355 360 365

Ile Asn Ala Asp Trp Ile Glu Ser Leu Thr Ala Thr Ser Glu Arg Ser

370 375 380

Arg Arg Leu Ser Pro Pro Ala Phe Arg Tyr Gln Leu Thr Glu Leu Ala
385 390 395 400

Arg Lys Ala Gly Lys Arg Ile Val Leu Pro Glu Gly Asp Glu Pro Arg
405 410 415

Thr Val Lys Ala Ala Ala Ile Cys Ala Glu Arg Gly Ile Ala Thr Cys
420 425 430

Val Leu Leu Gly Asn Pro Ala Glu Ile Asn Arg Val Ala Ala Ser Gln
435 440 445

Gly Val Glu Leu Gly Ala Gly Ile Glu Ile Val Asp Pro Glu Val Val
450 455 460

Arg Glu Ser Tyr Val Gly Arg Leu Val Glu Leu Arg Lys Asn Lys Gly
465 470 475 480

Met Thr Glu Thr Val Ala Arg Glu Gln Leu Glu Asp Asn Val Val Leu
485 490 495

Gly Thr Leu Met Leu Glu Gln Asp Glu Val Asp Gly Leu Val Ser Gly
500 505 510

Ala Val His Thr Thr Ala Asn Thr Ile Arg Pro Pro Leu Gln Leu Ile
515 520 525

Lys Thr Ala Pro Gly Ser Ser Leu Val Ser Ser Val Phe Phe Met Leu
530 535 540

Leu Pro Glu Gln Val Tyr Val Tyr Gly Asp Cys Ala Ile Asn Pro Asp
545 550 555 560

Pro Thr Ala Glu Gln Leu Ala Glu Ile Ala Ile Gln Ser Ala Asp Ser
565 570 575

Ala Ala Ala Phe Gly Ile Glu Pro Arg Val Ala Met Leu Ser Tyr Ser
580 585 590

Thr Gly Thr Ser Gly Ala Gly Ser Asp Val Glu Lys Val Arg Glu Ala
595 600 605

Thr Arg Leu Ala Gln Glu Lys Arg Pro Asp Leu Met Ile Asp Gly Pro
610 615 620

```
Leu Gln Tyr Asp Ala Ala Val Met Ala Asp Val Ala Lys Ser Lys Ala
625                 630                 635                 640

Pro Asn Ser Pro Val Ala Gly Arg Ala Thr Val Phe Ile Phe Pro Asp
                645                 650                 655

Leu Asn Thr Gly Asn Thr Thr Tyr Lys Ala Val Gln Arg Ser Ala Asp
            660                 665                 670

Leu Ile Ser Ile Gly Pro Met Leu Gln Gly Met Arg Lys Pro Val Asn
        675                 680                 685

Asp Leu Ser Arg Gly Ala Leu Val Asp Asp Ile Val Tyr Thr Ile Ala
    690                 695                 700

Leu Thr Ala Ile Gln Ser Ala Gln Gln Gln
705                 710
```

## Description

## FIELD OF THE INVENTION

The present invention relates to a biotechnological method for producing amino acids. In particular, the method may use alkanes as the starting material for production of L-amino acids.

## BACKGROUND OF THE INVENTION

Amino acids are especially useful as additives in animal feed and as nutritional supplements for human beings. They can also be used in infusion solutions and may function as synthetic intermediates for the manufacture of pharmaceuticals and agricultural chemicals. Compounds such as methionine, lysine, tryptophan and threonine are usually industrially produced to be used as food or feed additives and also in pharmaceuticals. In particular, methionine, an essential amino acid, which cannot be synthesized by animals, plays an important role in many body functions. L-methionine is presently being produced by chemical synthesis from hydrogen cyanide, acrolein and methyl mercaptan. These petroleum based starting materials such as acrolein and methyl mercaptan are obtained by cracking gasoline or petroleum which is bad for the environment. Also, since the costs for these starting materials will be linked to the price of petroleum, with the expected increase in petroleum prices in the future, prices of methionine will also increase relative to the increase in the petroleum prices. Similarly, lysine, an essential amino acid, also cannot be synthesized by animals. L-lysine is presently being produced by fermentation processes using high-performance strains of *Corynebacterium glutamicum* and *Escherichia coli* from sugar sources such as molasses, sucrose and/or glucose.

Production and consumption of agricultural products in general will grow particularly due to increased demand in developing countries - especially for beef and sugar. Additionally, a growing demand for bio-fuels is increasing the usage and price for sugar even further.

Since the market for amino acids will be affected by the increasing cost pressure to provide animal feed as well as the increasing price of the starting material sugar, the business will be squeezed from two sides.

There are currently four different production methods for amino acids. They include extraction, synthesis, fermentation, and enzymatic catalysis. Of these four methods, fermentation and enzymatic catalysis have the most economic and ecological advantages.

In order to maintain the competiveness of an efficient feed supplement with amino acid, there is a need to develop a production process for amino acids using an easily available and reasonably priced raw material.

Accordingly, there is a need in the art for a cheaper and more efficient biotechnological means of producing sugar-based amino acids.

## DESCRIPTION OF THE INVENTION

The present invention attempts to solve the problems above by providing a biotechnological means of producing at least one amino acid from at least one alkane. In particular, there is provided at least one genetically modified microbial cell that is capable of producing at least one amino acid from at least one alkane. The amino acid may be an L-amino acid and may be selected from the group consisting of tryptophan, lysine, threonine, methionine, O-acetyl homoserine, valine and isoleucine. The use of these genetically modified cells in a method to produce at least one amino acid may add flexibility to the production of these compounds by enabling the use of a readily available alternative petrochemical raw materials for the production of amino acids. Also, the use of whole-cell biocatalysts capable of integrating the entire means of converting alkanes to amino acids within them, makes the process of conversion simpler as only a small number of process steps are involved in the conversion. The reliance of amino acids on simple carbon sources as the carbon substrate is also eliminated.

According to one aspect of the present invention, there is provided a microbial cell for producing at least one L-amino acid from at least one short chain alkane, wherein the cell comprises:

(i) an increased expression relative to the wild type cell of Enzyme $E_1$ capable of converting the alkane to a corresponding 1-alkanol;
(ii) an increased expression relative to the wild type cell of Enzyme $E_2$ capable of converting the 1-alkanol of (i) to a corresponding aldehyde; and either
(iii)

(A)

- an increased expression relative to the wild type cell of Enzyme $E_3$ capable of converting the aldehyde of (ii) to a corresponding alkanoic acid; and

- a wild-type level expression of Enzyme $E_4$ or an increased expression relative to the wild type cell of Enzyme $E_4$ capable of converting the alkanoic acid of (iii) to a corresponding fatty acyl thioester; or

(B)

- an increased expression relative to the wild type cell of Enzyme $E_5$ capable of converting the aldehyde of (ii) to a corresponding fatty acyl thioester;

and

(iv) an increased expression relative to the wild type cell of Enzyme $E_6$ capable of converting the fatty acyl thioester of (iii) to a corresponding amino acid

Alkanes are saturated hydrocarbons that have various applications depending on the number of carbon atoms and on the structure of the alkane (i.e. branched, linear, cyclic etc.). Alkanes (technically, always acyclic or open-chain compounds) have the general chemical formula $C_nH_{2n+2}$. The short chain alkane used according to any aspect of the present invention may refer to at least one alkane with 1-4 carbon atoms. In particular, alkanes with 1 to 6 carbon atoms comprise, for example, methane, ethane, propane, butane, isobutene, pentane and hexane. More in particular, the short-chain alkane may be selected from the group consisting of methane, ethane, propane and butane. In one example, the short-chain alkane may be ethane, butane or propane.

**Enzyme $E_7$**

In particular, if the alkane used according to any aspect of the present invention may be a butane, the cell according to any aspect of the present invention may be genetically modified to increase expression relative to the wild type cell of at least one enzyme ($E_{7a}$). More in particular, the enzyme $E_{7a}$ may be selected from the group consisting of acyl-ACP synthetase ($E_g$) (EC 6.2.1.20), acyl-CoA synthetase ($E_f$) (EC 6.2.1.2, EC 6.2.1.3, EC 6.2.1.10), and the combination of butyrate kinase ($E_{hi}$), (EC 2.7.2.7) and phosphotransbutyrylase ($E_{ii}$) (EC 2.3.1.19). The increase in the expression of at least one $E_{7a}$ enzyme, amplifies the production of acetyl thioesters from butane. In particular, the increase in expression of at least one $E_{7a}$ enzyme relative to the wild-type cell intensifies the reaction: Butyrate -> Butyryl-thioester -> Acetyl-Thioester.

In particular, when the alkane used as a substrate according to any aspect of the present invention is a butane, the cell according to any aspect of the present invention may be genetically modified to increase the expression of at least one enzyme $E_{7a}$. The enzyme $E_{7a}$ may be selected from the group consisting of acyl-ACP synthetase ($E_g$) (EC 6.2.1.20), acyl-CoA synthetase ($E_f$) (EC 6.2.1.2, EC 6.2.1.3, EC 6.2.1.10), and the combination of fatty acyl kinase ($E_h$) of EC 2.7.2.1, EC 2.7.2.12, EC 2.7.2.15 or EC 2.7.2.7 and phosphotransacylase ($E_i$) of EC 2.3.1.8 or EC 2.3.1.19. In particular, enzyme $E_{7a}$ may be an acyl-ACP synthetase ($E_g$) comprising SEQ ID NO:21 or a variant thereof, or an acyl-CoA synthetase ($E_f$) comprising SEQ ID NO:22 or a variant thereof, or the combination of fatty acyl kinase ($E_h$) comprising SEQ ID NO:23 or a variant thereof and phosphotransacylase ($E_i$) comprising SEQ ID NO:24 or a variant thereof.

In one example, the alkane used according to any aspect of the present invention may be a propane, the cell according to any aspect of the present invention may be genetically modified to increase expression relative to the wild type cell of at least one enzyme ($E_{7b}$). More in particular, the enzyme $E_{7b}$ may be selected from the group consisting of acyl-ACP synthetase ($E_g$) (EC 6.2.1.20), acyl-CoA synthetase($E_f$) (EC 6.2.1.2, EC 6.2.1.3, EC 6.2.1.10), methylisocitrate hydro-lyase ($E_{7bi}$) (EC 4.2.1.99), methylisocitrate lyase ($E_{7bii}$) (EC 4.1.3.30), 2-Methylisocitrate dehydratase ($E_{7biii}$) (EC 4.2.1.79), 2-Methylcitrate synthase ($E_{7biv}$) (EC 2.3.3.5), combination of phosphotranspropionylase ($E_{iii}$) (EC 2.3.1.19, EC 2.3.1.8), propionate kinase ($E_{hii}$) (EC 2.7.2.15) and propionyl-CoA ligase ($E_{7bvii}$) (EC 6.2.1.17) and propionyl-CoA:acetate Coenzyme A transferase ($E_{7bviii}$)(EC 2.8.3.1). The increase in the expression of at least one $E_{7b}$ enzyme, amplifies the production of acetyl thioesters from propane. In particular, the increase in expression of at least one $E_{7b}$ enzyme relative to the wild-type cell intensifies the reaction: Propionate -> Propionyl-thioester -> Acetyl-Thioester.

In particular, when the alkane used as a substrate according to any aspect of the present invention is a propane, the cell according to any aspect of the present invention may be genetically modified to increase the expression of at least one enzyme $E_{7b}$. The enzyme $E_{7b}$ may be selected from the group consisting of acyl-ACP synthetase ($E_g$) (EC 6.2.1.20), acyl-CoA synthetase ($E_f$) (EC 6.2.1.2, EC 6.2.1.3, EC 6.2.1.10), methylisocitrate hydro-lyase ($E_{7bi}$) (EC 4.2.1.99), methylisocitrate lyase ($E_{7bii}$) (EC 4.1.3.30), 2-Methylisocitrate dehydratase ($E_{7biii}$) (EC 4.2.1.79), 2-Methylcitrate synthase ($E_{7biv}$) (EC 2.3.3.5), combination of phosphotranspropionylase ($E_{iii}$) (EC 2.3.1.19, EC 2.3.1.8) and propionate kinase ($E_{hii}$) (EC 2.7.2.15) and propionyl-CoA ligase ($E_{7bvii}$) (EC 6.2.1.17). In particular, enzyme $E_{7b}$ may be an acyl-ACP synthetase ($E_g$) comprising SEQ ID NO:21 or a variant thereof, or an acyl-CoA synthetase ($E_f$) comprising SEQ ID NO:22 or a variant thereof, methylisocitrate hydro-lyase ($E_{7bi}$) comprising SEQ ID NO:27, 94 or a variant thereof, a methylisocitrate lyase ($E_{7bii}$) comprising SEQ ID NO:28, 95, 96 or a variant thereof, a 2-Methylisocitrate dehydratase ($E_{7biii}$) comprising SEQ ID NO:29, 97, 98 or a variant thereof, a 2-Methylcitrate synthase ($E_{7biv}$) comprising SEQ ID NO:30, 99, 100 or a variant thereof or the combination of phosphotranspropionylase ($E_{iii}$) comprising SEQ ID NO:31, 101 or a variant thereof and propionate kinase ($E_{hii}$) comprising SEQ ID NO:26, 4 or a variant thereof, a propionyl-CoA ligase ($E_{7bvii}$) (EC 6.2.1.17) comprising SEQ ID NO:32 or a variant thereof or propionyl-CoA:acetate Coenzyme A transferase ($E_{7bviii}$) comprising SEQ ID NO:17 a variant thereof.

The cells according to any aspect of the present invention may be used to produce amino acids from all short-chain alkanes with high space-time yield, high carbon yield and high concentration in the culture supernatant. As a result of these advantages, an efficient workup is facilitated.

The phrase "wild type" as used herein in conjunction with a cell or microorganism may denote a cell with a genome make-up that is in a form as seen naturally in the wild. The term may be applicable for both the whole cell and for individual genes. The term 'wild type' may thus also include cells which have been genetically modified in other aspects (i.e. with regard to one or more genes) but not in relation to the genes of interest. The term "wild type" therefore does not include such cells where the gene sequences of the specific genes of interest have been altered at least partially by man using recombinant methods. A wild type cell according to any aspect of the present invention thus refers to a cell that has no genetic mutation with respect to the whole genome and/or a particular gene. Therefore, in one example, a wild type cell with respect to enzyme $E_1$ may refer to a cell that has the natural/ non-altered expression of the enzyme $E_1$ in the cell. The wild type cell with respect to enzyme $E_2$, $E_3$, $E_4$, $E_5$, $E_6$, $E_7$, etc. may be interpreted the same way and may refer to a cell that has the natural/ non-altered expression of the enzyme $E_2$, $E_3$, $E_4$, $E_5$, $E_6$, $E_7$, etc. respectively in the cell. A wild-type cell can also include a cell that has mutations from nature. However, a "wild type cell" relative to a genetically modified cell according to any aspect of the present invention, means a cell in which the mutation resulting in the production of a substance in a quantifiably reduced or increased amount has not occurred. For example, a wild-type cell according to any aspect of the present invention, relative to a genetically modified cell according to any aspect of the present invention with increased expression of enzymes $E_1$, $E_2$, $E_3$, $E_4$ and $E_6$, $E_7$, refers to a cell which has not been mutated to increase the expression of enzymes $E_1$, $E_2$, $E_3$, $E_4$ and $E_6$, $E_7$, using recombinant means. Similarly, a wild-type cell according to any aspect of the present invention, relative to a genetically modified cell according to any aspect of the present invention with increased expression of enzymes $E_1$, $E_2$, $E_5$ and $E_6$, refers to a cell which has not been mutated to increase the expression of enzymes $E_1$, $E_2$, $E_5$ and $E_6$, using recombinant means. Wild-type cells are therefore, reference, or standard, cells used according to any aspect of the present invention. A wild-type cell, thus need not be a cell normally found in nature, and often will be a recombinant or genetically altered cell line. However, the wild type cells according to any aspect of the present invention may not be genetically modified with reference to the enzymes $E_1$, $E_2$, $E_3$, $E_4$, $E_5$, $E_6$, and/or $E_7$.

In one example, in the cell according to any aspect of the present invention, the expression of enzyme $E_4$ is not altered. This means, the cell used according to any aspect of the present invention, expresses $E_4$ in its wild type form and in the wild type form the cell expresses $E_4$ in a detectable amount. The wild type cell therefore, expresses enzyme $E_4$ and the expression is sufficient to carry out the step of converting the alkanoic acid of (iii) to a corresponding fatty acyl thioester. In this example, there is thus no need to increase the expression of $E_4$ and the cell expresses the wild-type $E_4$ in unaltered/ unprocessed form.

In another example, the cell according to any aspect of the present invention may be genetically modified to increase the expression of enzyme $E_4$ relative to the wild type cell. The cell in this example may be genetically modified to overexpress enzyme $E_4$ relative to the wild-type cell so that the cell is capable of converting the alkanoic acid of (iii) to a corresponding fatty acyl thioester.

Any of the enzymes used according to any aspect of the present invention, may be an isolated enzyme. In particular, the enzymes used according to any aspect of the present invention may be used in an active state and in the presence of all cofactors, substrates, auxiliary and/or activating polypeptides or factors essential for its activity. The term "isolated", as used herein, means that the enzyme of interest is enriched compared to the cell in which it occurs naturally. The enzyme may be enriched by SDS polyacrylamide electrophoresis and/or activity assays. For example, the enzyme of interest may constitute more than 5, 10, 20, 50, 75, 80, 85, 90, 95 or 99 percent of all the polypeptides present in the preparation as judged by visual inspection of a polyacrylamide gel following staining with Coomassie blue dye.

The enzyme used according to any aspect of the present invention may be recombinant. The term "recombinant" as used herein, refers to a molecule or is encoded by such a molecule, particularly a polypeptide or nucleic acid that, as such, does not occur naturally but is the result of genetic engineering or refers to a cell that comprises a recombinant molecule. For example, a nucleic acid molecule is recombinant if it comprises a promoter functionally linked to a sequence encoding a catalytically active polypeptide and the promoter has been engineered such that the catalytically active polypeptide is overexpressed relative to the level of the polypeptide in the corresponding wild type cell that comprises the original unaltered nucleic acid molecule.

A skilled person would be able to use any method known in the art to genetically modify a cell or microorganism.

According to any aspect of the present invention, the genetically modified cell may be genetically modified so that in a defined time interval, within 2 hours, in particular within 8 hours or 24 hours, it forms at least once or twice, especially at least 10 times, at least 100 times, at least 1000 times or at least 10000 times amino acids than the wild-type cell. The increase in product formation can be determined for example by cultivating the cell according to any aspect of the present invention and the wild-type cell each separately under the same conditions (same cell density, same nutrient medium, same culture conditions) for a specified time interval in a suitable nutrient medium and then determining the amount of target product (amino acids) in the nutrient medium.

The genetically modified cell or microorganism may be genetically different from the wild type cell or microorganism. The genetic difference between the genetically modified microorganism according to any aspect of the present invention and the wild type microorganism may be in the presence of a complete gene, amino acid, nucleotide etc. in the genetically modified microorganism that may be absent in the wild type microorganism. In one example, the genetically modified microorganism according to any aspect of the present invention may comprise enzymes that enable the microorganism to produce more amino acids compared to the wild type cells. The wild type microorganism relative to the genetically modified microorganism of the present invention may have none or no detectable activity of the enzymes that enable the genetically modified microorganism to produce amino acids from alkanes. As used herein, the term 'genetically modified microorganism' may be used interchangeably with the term 'genetically modified cell'. The genetic modification according to any aspect of the present invention is carried out on the cell of the microorganism.

The cells according to any aspect of the present invention are genetically transformed according to any method known in the art. In particular, the cells may be produced according to the method disclosed in WO2013024114.

The phrase 'the genetically modified cell has an increased activity, in comparison with its wild type, in enzymes' as used herein refers to the activity of the respective enzyme that is increased by a factor of at least 2, in particular of at least 10, more in particular of at least 100, yet more in particular of at least 1000 and even more in particular of at least 10000.

The phrase "increased activity of an enzyme", as used herein is to be understood as increased intracellular activity. Basically, an increase in enzymatic activity can be achieved by increasing the copy number of the gene sequence or gene sequences that code for the enzyme, using a strong promoter or employing a gene or allele that codes for a corresponding enzyme with increased activity, altering the codon utilization of the gene, increasing the half-life of the mRNA or of the enzyme in various ways, modifying the regulation of the expression of the gene and optionally by combining these measures. Genetically modified cells used according to any aspect of the present invention are for example produced by transformation, transduction, conjugation or a combination of these methods with a vector that contains the desired gene, an allele of this gene or parts thereof and a vector that makes expression of the gene possible. Heterologous expression is in particular achieved by integration of the gene or of the alleles in the chromosome of the cell or an extrachromosomally replicating vector. In one example, a cell with an increased expression of an enzyme may refer to a cell with an overexpression of the enzyme relative to the wild type cell that has no or the normal expression of the enzyme. In particular, an increased activity of an enzyme relative to a wild-type cell, refers to the overexpression of the gene encoding the enzyme in the genetically modified cell.

In the same context, the phrase "decreased activity of an enzyme $E_x$" used with reference to any aspect of the present invention may be understood as meaning an activity decreased by a factor of at least 0.5, particularly of at least 0.1, more particularly of at least 0.01, even more particularly of at least 0.001 and most particularly of at least 0.0001. The phrase "decreased activity" also comprises no detectable activity ("activity of zero"). The decrease in the activity of a certain enzyme can be effected, for example, by selective mutation or by other measures known to the person skilled in the art for decreasing the activity of a certain enzyme. In particular, the person skilled in the art finds instructions for the modification and decrease of protein expression and concomitant lowering of enzyme activity by means of interrupting specific genes, for example at least in Dubeau *et al.* 2009. Singh & Röhm. 2008., Lee *et al.*, 2009 and the like. The decrease in the enzymatic activity in a cell according to any aspect of the present invention may be achieved by modification of a gene comprising one of the nucleic acid sequences, wherein the modification is selected from the group comprising, consisting of, insertion of foreign DNA in the gene, deletion of at least parts of the gene, point mutations in the gene sequence, RNA interference (siRNA), *antisense* RNA or modification (insertion, deletion or point mutations) of regulatory sequences, such as, for example, promoters and terminators or of ribosome binding sites, which flank the gene.

Foreign DNA is to be understood in this connection as meaning any DNA sequence which is "foreign" to the gene (and not to the organism), i.e. endogenous DNA sequences can also function in this connection as "foreign DNA". In this connection, it is particularly preferred that the gene is interrupted by insertion of a selection marker gene, thus the foreign DNA is a selection marker gene, wherein preferably the insertion was effected by homologous recombination in the gene locus.

The expression of the enzymes and genes mentioned above and all mentioned below is determinable by means of 1- and 2-dimensional protein gel separation followed by optical identification of the protein concentration in the gel with appropriate evaluation software.

If the increasing of an enzyme activity is based exclusively on increasing the expression of the corresponding gene, then the quantification of the increasing of the enzyme activity can be simply determined by a comparison of the 1- or 2-dimensional protein separations between wild type and genetically modified cell. A common method for the preparation of the protein gels with bacteria and for identification of the proteins is the procedure described by Hermann et al. (Electrophoresis, 22: 1712-23 (2001). The protein concentration can also be analysed by Western blot hybridization with an antibody specific for the protein to be determined (Sambrook et al., Molecular Cloning: a laboratory manual, 2nd Ed. Cold Spring Harbor Laboratory Press, Cold Spring Harbor, N.Y. USA, 1989) followed by optical evaluation with appropriate software for concentration determination (Lohaus and Meyer (1989) Biospektrum, 5: 32-39; Lottspeich (1999), Angewandte Chemie 111: 2630-2647). This method is also always an option when possible products of the reaction to be catalysed by the enzyme activity to be determined may be rapidly metabolized in the microorganism or else the activity in the wild type is itself too low for it to be possible adequately to determine the enzyme activity to be determined on the basis of the production formation.

In particular,

- the Enzyme $E_1$ is selected from the group consisting of P450 alkane hydroxylase ($E_a$) of EC 1.14.15.3- and AlkB alkane hydroxylase ($E_b$) of EC 1.14.15.3;

- the Enzyme $E_2$ is selected from the group consisting of P450 alkane hydroxylase ($E_a$) of EC 1.14.15.3-, AlkB alkane hydroxylase ($E_b$) of EC 1.14.15.3, alcohol oxidase ($E_c$) of EC 1.1.3.20 and alcohol dehydrogenase ($E_d$) of EC 1.1.1.1 or EC 1.1.1.2;

- the Enzyme $E_3$ is selected from the group consisting of P450 alkane hydroxylase ($E_a$) of EC 1.14.15.3-, AlkB alkane hydroxylase ($E_b$) of EC 1.14.15.3, aldehyde dehydrogenase ($E_e$) of EC 1.2.1.3, EC 1.2.1.4 or EC 1.2.1.5, alcohol oxidase ($E_c$) of EC 1.1.3.20, AlkJ alcohol dehydrogenase ($E_{di}$) of EC 1.1.99.- and alcohol dehydrogenase ($E_d$) of EC 1.1.1.1 or EC 1.1.1.2, wherein $E_c$, $E_{di}$, and $E_d$ are each capable of oxidizing an ω-hydroxy alkanoic acid ester directly to the corresponding ω-carboxy alkanoic acid ester;

- the Enzyme $E_4$ is selected from the group consisting of fatty acyl coenzyme A (CoA) synthase (FACS) ($E_f$) of EC 6.2.1.1, EC 6.2.1.2, EC 6.2.1.3, or EC 2.3.1.86; acyl-Acyl Carrier Protein (ACP) synthase ($E_g$) of EC 6.2.1.20 or EC 6.2.1.47; fatty acyl kinase ($E_h$) of EC 2.7.2.1, EC 2.7.2.12, EC 2.7.2.15 or EC 2.7.2.7 and phosphotransacylase ($E_i$) of EC 2.3.1.8 or EC 2.3.1.19; and fatty acyl coenzyme A synthase ($E_f$) of EC 6.2.1.1, EC 6.2.1.2 or EC 6.2.1.3 and fatty acyl-CoA:ACP transacylase ($E_j$) of EC 2.3.1.38 or EC 2.3.1.39;

- the Enzyme $E_5$ is selected from the group consisting of aldehyde dehydrogenase ($E_e$) of EC 1.2.1.3, EC 1.2.1.4 or EC 1.2.1.5, alcohol oxidase ($E_c$) of EC 1.1.3.20, AlkJ alcohol dehydrogenase ($E_{di}$) of EC 1.1.99.- and alcohol dehydrogenase ($E_d$) of EC 1.1.1.1 or EC 1.1.1.2;

- the Enzyme $E_6$ is capable of converting the fatty acyl thioester of any aspect of the present invention, in particular step (iii) to a corresponding amino acid.

The amino acid produced according to any aspect of the present invention may be an L-amino acid. In particular, the amino acid may be selected from the group consisting of lysine, threonine, methionine, valine, O-Acetyl homoserine, tryptophan, and isoleucine. More in particular, the amino acid produced according to any aspect of the present invention may be lysine, O-Acetyl homoserine or threonine.

## Enzyme $E_6$

The Enzyme $E_6$ may be capable of converting the fatty acyl thioester of any aspect of the present invention, in particular step (iii) to a corresponding amino acid.

In one example, when the target amino acid produced according to any aspect of the present invention is lysine, the enzymes $E_6$ may be selected from the group consisting of aspartate kinase ($E_{6a}$) (EC 2.7.2.4), aspartate semialdehyde dehydrogenase ($E_{6b}$) (EC 1.2.1.11), 4-hydroxy-tetrahydrodipicolinate synthase ($E_{6c}$) (EC 4.3.3.7), dihydrodipicolinate reductase ($E_{6d}$) (EC 1.17.1.8), diaminopimelate decarboxylase ($E_{6e}$) (EC 4.1.1.20), lysine exporter ($E_{6f}$) (TCDB families 2.A.124.1.1, 2.A.75.1.1 or 2.A.75.1.2), phosphoenolpyruvate (PEP) carboxylase ($E_{6g}$) (EC 4.1.1.31), proton-translocating transhydrogenase ($E_{6h}$) (EC 1.6.1.5), and pyruvate carboxylase ($E_{6i}$) (EC 6.4.1.1). In particular, $E_6$ may be an aspartate kinase ($E_{6a}$) comprising SEQ ID NO:1, 79 or a variant thereof, an aspartate semialdehyde dehydrogenase ($E_{6b}$) comprising SEQ ID NO:2, 82 or a variant thereof, a 4-hydroxy-tetrahydrodipicolinate synthase ($E_{6c}$) comprising SEQ ID NO:3 or a variant thereof, a dihydrodipicolinate reductase ($E_{6d}$) comprising SEQ ID NO:5 or a variant thereof, a diaminopimelate

decarboxylase ($E_{6e}$) comprising SEQ ID NO:6 or a variant thereof, a lysine exporter ($E_{6f}$) comprising SEQ ID NO:7, 8, 9 or a variant thereof, phosphoenolpyruvate (PEP) carboxylase ($E_{6g}$) comprising SEQ ID NO:10 or a variant thereof, proton-translocating transhydrogenase ($E_{6h}$) comprising SEQ ID NO:11, 20 or a variant thereof, and pyruvate carboxylase ($E_{6i}$) comprising SEQ ID NO:12 or a variant thereof. More in particular, the enzyme $E_6$ may be selected from the group consisting of aspartate kinase ($E_{6a}$) and 4-hydroxy-tetrahydrodipicolinate synthase ($E_{6c}$). Even more in particular, the enzyme $E_6$ may comprise the sequence SEQ ID NO:1, 3 or a variant thereof. In one example, the enzyme $E_6$ may consists of the sequence SEQ ID NO:1, 3 or a variant thereof.

In another example, when the target amino acid produced according to any aspect of the present invention is O-Acetyl homoserine, the enzymes $E_6$ may be selected from the group consisting of aspartate kinase ($E_{6a}$) (EC 2.7.2.4), aspartate semialdehyde dehydrogenase ($E_{6b}$) (EC 1.2.1.11), glyceraldehyde-3-phosphate dehydrogenase (NADP-dependent) ($E_{6j}$) (EC 1.2.1.9, EC 1.2.1.13, EC 1.2.1.59, EC 1.2.1.60), homoserine dehydrogenase (also known as a bifunctional aspartokinase I/homoserine dehydrogenase I ($E_{6k}$) (EC 1.1.1.3), homoserine kinase ($E_{6l}$) (EC 2.7.1.39), homoserine O-acetyltransferase ($E_{6s}$) (EC 2.3.1.31), phosphoenolpyruvate (PEP) carboxylase ($E_{6g}$) (EC 4.1.1.31), proton-translocating transhydrogenase ($E_{6h}$) (EC 1.6.1.5), pyruvate carboxylase ($E_{6i}$) (EC 6.4.1.1), O-Acetyl homoserine exporter ($E_{6ad}$) (TCDB classification 2.A.42.2.2; 2.A.7.3.6; 2.A.76.1.10; 2.A.76.1.2; 2.A.79.1.1; 2.A.95.1.4, 2.A.7.21.5, 2.A.76.1.1, 2.A.76.1.9). In particular, $E_6$ may be an aspartate kinase ($E_{6a}$) comprising SEQ ID NO:1, 79 or a variant thereof, an aspartate semialdehyde dehydrogenase ($E_{6b}$) comprising SEQ ID NO:2, 82 or a variant thereof, glyceraldehyde-3-phosphate dehydrogenase (NADP-dependent) ($E_{6j}$) comprising SEQ ID NO:13 or a variant thereof, homoserine dehydrogenase ($E_{6k}$) comprising SEQ ID NO:14, 51, 80 or a variant thereof, homoserine kinase ($E_{6l}$) comprising SEQ ID NO:15, 81 or a variant thereof, homoserine O-acetyltransferase ($E_{6s}$) comprising SEQ ID NO:16, 78 or a variant thereof, phosphoenolpyruvate (PEP) carboxylase ($E_{6g}$) comprising SEQ ID NO:10 or a variant thereof, a proton-translocating transhydrogenase ($E_{6h}$) comprising SEQ ID NO:11, 20 or a variant thereof, pyruvate carboxylase ($E_{6i}$) comprising SEQ ID NO:12 or a variant thereof, O-Acetyl homoserine exporter ($E_{6ad}$) comprising SEQ ID NO:19, 84, 85, 86 or variant thereof. More in particular, the enzyme $E_6$ may be selected from the group consisting of homoserine dehydrogenase (also known as a bifunctional aspartokinase I/homoserine dehydrogenase I ($E_{6k}$) and homoserine O-acetyltransferase ($E_{6s}$). Even more in particular, the enzyme $E_6$ may comprise the sequence SEQ ID NO:14, 51, 16, 78 or a variant thereof. In one example, the enzyme $E_6$ may consists of the sequence SEQ ID NO:14, 51, 16, 78 or a variant thereof.

In yet another example, when the target amino acid produced according to any aspect of the present invention is a threonine, the enzymes $E_6$ may be selected from the group consisting of aspartate kinase ($E_{6a}$) (EC 2.7.2.4), aspartate semialdehyde dehydrogenase ($E_{6b}$) (EC 1.2.1.11), glyceraldehyde-3-phosphate dehydrogenase (NADP-dependent) ($E_{6j}$) (EC 1.2.1.9, EC 1.2.1.13, EC 1.2.1.59, EC 1.2.1.60), homoserine dehydrogenase ($E_{6k}$) (EC 1.1.1.3), homoserine kinase ($E_{6l}$) (EC 2.7.1.39), phosphoenolpyruvate (PEP) carboxylase ($E_{6g}$) (EC 4.1.1.31), proton-translocating transhydrogenase ($E_{6h}$) (EC 1.6.1.5), pyruvate carboxylase ($E_{6i}$) (EC 6.4.1.1), threonine synthase ($E_{6m}$) (EC 4.2.3.1) and threonine exporter ($E_{6n}$) (TCDB families 2.A.7.3.6, 2.A.76.1.10 or 2.A.79.1.1). In particular, $E_6$ may be an aspartate kinase ($E_{6a}$) comprising SEQ ID NO:1, 79 or variant thereof, aspartate semialdehyde dehydrogenase ($E_{6b}$) comprising SEQ ID NO:2, 82 or variant thereof, glyceraldehyde-3-phosphate dehydrogenase (NADP-dependent) ($E_{6j}$) comprising SEQ ID NO:13 or variant thereof, homoserine dehydrogenase ($E_{6k}$) comprising SEQ ID NO:14, 51, 80 or variant thereof, homoserine kinase ($E_{6l}$) comprising SEQ ID NO:15, 81 or variant thereof, phosphoenolpyruvate (PEP) carboxylase ($E_{6g}$) comprising SEQ ID NO:10 or variant thereof, proton-translocating transhydrogenase ($E_{6h}$) comprising SEQ ID NO:11, 20 or variant thereof, pyruvate carboxylase ($E_{6i}$) comprising SEQ ID NO:12 or variant thereof, threonine synthase comprising SEQ ID NO:18, 83 or variant thereof and threonine exporter ($E_{6n}$) comprising SEQ ID NO:19, 84, 85, 86 or variant thereof. More in particular, $E_6$ may be selected from the group consisting of a feedback-resistant variant of aspartate kinase ($E_{6a}$) comprising SEQ ID NO:1 with a point mutation of T342I, or SEQ ID NO:79 with at least one point mutation selected from the group consisting of T311I, A279T, S301Y, A279V, S301F, T308I, S317A, R320G, G345D, S381F, Q404E, G408R, G277A, Q298A, T361A, E363A, and F364A, particularly with point mutation T3111, feedback-resistant variant of homoserine dehydrogenase ($E_{6k}$) comprising SEQ ID NO:14 with at least one point mutation selected from the group consisting of G378E, D375A, V379E, L380E, I392P, S393A, L394P and Q399T, SEQ ID NO:51 with point mutation S345P or SEQ ID NO:80, homoserine kinase ($E_{6l}$) comprising SEQ ID NO:15, 81 or a variant thereof and threonine exporter ($E_{6n}$) comprising SEQ ID NO:19, 84, 85, 86 or variant thereof. In one example, the enzyme $E_6$ may be a feedback-resistant variant of aspartate kinase ($E_{6a}$), or a feedback-resistant variant of homoserine dehydrogenase ($E_{6k}$). Examples of which, are provided at least in Li, Y., et al. Current status on metabolic engineering for the production of L-aspartate family amino acids and derivatives. Bioresour. Technol. (2017), particularly on page 8.

In a further example, when the target amino acid produced according to any aspect of the present invention is a methionine, the enzymes $E_6$ may be selected from the group consisting of aspartate kinase ($E_{6a}$) (EC 2.7.2.4), aspartate semialdehyde dehydrogenase ($E_{6b}$) (EC 1.2.1.11), cystathionine beta-lyase ($E_{6o}$) (EC 4.4.1.8), cystathionine gamma-synthase ($E_{6p}$) (EC 2.5.1.48), glyceraldehyde-3-phosphate dehydrogenase (NADP-dependent) ($E_{6j}$) (EC 1.2.1.9, EC 1.2.1.13, EC 1.2.1.59, EC 1.2.1.60), homocysteine transmethylase ($E_{6q}$) (EC 2.1.1.10 or EC 2.1.1.13), homoserine dehydrogenase ($E_{6k}$) (EC 1.1.1.3), homoserine O-succinyltransferase ($E_{6r}$) (EC 2.3.1.46), homoserine O-acetyltrans-

ferase ($E_{6s}$) (EC 2.3.1.31), methionine exporter ($E_{6t}$) (TCDB families 2.A.3.13.1, 2.A.76.1.5 or 2.A.78.1.3), O-acetyl homoserine sulfhydrylase ($E_{6u}$) (EC 2.5.1.49), O-succinyl homoserine sulfhydrylase ($E_{6v}$) (EC:2.5.1.-), phosphoenolpyruvate (PEP) carboxylase ($E_{6g}$) (EC 4.1.1.31), proton-translocating transhydrogenase ($E_{6h}$) (EC 1.6.1.5), and pyruvate carboxylase ($E_{6i}$) (EC 6.4.1.1). In particular, $E_6$ may be a feedback-resistant variant of aspartate kinase ($E_{6a}$) comprising SEQ ID NO:1 with a point mutation of T342I, or SEQ ID NO:79 with at least one point mutation selected from the group consisting of T311I, A279T, S301Y, A279V, S301F, T308I, S317A, R320G, G345D, S381F, Q404E, G408R, G277A, Q298A, T361A, E363A, and F364A, particularly with point mutation T311I.

In one example, when the target amino acid produced according to any aspect of the present invention is a valine, the enzymes $E_6$ may be selected from the group consisting of $\alpha$-acetohydroxy acid isomeroreductase ($E_{6w}$) (EC 1.1.1.86), acetolactate synthase ($E_{6x}$) (EC 2.2.1.6) also known as a acetohydroxyacid synthase or a acetohydroxybutanoate synthase, 2,3-Dihydroxy acid hydro-lyase ($E_{6y}$) (EC 4.2.1.9), glucose-6-phosphate dehydrogenase (NADP-dependent) ($E_{6z}$) (EC 1.1.1.49, EC 1.1.1.361, EC 1.1.1.363, EC 1.1.1.388), malic enzyme ($E_{6aa}$) (EC 1.1.1.39), proton-translocating transhydrogenase ($E_{6h}$) (EC 1.6.1.5), valine exporter ($E_{6ab}$) (TCDB classification 2.A.78.1.2, 2.A.76.1.5) and valine transaminase ($E_{6ac}$) EC 2.6.1.42.

In another example, when the target amino acid produced according to any aspect of the present invention is a tryptophan, the enzymes $E_6$ may be selected from the group consisting of anthranilate phosphoribosyl transferase ($E_{6ae}$) (EC 2.4.2.18), anthranilate synthase ($E_{6af}$) (EC 4.2.3.5), chorismate synthase ($E_{6ag}$) (EC 4.2.3.5), 2-Dehydro-3-deoxyphosphoheptonate aldolase ($E_{6ah}$) (EC 2.5.1.54), 3-Dehydroquinate synthase ($E_{6ai}$) (EC 4.2.3.4), 3-Dehydroquinate dehydratase ($E_{6aj}$) (EC 4.2.1.10), glucokinase ($E_{6ak}$) (EC 2.7.1.10, EC 2.7.1.1), glucose facilitator ($E_{6al}$) (TCDB classification 2.A.1.1.1), glucose permease ($E_{6am}$) (TCDB classification 2.A.1.1.65), indole-3-glycerol phosphate aldolase ($E_{6an}$) (EC 4.2.1.20), indole-3-glycerol phosphate synthase ($E_{6ao}$) (EC 4.1.1.48), isocitrate lyase ($E_{6ap}$) (EC 4.1.3.1), malate synthase ($E_{6aq}$) (EC 2.3.3.9), 3-Phosphoglycerate dehydrogenase ($E_{6ar}$) (EC 1.1.1.95, EC 1.1.1.399), phosphoribosylanthranilate isomerase ($E_{6as}$) (EC 5.3.1.24), phosphoserine aminotransferase ($E_{6at}$) (EC 2.6.1.52), phosphoserine phosphatase ($E_{6au}$) (EC 3.1.3.3), 3-Phosphoshikimate 1-carboxyvinyltransferase ($E_{6av}$) (EC 2.5.1.19), ribulose-5-phosphate epimerase ($E_{6aw}$) (EC 5.1.3.1), ribulose-5-phosphate isomerase ($E_{6ax}$) (EC 5.3.1.6), shikimate dehydrogenase ($E_{6ay}$) (EC 1.1.1.25, EC 1.1.1.282), shikimate kinase ($E_{6az}$) (EC 2.7.1.71), transaldolase ($E_{6ba}$) (EC 2.2.1.2), transketolase ($E_{6bb}$) (EC 2.2.1.1), tryptophan synthase ($E_{6bc}$) (EC 4.2.1.20), and tryptophan exporter ($E_{6bd}$) (TCDB classification 2.A.7.17.2). In particular, $E_6$ may selected from the group consisting of a feedback-resistant variant of anthranilate synthase ($E_{6af}$), a feedback-resistant variant of 2-Dehydro-3-deoxyphosphoheptonate aldolase ($E_{6ah}$), transketolase ($E_{6bb}$), glucose permease ($E_{6am}$). In one example, where the enzyme $E_6$ is a feedback-resistant variant of anthranilate synthase ($E_{6af}$) or a feedback-resistant variant of 2-Dehydro-3-deoxyphosphoheptonate aldolase ($E_{6ah}$), the enzymes are disclosed at least in Li, Y., et al. Current status on metabolic engineering for the production of L-aspartate family amino acids and derivatives. Bioresour. Technol. (2017), particularly on page 8.

In a further example, when the target amino acid produced according to any aspect of the present invention is an isoleucine, the enzymes $E_6$ may be selected from the group consisting of aspartate kinase ($E_{6a}$) (EC 2.7.2.4), aspartate semialdehyde dehydrogenase ($E_{6b}$) (EC 1.2.1.11), acetolactate synthase ($E_{6x}$) (EC 2.2.1.6) also known as an acetohydroxyacid synthase or a acetohydroxybutanoate synthase, $\alpha$-acetohydroxy acid isomeroreductase ($E_{6w}$) (EC 1.1.1.86), 2,3-Dihydroxy acid hydro-lyase ($E_{6y}$) (EC 4.2.1.19), glyceraldehyde-3-phosphate dehydrogenase (NADP-dependent) ($E_{6j}$) (EC 1.2.1.9, EC 1.2.1.13, EC 1.2.1.59, EC 1.2.1.60), homoserine dehydrogenase ($E_{6k}$) (EC 1.1.1.3), homoserine kinase ($E_{6l}$) (EC 2.7.1.39), isoleucine transaminase ($E_{6be}$) (EC 2.6.1.42), isoleucine exporter ($E_{6bf}$) (TCDB classification 2.A.78.1.2, 2.A.76.1.5), phosphoenolpyruvate (PEP) carboxylase ($E_{6g}$) (EC 4.1.1.31), pyruvate carboxylase ($E_{6i}$) (EC 6.4.1.1), PEP carboxykinase ($E_{6bg}$) (EC 4.1.1.32, EC 4.1.1.38, EC 4.1.1.49), threonine synthase ($E_{6m}$) (EC 4.2.3.1) and threonine deaminase ($E_{6bh}$) (EC 4.3.1.19). In particular, $E_6$ may be selected from the group consisting of a feedback-resistant variant of aspartate kinase ($E_{6a}$), homoserine dehydrogenase ($E_{6k}$), acetolactate synthase ($E_{6x}$), feedback-resistant variant of threonine dehydratase also known as threonine deaminase ($E_{6bh}$), homoserine kinase ($E_{6l}$), $\alpha$-acetohydroxy acid isomeroreductase ($E_{6w}$), 2,3-Dihydroxy acid hydro-lyase ($E_{6y}$), isoleucine transaminase ($E_{6be}$) and isoleucine exporter ($E_{6bf}$). In one example, the enzyme $E_6$ is a feedback-resistant variant of aspartate kinase ($E_{6a}$), homoserine dehydrogenase ($E_{6k}$), acetolactate synthase ($E_{6x}$), or a feedback-resistant variant of threonine deaminase ($E_{6bh}$) also known as dehydratase, the enzymes are disclosed at least in Li, Y., et al. Current status on metabolic engineering for the production of L-aspartate family amino acids and derivatives. Bioresour. Technol. (2017), particularly on page 8.

In addition to the cells according to any aspect of the present invention being genetically modified to increase the expression of the enzymes $E_1$, $E_2$, $E_3$, $E_4$, $E_5$, $E_6$ and optionally $E_{7a}$ or $E_{7b}$ depending on the substrate used, the cell according to any aspect of the present invention may also be genetically modified to decrease the expression of at least one enzyme $E_8$.

**Enzyme $E_8$**

In particular, the specific enzyme $E_8$ may be dependent on the target amino acid to be produced. Accordingly, if the cell according to any aspect of the present invention is genetically modified to produce lysine from a C1-C4 alkane, the cell is further genetically modified to decrease the expression of at least one enzyme $E_8$ selected from the group consisting of isocitrate dehydrogenase ($E_{8j}$) (EC 1.1.1.41, EC 1.1.1.42), lysine importer ($E_{8r}$) (TCDB classification 1.B.25.1.1, 2.A.3.1.18; 2.A.3.1.19; 2.A.3.1.2), PEP carboxykinase ($E_{6bg}$) (EC 4.1.1.32, EC 4.1.1.38, EC 4.1.1.49) and threonine deaminase ($E_{6bh}$) (EC 4.3.1.19), relative to the wild type cell. In particular, $E_8$ may be selected from the group consisting of isocitrate dehydrogenase ($E_{8j}$) (EC 1.1.1.41, EC 1.1.1.42), lysine importer ($E_{8r}$) (TCDB classification 1.B.25.1.1, 2.A.3.1.18; 2.A.3.1.19; 2.A.3.1.2), PEP carboxykinase ($E_{6bg}$) and threonine deaminase ($E_{6bh}$) (EC 4.3.1.19), relative to the wild type cell.

If the cell according to any aspect of the present invention is genetically modified to produce O-Acetyl homoserine from a C1-C4 alkane, the cell is further genetically modified to decrease the expression of at least one enzyme $E_8$ selected from the group consisting of diaminopimelate decarboxylase ($E_{6e}$) (EC 4.1.1.20), homoserine kinase ($E_{6l}$) (EC 2.7.1.39), homoserine O-succinyltransferase ($E_{6r}$) (EC 2.3.1.46), isocitrate dehydrogenase ($E_{8j}$) (EC 1.1.1.41, EC 1.1.1.42), PEP carboxykinase ($E_{6bg}$) (EC 4.1.1.32, EC 4.1.1.38, EC 4.1.1.49), threonine deaminase ($E_{6h}$) (EC 4.3.1.19), O-acetyl homoserine sulfhydrylase ($E_{6u}$) (EC 2.5.1.49), O-succinyl homoserine sulfhydrylase ($E_{6v}$) (EC 2.5.1.48), and O-Acetyl homoserine importer ($E_{8k}$) (TCDB classification 2.A.1.53.1, 2.A.23.4.1, 2.A.42.2.2), relative to the wild type cell.

If the cell according to any aspect of the present invention is genetically modified to produce threonine from a C1-C4 alkane, the cell is further genetically modified to decrease the expression of at least one enzyme $E_8$ selected from the group consisting of diaminopimelate decarboxylase ($E_{6e}$) (EC 4.1.1.20), homoserine dehydrogenase ($E_{6k}$) (EC 1.1.1.3), isocitrate dehydrogenase ($E_{6j}$) (EC 1.1.1.41, EC 1.1.1.42), PEP carboxykinase ($E_{6bg}$) (EC 4.1.1.32, EC 4.1.1.38, EC 4.1.1.49), serine hydroxymethyltransferase ($E_{8l}$) (EC 2.1.2.1), threonine aldolase ($E_{8m}$) (EC 4.1.2.48), threonine dehydrogenase ($E_{8n}$) (EC 1.1.1.103), threonine deaminase ($E_{6bh}$) (EC 4.3.1.19), and threonine importer ($E_{8s}$) (TCDB classification 2.A.1.53.1, 2.A.23.4.1, 2.A.42.2.2), relative to the wild type cell.

If the cell according to any aspect of the present invention is genetically modified to produce methionine from a C1-C4 alkane, the cell is further genetically modified to decrease the expression of at least one enzyme $E_8$ selected from the group consisting of diaminopimelate decarboxylase ($E_{6e}$) (EC 4.1.1.20), homoserine kinase ($E_{6l}$) (EC 2.7.1.39), isocitrate dehydrogenase ($E_{8j}$) (EC 1.1.1.41, EC 1.1.1.42), PEP carboxykinase ($E_{6bg}$) (EC 4.1.1.32, EC 4.1.1.38, EC 4.1.1.49), threonine deaminase ($E_{6bh}$) (EC 4.3.1.19), and methionine importer ($E_{8t}$) (TCDB classification 2.A.22.4.3, 3.A.1.24.3; 3.A.1.24.2; 3.A.1.24.1; 3.A.1.24.4; 3.A.1.24.6; 3.A.1.3.24), relative to the wild type cell.

If the cell according to any aspect of the present invention is genetically modified to produce valine from a C1-C4 alkane, the cell is further genetically modified to decrease the expression of at least one enzyme $E_8$ selected from the group consisting of alanine aminotransferase ($E_{8a}$) (EC 2.6.1.2, EC 2.6.1.12, EC 2.6.1.32), dihydrolipoamide acetyltransferase ($E_{8b}$) (EC 2.3.1.12), 2-Isopropylmalate synthase ($E_{8c}$) (EC 2.3.3.13), malate dehydrogenase ($E_{8d}$) (EC 1.1.1.37), 3-Methyl-2-oxobutanoate hydroxymethyl transferase ($E_{8e}$) (EC 2.1.2.11), pantoate-beta-alanine ligase ($E_{8f}$) (EC 6.3.2.1), phosphoenolpyruvate (PEP) carboxylase ($E_{6g}$) (EC 4.1.1.31), pyruvate dehydrogenase ($E_{8g}$) (EC 1.2.4.1), pyruvate:quinone oxidoreductase ($E_{8h}$) (EC 1.2.5.1), valine importer ($E_{8i}$) (TCDB classification 2.A.1.53.2, 2.A.26.1.9, 2.A.3.3.23, 3.A.1.4.1, 3.A.1.3.23), relative to the wild type cell.

If the cell according to any aspect of the present invention is genetically modified to produce tryptophan from a C1-C4 alkane, the cell is further genetically modified to decrease the expression of at least one enzyme $E_8$ selected from the group consisting of chorismate mutase ($E_{8l}$) (EC 5.4.99.5), glucose-specific PEP-dependent phosphotransferase system ($E_{8m}$) (EC 2.7.1.199), phosphoglucoisomerase ($E_{8n}$) (EC 5.3.1.9), prephenate dehydratase ($E_{8o}$) EC 4.2.1.51, pyruvate carboxylase ($E_{6i}$) (EC 6.4.1.1), pyruvate kinase ($E_{8p}$) (EC 2.7.1.40) and tryptophan importer ($E_{8q}$) (TCDB classification 2.A.22.4.1, 2.A.22.5.3, 2.A.3.1.22, 2.A.42.1.2, 2.A.42.1.3, 2.A.88.4.1, 3.A.1.34.1, 2.A.3.1.12, 2.A.3.1.3), relative to the wild type cell.

If the cell according to any aspect of the present invention is genetically modified to produce isoleucine from a C1-C4 alkane, the cell is further genetically modified to decrease the expression of at least one enzyme $E_8$ selected from the group consisting of diaminopimelate decarboxylase ($E_{6e}$) (EC 4.1.1.20), isocitrate dehydrogenase ($E_{8j}$) (EC 1.1.1.41, EC 1.1.1.42), isoleucine importer ($E_{8u}$) (TCDB classification 2.A.1.53.2, 2.A.26.1.9, 2.A.3.3.23, 3.A.1.4.1, 3.A.1.3.23), serine hydroxymethyltransferase ($E_{8l}$) (EC 2.1.2.1), threonine aldolase ($E_{8m}$) (EC 4.1.2.48), and threonine dehydrogenase ($E_{8n}$) (EC 1.1.1.103), relative to the wild type cell.

- **Lysine**

Lysine may be the target amino acid that may be produced from at least one alkane selected from the group consisting of C1-C4 alkane according to any aspect of the present invention. In particular, the cell according to any aspect of the present invention may be genetically modified to increase the expression relative to the wild type cell of at least one of the following enzymes $E_1$- $E_6$. More in particular, the cell according to any aspect of the present invention which is used to produce lysine as the target amino acid, may be genetically modified to increase the expression of all the enzymes $E_1$-$E_6$. Even more in particular, $E_1$- $E_6$ are:

- the Enzyme $E_1$ is selected from the group consisting of P450 alkane hydroxylase ($E_a$) of EC 1.14.15.3- and AlkB alkane hydroxylase ($E_b$) of EC 1.14.15.3;

- the Enzyme $E_2$ is selected from the group consisting of P450 alkane hydroxylase ($E_a$) of EC 1.14.15.3-, AlkB alkane hydroxylase ($E_b$) of EC 1.14.15.3, alcohol oxidase ($E_c$) of EC 1.1.3.20 and alcohol dehydrogenase ($E_d$) of EC 1.1.1.1 or EC 1.1.1.2;

- the Enzyme $E_3$ is selected from the group consisting of P450 alkane hydroxylase ($E_a$) of EC 1.14.15.3-, AlkB alkane hydroxylase ($E_b$) of EC 1.14.15.3, aldehyde dehydrogenase ($E_e$) of EC 1.2.1.3, EC 1.2.1.4 or EC 1.2.1.5, alcohol oxidase ($E_c$) of EC 1.1.3.20, AlkJ alcohol dehydrogenase ($E_{di}$) of EC 1.1.99.- and alcohol dehydrogenase ($E_d$) of EC 1.1.1.1 or EC 1.1.1.2, wherein $E_c$, $E_{di}$, and $E_d$ are each capable of oxidizing an ω-hydroxy alkanoic acid ester directly to the corresponding ω-carboxy alkanoic acid ester;

- the Enzyme $E_4$ is selected from the group consisting of fatty acyl coenzyme A (CoA) synthase (FACS) ($E_f$) of EC 6.2.1.1, EC 6.2.1.2, EC 6.2.1.3, or EC 2.3.1.86; acyl-Acyl Carrier Protein (ACP) synthase ($E_g$) of EC 6.2.1.20 or EC 6.2.1.47; fatty acyl kinase ($E_h$) of EC 2.7.2.1, EC 2.7.2.12, EC 2.7.2.15 or EC 2.7.2.7 and phosphotransacylase ($E_i$) of EC 2.3.1.8 or EC 2.3.1.19; and fatty acyl coenzyme A synthase (Ef) of EC 6.2.1.1, EC 6.2.1.2 or EC 6.2.1.3 and fatty acyl-CoA:ACP transacylase ($E_j$) of EC 2.3.1.38 or EC 2.3.1.39;

- the Enzyme $E_5$ is selected from the group consisting of aldehyde dehydrogenase ($E_e$) of EC 1.2.1.3, EC 1.2.1.4 or EC 1.2.1.5, alcohol oxidase ($E_c$) of EC 1.1.3.20, AlkJ alcohol dehydrogenase ($E_{di}$) of EC 1.1.99.- and alcohol dehydrogenase ($E_d$) of EC 1.1.1.1 or EC 1.1.1.2; and

- the Enzyme $E_6$ is capable of converting the fatty acyl thioester of any aspect of the present invention, in particular step (iii) to a corresponding amino acid.

The Enzyme $E_6$ capable of converting the fatty acyl thioester of any aspect of the present invention, in particular step (iii) to the lysine may be selected from the group consisting of aspartate kinase ($E_{6a}$) (EC 2.7.2.4), aspartate semialdehyde dehydrogenase ($E_{6b}$) (EC 1.2.1.11), 4-hydroxy-tetrahydrodipicolinate synthase ($E_{6c}$) (EC 4.3.3.7), dihydrodipicolinate reductase ($E_{6d}$) (EC 1.17.1.8), diaminopimelate decarboxylase ($E_{6e}$) (EC 4.1.1.20), lysine exporter ($E_{6f}$) (TCDB families 2.A.124.1.1, 2.A.75.1.1 or 2.A.75.1.2), phosphoenolpyruvate (PEP) carboxylase ($E_{6g}$) (EC 4.1.1.31), proton-translocating transhydrogenase ($E_{6h}$) (EC 1.6.1.5), and pyruvate carboxylase ($E_{6i}$) (EC 6.4.1.1). In particular, $E_6$ may be an aspartate kinase ($E_{6a}$) comprising SEQ ID NO:1, 79 or a variant thereof, an aspartate semialdehyde dehydrogenase ($E_{6b}$) comprising SEQ ID NO:2, 82 or a variant thereof, a 4-hydroxy-tetrahydrodipicolinate synthase ($E_{6c}$) comprising SEQ ID NO:3 or a variant thereof, a dihydrodipicolinate reductase ($E_{6d}$) comprising SEQ ID NO:5 or a variant thereof, a diaminopimelate decarboxylase ($E_{6e}$) comprising SEQ ID NO:6 or a variant thereof, a lysine exporter ($E_{6f}$) comprising SEQ ID NO:7, 8, 9 or a variant thereof, , phosphoenolpyruvate (PEP) carboxylase ($E_{6g}$) comprising SEQ ID NO:10 or a variant thereof, proton-translocating transhydrogenase ($E_{6h}$) comprising SEQ ID NO:11, 20 or a variant thereof, and pyruvate carboxylase ($E_{6i}$) comprising SEQ ID NO:12 or a variant thereof. More in particular, the enzyme $E_6$ may be selected from the group consisting of aspartate kinase ($E_{6a}$) and 4-hydroxy-tetrahydrodipicolinate synthase ($E_{6c}$). Even more in particular, the enzyme $E_6$ may comprise the sequence SEQ ID NO:1, 3 or a variant thereof. In one example, the enzyme $E_6$ may consists of the sequence SEQ ID NO:1, 3 or a variant thereof.

The cell capable of producing lysine according to any aspect of the present invention may also be genetically modified to decrease the expression of at least one enzyme $E_8$ selected from the group consisting of isocitrate dehydrogenase ($E_{8j}$) (EC 1.1.1.41, EC 1.1.1.42), lysine importer ($E_{8r}$) (TCDB classification 1.B.25.1.1, 2.A.3.1.18; 2.A.3.1.19; 2.A.3.1.2), PEP carboxykinase ($E_{6bg}$) (EC 4.1.1.32, EC 4.1.1.38, EC 4.1.1.49) and threonine deaminase ($E_{6bh}$) (EC 4.3.1.19), relative to the wild type cell.

Accordingly, a cell capable of producing lysine from at least one C1-C4 alkane, may be genetically modified to increase the expression of $E_1$, $E_2$, $E_3$, $E_4$, $E_5$, and $E_6$, and decrease the expression of $E_8$ relative to the wild type cell.

In one example, when the substrate alkane is a butane, the cell according to any aspect of the present invention used to produce lysine, may be further genetically modified to increase expression relative to the wild type cell of at least one further enzyme ($E_{7a}$). More in particular, the enzyme $E_{7a}$ may be selected from the group consisting of acyl-ACP synthetase ($E_g$) (EC 6.2.1.20), acyl-CoA synthetase ($E_f$) (EC 6.2.1.2, EC 6.2.1.3, EC 6.2.1.10), and the combination of butyrate kinase ($E_{hi}$), (EC 2.7.2.7) and phosphotransbutyrylase ($E_{ii}$) (EC 2.3.1.19). In particular, enzyme $E_{7a}$ may be an

acyl-ACP synthetase ($E_g$) comprising SEQ ID NO:21 or a variant thereof, or an acyl-CoA synthetase ($E_f$) comprising SEQ ID NO:22 or a variant thereof, or the combination of butyrate kinase ($E_{hi}$) comprising SEQ ID NO:25 or a variant thereof and phosphotransacylase ($E_i$) comprising SEQ ID NO:24 or a variant thereof.

In another example, when the substrate alkane is a propane, the cell according to any aspect of the present invention used to produce lysine, may be further genetically modified to increase expression relative to the wild type cell of at least one further enzyme ($E_{7b}$). More in particular, the enzyme $E_{7b}$ may be selected from the group consisting of acyl-ACP synthetase ($E_g$) (EC 6.2.1.20), acyl-CoA synthetase ($E_f$) (EC 6.2.1.2, EC 6.2.1.3, EC 6.2.1.10), methylisocitrate hydro-lyase ($E_{7bi}$) (EC 4.2.1.99), methylisocitrate lyase ($E_{7bii}$) (EC 4.1.3.30), 2-Methylisocitrate dehydratase ($E_{7biii}$) (EC 4.2.1.79), 2-Methylcitrate synthase ($E_{7biv}$) (EC 2.3.3.5), combination of phosphotranspropionylase ($E_{iii}$) (EC 2.3.1.19, EC 2.3.1.8) and propionate kinase ($E_{hii}$) (EC 2.7.2.15) and propionyl-CoA ligase ($E_{7bvii}$) (EC 6.2.1.17). Even more in particular, the enzyme $E_{7b}$ may be an acyl-ACP synthetase ($E_g$) comprising SEQ ID NO:21 or a variant thereof, or an acyl-CoA synthetase ($E_f$) comprising SEQ ID NO:22 or a variant thereof, or a methylisocitrate hydro-lyase ($E_{7bi}$) comprising SEQ ID NO:27, 94 or a variant thereof, a methylisocitrate lyase ($E_{7bii}$) comprising SEQ ID NO:28, 95, 96 or a variant thereof, a 2-Methylisocitrate dehydratase ($E_{7biii}$) comprising SEQ ID NO:29, 97, 98 or a variant thereof, a 2-Methylcitrate synthase ($E_{7biv}$) comprising SEQ ID NO:30, 99, 100 or a variant thereof or the combination of phosphotranspropionylase ($E_{iii}$) comprising SEQ ID NO:31, 101 or a variant thereof and propionate kinase ($E_{hii}$) comprising SEQ ID NO:26, 4 or a variant thereof, a propionyl-CoA ligase ($E_{7bvii}$) (EC 6.2.1.17) comprising SEQ ID NO:32 or a variant thereof or propionyl-CoA:acetate Coenzyme A transferase ($E_{7bviii}$) comprising SEQ ID NO:17 a variant thereof.

In particular, according to any aspect of the present invention, the cell may be genetically modified to increase the expression of all the enzymes $E_1$- $E_6$ for production of lysine from at least one C1-C4 alkane, wherein, $E_1$- $E_6$ are:

- $E_1$ is a butane monoxygenase ($E_c$) (EC 1.14.13.230), preferably comprising the sequences with accession numbers AAM 19732.1, AAM19730.1, AAM19728.1, AAM19727.1, AAM 19729.1, ABU68845.2, WP_031430811, AAM19731.1, WP_003609331.1 or variants thereof;
- $E_2$ is an alcohol dehydrogenase ($E_d$) (EC 1.1.1.1 or EC 1.1.1.2), preferably comprising SEQ ID NO:91 or a variant thereof;
- $E_3$ is an aldehyde dehydrogenase ($E_e$) (EC 1.2.1.3, EC 1.2.1.4 or EC 1.2.1.5), preferably comprising SEQ ID NO:42 or a variant thereof;
- $E_4$ is fatty acyl CoA synthase (FACS) (Ef) (EC 6.2.1.1, EC 6.2.1.2 or EC 6.2.1.3), preferably comprising SEQ ID NO:88 or variant thereof; and
- $E_6$ is selected from the group consisting of:

    (i) a feedback-resistant variant of aspartate kinase ($E_{6a}$), preferably comprising SEQ ID NO:1 with a point mutation of T342I, or SEQ ID NO:79 with at least one point mutation selected from the group consisting of T311I, A279T, S301Y, A279V, S301F, T308I, S317A, R320G, G345D, S381F, Q404E, G408R, G277A, Q298A, T361A, E363A, and F364A, and
    (ii) a feedback-resistant variant of 4-hydroxy-tetrahydrodipicolinate synthase ($E_{6c}$) (EC 4.3.3.7), preferably comprising SEQ ID NO:3 or a variant thereof comprising point mutations G84T, G250A and/or A251C;
    preferably is $E_6$ a combination of $E_{6a}$ and $E_{6c}$.

- **O-acetyl Homoserine**

O-acetyl Homoserine may be the target amino acid that may be produced from at least one alkane selected from the group consisting of C1-C4 alkane according to any aspect of the present invention. In particular, the cell according to any aspect of the present invention may be genetically modified to increase the expression relative to the wild type cell of at least one of the following enzymes $E_1$- $E_6$. More in particular, the cell according to any aspect of the present invention which is used to produce O-acetyl Homoserine as the target amino acid, may be genetically modified to increase the expression of all the enzymes $E_1$- $E_6$. Even more in particular, $E_1$- $E_6$ are:

- the Enzyme $E_1$ is selected from the group consisting of P450 alkane hydroxylase ($E_a$) of EC 1.14.15.3- and AlkB alkane hydroxylase ($E_b$) of EC 1.14.15.3;
- the Enzyme $E_2$ is selected from the group consisting of P450 alkane hydroxylase ($E_a$) of EC 1.14.15.3-, AlkB alkane hydroxylase ($E_b$) of EC 1.14.15.3, alcohol oxidase ($E_c$) of EC 1.1.3.20 and alcohol dehydrogenase ($E_d$) of EC 1.1.1.1 or EC 1.1.1.2;
- the Enzyme $E_3$ is selected from the group consisting of P450 alkane hydroxylase ($E_a$) of EC 1.14.15.3-, AlkB alkane hydroxylase ($E_b$) of EC 1.14.15.3, aldehyde dehydrogenase ($E_e$) of EC 1.2.1.3, EC 1.2.1.4 or EC 1.2.1.5, alcohol oxidase ($E_c$) of EC 1.1.3.20, AlkJ alcohol dehydrogenase ($E_{di}$) of EC 1.1.99.- and alcohol dehydrogenase ($E_d$) of EC 1.1.1.1 or EC 1.1.1.2, wherein $E_c$, $E_{di}$, and $E_d$ are each capable of oxidizing an ω-hydroxy alkanoic acid ester directly to the corresponding ω-carboxy alkanoic acid ester;

- the Enzyme $E_4$ is selected from the group consisting of fatty acyl coenzyme A (CoA) synthase (FACS) ($E_f$) of EC 6.2.1.1, EC 6.2.1.2, EC 6.2.1.3, or EC 2.3.1.86; acyl-Acyl Carrier Protein (ACP) synthase ($E_g$) of EC 6.2.1.20 or EC 6.2.1.47; fatty acyl kinase ($E_h$) of EC 2.7.2.1, EC 2.7.2.12, EC 2.7.2.15 or EC 2.7.2.7 and phosphotransacylase ($E_i$) of EC 2.3.1.8 or EC 2.3.1.19; and fatty acyl coenzyme A synthase ($E_f$) of EC 6.2.1.1, EC 6.2.1.2 or EC 6.2.1.3 and fatty acyl-CoA:ACP transacylase ($E_j$) of EC 2.3.1.38 or EC 2.3.1.39;
- the Enzyme $E_5$ is selected from the group consisting of aldehyde dehydrogenase ($E_e$) of EC 1.2.1.3, EC 1.2.1.4 or EC 1.2.1.5, alcohol oxidase ($E_c$) of EC 1.1.3.20, AlkJ alcohol dehydrogenase ($E_{di}$) of EC 1.1.99.- and alcohol dehydrogenase ($E_d$) of EC 1.1.1.1 or EC 1.1.1.2; and
- the Enzyme $E_6$ is capable of converting the fatty acyl thioester of any aspect of the present invention, in particular step (iii) to a corresponding amino acid.

The Enzyme $E_6$ capable of converting the fatty acyl thioester of any aspect of the present invention, in particular step (iii) to the o-actyl homoserine may be selected from the group consisting of aspartate kinase ($E_{6a}$) (EC 2.7.2.4), aspartate semialdehyde dehydrogenase ($E_{6b}$) (EC 1.2.1.11), glyceraldehyde-3-phosphate dehydrogenase (NADP-dependent) ($E_{6j}$) (EC 1.2.1.9, EC 1.2.1.13, EC 1.2.1.59, EC 1.2.1.60), homoserine dehydrogenase ($E_{6k}$) (EC 1.1.1.3), homoserine kinase ($E_{6l}$) (EC 2.7.1.39), phosphoenolpyruvate (PEP) carboxylase ($E_{6g}$) (EC 4.1.1.31), proton-translocating transhydrogenase ($E_{6h}$) (EC 1.6.1.5), pyruvate carboxylase ($E_{6i}$) (EC 6.4.1.1), threonine synthase ($E_{6m}$) (EC 4.2.3.1), and threonine exporter ($E_{6n}$) (TCDB families 2.A.7.3.6, 2.A.76.1.10 or 2.A.79.1.1). In particular, $E_6$ may be an aspartate kinase ($E_{6a}$) comprising SEQ ID NO:1, 79 or a variant thereof, an aspartate semialdehyde dehydrogenase ($E_{6b}$) comprising SEQ ID NO:2, 82 or a variant thereof, glyceraldehyde-3-phosphate dehydrogenase (NADP-dependent) ($E_{6j}$) comprising SEQ ID NO:13 or a variant thereof, homoserine dehydrogenase ($E_{6k}$) comprising SEQ ID NO:14, 51, 80 or a variant thereof, homoserine kinase ($E_{6l}$) comprising SEQ ID NO:15, 81 or a variant thereof, homoserine O-acetyltransferase ($E_{6s}$) comprising SEQ ID NO:16, 78, 87 or a variant thereof, phosphoenolpyruvate (PEP) carboxylase ($E_{6g}$) comprising SEQ ID NO:10 or a variant thereof, a proton-translocating transhydrogenase ($E_{6h}$) comprising SEQ ID NO:11, 20 or a variant thereof, pyruvate carboxylase ($E_{6i}$) comprising SEQ ID NO:12 or a variant thereof, O-Acetyl homoserine exporter ($E_{6ad}$) comprising SEQ ID NO:19, 84, 85, 86 or a variant thereof. More in particular, $E_6$ may be a feedback-resistant variant of aspartate kinase ($E_{6a}$) comprising SEQ ID NO:1 with a point mutation of T342I, or SEQ ID NO:79 with at least one point mutation selected from the group consisting of T3111, A279T, S301Y, A279V, S301F, T308I, S317A, R320G, G345D, S381F, Q404E, G408R, G277A, Q298A, T361A, E363A, and F364A, particularly with point mutation T3111, may be a feedback-resistant variant of homoserine dehydrogenase ($E_{6k}$) comprising SEQ ID NO:14 with at least one point mutation selected from the group consisting of G378E, D375A, V379E, L380E, I392P, S393A, L394P and Q399T, SEQ ID NO:51 with point mutation S345F or SEQ ID NO:80, or may be a feedback-resistant variant of homoserine O-acetyltransferase ($E_{6s}$) comprising SEQ ID NO:78 with point mutation Y294C.

Even more in particular, the enzyme $E_6$ may be selected from the group consisting of a feedback resistant variant of homoserine dehydrogenase (also known as a bifunctional aspartokinase I/homoserine dehydrogenase I ($E_{6k}$), homoserine O-acetyltransferase ($E_{6s}$) and a feedback-resistant variant of aspartate kinase ($E_{6a}$). Even more in particular, the enzyme $E_6$ may comprise the sequence SEQ ID NO:14, 51, 16, 78 or a variant thereof. In one example, the enzyme $E_6$ may consists of the sequence SEQ ID NO:14, 51, 16, 78 or a variant thereof.

The cell capable of producing o-acetyl homoserine according to any aspect of the present invention may also be genetically modified to decrease the expression of at least one enzyme $E_8$ selected from the group consisting of decarboxylase ($E_{6e}$) (EC 4.1.1.20), homoserine kinase ($E_{6l}$) (EC 2.7.1.39), homoserine O-succinyltransferase ($E_{6r}$) (EC 2.3.1.46), isocitrate dehydrogenase ($E_{8j}$) (EC 1.1.1.41, EC 1.1.1.42), PEP carboxykinase ($E_{6bg}$) (EC 4.1.1.32, EC 4.1.1.38, EC 4.1.1.49), threonine deaminase ($E_{6h}$) (EC 4.3.1.19), O-acetyl homoserine sulfhydrylase ($E_{6u}$) (EC 2.5.1.49), O-succinyl homoserine sulfhydrylase ($E_{6v}$) (EC 2.5.1.48), and O-Acetyl homoserine importer ($E_{8k}$) (TCDB classification 2.A.1.53.1, 2.A.23.4.1, 2.A.42.2.2), relative to the wild type cell. Accordingly, a cell capable of producing o-acetyl homoserine from at least one C1-C4 alkane, may be genetically modified to increase the expression of $E_1$, $E_2$, $E_3$, $E_4$, $E_5$, and $E_6$, and decrease the expression of $E_8$ relative to the wild type cell.

In one example, when the substrate alkane is a butane, the cell according to any aspect of the present invention used to produce o-acetyl homoserine, may be further genetically modified to increase expression relative to the wild type cell of at least one further enzyme ($E_{7a}$). More in particular, the enzyme $E_{7a}$ may be selected from the group consisting of acyl-ACP synthetase ($E_g$) (EC 6.2.1.20), acyl-CoA synthetase ($E_f$) (EC 6.2.1.2, EC 6.2.1.3, EC 6.2.1.10), and the combination of butyrate kinase ($E_{hi}$), (EC 2.7.2.7) and phosphotransbutyrylase ($E_{ii}$) (EC 2.3.1.19). In particular, enzyme $E_{7a}$ may be an acyl-ACP synthetase ($E_g$) comprising SEQ ID NO:21 or a variant thereof, or an acyl-CoA synthetase ($E_f$) comprising SEQ ID NO:22 or a variant thereof, or the combination of butyrate kinase ($E_{hi}$) comprising SEQ ID NO:25 or a variant thereof and phosphotransacylase ($E_i$) comprising SEQ ID NO:24 or a variant thereof.

In another example, when the substrate alkane is a propane, the cell according to any aspect of the present invention used to produce o-acetyl homoserine, may be further genetically modified to increase expression relative to the wild type cell of at least one further enzyme ($E_{7b}$). More in particular, the enzyme $E_{7b}$ may be selected from the group consisting of acyl-ACP synthetase ($E_g$) (EC 6.2.1.20), acyl-CoA synthetase ($E_f$) (EC 6.2.1.2, EC 6.2.1.3, EC 6.2.1.10), methylisocitrate hydro-lyase ($E_{7bi}$) (EC 4.2.1.99), methylisocitrate lyase ($E_{7bii}$) (EC 4.1.3.30), 2-Methylisocitrate dehydratase ($E_{7biii}$) (EC 4.2.1.79), 2-Methylcitrate synthase ($E_{7biv}$) (EC 2.3.3.5), combination of phosphotranspropionylase ($E_{iii}$) (EC 2.3.1.19, EC 2.3.1.8) and propionate kinase ($E_{hii}$) (EC 2.7.2.15) and propionyl-CoA ligase ($E_{7bvii}$) (EC 6.2.1.17). Even more in particular, the enzyme $E_{7b}$ may be an acyl-ACP synthetase ($E_g$) comprising SEQ ID NO:21 or a variant thereof, or an acyl-CoA synthetase ($E_f$) comprising SEQ ID NO:22 or a variant thereof, or a methylisocitrate hydro-lyase ($E_{7bi}$) comprising SEQ ID NO:27, 94 or a variant thereof, a methylisocitrate lyase ($E_{7bii}$) comprising SEQ ID NO:28, 95, 96 or a variant thereof, a 2-Methylisocitrate dehydratase ($E_{7biii}$) comprising SEQ ID NO:29, 97, 98 or a variant thereof, a 2-Methylcitrate synthase ($E_{7biv}$) comprising SEQ ID NO:30, 99, 100 or a variant thereof or the combination of phosphotranspropionylase ($E_{iii}$) comprising SEQ ID NO:31, 101 or a variant thereof and propionate kinase ($E_{hii}$) comprising SEQ ID NO:26, 4 or a variant thereof, a propionyl-CoA ligase ($E_{7bvii}$) (EC 6.2.1.17) comprising SEQ ID NO:32 or a variant thereof or propionyl-CoA:acetate Coenzyme A transferase ($E_{7bviii}$) comprising SEQ ID NO:17 a variant thereof.

In particular, according to any aspect of the present invention, the cell may be genetically modified to increase the expression of all the enzymes $E_1$- $E_6$, wherein, $E_1$- $E_6$ are:

- $E_1$ is a butane monoxygenase ($E_c$) (EC 1.14.13.230), preferably comprising the sequences with accession numbers AAM19732.1, AAM19730.1, AAM19728.1, AAM19727.1, AAM 19729.1, and ABU68845.2 or variants thereof;
- $E_2$ is an alcohol dehydrogenase ($E_d$) (EC 1.1.1.1 or EC 1.1.1.2), preferably comprising SEQ ID NO:91 or a variant thereof;
- $E_3$ is an aldehyde dehydrogenase ($E_e$) (EC 1.2.1.3, EC 1.2.1.4 or EC 1.2.1.5), preferably comprising SEQ ID NO:42 or a variant thereof;
- $E_4$ is fatty acyl CoA synthase (FACS) ($E_f$) (EC 6.2.1.1, EC 6.2.1.2 or EC 6.2.1.3), preferably comprising SEQ ID NO:88 or variant thereof; and
- $E_6$ is selected from the group consisting of:

  (i) a feedback resistant variant of homoserine dehydrogenase ($E_{6k}$), preferably comprising SEQ ID NO:14 with at least one point mutation selected from the group consisting of G378E, D375A, V379E, L380E, I392P, S393A, L394P and Q399T, SEQ ID NO:51 with point mutation S345P or SEQ ID NO:80,
  (ii) a feedback-resistant variant of aspartate kinase ($E_{6a}$) comprising SEQ ID NO:1 with a point mutation of T342I, or SEQ ID NO:79 with at least one point mutation selected from the group consisting of T311I, A279T, S301Y, A279V, S301F, T308I, S317A, R320G, G345D, S381F, Q404E, G408R, G277A, Q298A, T361A, E363A, and F364A, and
  (iii) a feedback-resistant variant of homoserine O-acetyltransferase ($E_{6s}$) comprising SEQ ID NO:78 with point mutation Y294C;
  preferably is $E_6$ a combination of $E_{6k}$, $E_{6a}$ and $E_{6s}$.

- **Threonine**

Threonine may be the target amino acid that may be produced from at least one alkane selected from the group consisting of C1-C4 alkane according to any aspect of the present invention. In particular, the cell according to any aspect of the present invention may be genetically modified to increase the expression relative to the wild type cell of at least one of the following enzymes $E_1$- $E_6$. More in particular, the cell according to any aspect of the present invention which is used to produce threonine as the target amino acid, may be genetically modified to increase the expression of all the enzymes $E_1$- $E_6$. Even more in particular, $E_1$- $E_6$ are:

- the Enzyme $E_1$ is selected from the group consisting of P450 alkane hydroxylase ($E_a$) of EC 1.14.15.3- and AlkB alkane hydroxylase ($E_b$) of EC 1.14.15.3;
- the Enzyme $E_2$ is selected from the group consisting of P450 alkane hydroxylase ($E_a$) of EC 1.14.15.3-, AlkB alkane hydroxylase ($E_b$) of EC 1.14.15.3, alcohol oxidase ($E_c$) of EC 1.1.3.20 and alcohol dehydrogenase ($E_d$) of EC 1.1.1.1 or EC 1.1.1.2;
- the Enzyme $E_3$ is selected from the group consisting of P450 alkane hydroxylase ($E_a$) of EC 1.14.15.3-, AlkB alkane hydroxylase ($E_b$) of EC 1.14.15.3, aldehyde dehydrogenase ($E_e$) of EC 1.2.1.3, EC 1.2.1.4 or EC 1.2.1.5, alcohol oxidase ($E_c$) of EC 1.1.3.20, AlkJ alcohol dehydrogenase ($E_{di}$) of EC 1.1.99.- and alcohol dehydrogenase ($E_d$) of EC 1.1.1.1 or EC 1.1.1.2, wherein $E_c$, $E_{di}$, and $E_d$ are each capable of oxidizing an ω-hydroxy alkanoic acid ester directly to the corresponding ω-carboxy alkanoic acid ester;
- the Enzyme $E_4$ is selected from the group consisting of fatty acyl coenzyme A (CoA) synthase (FACS) ($E_f$) of EC 6.2.1.1, EC 6.2.1.2, EC 6.2.1.3, or EC 2.3.1.86; acyl-Acyl Carrier Protein (ACP) synthase ($E_g$) of EC 6.2.1.20 or EC 6.2.1.47; fatty acyl kinase ($E_h$) of EC 2.7.2.1, EC 2.7.2.12, EC 2.7.2.15 or EC 2.7.2.7 and phosphotransacylase ($E_i$) of EC 2.3.1.8 or EC 2.3.1.19; and fatty acyl coenzyme A synthase ($E_f$) of EC 6.2.1.1, EC 6.2.1.2 or EC 6.2.1.3 and fatty acyl-CoA:ACP transacylase ($E_j$) of EC 2.3.1.38 or EC 2.3.1.39;

- the Enzyme $E_5$ is selected from the group consisting of aldehyde dehydrogenase ($E_e$) of EC 1.2.1.3, EC 1.2.1.4 or EC 1.2.1.5, alcohol oxidase ($E_c$) of EC 1.1.3.20, AlkJ alcohol dehydrogenase ($E_{di}$) of EC 1.1.99.- and alcohol dehydrogenase ($E_d$) of EC 1.1.1.1 or EC 1.1.1.2; and
- the Enzyme $E_6$ is capable of converting the fatty acyl thioester of any aspect of the present invention, in particular step (iii) to a corresponding amino acid.

The Enzyme $E_6$ capable of converting the fatty acyl thioester of any aspect of the present invention, in particular step (iii) to the threonine may be selected from the group consisting of $E_6$ may be selected from the group consisting of aspartate kinase ($E_{6a}$) (EC 2.7.2.4), aspartate semialdehyde dehydrogenase ($E_{6b}$) (EC 1.2.1.11), glyceraldehyde-3-phosphate dehydrogenase (NADP-dependent) ($E_{6j}$) (EC 1.2.1.9, EC 1.2.1.13, EC 1.2.1.59, EC 1.2.1.60), homoserine dehydrogenase ($E_{6k}$) (EC 1.1.1.3), homoserine kinase ($E_{6l}$) (EC 2.7.1.39), phosphoenolpyruvate (PEP) carboxylase ($E_{6g}$) (EC 4.1.1.31), proton-translocating transhydrogenase ($E_{6h}$) (EC 1.6.1.5), pyruvate carboxylase ($E_{6i}$) (EC 6.4.1.1), threonine synthase ($E_{6m}$) (EC 4.2.3.1) and threonine exporter ($E_{6n}$) (TCDB families 2.A.7.3.6, 2.A.76.1.10 or 2.A.79.1.1). In particular, $E_6$ may be an aspartate kinase ($E_{6a}$) comprising SEQ ID NO:1, 79 or variant thereof, aspartate semialdehyde dehydrogenase ($E_{6b}$) comprising SEQ ID NO:2, 82 or variant thereof, glyceraldehyde-3-phosphate dehydrogenase (NADP-dependent) ($E_{6j}$) comprising SEQ ID NO:13 or variant thereof, homoserine dehydrogenase ($E_{6k}$) comprising SEQ ID NO:14, 51, 80 or variant thereof, homoserine kinase ($E_{6l}$) comprising SEQ ID NO:15, 81 or variant thereof, phosphoenolpyruvate (PEP) carboxylase ($E_{6g}$) comprising SEQ ID NO:10 or variant thereof, proton-translocating transhydrogenase ($E_{6h}$) comprising SEQ ID NO:11, 20 or variant thereof, pyruvate carboxylase ($E_{6i}$) comprising SEQ ID NO:12 or variant thereof, threonine synthase comprising SEQ ID NO:18, 83 or variant thereof and threonine exporter ($E_{6n}$) comprising SEQ ID NO:19, 84, 85, 86 or variant thereof. More in particular, $E_6$ may be selected from the group consisting of a feedback-resistant variant of aspartate kinase ($E_{6a}$) comprising SEQ ID NO:1 with a point mutation of T342I, or SEQ ID NO:79 with at least one point mutation selected from the group consisting of T3111, A279T, S301Y, A279V, S301F, T308I, S317A, R320G, G345D, S381F, Q404E, G408R, G277A, Q298A, T361A, E363A, and F364A, particularly with point mutation T3111, feedback-resistant variant of homoserine dehydrogenase ($E_{6k}$) comprising SEQ ID NO:14 with at least one point mutation selected from the group consisting of G378E, D375A, V379E, L380E, I392P, S393A, L394P and Q399T, SEQ ID NO:51 with point mutation S345P or SEQ ID NO:80, homoserine kinase ($E_{6l}$) comprising SEQ ID NO:15, 81 or a variant thereof and threonine exporter ($E_{6n}$) comprising SEQ ID NO:19, 84, 85, 86 or variant thereof. In one example, the enzyme $E_6$ may be a feedback-resistant variant of aspartate kinase ($E_{6a}$), or a feedback-resistant variant of homoserine dehydrogenase ($E_{6k}$). Examples of which, are provided at least in Li, Y., et al. Current status on metabolic engineering for the production of L-aspartate family amino acids and derivatives. Bioresour. Technol. (2017), particularly on page 8.

The cell capable of producing threonine according to any aspect of the present invention may also be genetically modified to decrease the expression of at least one enzyme $E_8$ selected from the group consisting of diaminopimelate decarboxylase ($E_{6e}$) (EC 4.1.1.20), homoserine dehydrogenase ($E_{6k}$) (EC 1.1.1.3), isocitrate dehydrogenase ($E_{6j}$) (EC 1.1.1.41, EC 1.1.1.42), PEP carboxykinase ($E_{6bg}$) (EC 4.1.1.32, EC 4.1.1.38, EC 4.1.1.49), serine hydroxymethyltransferase ($E_{8l}$) (EC 2.1.2.1), threonine aldolase ($E_{8m}$) (EC 4.1.2.48), threonine dehydrogenase ($E_{8n}$) (EC 1.1.1.103), threonine deaminase ($E_{6bh}$) (EC 4.3.1.19), and threonine importer ($E_{8s}$) (TCDB classification 2.A.1.53.1, 2.A.23.4.1, 2.A.42.2.2), relative to the wild type cell. Accordingly, a cell capable of producing threonine from at least one C1-C4 alkane, may be genetically modified to increase the expression of $E_1$, $E_2$, $E_3$, $E_4$, $E_5$, and $E_6$, and decrease the expression of $E_8$ relative to the wild type cell.

In one example, when the substrate alkane is a butane, the cell according to any aspect of the present invention used to produce threonine, may be further genetically modified to increase expression relative to the wild type cell of at least one further enzyme ($E_{7a}$). More in particular, the enzyme $E_{7a}$ may be selected from the group consisting of acyl-ACP synthetase ($E_g$) (EC 6.2.1.20), acyl-CoA synthetase ($E_f$) (EC 6.2.1.2, EC 6.2.1.3, EC 6.2.1.10), and the combination of butyrate kinase ($E_{hi}$), (EC 2.7.2.7) and phosphotransbutyrylase ($E_{ii}$) (EC 2.3.1.19). In particular, enzyme $E_{7a}$ may be an acyl-ACP synthetase ($E_g$) comprising SEQ ID NO:21 or a variant thereof, or an acyl-CoA synthetase ($E_f$) comprising SEQ ID NO:22 or a variant thereof, or the combination of butyrate kinase ($E_{hi}$) comprising SEQ ID NO:25 or a variant thereof and phosphotransacylase ($E_i$) comprising SEQ ID NO:24 or a variant thereof.

In another example, when the substrate alkane is a propane, the cell according to any aspect of the present invention used to produce threonine, may be further genetically modified to increase expression relative to the wild type cell of at least one further enzyme ($E_{7b}$). More in particular, the enzyme $E_{7b}$ may be selected from the group consisting of acyl-ACP synthetase ($E_g$) (EC 6.2.1.20), acyl-CoA synthetase ($E_f$) (EC 6.2.1.2, EC 6.2.1.3, EC 6.2.1.10), methylisocitrate hydro-lyase ($E_{7bi}$) (EC 4.2.1.99), methylisocitrate lyase ($E_{7bii}$) (EC 4.1.3.30), 2-Methylisocitrate dehydratase ($E_{7biii}$) (EC 4.2.1.79), 2-Methylcitrate synthase ($E_{7biv}$) (EC 2.3.3.5), combination of phosphotranspropionylase ($E_{iii}$) (EC 2.3.1.19, EC 2.3.1.8) and propionate kinase ($E_{hii}$) (EC 2.7.2.15) and propionyl-CoA ligase ($E_{7bvii}$) (EC 6.2.1.17). Even more in particular, the enzyme $E_{7b}$ may be an acyl-ACP synthetase ($E_g$) comprising SEQ ID NO:21 or a variant thereof, or an acyl-CoA synthetase (Ef) comprising SEQ ID NO:22 or a variant thereof, or a methylisocitrate hydro-lyase ($E_{7bi}$) comprising SEQ ID NO:27, 94 or a variant thereof, a methylisocitrate lyase ($E_{7bii}$) comprising SEQ ID NO:28, 95, 96 or a variant thereof, a 2-Methylisocitrate dehydratase ($E_{7biii}$) comprising SEQ ID NO:29, 97, 98 or a variant thereof, a 2-Methylcitrate synthase ($E_{7biv}$) comprising SEQ ID NO:30, 99, 100 or a variant thereof or the combination of phosphotranspropionylase ($E_{iii}$) comprising SEQ ID NO:31, 101 or a variant thereof and propionate kinase ($E_{hii}$) comprising SEQ ID NO:26, 4 or a variant thereof, a propionyl-CoA ligase ($E_{7bvii}$) (EC 6.2.1.17) comprising SEQ ID NO:32 or a variant thereof or propionyl-CoA:acetate Coenzyme A transferase ($E_{7bviii}$) comprising SEQ ID NO:17 a variant thereof.

In particular, according to any aspect of the present invention, the cell may be genetically modified to increase the expression of all the enzymes $E_1$- $E_6$, wherein, $E_1$- $E_6$ are:

- $E_1$ is a butane monoxygenase ($E_c$) (EC 1.14.13.230), preferably comprising the sequences with accession numbers AAM 19732.1, AAM19730.1, AAM19728.1, AAM19727.1, AAM 19729.1, and ABU68845.2 or variants thereof;
- $E_2$ is an alcohol dehydrogenase ($E_d$) (EC 1.1.1.1 or EC 1.1.1.2), preferably comprising SEQ ID NO:91 or a variant thereof;
- $E_3$ is an aldehyde dehydrogenase ($E_e$) (EC 1.2.1.3, EC 1.2.1.4 or EC 1.2.1.5), preferably comprising SEQ ID NO:42 or a variant thereof;
- $E_4$ is fatty acyl CoA synthase (FACS) ($E_f$) (EC 6.2.1.1, EC 6.2.1.2 or EC 6.2.1.3), preferably comprising SEQ ID NO:88 or variant thereof; and
- $E_6$ is selected from the group consisting of:

  (i) feedback-resistant variant of homoserine dehydrogenase ($E_{6k}$) comprising SEQ ID NO:14 with at least one point mutation selected from the group consisting of G378E, D375A, V379E, L380E, I392P, S393A, L394P and Q399T, or SEQ ID NO:51 with point mutation S345P;
  (ii) feedback-resistant variant of aspartate kinase ($E_{6a}$) comprising SEQ ID NO:1 with a point mutation of T342I, or SEQ ID NO:79 with at least one point mutation selected from the group consisting of T311I, A279T, S301Y, A279V, S301F, T308I, S317A, R320G, G345D, S381F, Q404E, G408R, G277A, Q298A, T361A, E363A, and F364A, particularly with point mutation T311I;
  (iii) homoserine kinase comprising SEQ ID NO:15, 81 or a variant thereof;
  (iv) threonine synthase comprising SEQ ID NO:18, 83 or variant thereof; and
  (v) threonine exporter ($E_{6n}$) comprising SEQ ID NO:19.

**Enzyme $E_1$**

Enzyme $E_1$ may be capable of converting at least one alkane to the corresponding 1-alkanol. In particular, $E_1$ may be at least one P450 alkane hydroxylase/ monooxygenase ($E_a$) of EC 1.14.15.1, AlkB alkane hydroxylase ($E_b$) of EC 1.14.15.3, methane monooxygenase ($E_{ai}$) of EC 1.14.13.25 or EC 1.14.18.3, propane monooxygenase ($E_{aii}$) of EC 1.14.13.227, and/or butane monooxygenase ($E_{aiii}$) of EC 1.14.13.230.

The P450 alkane hydroxylase ($E_a$) is a component of a reaction system comprising

- two enzyme components cytochrome P450 alkane hydroxylase and NAD(P)H cytochrome P450 oxidoreductase of EC 1.6.2.4 or
- three enzyme components cytochrome P450 alkane hydroxylase of the CYP153 type, ferredoxin NAD(P)+ reductases of EC 1.18.1.2 or EC 1.18.1.3 and ferredoxin.

The AlkB alkane hydroxylase ($E_{1b}$) is a component of a reaction system comprising

- AlkB alkane hydroxylases of EC 1.14.15.3 which is a component of a reaction system comprising three enzyme components AlkB alkane hydroxylase of EC 1.14.15.3, AlkT rubredoxin NAD(P)+ reductase of EC 1.18.1.1 or of EC 1.18.1.4 and rubredoxin AlkG.

The P450 alkane hydroxylase ($E_a$) may be a methane monooxygenase ($E_{ai}$) (EC 1.14.13.25 or EC 1.14.18.3), propane monooxygenase ($E_b$) (EC 1.14.13.227) or butane monooxygenase ($E_c$) (EC 1.14.13.230).

In particular, $E_1$ may be an AlkB alkane hydroxylase ($E_b$) also known as an alkane monooxygenase. More in particular, $E_1$ may comprise sequence identity of at least 50% to the alkane monooxygenase from *Pseudomonas putida* GPo1 encoded by alkBGT. Even more in particular, $E_1$ may comprise sequence identity of at least 50% to the polypeptide YP_001185946.1. More in particular, $E_1$ may comprise a polypeptide with sequence identity of at least 50, 60, 65, 70, 75, 80, 85, 90, 91, 94, 95, 98 or 100% to a polypeptide YP_001185946.1.

In another example, $E_1$ may be a butane monooxygenase ($E_{aiii}$) of EC 1.14.13.230 that comprises a gene cluster comprising butane monooxygenase hydroxylase BMOH alpha subunit (bmoX), butane monooxygenase beta subunit (bmoY), butane monooxygenase gamma subunit (bmoZ), butane monooxygenase regulatory protein (bmoB), butane monooxygenase reductase (bmoC_1), bmoG (similar to groEL from *E. coli*) and three putative ORF. In particular, the butane monooxygenase ($E_{aiii}$) may be from *Thauera butanivorans*. More in particular, the butane monooxygenase operon may comprise SEQ ID NO:35.

## Enzyme $E_2$

Enzyme $E_2$ may be capable of converting a 1-alkanol to the corresponding 1-alkanal. In particular, $E_2$ may be at least one P450 alkane hydroxylases ($E_a$) of EC 1.14.15.3, AlkB alkane hydroxylases ($E_b$) of EC 1.14.15.3, alcohol oxidase ($E_c$) of EC 1.1.3.20 or alcohol dehydrogenase ($E_d$) of EC 1.1.1.1 or EC 1.1.1.2. More in particular, $E_2$ may be selected from the group consisting of P450 alkane hydroxylase ($E_a$), AlkB alkane hydroxylase ($E_b$), alcohol oxidase ($E_c$) of EC 1.1.3.20, AlkJ alcohol dehydrogenase ($E_{di}$), and alcohol dehydrogenase ($E_{dii}$) of EC 1.1.1.1 or EC 1.1.1.2.

In particular, $E_2$ may be an AlkB alkane hydroxylase ($E_b$) also known as an alkane monooxygenase. More in particular, $E_2$ may comprise sequence identity of at least 50% to the alkane monooxygenase from *Pseudomonas putida* GPo1 encoded by alkBGT. Even more in particular, $E_2$ may comprise sequence identity of at least 50% to the polypeptide YP_001185946.1. More in particular, $E_2$ may comprise a polypeptide with sequence identity of at least 50, 60, 65, 70, 75, 80, 85, 90, 91, 94, 95, 98 or 100% to a polypeptide YP_001185946.1.

In one example, $E_2$ may be an alcohol oxidase ($E_c$) that may be selected from the group consisting of AAS46878.1, ACX81419.1, AAS46879.1, CAB75353.1, AAS46880.1, XP_712350.1, XP_002422236.1, XP_712386.1, EEQ43775.1, CAB75351.1, CAB75352.1, XP_002548766.1, and XP_002548765.1.

In a further example, $E_2$ may be an AlkJ alcohol dehydrogenase ($E_{di}$) and may be selected from the group consisting of Q00593.1, Q9WWW2.1, ZP_00957061.1, YP_957894.1, CAC38030.1, YP_694430.1, YP_957725.1, and YP_001672216.1.

In another example, $E_2$ may be an alcohol dehydrogenase ($E_{dii}$) and may be selected from the group consisting of *AdhE, AdhP, YjgB, YqhD, GldA, EutG, YiaY, AdhE, AdhP, YhhX, YahK, HdhA, HisD, SerA, Tdh, Ugd, Udg, Gmd, YefA, YbiC, YdfG, YeaU, TtuC, YeiQ, YgbJ, YgcU, YgcT, YgcV, YggP, YgjR, YliI, YqiB, YzzH, LdhA, GapA, Epd, Dld, GatD, Gcd, GlpA, GlpB, GlpC, GlpD, GpsA and YphC* from bacteria, in particular *E. coli.*

## Enzyme $E_3$

Enzyme $E_3$ may be capable of converting at least one 1-alkanal to the corresponding alkanoic acid. In particular, $E_3$ may be capable of converting formaldehyde, acetaldehyde, propanal and/or butanal to the corresponding fatty acid. In particular, $E_3$ may be selected from the group consisting of P450 alkane hydroxylases ($E_a$) of EC 1.14.15.3-, AlkB alkane hydroxylases ($E_b$) of EC 1.14.15.3, bifunctional alcohol oxidases ($E_c$) of EC 1.1.3.20, bifunctional AlkJ alcohol dehydrogenases ($E_{di}$) or bifunctional alcohol dehydrogenases ($E_d$) of EC 1.1.1.1 or EC 1.1.1.2, capable of oxidizing an 1-alkanol via an 1-alkanal directly to the corresponding alkanoic acid, and aldehyde dehydrogenases ($E_e$).

Enzyme $E_3$ may be an aldehyde dehydrogenase ($E_e$) (EC 1.2.1.3, EC 1.2.1.4 or EC 1.2.1.5), that may be capable of catalyzing the conversion of ω-oxoalkanoic acid (ester) = ω-carboxyalkanoic acid (ester).

In one example, $E_e$ may be capable of specifically catalysing the following reaction:

$$\omega\text{-oxoalkanoic acid (ester)} + NAD(P)^+ = \omega\text{-carboxyalkanoic acid (ester)} + NAD(P)H + H^+$$

In this case, enzyme $E_e$ may be an aldehyde dehydrogenase of EC 1.2.1.3, EC 1.2.1.4 or EC 1.2.1.5, and may be selected from the group consisting of Prr, Usg, MhpF, AstD, GdhA, FrmA, Feab, Asd, Sad, PuuE, GabT, YgaW, BetB, PutA, PuuC, FeaB, AldA, Prr, EutA, GabD, AldB, TynA and Ynel from bacteria, in particular *E. coli.*

In another example, enzyme $E_3$ may be capable of catalysing the following reaction:

$$\omega\text{-oxoalkanoic acid (ester)} + O_2 = \omega\text{-carboxyalkanoic acid (ester)} + H_2O_2$$

In this case, $E_3$ may be a fatty alcohol oxidases ($E_c$) of EC 1.1.3.20.

**Enzyme $E_4$**

The Enzyme $E_4$ may be capable of converting at least one alkanoic acid to the corresponding fatty acyl thioester. In particular, short-chain fatty acids, such as acetic, propanoic and/ or butyric acid may be converted to the corresponding fatty acyl thioester, such as fatty acyl-Coenzyme A, fatty acyl-ACP, fatty acyl-S-4-phosphopantotheine with the 4-phosphopantotheine group residing in a polypeptide chain and the like.

In particular, $E_4$ may be selected from the group consisting of fatty acyl coenzyme A (CoA) synthase ($E_f$) of EC 6.2.1.1, EC 6.2.1.2 or EC 6.2.1.3; Acyl-Acyl Carrier Protein (ACP) synthase ($E_g$) of EC 6.2.1.20 or EC 6.2.1.47; Fatty acyl kinase ($E_h$) of EC 2.7.2.1, EC 2.7.2.12, EC 2.7.2.15 or EC 2.7.2.7 and phosphotransacylase ($E_i$) of EC 2.3.1.8 or EC 2.3.1.19; and fatty acyl coenzyme A synthase ($E_f$) of EC 6.2.1.1, EC 6.2.1.2 or EC 6.2.1.3 and fatty acyl-CoA:ACP transacylase ($E_j$) of EC 2.3.1.38 or EC 2.3.1.39.

In particular, $E_4$ may be

(a) fatty acyl CoA synthase (FACS) ($E_f$) of EC 6.2.1.1, EC 6.2.1.2 or EC 6.2.1.3;

(b) acyl-acyl-ACP synthase ($E_g$) of EC 6.2.1.20 or EC 6.2.1.47;

(c) combination of fatty acyl kinase ($E_h$) of EC 2.7.2.1, EC 2.7.2.12, EC 2.7.2.15 or EC 2.7.2.7 and phosphotransacylase ($E_i$) of EC 2.3.1.8 or EC 2.3.1.19; or

(d) combination of fatty acyl CoA synthase ($E_f$) of EC 6.2.1.1, EC 6.2.1.2 or EC 6.2.1.3 and fatty acyl-CoA:ACP transacylase ($E_j$) of EC EC 2.3.1.38 or EC 2.3.1.39

The Enzyme $E_f$ may be capable of catalysing the conversion of a fatty acid to acyl-CoA. A skilled person would appreciate that some fatty acyl-CoA synthase peptides will catalyse other reactions as well, for example some acyl-CoA synthase peptides will accept other substrates in addition to fatty acids. The Enzyme $E_j$, (acyl-CoA (coenzyme A):ACP (acyl carrier protein) transacylases may be capable of catalysing the process of conversion of dodecanoyl-CoA thioester to dodecanoyl-ACP thioester.

More in particular, $E_4$ may be fatty acyl CoA synthase (FACS) ($E_f$) with SEQ ID NO:88 or variant thereof. In another example, $E_4$ may be a combination of fatty acyl kinase ($E_h$) with SEQ ID NO:89, 90 or a variant thereof and phosphotransacylase ($E_i$) comprising SEQ ID NO:24 or a variant thereof.

**Enzyme $E_5$**

Enzyme $E_5$ may be capable of converting a short-chain aldehyde to a corresponding fatty acyl thioester. In particular, $E_5$ may convert aldehydes such as acetaldehyde, propanal or butanal to a corresponding fatty acyl thioester, such as fatty acyl-Coenzyme A, fatty acyl-ACP or fatty acyl-S-4-phosphopantotheine with the 4-phosphopantotheine group residing in a polypeptide chain and the like. Even more in particular, the Enzyme $E_5$ may be an aldehyde dehydrogenase ($E_e$) (EC 1.2.1.3, EC 1.2.1.4 or EC 1.2.1.5) or an alcohol oxidase ($E_c$) (EC 1.1.3.20).

The enzymes $E_1$ to $E_8$ may comprise a polypeptide sequence wherein up to 60%, preferably up to 25%, particularly up to 15%, in particular up to 10, 9, 8, 7, 6, 5, 4, 3, 2 or 1% of the amino acid residues are modified compared to the reference sequences known in the art. A skilled person may easily obtain the sequences of the relevant enzymes, $E_1$ to $E_8$ from Genebank (https://www.ncbi.nlm.nih.gov/genbank/) and using the methods known in the art obtain the cell according to any aspect of the present invention. For example, sequences labelled by accession numbers on genebank may be modified by deletion, insertion, substitution or a combination thereof and which still possess at least 50%, preferably 65%, particularly preferably 80%, in particular more than 90% of the activity of the protein with the corresponding, reference sequence, wherein 100% activity of the reference protein is understood to mean the increasing of the activity of the cells used as a biocatalyst, i.e. the quantity of substance converted per unit time based on the cell quantity used (units per gram cell dry weight [U/g CDW]) in comparison to the activity of the biocatalyst in the absence of the reference protein.

Modifications of amino acid residues of a given polypeptide sequence which lead to no significant modifications of the properties and function of the given polypeptide are known to those skilled in the art. Thus for example many amino acids can often be exchanged for one another without problems; examples of such suitable amino acid substitutions are: Ala by Ser; Arg by Lys; Asn by Gln or His; Asp by Glu; Cys by Ser; Gln by Asn; Glu by Asp; Gly by Pro; His by Asn or Gln; Ile by Leu or Val; Leu by Met or Val; Lys by Arg or Gln or Glu; Met by Leu or Ile; Phe by Met or Leu or Tyr; Ser by Thr; Thr by Ser; Trp by Tyr; Tyr by Trp or Phe; Val by Ile or Leu. It is also known that modifications, particularly at the N- or C-terminus of a polypeptide in the form of for example amino acid insertions or deletions, often exert no significant influence on the function of the polypeptide.

The accession numbers stated in connection with the present invention mentioned throughout this specification correspond to the NCBI ProteinBank database entries with the date 27.06.2018; as a rule, the version number of the entry is identified here by "numerals" such as for example ".1".

All stated percentages (%) are, unless otherwise stated, mass percent.

According to any aspect of the present invention, the microbial cell may be selected from the species of bacteria, preferably selected from the group consisting of, *Abiotrophia, Acaryochloris, Accumulibacter, Acetivibrio, Acetobacter, Acetohalobium, Acetonema, Achromobacter, Acidaminococcus, Acidimicrobium, Acidiphilium, Acidithiobacillus, Acidobacterium, Acidothermus, Acidovorax, Acinetobacter, Actinobacillus, Actinomyces, Actinosynnema, Aerococcus, Aeromicrobium, Aeromonas, Afipia, Aggregatibacter, Agrobacterium, Ahrensia, Akkermansia, Alcanivorax, Alicycliphilus, Alicyclobacillus, Aliivibrio, Alkalilimnicola, Alkaliphilus, Allochromatium, Alteromonadales, Alteromonas, Aminobacterium, Aminomonas, Ammonifex, Amycolatopsis, Amycolicicoccus, Anabaena, Anaerobaculum, Anaerococcus, Anaerofustis, Anaerolinea, Anaeromyxobacter, Anaerostipes, Anaerotruncus, Anaplasma, Anoxybacillus, Aquifex, Arcanobacterium, Arcobacter, Aromatoleum, Arthrobacter, Arthrospira, Asticcacaulis, Atopobium, Aurantimonas, Azoarcus, Azorhizobium, Azospirillum, Azotobacter, Bacillus, Bartonella, Basfia, Baumannia, Bdellovibrio, Beggiatoa, Beijerinckia, Bermanella, Beutenbergia, Bifidobacterium, Bilophila, Blastopirellula, Blautia, Blochmannia, Bordetella, Borrelia, Brachybacterium, Brachyspira, Bradyrhizobium, Brevibacillus, Brevibacterium, Brevundimonas, Brucella, Buchnera, Bulleidia, Burkholderia, Butyrivibrio, Caldalkalibacillus, Caldanaerobacter, Caldicellulosiruptor, Calditerrivibrio, Caminibacter, Campylobacter, Carboxydibrachium, Carboxydothermus, Cardiobacterium, Carnobacterium, Carsonella, Catenibacterium, Catenulispora, Catonella, Caulobacter, Cellulomonas, Cellvibrio, Centipeda, Chelativorans, Chloroflexus, Chromobacterium, Chromohalobacter, Chthoniobacter, Citreicella, Citrobacter, Citromicrobium, Clavibacter, Cloacamonas, Clostridium, Collinsella, Colwellia, Comamonas, Conexibacter, Congregibacter, Coprobacillus, Coprococcus, Coprothermobacter, Coraliomargarita, Coriobacterium, corrodens, Corynebacterium, Coxiella, Crocosphaera, Cronobacter, Cryptobacterium, Cupriavidus, Cyanobium, Cyanothece, Cylindrospermopsis, Dechloromonas, Deferribacter, Dehalococcoides, Dehalogenimonas, Deinococcus, Delftia, Denitrovibrio, Dermacoccus, Desmospora, Desulfarculus, Desulphateibacillum, Desulfitobacterium, Desulfobacca, Desulfobacterium, Desulfobulbus, Desulfococcus, Desulfohalobium, Desulfomicrobium, Desulfonatronospira, Desulforudis, Desulfotalea, Desulfotomaculum, Desulfovibrio, Desulfurispirillum, Desulfurobacterium, Desulfuromonas, Dethiobacter, Dethiosulfovibrio, Dialister, Dichelobacter, Dickeya, Dictyoglomus, Dietzia, Dinoroseobacter, Dorea, Edwardsiella, Ehrlichia, Eikenella, Elusimicrobium, Endoriftia, Enhydrobacter, Enterobacter, Enterococcus, Epulopiscium, Erwinia, Erysipelothrix, Erythrobacter, Escherichia, Ethanoligenens, Eubacterium, Eubacterium, Exiguobacterium, Faecalibacterium, Ferrimonas, Fervidobacterium, Fibrobacter, Finegoldia, Flexistipes, Francisella, Frankia, Fructobacillus, Fulvimarina, Fusobacterium, Gallibacterium, Gallionella, Gardnerella, Gemella, Gemmata, Gemmatimonas, Geobacillus, Geobacter, Geodermatophilus, Glaciecola, Gloeobacter, Glossina, Gluconacetobacter, Gordonia, Granulibacter, Granulicatella, Grimontia, Haemophilus, Hahella, Halanaerobiumns, Haliangium, Halomonas, Halorhodospira, Halothermothrix, Halothiobacillus, Hamiltonella, Helicobacter, Heliobacterium, Herbaspirillum, Herminiimonas, Herpetosiphon, Hippea, Hirschia, Histophilus, Hodgkinia, Hoeflea, Holdemania, Hydrogenivirga, Hydrogenobaculum, Hylemonella, Hyphomicrobium, Hyphomonas, Idiomarina, Ilyobacter, Intrasporangium, Isoptericola, Isosphaera, Janibacter, Janthinobacterium, Jonesia, Jonquetella, Kangiella, Ketogulonicigenium, Kineococcus, Kingella, Klebsiella, Kocuria, Koribacter, Kosmotoga, Kribbella, Ktedonobacter, Kytococcus, Labrenzia, Lactobacillus, Lactococcus, Laribacter, Lautropia, Lawsonia, Legionella, Leifsonia, Lentisphaera, Leptolyngbya, Leptospira, Leptothrix, Leptotrichia, Leuconostoc, Liberibacter, Limnobacter, Listeria, Loktanella, Lutiella, Lyngbya, Lysinibacillus, Macrococcus, Magnetococcus, Magnetospirillum, Mahella, Mannheimia, Maricaulis, Marinithermus, Marinobacter, Marinomonas, Mariprofundus, Maritimibacter, Marvinbryantia, Megasphaera, Meiothermus, Melissococcus, Mesorhizobium, Methylacidiphilum, Methylibium, Methylobacillus, Methylobacter, Methylobacterium, Methylococcus, Methylocystis, Methylomicrobium, Methylophaga, Methylophilales, Methylosinus, Methyloversatilis, Methylovorus, Microbacterium, Micrococcus, Microcoleus, Microcystis, Microlunatus, Micromonospora, Mitsuokella, Mobiluncus, Moorella, Moraxella, Moritella, Mycobacterium, Myxococcus, Nakamurella, Natranaerobius, Neisseria, Neorickettsia, Neptuniibacter, Nitratifractor, Nitratiruptor, Nitrobacter, Nitrococcus, Nitrosomonas, Nitrosospira, Nitrospira, Nocardia, Nocardioides, Nocardiopsis, Nodularia, Nostoc, Novosphingobium, Oceanibulbus, Oceanicaulis, Oceanicola, Oceanithermus, Oceanobacillus, Ochrobactrum, Octadecabacter, Odyssella, Oligotropha, Olsenella, Opitutus, Oribacterium, Orientia, Ornithinibacillus, Oscillatoria, Oscillochloris, Oxalobacter, Paenibacillus, Pantoea, Paracoccus, Parascardovia, Parasutterella, Parvibaculum, Parvimonas, Parvularcula, Pasteurella, Pasteuria, Pectobacterium, Pediococcus, Pedosphaera, Pelagibaca, Pelagibacter, Pelobacter, Pelotomaculum, Peptoniphilus, Peptostreptococcus, Persephonella, Petrotoga, Phaeobacter, Phascolarctobacterium, Phenylobacterium, Photobacterium, Pirellula, Planctomyces, Planococcus, Plesiocystis, Polaromonas, Polaromonas, Polymorphum, Polynucleobacter, Poribacteria, Prochlorococcus, Propionibacterium, Proteus, Providencia, Pseudoalteromonas, Pseudoflavonifractor, Pseudomonas, Pseudonocardia, Pseudoramibacter, Pseudovibrio, Pseudoxanthomonas, Psychrobacter, Psychromonas, Puniceispirillum, Pusillimonas, Pyramidobacter, Rahnella, Ralstonia, Raphidiopsis, Regiella, Reinekea, Renibacterium, Rhizobium, Rhodobacter, Rhodococcus, Rhodoferax, Rhodomicrobium, Rhodopirellula, Rhodopseudomonas, Rhodospirillum, Rickettsia, Rickettsiella, Riesia, Roseburia, Roseibium, Ro-*

*seiflexus, Roseobacter, Roseomonas, Roseovarius, Rothia, Rubrivivax, Rubrobacter, Ruegeria, Ruminococcus, Ruthia, Saccharomonospora, Saccharophagus, Saccharopolyspora, Sagittula, Salinispora, Salmonella, Sanguibacte, Scardovia, Sebaldella, Segniliparus, Selenomonas, Serratia, Shewanella, Shigella, Shuttleworthia, Sideroxydans, Silicibacter, Simonsiella, Sinorhizobium, Slackia, Sodalis, Solibacter, Solobacterium, Sorangium, Sphaerobacter, Sphingobium, Sphingomonas, Sphingopyxis, Spirochaeta, Sporosarcina, Stackebrandtia, Staphylococcus, Starkeya, Stenotrophomonas, Stigmatella, Streptobacillus, Streptococcus, Streptomyces, Streptosporangium, Subdoligranulum, subvibrioides, Succinatimonas, Sulfitobacter, Sulfobacillus, Sulfuricurvum, Sulfurihydrogenibium, Sulfurimonas, Sulfurospirillum, Sulfurovum, Sutterella, Symbiobacterium, Synechocystis, Syntrophobacter, Syntrophobotulus, Syntrophomonas, Syntrophothermus, Syntrophus, taiwanensis, Taylorella, Teredinibacter, Terriglobus, Thalassiobium, Thauera, Thermaerobacter, Thermanaerovibrio, Thermincola, Thermoanaerobacter, Thermoanaerobacterium, Thermobaculum, Thermobifida, Thermobispora, Thermocrinis, Thermodesulphateator, Thermodesulfobacterium, Thermodesulfobium, Thermodesulfovibrio, Thermomicrobium, Thermomonospora, Thermosediminibacter, Thermosinus, Thermosipho, Thermosynechococcus, Thermotoga, Thermovibrio, Thermus, Thioalkalimicrobium, Thioalkalivibrio, Thiobacillus, Thiomicrospira, Thiomonas, Tolumonas, Treponema, tribocorum, Trichodesmium, Tropheryma, Truepera, Tsukamurella, Turicibacter, Variovorax, Veillonella, Verminephrobacter, Verrucomicrobium, Verrucosispora, Vesicomyosocius, Vibrio, Vibrionales, Victivallis, Weissella, Wigglesworthia, Wolbachia, Wolinella, Xanthobacter, Xanthomonas, Xenorhabdus, Xylanimonas, Xylella, Yersinia, Zinderia and Zymomonas,*

In particular, the microbial cell may be from *E. coli, Pseudomonas* sp., *Pseudomonas fluorescens, Pseudomonas putida, Pseudomonas stutzeri, Acinetobacter* sp., *Burkholderia* sp., *Burkholderia thailandensis,* Cyanobakterien, *Klebsiella* sp., *Klebsiella oxytoca, Salmonella sp., Rhizobium sp.* and *Rhizobium meliloti, Bacillus* sp., *Bacillus subtilis, Clostridium* sp., *Corynebacterium* sp., *Corynebacterium glutamicum, Brevibacterium* sp., *Chlorella* sp. and *Nostoc* sp.. More in particular, the microbial cell may be from *E. coli.*

## EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Example 1

*Formation of o-Acetyl-L-homoserine from ethane with Escherichia coli.*

For the biotransformation of ethane to o-Acetyl-L-homoserine the genetically modified strain *Escherichia coli* CGSC 12149 lysCfbr_Ec thrAfbr_Ec pACYC184 {PalkS} [alkS_PpGPo1] {PalkB} [bmoXYBZ_Tb PROKKA_02001_Tb PROKKA_02000_Tb bmoC_1_Tb PROKKA_01998_Tb bmoG_Tb] pBR322 {PalkS} [alkS_PpGPo1] {PalkB} [adhA_Cg aldH_Cg] {Placuv5}[metX_Cg]{Ptac}[thrA_fbr_Ec] was used. This strain harbours the following characteristics:

i. Modification of the *E. coli* CGSC 12149 lysC gene (SEQ ID NO:33), encoding a feedback resistant variant of aspartokinase 3.

ii. Modification of the *E. coli* CGSC 12149 thrA gene (SEQ ID NO:34), encoding a feedback resistant variant of bifunctional aspartokinase 1 / homoserine dehydrogenase 1 (using a natural promotor).

iii. Expression of *Thauera butanivorans* DSM 2080 butane monooxygenase operon (SEQ ID NO:35), comprising of bmoX_Tb (butane monooxygenase hydroxylase BMOH alpha subunit), bmoY_Tb (butane monooxygenase beta subunit), bmoZ_Tb (butane monooxygenase gamma subunit), bmoB_Tb (butane monooxygenase regulatory protein), bmoC_1_Tb (butane monooxygenase reductase), bmoG_Tb (similar to groEL from *E. coli*) and three putative ORF PROKKA_02001_Tb, PROKKA_02000_Tb and PROKKA_01998_Tb.

iv. Expression of *Corynebacterium glutamicum* ATCC 13032 adhA_Cg (SEQ ID NO:36), encoding Zn-dependent alcohol dehydrogenases and aldH_Cg (SEQ ID NO:37), encoding NAD-dependent aldehyde dehydrogenases Cgl2796 genes.

v. Expression of *Corynebacterium glutamicum* ATCC 13032 metX gene (SEQ ID NO:38), encoding homoserine O-acetyl transferase.

vi. Modification and expression of the *E. coli* CGSC 12149 thrA gene (SEQ ID NO:34), encoding a feedback resistant variant of bifunctional aspartokinase 1 / homoserine dehydrogenase 1 (using an overexpression system).

These characteristics were brought about by:

i. Replacement of *E. coli* CGSC 12149 thrA gene by another allele of thrA, encoding a feedback resistant variant of bifunctional aspartokinase 1 / homoserine dehydrogenase 1 (point mutation at bp 1034 from C to T (SEQ ID NO:34), Ser345Phe SEQ ID NO:51), with pKO3 derivative 4-49 (SEQ ID NO:39).

ii. Replacement of *E. coli* CGSC 12149 lysC gene by another allele of lysC, encoding a feedback resistant variant aspartokinase 3 (point mutation at bp 1055 from C to T (SEQ ID NO:33), T342I (SEQ ID NO:1), with pKO3 derivative 4-47 (SEQ ID NO:40).

iii. Introduction of plasmid pACYC184 {PalkS} [alkS_PpGPo1] {PalkB} [bmoXYBZ_Tb PROKKA_02001_Tb PROKKA_02000_Tb bmoC_1_Tb PROKKA_01998_Tb bmoG_Tb] (SEQ ID NO:41)

iv. Introduction of plasmid pBR322 {PalkS} [alkS_PpGPo1] {PalkB} [adhA_Cg aldH_Cg] [blaA_Ec] {Placuv5} [metX_Cg]{Ptac}[thrA_fbr_Ec] (SEQ ID NO:73)

• Construction of pKO3 modification vectors

For construction of pKO3 derivatives for gene deletion and/or allelic replacement homologous sequences up- and downstream of the target genes were amplified by PCR from genomic DNA of *Escherichia coli* W3110 using the following primers. Homologous ends for assembly cloning were introduced within the primers.

| | |
|---|---|
| lysCfbr homologue sequence 1 | SEQ ID NOs: 43, 44 |
| lysCfbr homologue sequence 2 | SEQ ID NOs: 45, 46 |
| thrAfbr homologue sequence 1 | SEQ ID NOs: 47, 48 |
| thrAfbr homologue sequence 2 | SEQ ID NOs: 49, 50 |

The PCR was performed with Phusion® High-Fidelity Master Mix according to the manufacturer (New England Biolabs, Ipswitch, MA, USA). The thermal cycle profile was 3 min at 98 °C for initial denaturation, 35 cycles: 10 sec at 98 °C, 30 sec at 60 °C to 68 °C (gradient), 20 sec at 72 °C and a final 10 min hold step at 72 °C. Purification of PCR products was performed by gel extraction or PCR purification according to the manufacturer of purification kits (QiaQuick PCR Purification Kit and QiaQuick Gel Extraction Kit, Qiagen, Hilden, Germany). Purified PCR products were assembled into NotI restricted pKO3 plasmid using NEBuilder® HiFi DNA Assembly Master Mix according to the manufacturers manual (New England Biolabs, Ipswitch, MA, USA). Transformation of *E. coli* DH10β was performed according to the manufacturer (New England Biolabs, Ipswitch, MA, USA). The final plasmids were verified by restriction analysis and DNA sequencing.

• Construction of pJAG-4-48

For construction of pCDF derivative for gene expression of thrA, encoding a feedback resistant variant of bifunctional aspartokinase 1 / homoserine dehydrogenase 1 (point mutation at bp 1034 from C to T (SEQ ID NO:34), Ser345Phe, (SEQ ID NO:51) from *Escherichia coli* W3110 and metX_Cg, encoding Homoserine-O-Acetyltransferase from *Corynebacterium glutamicum* ATCC 13032 (SEQ ID NO:16) target genes were amplified by PCR from genomic DNA of *Escherichia coli* W3110 or *Corynebacterium glutamicum* ATCC 13032 (i.e. SEQ ID NO:34 or 52) respectively using the following primers. Homologous ends for assembly cloning were introduced within the primers. The point mutation of *thrA* that leads to a feedback resistant variant was implemented within the forward primer. The gene *thrA* was cloned downstream of a *tac* promotor (SEQ ID NO:53) which was amplified by PCR from another vector. Following primers were used for amplification:

| | |
|---|---|
| metX_Cg | SEQ ID NOs: 54, 55 |
| *tac* promotor | SEQ ID NOs: 56, 57 |
| thrA part 1 | SEQ ID NOs: 58, 59 |
| thrA part 2 | SEQ ID NOs: 60, 61 |

The PCR was performed with Phusion® High-Fidelity Master Mix according to the manufacturer (New England Biolabs, Ipswitch, MA, USA). The thermal cycle profile was 3 min at 98 °C for initial denaturation, 35 cycles: 10 sec at 98 °C, 30 sec at 60 °C to 70 °C (gradient), 45 sec at 72 °C and a final 10 min hold step at 72 °C. Purification of PCR products was performed by gel extraction or PCR purification according to the manufacturer of purification kits (QiaQuick PCR Purification Kit and QiaQuick Gel Extraction Kit, Qiagen, Hilden, Germany).

Purified PCR products were assembled into *NdeI* and *XbaI* restricted pJ281_alaT_C.gl._TA_C.v.(Ct) (SEQ ID NO:62) plasmid using NEBuilder® HiFi DNA Assembly Master Mix according to the manufacturers manual (New England Biolabs, Ipswitch, MA, USA). Transformation of *E. coli* DH10β was performed according to the manufacturer (New England Biolabs, Ipswitch, MA, USA). The final plasmid was verified by restriction analysis and DNA sequencing (SEQ ID NO:63).

• Construction of HM-p-25

For expression of *Thauera butanivorans* DSM 2080 butane monooxygenase operon (SEQ ID NO:35), comprising of bmoX_Tb (butane monooxygenase hydroxylase BMOH alpha subunit), bmoY_Tb (butane monooxygenase beta subunit), bmoZ_Tb (butane monooxygenase gamma subunit), bmoB_Tb (butane monooxygenase regulatory protein), bmoC_1_Tb (butane monooxygenase reductase), bmoG_Tb (similar to groEL from *E. coli*) and three putative ORF PROKKA_02001_Tb, PROKKA_02000_Tb and PROKKA_01998_Tb the whole sequence was amplified from chromosomal DNA of *Thauera butanivorans* DSM 2018 and subcloned into a basal vector. From this vector, the whole operon was subcloned into a vector comprising a) pACYC184 backbone b) DCPK induction system (SEQ ID NO. 64) and c) full *bmo* operon sequence under DCPK control (SEQ ID NO:35). The final plasmid was verified by restriction analysis and DNA sequencing (SEQ ID NO:41) with sequence part b) spanning 12129bp - 36 bp, sequence part c) spanning 37 - 7885 bp and sequence part a) spanning the remaining vector sequence.

• Construction of AH-p-125

For construction of pBR322 derivative for gene expression of *Corynebacterium glutamicum* ATCC 13032 adhA_Cg (SEQ ID NO:36), encoding Zn-dependent alcohol dehydrogenases and aldH_Cg (SEQ ID NO:37), encoding NAD-dependent aldehyde dehydrogenases Cgl2796 target genes were amplified by PCR from genomic DNA of *C.glutamicum* ATCC 13032 using the following primers. Homologous ends for assembly cloning were introduced within the primers SEQ ID NOs: 65-68.

The PCR was performed with Phusion® High-Fidelity Master Mix according to the manufacturer (New England Biolabs, Ipswitch, MA, USA), 2 μl of 25 mM MgCl2 was added to each 25 μl reaction. The thermal cycle profile was 3 min at 98 °C for initial denaturation, 40 cycles: 10 sec at 98 °C, 30 sec at 65 °C +/- 1,5 °C (gradient), 55 sec at 72 °C and a final 5 min hold step at 72 °C. Purification of PCR products was performed by gel extraction or PCR purification according to the manufacturer of purification kits (QiaQuick PCR Purification Kit and QiaQuick Gel Extraction Kit, Qiagen, Hilden, Germany).

Purified PCR products were assembled into AgeI restricted AH-p-123 plasmid bringing DCPK induction system (SEQ ID NO:64) using NEBuilder® HiFi DNA Assembly Master Mix according to the manufacturers manual (New England Biolabs, Ipswitch, MA, USA). Transformation of E. coli DH10β was performed according to the manufacturer (New England Biolabs, Ipswitch, MA, USA). The final plasmid was verified by restriction analysis and DNA sequencing (SEQ ID NO:69).

• Construction of HM-p-50

For construction of an *E. coli* expression vector for *thrA*, encoding a feedback resistant variant of aspartate kinase from *E. coli* W3110 and *metX,* encoding homoserine acetyl transferase from *C.glutamicum* ATCC 13032, both genes including lacUV5 promotor (metX_Cg) and *tac* promotor (thrAfbr_Ec) were amplified by PCR from plasmid 4-52 (SEQ ID NO:70) with the primers SEQ ID NO:71 and SEQ ID NO:72.

Purified PCR products were assembled into *SalI* restricted AH-p-125 (SEQ ID NO:69) plasmid using NEBuilder® HiFi DNA Assembly Master Mix according to the manufacturers manual (New England Biolabs, Ipswitch, MA, USA). Transformation of E. coli DH10β was performed according to the manufacturer (New England Biolabs, Ipswitch, MA, USA). The final plasmid was verified by restriction analysis and DNA sequencing (SEQ ID NO:73).

• Construction of strain GAO-EC-147

*E. coli* CGSC 12149 wild type was modified according to pKO3 procedure (Link AJ, Phillips D, Church GM.. J Bateriol. 179(20):6228-37) with plasmids according to SEQ ID NO:39 and SEQ ID NO:40. Two rounds of modifications lead to *E. coli* CGSC 12149 lysCfbr_Ec thrAfbr_Ec. This strain was transformed with plasmids according to SEQ ID NOs: 74 and 75. Transformation of *E. coli* derivatives was performed via electroporation as known in the art. This work resulted in *E. coli* strain GAO-EC-147.

• **DASGIP TESTING GAO-EC-147**

- **Materials and methods**

Working with highly combustible gases in atmospheres containing significant amounts of oxygen (air for example) requires some special safety precautions. Generally, gassing of the fermenters is done with an ethane/air mixtures above the upper explosion limit (UEL) of ≈ 15 vol.% ethane in air. The composition of the gas mix is ethane/air 0.25/0.75.

All biotransformation experiments were conducted in a DASGIP-fermenter system in glass vessels with a working volume of 150-300 mL. Two 8fold pump modules are connected to the fermenters. Those can either be used for a two side pH-control of eight fermenters in parallel or for a pH control with base plus glucose feeding. A third external pump can be used additionally with a constant feeding rate; this pump is not connected and controlled by the DASGIP control programme.

All vessels are equipped with a pH and a dO2 probe. Those probes are connected to a control module and the corresponding signals serve as trigger for acid/base feed for pH control and for the stirrers for dO2 control respectively. In order to avoid possible sources of ignition that could occur with conventionally used thermos blocs, the temperature is controlled by immersion of the vessels into a tempered water bath. For the same reason - elimination of ignition sources - no overhead stirrers, but submergible magnetic stirrers are used for agitation of the fermenter content.

- **Media**

*(i) LB-medium:*

25 g LB-broth are dissolved in distilled water and autoclaved for 20 min. at 121°C.

*(ii) M9-medium without C-source:*

For 1 L medium, 8.52 g Na2HPO4, 3.00 g KH2PO4, 0.50 g NaCl, and 2.00 g NH4Cl are dissolved in approximately 900 mL distilled water. pH is adjusted to 7.0 with a diluted NH3-solution and distilled water is added to a final volume of 1000 mL. The solution is autoclaved and 2 mL of a MgSO4 solution (1 mol/L) and 1 mL of US3 trace element solution are added under sterile conditions.

*(iii) Trace element solution US3:*

For 1000 mL trace element solution US3, 40 mL HCl (37%), 1.9 g MnCl2*4 H2O, 1.9 g ZnSO4*7 H2O, 0.9 g Na-EDTA*2 H2O, 0.3 g H3BO3, 0.3 g Na2MoO4*2 H2O, 4.7 g CaCl2*2 H2O, 17.8 g FeSO4*7H2O, 0.2 g CuCl2*2H2O are dissolved one by one in 900 mL distilled water. Distilled water is added to a final volume of 1000 mL and the solution is filter sterilised (0.22 μm, PTFE membrane).

*(iv) MgSO4-solution (1M):*

246,47 g MgSO4*7H2O were dissolved in 1L distilled water and filter sterilised (0.22 μm, PTFE membrane).

*(v) MgSO4-solution (200 g/L):*

200 g MgSO4*7H2O were dissolved in 1L distilled water and filter sterilised (0.22 μm, PTFE membrane).

*(vi) NH4Cl-solution (220 g/L):*

220 g NH4Cl were dissolved in 1L distilled water and filter sterilised (0.22 μm, PTFE membrane).

*(vii) Glucose feed:*

550 g glucose*H2O were dissolved at ≈50° C in distilled water to give a final volume of 850 mL. The solution was sterilised by autoclaving it at 121° C for 20 min. For a glucose feed solution, 150 mL of sterile, distilled water were added under sterile conditions.

- **Growth and induction in fermenter**

For experiments with growth and induction in the main DASGIP-fermenter, only one preculture step is required. 100 mL shaking flasks are filled with 25 mL LB-medium, the respective amount of antibiotic and inoculated from a cryo culture. After cultivation at 37°C and 180 rpm, fermenters are inoculated from the LB-preculture with an OD of 0.1. The fermenters contain 190 mL M9 medium with a batch glucose concentration of 4 g/L and an antibiotic according to the cultivated strain. When the measured $dO_2$-increases due to glucose depletion, the glucose feed is started (0.4 g/Lh) and the inductor is added to the fermenter (1.5 μl DCPK, 1 mM IPTG, approximately after 22 h). Gas flow was set to 4.5 NL/H, after 25 h glucose feed was shut down and cultures were growing on ethane as sole carbon source. DO was set at 30 % as lower level and controlled by stirring speed, pH was set up 7.0 and controlled by 220 g/L $NH_4Cl$ when necessary.

- ***Analytics***

• *Quantification of ethanol and acetate by HPLC*

The quantification of ethanol and acetate in fermentation samples is carried out by HPLC. The quantification is based on an external calibration with the respective standards.

*Chemicals*

Ethanol (e.g.Sigma-Aldrich, >99% (GC), purum); natrium acetate (e.g. Merck); sulfuric acid (e.g. Merck); deionized water (Purification by a Millipore system)

*Sample preparation*

The aqueous fermentation samples are sterile-filtered and diluted by 20 mmolar aqueous sulfuric acid. Possible precipitates are separated by centrifugation.

*HPLC conditions*

| | |
|---|---|
| HPLC system | Agilent Technologies 1200 Series |
| HPLC column | Aminex HPX-87H (300mm x 7,8mm) (Bio-rad) |
| Eluent | 10 mmolar aqueous sulfuric acid |
| Column temperature | 40 °C |
| Flow rate | 0.6 mL/min |
| Detector | RID (Agilent G1362A-B) and DAD (210 nm) (Agilent G1315C-B) |
| Detector temperature | 35°C (RID) |
| Injection volume | 20 μL |
| Retention times | acetate 14.5 min; ethanol 20.5 min |

• *Quantification of amino acids by HPLC*

The quantification of amino acids is carried out by HPLC after derivatization with ortho-phthaldialdehyde. The quantification is based on an external calibration with the respective standards.

*Chemicals*

NaOH 32 % (e.g.. Fluka); methanol HPLC grade (e.g.Honeywell); n-propanol (e.g. Sigma-Aldrich); o-phthaldialdehyde (e.g.Roth); boric acid (e.g. Merck); mercaptoethanol (e.g. Sigma-Aldrich); formic acid (e.g. Sigma-Aldrich); acetonitrile HPLC grade (e.g. Sigma-Aldrich); Brij35 25 % in water (e.g.Sigma-Aldrich); deionized water (Purification by a Millipore system); aspartic acid (e.g. Sigma-Aldrich); homoserine (e.g. Sigma-Aldrich); threonine (e.g. Sigma-Aldrich);

glycine (e.g. Merck); acetylhomoserine (e.g. Chemos); methionine (e.g.Acros); valine (e.g. Merck; isoleucine(e.g. Roth); lysine (e.g. Sigma-Aldrich);

*Preparation of OPA reagent*

1000 mg o-phthaldialdehyde is dissolved in 10 ml methanol, 90 ml borate buffer (pH 10.4) is added, 500 μl mercaptoethanol is added. The reagent is stored in the fridge overnight. Then 100 μl mercaptoethanol is added.

*Preparation of borat buffer (0,4 mol/L)*

38,1 g $Na_2B_4O_7$ * 10 $H_2O$ is dissolved in 1 L water, pH value is adjusted to 10.4 by 10 mol/L NaOH, 1 mL Brij35 25 % is added

*Sample preparation*

The fermentation samples are diluted by n-propanol and centrifuged. The clear supernatant is used for analysis.

*HPLC conditions*

| | |
|---|---|
| HPLC system | Agilent Technologies 1200 Series |
| pre column | HPLC KrudKatcher Ultra HPLC In-Line Filter; 0.5 u Porosity x 0.004 ID (Phenomenex) |
| column | Kinetex XB-C18; 100 x 4.6 mm; 2.6 μm; 100A; (Phenomenex) |
| Eluent A | 95% water, 5% methanol, 0.1% formic acid |
| Eluent B | 90% acetonitrile, 5% water, 5% methanol, 0,1% formic acid |

Gradient profile

| | time [min] | eluent B [%] | flow rate [ml/min] | max. pressure [bar] |
|---|---|---|---|---|
| 1 | 0 | 10 | 0,6 | 400 |
| 2 | 1 | 10 | 0,6 | 400 |
| 3 | 5,5 | 35 | 0,6 | 400 |
| 4 | 6,5 | 35 | 0,6 | 400 |
| 5 | 13 | 70 | 0,6 | 400 |
| 6 | 13,1 | 100 | 0,6 | 400 |
| 7 | 16 | 100 | 0,6 | 400 |
| 8 | 16,1 | 10 | 0,6 | 400 |
| 9 | 21 | 10 | 0,6 | 400 |

| | |
|---|---|
| Column temperature | 30 °C |
| Flow rate | 0.6 mL/min |
| Detector | FLD (Agilent G1321A) |
| | PMT Gain 5, excitation wavelength 330mm, emission wavelength 450 nm |

(continued)

| Injection program (derivatization) | # | Command |
|---|---|---|
| | 1 | DRAW 4.5 μL from Vial 1*, def. speed, def. offset |
| | 2 | DRAW 1.5 μL from sample, def. speed, def. offset |
| | 3 | DRAW 0.5 μL from air, def. speed |
| | 4 | NEEDLE wash in flush Port. 15.0 sec |
| | 5 | DRAW 4.5 μL from Vial 1, def. speed, def. offset |
| | 6 | MIX 11.0 μL in seat, def. speed, 1 times |
| | 7 | WAIT 1.00 min |
| | 8 | INJECT |
| | 9 | WAIT 0.50 min |
| | 10 | Switch VALVE to "Bypass" |
| | 11 | NEEDLE wash in flush Port. 10.0 sec |
| | 12 | Draw 100.0 μL from Vial 2*, def. speed, def. offset |
| | 13 | EjeCt 100.0 μL from Vial 2, def. speed, def. offset |
| | 14 | Draw 100.0 μL from Vial 3*, def. speed, def. offset |
| | 15 | EjeCt 100.0 μL from Vial 3, def. speed, def. offset |
| | 16 | Valve mainpass |
| | *Vial 1 OPA-Reagenz | |
| | *Vial 2 water | |
| | *Vial 3 55 Vol.-% n-propanol in water | |
| Retention times | aspartic acid 8.6 min; homoserine 9.1 min; threonine 9.8 min; glycine 10.1 min; acetylhomoserine 11.6 min; methionine 13.3 min; valine 13.8 min; isoleucine 14.7 min; lysine 15.1 min | |

• *Implementation of μ-GC online measurements of ethane, oxygen, nitrogen, and, carbon dioxide and determination of transfer rates and Connection of fermenters to the μ-GC*

All fermenters were equipped with sterile filters (0.22μm) with NPT-thread to ensure tightness of the off-gas stream and enable mass balancing. Behind the sterile filters, a tee was installed with the main off-gas stream to the fume hood and a side branch for GC measurements. The side branch (1/16" stainless steel tubing) was connected to a 16 port VICI-valve that is directly connected to the GC. The 16-port valve is controlled by the GC-software. In the μ-GC, a sampling pump is integrated which takes actively samples from the off-gas stream. To make sure, the sample represents the actual fermenter gas composition, the sampling time is 30 s at a flow rate of 9 mL/min to flush the whole sampling line. A second tee is installed in the gas supply of fermenter/unit No1 and No5 to be able to measure the actual gas inlet as a representative for all fermenters (For fermenters 1-4 and 5-8 respectively).

• *Calibration*

For the calibration of the μ-GC, three test gas mixtures were used with a composition of ethane/CO2/N2/O2 of 1: 25/10/50.7/13.65; 2: 30/5/50.7/13.65; 3:35/1/49.92/13.44. Mixture 2 is used as quality control; mixtures 1 and 3 are used for a two point linear calibration. A quality control with mixture 2 is carried out every 30 days. The calibration is done at the installation of the μ-GC, every time, the method is changed, and when the quality control is out of the specification.

• *GC-parameters*

The μ-GC is equipped with four modules containing four different columns which can be analysed independently by four thermal conductivity detectors (TCD). All four columns are heated in a common oven to 80°C. Column No 1 is a 10 m mol sieve 5 Å (MS5A) with a heated injector (110°C). To avoid deterioration of the column by water and other contaminants, a backflush of 10 s is set. The column runs at 170 kPa static pressure mode with argon as carrier gas. Column No 1 is used to analyse permanent gases such as oxygen (29.0 s retention time), and nitrogen (30.8 s retention time) with a total runtime of 180 s. With argon as carrier gas, the signal has to be inverted and an approximately two times reduced sensitivity for nitrogen and oxygen is observed compared to helium as carrier gas. Column No 2 is a 10 m PPU column. The backflush is 16 s, the injector temperature 110°C and the pressure is kept at 150 kPa in static pressure mode with a total runtime of 180 s. On column No 2, carbon dioxide and ethane are analysed with retention times of 31.6 s and 34.5 s respectively. On column No3 and No4, higher molecules can be analysed, for the actual analytical task, they are not necessary.

• *Online ethane, oxygen, and carbon dioxide measurements using nitrogen as internal standard*

For the gassing of the fermenters, either pressurised air or a gas mixing unit (pressurised air plus pure ethane). While passing the fermentation broth the gas composition is changed by oxygen, and ethane consumption, carbon dioxide formation and dilution by saturation with steam. In the case of diluted liquid samples, the consumption of only one analyte does not influence the concentration of the other analytes as there is nearly no change in the total volume. For non-diluted gaseous samples, with all analytes present in significant amounts, the consumption or formation of one analyte drastically influences the concentration (vol.-%) of the other analytes. Therefore, an internal standard is needed. In the actual gas composition, nitrogen is used as an internal standard, as it is neither consumed nor produced during biotransformation and almost insoluble in water. Thus, the dilution factor $F_{dil}$ respectively the change in the gas flow rate inlet vs. outlet is calculated using the respective nitrogen concentrations:

$$F_{dil} = \frac{N_{2,in}}{N_{2,out}}$$

With:

N2, in = volume fraction nitrogen inlet
N2, out = volume fraction nitrogen concentration outlet in %

The actual ethane consumption □ $V_{ethane}$ is then calculated from the difference in ethane volume fraction in the inlet - outlet taking the dilution factor $F_{dil}$ into account:

$$\Delta V_{ethane} = \dot{V} \cdot \left(\frac{x_{ethane,in}}{100} - \frac{x_{ethane,out} \cdot F_{dil}}{100}\right)$$

With:

$V$ = total flow rate in L/h
$X_{ethane,in}$ = volume fraction ethane in
$X_{ethane,out}$ = volume fraction ethane out

The calculated ethane volume consumed is converted into the respective amount of ethane [mol] using the ideal gas law.

$$p \cdot V = n \cdot R \cdot T$$

With:

*p = pressure [Pa]*
*V = volume [m³]*

*n = amount of substance [mol]*

$$R = \text{Gas constant} = 8.3145\ J \cdot mol^{-1} \cdot K^{-1}$$

*T = temperature [K]*

With these data, the volumetric ethane uptake rate (EUR, mmol*$L^{-1}$*$h^{-1}$), oxygen uptake/transfer rate (OUR/OTR, mmol*$L^{-1}$*$h^{-1}$) and the carbon dioxide transfer rate (CTR, mmol*$L^{-1}$*$h^{-1}$) are determined, as well as the specific EUR in $mg_{ethane}/(g_{CDW} * h)$.

***Results***

After 14 h until 40 h process time ethane uptake rate EUR is exceeding 90 mg ethane per g dry weight and hour.

484 mg/L o-Acetyl-L-homoserine were produced in 48.5 h process time with ethane as sole carbon source. Corresponding control strains equipped with expression systems comprising SEQ ID NO:35 and SEQ ID NO:46 did not show any production of o-Acetyl-L-homoserine while both other systems were functional.

**Example 2**

*Formation of Lysine from ethane with Escherichia coli.*

For the biotransformation of ethane to L-lysine the genetically modified strain *E.coli* CGSC 12149 lysCfbr_Ec thrAfbr_Ec pACYC184 {PalkS} [alkS_PpGPo1] {PalkB} [bmoXYBZ_Tb PROKKA_02001_Tb PROKKA_02000_Tb bmoC_1_Tb PROKKA_01998_Tb bmoG_Tb] pBR322 {PalkS} [alkS_PpGPo1] {PalkB} [adhA_Cg aldH_Cg] {Placuv5} [metX_Cg]{Ptac}[thrA_fbr_Ec] was used. This strain harbors the following characteristics:

i) Modification of the *E. coli* CGSC 12149 lysC gene (SEQ ID NO:33), encoding a feedback resistant variant of aspartokinase 3.
ii) Expression of *Thauera butanivorans* DSM 2080 butane monooxygenase operon (SEQ ID NO:35), comprising of bmoX_Tb (butane monooxygenase hydroxylase BMOH alpha subunit), bmoY_Tb (butane monooxygenase beta subunit), bmoZ_Tb (butane monooxygenase gamma subunit), bmoB_Tb (butane monooxygenase regulatory protein), bmoC_1_Tb (butane monooxygenase reductase), bmoG_Tb (similar to groEL from *E. coli*) and three putative ORF PROKKA_02001_Tb, PROKKA_02000_Tb and PROKKA_01998_Tb.
iii) Expression of *Corynebacterium glutamicum* ATCC 13032 adhA_Cg (SEQ ID NO:36), encoding Zn-dependent alcohol dehydrogenases and aldH_Cg (SEQ ID NO:37), encoding NAD-dependent aldehyde dehydrogenases Cgl2796 genes.
iv) Modification and expression of the *E. coli* W3110 dapA gene (SEQ ID NO:76), encoding a feedback resistant variant of 4-hydroxy-tetrahydrodipicolinate synthase (SEQ ID NO:3) with G84T G250A A251C leading to dapAmod3_Ec.

These characteristics were brought about by:

i. Replacement of *E. coli* CGSC 12149 lysC gene by another allele of lysC, encoding a feedback resistant variant aspartokinase 3 (point mutation at bp 1055 from C to T (SEQ ID NO:33), T342I (SEQ ID NO:1) with pKO3 derivative 4-47 (SEQ ID NO:40).

ii. Introduction of plasmid pACYC184 {PalkS} [alkS_PpGPo1] {PalkB} [bmoXYBZ_Tb PROKKA_02001_Tb PROKKA_02000_Tb bmoC_1_Tb PROKKA_01998_Tb bmoG_Tb] {PlacUV5} [adhA_Cg aldH_Cg] (SEQ ID NO:41)

iii. Introduction of plasmid pBR322 {PlacUV5} [dapAmod3_Ec] (SEQ ID NO:74)

• Construction of pKO3 modification vectors

For construction of pKO3 derivatives for gene deletion and/or allelic replacement homologous sequences up- and downstream of the target genes were amplified by PCR from genomic DNA of *E. coli* W3110 using the primers of SEQ ID NOs: 43-46. Homologous ends for assembly cloning were introduced within the primers. The PCR was performed with Phusion® High-Fidelity Master Mix according to the manufacturer (New England Biolabs, Ipswitch, MA, USA). The thermal cycle profile was 3 min at 98 °C for initial denaturation, 35 cycles: 10 sec at 98 °C, 30 sec at 60 °C to 68 °C (gradient), 20 sec at 72 °C and a final 10 min hold step at 72 °C. Purification of PCR products was performed by gel extraction or PCR purification according to the manufacturer of purification kits (QiaQuick PCR Purification Kit and QiaQuick Gel Extraction Kit, Qiagen, Hilden, Germany). Purified PCR products were assembled into *Not*I restricted pKO3 plasmid using NEBuilder® HiFi DNA Assembly Master Mix according to the manufacturers manual (New England Biolabs, Ipswitch, MA, USA). Transformation of *E. coli* DH10β was performed according to the manufacturer (New England Biolabs, Ipswitch, MA, USA). The final plasmids were verified by restriction analysis and DNA sequencing.

• Construction of HM-p-48

Plasmid HM-p-54 (SEQ ID NO:74) is based on plasmid HM-p-25 (SEQ ID NO:41) comprising butane monooxygenase operon of Thauera butanivorans DSM 2080 (SEQ ID NO. 3), comprising of bmoX_Tb (butane monooxygenase hydroxylase BMOH alpha subunit), bmoY_Tb (butane monooxygenase beta subunit), bmoZ_Tb (butane monooxygenase gamma subunit), bmoB_Tb (butane monooxygenase regulatory protein), bmoC_1_Tb (butane monooxygenase reductase), bmoG_Tb (similar to groEL from *E. coli*) and three putative ORF PROKKA_02001_Tb, PROKKA_02000_Tb and PROKKA_01998_Tb. Additionally, gene expression of *C.glutamicum* ATCC 13032 adhA_Cg (SEQ ID NO:36), encoding Zn-dependent alcohol dehydrogenases and aldH_Cg (SEQ ID NO:37), encoding NAD-dependent aldehyde dehydrogenases Cgl2796 was enabled by amplifying genes by PCR from AP-p-125 (SEQ ID NO:69) including lacUV5 promotor region (SEQ ID NO:77). Homologous ends for assembly cloning were introduced within the primers. The final plasmid was verified by restriction analysis and DNA sequencing (SEQ ID NO:75).

• Construction of HM-p-54

For expression of the *E. coli* W3110 dapA gene (DNA: SEQ ID NO:76; Protein: SEQ ID NO:3), encoding a feedback resistant variant of 4-hydroxy-tetrahydrodipicolinate synthase with G84T G250A A251C leading to dapAmod3_Ec was ordered as synthetic gene construct (SEQ ID NO:76). This synthetic gene was fused to a lacUV5 promotor by in vitro recombination and cloned into pBR322 base vector. The final plasmid was verified by restriction analysis and sequencing (SEQ ID NO:74).

• Construction of strain GAO-EC-149

*E. coli* CGSC 12149 wild type was modified according to pKO3 procedure (Link AJ, Phillips D, Church GM. J Bateriol. 179(20):6228-37) with plasmid according to SEQ ID NO:40. Modifications lead to *E. coli* CGSC 12149 lysCfbr_Ec. This strain was transformed with plasmids according to SEQ ID NO:74 and SEQ ID NO:75. Transformation of *E. coli* derivatives was performed via electroporation as known in the art. This work resulted in *E. coli* strain GAO-EC-149.

• **DASGIP TESTING GAO-EC-149**

Materials, methods and analytics are the same as Example 1.

**Results**

After 14 h until 40 h process time ethane uptake rate EUR exceeding 60 mg ethane per g dry weight and hour.

1211 mg/L L-lysine in 48.5 h process time were produced, thereby half was produced while glucose feed was still running (14 h process time), remaining 480 mg/L L-lysine was produced with ethane as sole carbon source. Corresponding control strains equipped with expression systems comprising SEQ ID NO:35 and SEQ ID NO:46 did not show any production of L-lysine while both other systems were functional.

**Example 3**

As listed in table 1 different amino acids are produced by various bacteria with increased expression of the specific enzymes as referenced in the tables. An alkane mixture comprising ethane, propane and butane at a weight ratio of 1:1:1 is used as alkane. All enzyme entries are NCBI accession numbers. For the enzymes E6 of the type E6a, E6c, E6e, E6k, E6l and E6s also feedback-insensitive variants of the sequences indicated may be used.

| # | Host cell | E1 | E2 | E3 | E4 | E6, in [] type of E6 | Amino acid to be produced |
|---|---|---|---|---|---|---|---|
| 1 | E. coli | AAM19727.1 and AAM19728.1 and AAM19729.1 and AAM19730.1 and AAM19731.1 and AAM19732.1 and ABU68845.2 | BAA36121.1 | BAA36121.1 | None, or P27550.2, or APC52536.1, and P0A9M8.2, or BAA16336.1 | BAE77370.1 [a] | Threonine |
| 1 | E. coli | AAM19727.1 and AAM19728.1 and AAM19729.1 and AAM19730.1 and AAM19731.1 and AAM19732.1 and ABU68845.2 | WP_ 011015397.1 | WP_ 011015386.1 | None, or P27550.2, or APC52536.1, and P0A9M8.2, or BAA16336.1 | BAE77859.1 [b] | Threonine |
| 1 | E. coli | AAM19727.1 and AAM19728.1 and AAM19729.1 and AAM19730.1 and AAM19731.1 and AAM19732.1 and ABU68845.2 | NP_745969.1 | NP_742708.1 | None, or P27550.2, or APC52536.1, and P0A9M8.2, or BAA16336.1 | TLD77709.1 [n] | Threonine |
| 2 | E. coli | AAM19727.1 and AAM19728.1 and AAM19729.1 and AAM19730.1 and AAM19731.1 and AAM19732.1 and ABU68845.2 | BAA36121.1 | NP_744824.1 | None, or P27550.2, or APC52536.1, and P0A9M8.2, or BAA16336.1 | APC53474.1 [g] | O-Acetylhomoserine |
| 2 | E. coli | AAM19727.1 and AAM19728.1 and AAM19729.1 and AAM19730.1 and AAM19731.1 and AAM19732.1 and ABU68845.2 | WP_ 011015397.1 | WP_ 011015386.1 | None, or P27550.2, or APC52536.1, and P0A9M8.2, or BAA16336.1 | APC51865.1 [h] | O-Acetylhomoserine |
| 2 | E. coli | AAM19727.1 and AAM19728.1 and AAM19729.1 and AAM19730.1 and AAM19731.1 and AAM19732.1 and ABU68845.2 | NP_745969.1 | NP_742708.1 | None, or P27550.2, or APC52536.1, and P0A9M8.2, or BAA16336.1 | NP_ 747448.1 [i] | O-Acetylhomoserine |
| 3 | E. coli | AAM19727.1 and AAM19728.1 and AAM19729.1 and AAM19730.1 and AAM19731.1 and AAM19732.1 and ABU68845.2 | BAA36121.1 | NP_744824.1 | None, or P27550.2, or APC52536.1, and P0A9M8.2, or BAA16336.1 | BAB96580.2 [l] | Methionine |
| 3 | E. coli | AAM19727.1 and AAM19728.1 and AAM19729.1 and AAM19730.1 and AAM19731.1 and AAM19732.1 and ABU68845.2 | WP_ 011015397.1 | WP_ 011015386.1 | None, or P27550.2, or APC52536.1, and P0A9M8.2, or BAA16336.1 | BAB96579.2 [k] | Methionine |

(continued)

| # | Host cell | E1 | E2 | E3 | E4 | E6, in [] type of E6 | Amino acid to be produced |
|---|---|---|---|---|---|---|---|
| 3 | E. coli | AAM19727.1 and AAM19728.1 and AAM19729.1 and AAM19730.1 and AAM19731.1 and AAM19732.1 and ABU68845.2 | BAA36121.1 | BAA36121.1 | None, or P27550.2, or APC52536.1, and P0A9M8.2, or BAA16336.1 | APC51864.1 [h] | Methionine |
| 4 | E. coli | AAM19727.1 and AAM19728.1 and AAM19729.1 and AAM19730.1 and AAM19731.1 and AAM19732.1 and ABU68845.2 | WP_ 011015397.1 | WP_ 011015386.1 | None, or P27550.2, or APC52536.1, and P0A9M8.2, or BAA16336.1 | BAA16355.1 [c] | Lysine |
| 4 | E. coli | AAM19727.1 and AAM19728.1 and AAM19729.1 and AAM19730.1 and AAM19731.1 and AAM19732.1 and ABU68845.2 | NP_745969.1 | NP_742708.1 | None, or P27550.2, or APC52536.1, and P0A9M8.2, or BAA16336.1 | CAF19965.1 [f] | Lysine |
| 4 | E. coli | AAM19727.1 and AAM19728.1 and AAM19729.1 and AAM19730.1 and AAM19731.1 and AAM19732.1 and ABU68845.2 | BAA36121.1 | NP_744824.1 | None, or P27550.2, or APC52536.1, and P0A9M8.2, or BAA16336.1 | BAB96600.1 [d] | Lysine |
| 5 | C. glutamicum | AAM19727.1 and AAM19728.1 and AAM19729.1 and AAM19730.1 and AAM19731.1 and AAM19732.1 and ABU68845.2 | WP_ 011015397.1 | WP_ 011015386.1 | None, or BAC00146.1, and BAC00147.1, or P27550.2 | BAB97645.1 [a] | Threonine |
| 5 | C. glutamicum | AAM19727.1 and AAM19728.1 and AAM19729.1 and AAM19730.1 and AAM19731.1 and AAM19732.1 and ABU68845.2 | NP_745969.1 | NP_742708.1 | None, or BAC00146.1, and BAC00147.1, or P27550.2 | CAF19888.1 [l] | Threonine |
| 5 | C. glutamicum | AAM19727.1 and AAM19728.1 and AAM19729.1 and AAM19730.1 and AAM19731.1 and AAM19732.1 and ABU68845.2 | WP_ 011015397.1 | WP_ 011015386.1 | None, or BAC00146.1, and BAC00147.1, or P27550.2 | BAB98978.1 [g] | Threonine |
| 6 | C. glutamicum | AAM19727.1 and AAM19728.1 and AAM19729.1 and AAM19730.1 and AAM19731.1 and AAM19732.1 and ABU68845.2 | NP_745969.1 | NP_742708.1 | None, or BAC00146.1, and BAC00147.1, or P27550.2 | BAB98576.1 [k] | O-Acetylhomoserine |

(continued)

| # | Host cell | E1 | E2 | E3 | E4 | E6, in [] type of E6 | Amino acid to be produced |
|---|---|---|---|---|---|---|---|
| 6 | C. glutamicum | AAM19727.1 and AAM19728.1 and AAM19729.1 and AAM19730.1 and AAM19731.1 and AAM19732.1 and ABU68845.2 | WP_ 011015397.1 | WP_ 011015386.1 | None, or BAC00146.1, and BAC00147.1, or P27550.2 | NP_ 747447.1 [i] | O-Acetylhomoserine |
| 6 | C. glutamicum | AAM19727.1 and AAM19728.1 and AAM19729.1 and AAM19730.1 and AAM19731.1 and AAM19732.1 and ABU68845.2 | NP_745969.1 | NP_742708.1 | None, or BAC00146.1, and BAC00147.1, or P27550.2 | BAB98082.1 [i] | O-Acetylhomoserine |
| 7 | C. glutamicum | AAM19727.1 and AAM19728.1 and AAM19729.1 and AAM19730.1 and AAM19731.1 and AAM19732.1 and ABU68845.2 | NP_745969.1 | NP_742708.1 | None, or BAC00146.1, and BAC00147.1, or P27550.2 | NP_ 599504.1 [a] | Methionine |
| 7 | C. glutamicum | AAM19727.1 and AAM19728.1 and AAM19729.1 and AAM19730.1 and AAM19731.1 and AAM19732.1 and ABU68845.2 | WP_ 011015397.1 | WP_ 011015386.1 | None, or BAC00146.1, and BAC00147.1, or P27550.2 | CAF19359.1 [o] | Methionine |
| 7 | C. glutamicum | AAM19727.1 and AAM19728.1 and AAM19729.1 and AAM19730.1 and AAM19731.1 and AAM19732.1 and ABU68845.2 | NP_745969.1 | NP_742708.1 | None, or BAC00146.1, and BAC00147.1, or P27550.2 | CAF21108.1 [u] | Methionine |
| 8 | C. glutamicum | AAM19727.1 and AAM19728.1 and AAM19729.1 and AAM19730.1 and AAM19731.1 and AAM19732.1 and ABU68845.2 | BAA36121.1 | BAA36121.1 | None, or BAC00146.1, and BAC00147.1, or P27550.2 | CAF20314.1 [d] | Lysine |
| 8 | C. glutamicum | AAM19727.1 and AAM19728.1 and AAM19729.1 and AAM19730.1 and AAM19731.1 and AAM19732.1 and ABU68845.2 | WP_ 011015397.1 | WP_ 011015386.1 | None, or BAC00146.1, and BAC00147.1, or P27550.2 | CAF19884.1 [e] | Lysine |
| 8 | C. glutamicum | AAM19727.1 and AAM19728.1 and AAM19729.1 and AAM19730.1 and AAM19731.1 and AAM19732.1 and ABU68845.2 | NP_745969.1 | NP_742708.1 | None, or BAC00146.1, and BAC00147.1, or P27550.2 | P75826.2 [f] | Lysine |

(continued)

| # | Host cell | E1 | E2 | E3 | E4 | E6, in [] type of E6 | Amino acid to be produced |
|---|---|---|---|---|---|---|---|
| 8 | C. glutamicum | AAM19727.1 and AAM19728.1 and AAM19729.1 and AAM19730.1 and AAM19731.1 and AAM19732.1 and ABU68845.2 | BAA36121.1 | BAA36121.1 | None, or BAC00146.1, and BAC00147.1, or P27550.2 | BAE76111.1 [f] | Lysine |
| 8 | C. glutamicum | AAM19727.1 and AAM19728.1 and AAM19729.1 and AAM19730.1 and AAM19731.1 and AAM19732.1 and ABU68845.2 | WP_ 011015397.1 | WP_ 011015386.1 | None, or BAC00146.1, and BAC00147.1, or P27550.2 | CAF19884.1 [e] | Lysine |
| 9 | P. putida | AAM19727.1 and AAM19728.1 and AAM19729.1 and AAM19730.1 and AAM19731.1 and AAM19732.1 and ABU68845.2 | NP_745969.1 | NP_742708.1 | None, or APC52536.1, and P0A9M8.2, or NP_746598.2, or NP_746811.1 | NP_ 746584.1 [a] | Threonine |
| 9 | P. putida | AAM19727.1 and AAM19728.1 and AAM19729.1 and AAM19730.1 and AAM19731.1 and AAM19732.1 and ABU68845.2 | NP_745969.1 | NP_742708.1 | None, or APC52536.1, and P0A9M8.2, or NP_746598.2, or NP_746811.1 | NP_ 747448.1 [l] | Threonine |
| 9 | P. putida | AAM19727.1 and AAM19728.1 and AAM19729.1 and AAM19730.1 and AAM19731.1 and AAM19732.1 and ABU68845.2 | BAA36121.1 | BAA36121.1 | None, or APC52536.1, and P0A9M8.2, or NP_746598.2, or NP_746811.1 | NP_ 744388.1 [n] | Threonine |
| 10 | P. putida | AAM19727.1 and AAM19728.1 and AAM19729.1 and AAM19730.1 and AAM19731.1 and AAM19732.1 and ABU68845.2 | BAA36121.1 | BAA36121.1 | wildtype | NP_ 743662.1 [k] | O-Acetylhomoserine |
| 10 | P. putida | AAM19727.1 and AAM19728.1 and AAM19729.1 and AAM19730.1 and AAM19731.1 and AAM19732.1 and ABU68845.2 | WP_ 011015397.1 | WP_ 011015386.1 | wildtype | BAA35485.1 [ad] | O-Acetylhomoserine |
| 10 | P. putida | AAM19727.1 and AAM19728.1 and AAM19729.1 and AAM19730.1 and AAM19731.1 and AAM19732.1 and ABU68845.2 | NP_745969.1 | NP_742708.1 | wildtype | NP_ 747198.1 [s] | O-Acetylhomoserine |

(continued)

| # | Host cell | E1 | E2 | E3 | E4 | E6, in [] type of E6 | Amino acid to be produced |
|---|---|---|---|---|---|---|---|
| 11 | P. putida | AAM19727.1 and AAM19728.1 and AAM19729.1 and AAM19730.1 and AAM19731.1 and AAM19732.1 and ABU68845.2 | BAA36121.1 | NP_744824.1 | wildtype | NP_ 744143.1 [b] | Methionine |
| 11 | P. putida | AAM19727.1 and AAM19728.1 and AAM19729.1 and AAM19730.1 and AAM19731.1 and AAM19732.1 and ABU68845.2 | WP_ 011015397.1 | WP_ 011015386.1 | wildtype | NP_ 742819.1 [q] | Methionine |
| 11 | P. putida | AAM19727.1 and AAM19728.1 and AAM19729.1 and AAM19730.1 and AAM19731.1 and AAM19732.1 and ABU68845.2 | NP_745969.1 | NP_742708.1 | wildtype | BAE78143.1 [t] | Methionine |
| 12 | P. putida | AAM19727.1 and AAM19728.1 and AAM19729.1 and AAM19730.1 and AAM19731.1 and AAM19732.1 and ABU68845.2 | WP_ 011015397.1 | WP_ 011015386.1 | wildtype | NP_ 744186.1 [c] | Lysine |
| 12 | P. putida | AAM19727.1 and AAM19728.1 and AAM19729.1 and AAM19730.1 and AAM19731.1 and AAM19732.1 and ABU68845.2 | NP_745969.1 | NP_742708.1 | wildtype | NP_ 747328.1 [e] | Lysine |
| 12 | P. putida | AAM19727.1 and AAM19728.1 and AAM19729.1 and AAM19730.1 and AAM19731.1 and AAM19732.1 and ABU68845.2 | BAA36121.1 | NP_744824.1 | wildtype | NP_ 746833.1 [d] | Lysine |

## Claims

1. A microbial cell for producing at least one L-amino acid from at least one C1-C4 alkane, wherein the cell comprises :

   (i) an increased expression relative to the wild type cell of Enzyme $E_1$ capable of converting the alkane to a corresponding 1-alkanol;
   (ii) an increased expression relative to the wild type cell of Enzyme $E_2$ capable of converting the 1-alkanol of (i) to a corresponding aldehyde; and either
   (iii)

   (A)

   - an increased expression relative to the wild type cell of Enzyme $E_3$ capable of converting the aldehyde of (ii) to a corresponding alkanoic acid; and
   - a wild-type level expression of Enzyme $E_4$ or an increased expression relative to the wild type cell of Enzyme $E_4$ capable of converting the alkanoic acid of (iii) to a corresponding fatty acyl thioester; or

   (B)

   - an increased expression relative to the wild type cell of Enzyme $E_5$ capable of converting the aldehyde of (ii) to a corresponding fatty acyl thioester;

   and
   (iv) an increased expression relative to the wild type cell of Enzyme $E_6$ capable of converting the fatty acyl thioester of (iii) to a corresponding amino acid.

2. The cell according to claim 1, wherein the amino acid produced is selected from the group consisting of lysine, threonine, and O-acetyl homoserine.

3. The cell according to either claim 1 or 2, wherein the amino acid produced is lysine and

   - the Enzyme $E_1$ is selected from the group consisting of P450 alkane hydroxylase ($E_a$) of EC 1.14.15.3-, AlkB alkane hydroxylase ($E_b$) of EC 1.14.15.3 from the AlkBGT component, methane monooxygenase ($E_k$) of EC 1.14.18.3, 1.14.99.39 or 1.14.13.25, propane monooxygenase ($E_l$) of EC. 1.14.13.227 and butane monooxygenase ($E_m$) of EC 1.14.13.230;

- the Enzyme $E_2$ is selected from the group consisting of P450 alkane hydroxylase ($E_a$) of EC 1.14.15.3-, AlkB alkane hydroxylase ($E_b$) of EC 1.14.15.3 from the AlkBGT component, alcohol oxidase ($E_c$) of EC 1.1.3.20 and alcohol dehydrogenase ($E_d$) of EC 1.1.1.1 or EC 1.1.1.2;
- the Enzyme $E_3$ is selected from the group consisting of P450 alkane hydroxylase ($E_a$) of EC 1.14.15.3-, AlkB alkane hydroxylase ($E_b$) of EC 1.14.15.3 from the AlkBGT component, aldehyde dehydrogenase ($E_e$) of EC 1.2.1.3, EC 1.2.1.4 or EC 1.2.1.5, bifunctional alcohol oxidase ($E_c$) of EC 1.1.3.20, bifunctional AlkJ alcohol dehydrogenase ($E_{di}$) of EC 1.1.99 and bifunctional alcohol dehydrogenase ($E_{dii}$) of EC 1.1.1.1 or EC 1.1.1.2, wherein $E_c$, $E_{di}$, and $E_{dii}$ are capable of oxidizing an ω-hydroxy alkanoic acid ester directly to the corresponding ω-carboxy alkanoic acid ester;
- the Enzyme $E_4$ is selected from the group consisting of fatty acyl coenzyme A (CoA) synthase ($E_f$) of EC 6.2.1.1, EC 6.2.1.2 or EC 6.2.1.3, acyl-Acyl Carrier Protein (ACP) synthase ($E_g$) of EC 6.2.1.20 or EC 6.2.1.47, a combination of fatty acyl kinase ($E_h$) of EC 2.7.2.1, EC 2.7.2.12, EC 2.7.2.15 or EC 2.7.2.7 and phosphotransacylase ($E_i$) of EC 2.3.1.8 or EC 2.3.1.19, and a combination of fatty acyl-CoA synthase ($E_f$) of EC 6.2.1.1, EC 6.2.1.2 or EC 6.2.1.3 and a fatty acyl-CoA:ACP transacylase ($E_j$) of EC 2.3.1.38 or EC 2.3.1.39;
- the Enzyme $E_5$ is an CoA-linked aldehyde dehydrogenase ($E_{ei}$) of EC 1.2.1.10 or EC 1.2.1.87; and
- the Enzyme $E_6$ is selected from the group consisting of aspartate kinase ($E_{6a}$) (EC 2.7.2.4), aspartate semialdehyde dehydrogenase ($E_{6b}$) (EC 1.2.1.114-hydroxy-tetrahydrodipicolinate synthase ($E_{6c}$) (EC 1.4.1.16), dihydrodipicolinate reductase ($E_{6d}$) (EC 1.17.1.8), diaminopimelate decarboxylase ($E_{6e}$) (EC 4.1.1.20), lysine exporter ($E_{6f}$) (TCDB families 2.A.124.1.1, 2.A.75.1.1 or 2.A.75.1.2), phosphoenolpyruvate (PEP) carboxylase ($E_{6g}$) (EC 4.1.1.31), proton-translocating transhydrogenase ($E_{6h}$) (EC 1.6.1.5), and pyruvate carboxylase ($E_{6i}$) (EC 6.4.1.1).

**4.** The cell according to claim 3, wherein the enzyme $E_6$ is selected from the group consisting of aspartate kinase ($E_{6a}$) and 4-hydroxy-tetrahydrodipicolinate synthase ($E_{6c}$) (EC 1.4.1.16).

**5.** The cell according to either claim 3 or 4, wherein the enzyme $E_6$ is a feedback resistant variant of aspartate kinase ($E_{6a}$) comprising SEQ ID NO:1, or a feedback resistant variant of 4-hydroxy-tetrahydrodipicolinate synthase ($E_{6c}$) (EC 1.4.1.16) comprising SEQ ID NO:3.

**6.** The cell according to any one of the preceding claims, wherein the amino acid produced is lysine and the enzyme

- $E_1$ is a butane monoxygenase ($E_c$) (EC 1.14.13.230), preferably comprising the sequences with accession numbers AAM19732.1, AAM19730.1, AAM19728.1, AAM19727.1, AAM19729.1, and ABU68845.2 or variants thereof;
- $E_2$ is an alcohol dehydrogenase ($E_d$) (EC 1.1.1.1 or EC 1.1.1.2), preferably comprising SEQ ID NO:91 or a variant thereof;
- $E_3$ is an aldehyde dehydrogenase ($E_e$) (EC 1.2.1.3, EC 1.2.1.4 or EC 1.2.1.5), preferably comprising SEQ ID NO:42 or a variant thereof;
- $E_4$ is fatty acyl CoA synthase (FACS) ($E_f$) (EC 6.2.1.1, EC 6.2.1.2 or EC 6.2.1.3), preferably comprising SEQ ID NO:88 or variant thereof; and
- $E_6$ is at least one enzyme selected from the group consisting of:

(i) a feedback-resistant variant of aspartate kinase ($E_{6a}$), preferably comprising SEQ ID NO:1 with a point mutation of T342I, or SEQ ID NO:79 with at least one point mutation selected from the group consisting of T311I, A279T, S301Y, A279V, S301F, T308I, S317A, R320G, G345D, S381F, Q404E, G408R, G277A, Q298A, T361A, E363A, and F364A, and
(ii) a feedback-resistant variant of 4-hydroxy-tetrahydrodipicolinate synthase ($E_{6c}$) (EC 1.4.1.16), preferably comprising SEQ ID NO:3 or a variant thereof comprising point mutations G84T, G250A and/or A251C;

**7.** The cell according to either claim 1 or 2, wherein the amino acid produced is O-acetyl homoserine and

- the Enzyme $E_1$ is selected from the group consisting of P450 alkane hydroxylase ($E_a$) of EC 1.14.15.3-, AlkB alkane hydroxylase ($E_b$) of EC 1.14.15.3 from the AlkBGT component, methane monooxygenase ($E_k$) of EC 1.14.18.3, 1.14.99.39 or 1.14.13.25, propane monooxygenase ($E_l$) of EC. 1.14.13.227 and butane monooxygenase ($E_m$) of EC 1.14.13.230;
- the Enzyme $E_2$ is selected from the group consisting of P450 alkane hydroxylase ($E_a$) of EC 1.14.15.3-, AlkB alkane hydroxylase ($E_b$) of EC 1.14.15.3 from the AlkBGT component, alcohol oxidase ($E_c$) of EC 1.1.3.20 and alcohol dehydrogenase ($E_d$) of EC 1.1.1.1 or EC 1.1.1.2;

- the Enzyme $E_3$ is selected from the group consisting of P450 alkane hydroxylase ($E_a$) of EC 1.14.15.3-, AlkB alkane hydroxylase ($E_b$) of EC 1.14.15.3 from the AlkBGT component, aldehyde dehydrogenase ($E_e$) of EC 1.2.1.3, EC 1.2.1.4 or EC 1.2.1.5, bifunctional alcohol oxidase ($E_c$) of EC 1.1.3.20, bifunctional AlkJ alcohol dehydrogenase ($E_{di}$) of EC 1.1.99 and bifunctional alcohol dehydrogenase ($E_{dii}$) of EC 1.1.1.1 or EC 1.1.1.2, wherein $E_c$, $E_{di}$, and $E_{dii}$ are capable of oxidizing an ω-hydroxy alkanoic acid ester directly to the corresponding ω-carboxy alkanoic acid ester;
- the Enzyme $E_4$ is selected from the group consisting of fatty acyl coenzyme A (CoA) synthase ($E_f$) of EC 6.2.1.1, EC 6.2.1.2 or EC 6.2.1.3, acyl-Acyl Carrier Protein (ACP) synthase ($E_g$) of EC 6.2.1.20 or EC 6.2.1.47, a combination of fatty acyl kinase ($E_h$) of EC 2.7.2.1, EC 2.7.2.12, EC 2.7.2.15 or EC 2.7.2.7 and phosphotransacylase ($E_l$) of EC 2.3.1.8 or EC 2.3.1.19, and a combination of fatty acyl-CoA synthase ($E_f$) of EC 6.2.1.1, EC 6.2.1.2 or EC 6.2.1.3 and a fatty acyl-CoA:ACP transacylase ($E_j$) of EC 2.3.1.38 or EC 2.3.1.39;
- the Enzyme $E_5$ is an CoA-linked aldehyde dehydrogenase ($E_{ei}$) of EC 1.2.1.10 or EC 1.2.1.87; and
- the Enzyme $E_6$ is selected from the group consisting of aspartate kinase ($E_{6a}$) (EC 2.7.2.4), aspartate semialdehyde dehydrogenase ($E_{6b}$) (EC 1.2.1.11), glyceraldehyde-3-phosphate dehydrogenase (NADP-dependent) ($E_{6j}$) (EC 1.2.1.9, EC 1.2.1.13, EC 1.2.1.59, EC 1.2.1.60), homoserine dehydrogenase ($E_{6k}$) (EC 1.1.1.3), homoserine kinase ($E_{6l}$) (EC 2.7.1.39), phosphoenolpyruvate (PEP) carboxylase ($E_{6g}$) (EC 4.1.1.31), proton-translocating transhydrogenase ($E_{6h}$) (EC 1.6.1.5), pyruvate carboxylase ($E_{6i}$) (EC 6.4.1.1), homoserine O-acetyltransferase ($E_{6s}$) (EC 2.3.1.31), and O-acetyl homoserine exporter ($E_{6ad}$) (TCDB classification 2.A.42.2.2; 2.A.7.3.6; 2.A.76.1.10; 2.A.76.1.2; 2.A.79.1.1; 2.A.95.1.4, 2.A.7.21.5, 2.A.76.1.1, 2.A.76.1.9).

**8.** The cell according to claim 7, wherein the enzyme $E_6$ is selected from the group consisting of aspartate kinase ($E_{6a}$), homoserine dehydrogenase ($E_{6k}$) and homoserine O-acetyltransferase ($E_{6s}$).

**9.** The cell according to either claim 6 or 7, wherein the enzyme $E_6$ is homoserine dehydrogenase ($E_{6k}$) comprising SEQ ID NO:14, 51, 80 or a variant thereof, or a homoserine O-acetyltransferase ($E_{6s}$) comprising SEQ ID NO:16, 78 or a variant thereof.

**10.** The cell according to any one of the preceding claims, wherein the amino acid produced is O-acetyl homoserine and the enzyme

- $E_1$ is a butane monoxygenase ($E_c$) (EC 1.14.13.230), preferably comprising the sequences with accession numbers AAM19732.1, AAM19730.1, AAM19728.1, AAM19727.1, AAM19729.1, and ABU68845.2 or variants thereof;
- $E_2$ is an alcohol dehydrogenase ($E_d$) (EC 1.1.1.1 or EC 1.1.1.2), preferably comprising SEQ ID NO:91 or a variant thereof;
- $E_3$ is an aldehyde dehydrogenase ($E_e$) (EC 1.2.1.3, EC 1.2.1.4 or EC 1.2.1.5), preferably comprising SEQ ID NO:42 or a variant thereof;
- $E_4$ is fatty acyl CoA synthase (FACS) ($E_f$) (EC 6.2.1.1, EC 6.2.1.2 or EC 6.2.1.3), preferably comprising SEQ ID NO:88 or variant thereof; and
- $E_6$ is at least one enzyme selected from the group consisting of:

(i) a feedback resistant variant of homoserine dehydrogenase ($E_{6k}$), preferably comprising SEQ ID NO:14 with at least one point mutation selected from the group consisting of G378E, D375A, V379E, L380E, I392P, S393A, L394P and Q399T, SEQ ID NO:51 with point mutation S345P or SEQ ID NO:80,
(ii) a feedback-resistant variant of aspartate kinase ($E_{6a}$) comprising SEQ ID NO:1 with a point mutation of T342I, or SEQ ID NO:79 with at least one point mutation selected from the group consisting of T311I, A279T, S301Y, A279V, S301F, T308I, S317A, R320G, G345D, S381F, Q404E, G408R, G277A, Q298A, T361A, E363A, and F364A, and
(iii) a feedback-resistant variant of homoserine O-acetyltransferase ($E_{6s}$) comprising SEQ ID NO:78 with point mutation Y294C

**11.** The cell according to any one of the preceding claims, wherein the cell is selected from the group consisting of *Acinetobacter sp., Bacillus sp., Brevibacterium sp., Burkholderia sp., Chlorella sp., Clostridium sp., Corynebacterium sp., Cyanobakterien*, *Escherichia sp., Pseudomonas sp., Klebsiella sp., Salmonella sp., Rhizobium sp., Saccharomyces sp., Pichia sp., Nostoc sp.*.

**12.** The cell according to any one of the preceding claims, wherein the cell is selected from the group consisting of *Bacillus subtilis, Burkholderia thailandensis, Corynebacterium glutamicum, E. coli, Klebsiella oxytoca, Pseudomonas*

*fluorescens, Pseudomonas putida, Pseudomonas stutzeri, Rhizobium meliloti, Saccharomyces cerevisiae* and *Pichia pastoris.*

**13.** A method of producing at least one amino acid, wherein the method comprises a step of contacting at least one cell according to any one of claims 1 to 12 with at least one C1-C4 alkane.

**14.** The method according to claim 13, wherein the alkane is ethane or butane and the amino acid is lysine or o-acetyl homoserine.

**15.** Use of at least one cell according to any one claims 1 to 12 for producing at least one amino acid from at least one C1-C4 alkane.

Europäisches Patentamt
European Patent Office
Office européen des brevets

# EUROPEAN SEARCH REPORT

Application Number
EP 19 19 2017

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/161043 A1 (UNIV RICE WILLIAM M [US]) 6 October 2016 (2016-10-06)<br>* the whole document *<br>* in particular paragraphs [0029], [0057], [0108], Table F + Fig. 5 and claim 41 *<br>----- | 1-15 | INV.<br>C12N9/02<br>C12N9/04<br>C12N9/00<br>C12N9/12<br>C12N9/10<br>C12N9/06<br>C12N9/88<br>C12P13/04<br>C12P13/06<br>C12P13/08 |
| X | WO 2017/087731 A1 (IND MICROBES INC [US]) 26 May 2017 (2017-05-26)<br>* the whole document *<br>* in particular paragraphs [0024], [0025], pages 35-37, Table 1; pages 74-75 ,Table 16; page 91, examples 28 and 29 *<br>----- | 1-15 | |
| A | LI YANJUN ET AL: "Current status on metabolic engineering for the production of l-aspartate family amino acids and derivatives",<br>BIORESOURCE TECHNOLOGY,<br>vol. 245, 24 May 2017 (2017-05-24), pages 1588-1602, XP085238080,<br>ISSN: 0960-8524, DOI: 10.1016/J.BIORTECH.2017.05.145<br>* the whole document *<br>* in particular Figs 3 and 4; Table 1 *<br>----- | 1-15 | TECHNICAL FIELDS SEARCHED (IPC)<br>C12N<br>C12P |
| A | DATABASE Geneseq [Online]<br><br>29 June 2017 (2017-06-29),<br>"E. coli W3110 mutant homoserine succinyl transferase, SEQ ID 4.",<br>XP002787548,<br>retrieved from EBI accession no. GSP:BDW62924<br>Database accession no. BDW62924<br>* homoserine O-acetyltransferase with Y294C mutation *<br>& CN 105 886 449 A (UNIV ZHEJIANG TECHNOLOGY) 24 August 2016 (2016-08-24)<br>-----<br>-/-- | 1-15 | |

The present search report has been drawn up for all claims

2

| Place of search | Date of completion of the search | Examiner |
|---|---|---|
| Munich | 15 October 2019 | Dumont, Elisabeth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 19 19 2017

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2018/019867 A1 (EVONIK DEGUSSA GMBH [DE]) 1 February 2018 (2018-02-01) * homoserine O-acetyltransferase; sequence 1 * ----- | 1-15 | |
| A | US 2018/135031 A1 (BAE HYUN WON [KR] ET AL) 17 May 2018 (2018-05-17) * fatty acyl CoA synthase (FACS); sequence 14 * ----- | 1-15 | |
| A | WO 2013/093737 A1 (BASF SE [DE]; WITTMANN CHRISTOPH [DE]; KIND STEFANIE [DE]; BECKER JUDI) 27 June 2013 (2013-06-27) * homoserine dehydrogenase; sequence 16 * ----- | 1-15 | |

TECHNICAL FIELDS SEARCHED (IPC)

The present search report has been drawn up for all claims

| Place of search | Date of completion of the search | Examiner |
|---|---|---|
| Munich | 15 October 2019 | Dumont, Elisabeth |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT ON EUROPEAN PATENT APPLICATION NO. EP 19 19 2017

This annex lists the patent family members relating to the patent documents cited in the above-mentioned European search report. The members are as contained in the European Patent Office EDP file on
The European Patent Office is in no way liable for these particulars which are merely given for the purpose of information.

15-10-2019

| Patent document cited in search report | Publication date | Patent family member(s) | Publication date |
|---|---|---|---|
| WO 2016161043 A1 | 06-10-2016 | AU 2016243669 A1 | 16-11-2017 |
| | | US 2018355394 A1 | 13-12-2018 |
| | | WO 2016161043 A1 | 06-10-2016 |
| WO 2017087731 A1 | 26-05-2017 | AU 2016358063 A1 | 31-05-2018 |
| | | CA 3005460 A1 | 26-05-2017 |
| | | CN 108779429 A | 09-11-2018 |
| | | EP 3377612 A1 | 26-09-2018 |
| | | US 2019032028 A1 | 31-01-2019 |
| | | WO 2017087731 A1 | 26-05-2017 |
| WO 2018019867 A1 | 01-02-2018 | CN 109790106 A | 21-05-2019 |
| | | EP 3490969 A1 | 05-06-2019 |
| | | JP 2019523271 A | 22-08-2019 |
| | | US 2019264245 A1 | 29-08-2019 |
| | | WO 2018019867 A1 | 01-02-2018 |
| US 2018135031 A1 | 17-05-2018 | BR 112016023337 A2 | 16-01-2018 |
| | | CN 106574232 A | 19-04-2017 |
| | | EP 3130663 A1 | 15-02-2017 |
| | | JP 6495940 B2 | 03-04-2019 |
| | | JP 2017510280 A | 13-04-2017 |
| | | JP 2019030316 A | 28-02-2019 |
| | | KR 20150117337 A | 20-10-2015 |
| | | US 2018135031 A1 | 17-05-2018 |
| | | WO 2015156583 A1 | 15-10-2015 |
| WO 2013093737 A1 | 27-06-2013 | EP 2794852 A1 | 29-10-2014 |
| | | US 2014356916 A1 | 04-12-2014 |
| | | WO 2013093737 A1 | 27-06-2013 |



For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013024114 A


### Non-patent literature cited in the description


- **HERMANN et al.** *Electrophoresis,* 2001, vol. 22, 1712-23
- **SAMBROOK et al.** Molecular Cloning: a laboratory manual. Cold Spring Harbor Laboratory Press, 1989
- **LOHAUS ; MEYER.** *Biospektrum,* 1989, vol. 5, 32-39
- **LOTTSPEICH.** *Angewandte Chemie,* 1999, vol. 111, 2630-2647
- **LI, Y. et al.** Current status on metabolic engineering for the production of L-aspartate family amino acids and derivatives. *Bioresour. Technol.,* 2017
- **LINK AJ ; PHILLIPS D ; CHURCH GM.** *J Bateriol.,* vol. 179 (20), 6228-37